# EUROPÄISCHE PATENTSCHRIFT

(11) **EP 0 741 700 B1**
(45) Veröffentlichungstag und Bekanntmachung des Hinweises auf die Patenterteilung: **07.10.2009**
(21) Anmeldenummer: 95905627.6
(22) Anmeldetag: 16.01.1995
(51) Int. Cl.: C07D 207/38, A01N 43/36, C07D 209/54

(54) **1-H-3-ARYL-PYRROLIDIN-2,4-DION-DERIVATE ALS SCHÄDLINGSBEKÄMPFUNGSMITTEL**
1-H-3-ARYLPYRROLIDINE-2,4-DIONE DERIVATIVES AS PEST-CONTROL AGENTS
DERIVES DE 1-H-3-ARYL-PYRROLIDINE-2,4-DIONE UTILISES COMME PESTICIDES

(30) Priorität: 28.01.1994 DE 4402531; 20.07.1994 DE 4425617
(43) Veröffentlichungstag der Anmeldung: 13.11.1996
(73) Patentinhaber: Bayer CropScience AG, 40789 Monheim (DE)
(72) Erfinder: FISCHER, Reiner, D-40789 Monheim (DE); BRETSCHNEIDER, Thomas, D-53797 Lohmar (DE); KRÜGER, Bernd-Wieland, D-51467 Bergisch Gladbach (DE); RUTHER, Michael, D-40789 Monheim (DE); ERDELEN, Christoph, D-42799 Leichlingen (DE); WACHENDORFF-NEUMANN, Ulrike, D-40789 Monheim (DE)
(86) Internationale Anmeldenummer: PCT/EP1995/000150
(87) Internationale Veröffentlichungsnummer: WO 1995/020572

(56) Entgegenhaltungen:
- EP-A- 0 456 063
- EP-A- 0 521 334
- EP-A- 0 613 885
- DE-A- 4 326 909

## Beschreibung

Die Erfindung betrifft neue 1-H-3-Aryl-pyrrolidin-2,4-dion-Derivate, mehrere Verfahren zu ihrer Herstellung und ihre Verwendung als Schädlingsbekämpfungsmittel, insbesondere als Insektizide und Akarizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985 1095) synthetisiert. Eine biologische Wirksamkeit dieser Verbindungen wurde nicht beschrieben.

In EP-A 0 262 399 und GB-A 2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A 355 599 und EP 415 211) sowie substituierte monocyclische 3-Aryl-pyrrolidin-2,4-dion-Derivate (EP-A 377 893 und EP 442 077).

Weiterhin bekannt sind polycyclische 3-Arylpyrrolidin-2,4-dion-Derivate (EP 442 073) sowie 1-H-3-Arylpyrrolidin-dion-Derivate (EP 456 063 und EP 521 334).

Es wurden nun neue substituierte 1-H-3-Aryl-pyrrolidin-2,4-dion-Derivate der Formel (I) 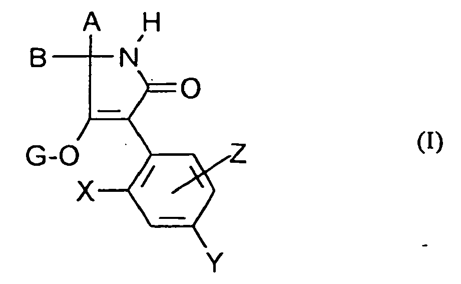 gefunden,
in welcher
- A: für Wasserstoff, für jeweils gegebenenfalls durch Halogen substituiertes C₁-C₈-Alkyl, C₃-C₄-Alkenyl, C₁-C₆-Alkoxy-C₂-C₄-alkyl, C₁-C₄-Polyalkoxy-C₂-C₄-alkyl, C₁-C₆₋Alkylthio-C₂-C₄-alkyl, Cycloalkyl mit 3 bis 6 Ringatomen, das durch 1 bis 2 Sauerstoff- und/oder Schwefelatome unterbrochen sein kann oder für jeweils gegebenenfalls durch Fluor, Chlor, Brom, Methyl, Ethyl, Propyl, iso-Propyl, Methoxy, Ethoxy, Trifluormethyl und/oder Nitro substituiertes Phenyl, Thienyl, Pyridyl, Imidazolyl, Pyrazolyl, Triazolyl, Indolyl, Thiazol oder Phenyl-C₁-C₃-alkyl steht,
- B: für Wasserstoff, C₁-C₈-Alkyl oder C₁-C₄-Alkoxyalkyl steht oder
- A, B: und das Kohlenstoffatom an das sie gebunden sind, für einen gesättigten oder ungesättigten C₃-C₈-Spirocyclus stehen, der gegebenenfalls einfach oder mehrfach durch Methyl, Ethyl, Propyl, Isopropyl, Butyl, iso-Butyl, sec.-Butyl, tert,-Butyl, Cyclohexyl, Trifluormethyl, Methoxy, Ethoxy, Propoxy, iso-Propoxy, Butoxy, iso-Butoxy, sek.-Butoxy, tert.-Butoxy, Methylthio, Ethylthio, Fluor, Chlor oder Phenyl substituiert und gegebenenfalls durch Sauerstoff oder Schwefel unterbrochen ist oder
- A, B: und das Kohlenstoffatom, an das sie gebunden sind, für einen C₃-C₆-Spirocyclus stehen, der durch eine gegebenenfalls durch ein Sauerstoff- oder Schwefelatom unterbrochene Alkylendiyl- oder durch eine Alkylendioxyl-Gruppe substituiert ist, die mit dem Kohlenstoffatom, an das sie gebunden ist, einen weiteren fünf- bis siebengliedrigen Spirocyclus bildet oder
- A,B: und das Kohlenstoffatom, an das sie gebunden sind, für einen C₃-C₆-Spirocyclus stehen, bei dem zwei Substituenten gemeinsam für einen gesättigten oder ungesättigten fünf- oder sechsgliedrigen Cyclus stehen, der durch Sauerstoff oder Schwefel unterbrochen sein kann,
- X: für Chlor steht,
- Y: für Methoxy steht,
- Z: für Wasserstoff steht,
oder
- X: für Chlor steht,
- Y: für Wasserstoff steht,
- Z: für Methoxy in der 6-Possition steht,
oder
- X: für Methoxy steht,
- Y: für Chlor steht,
- Z: für Wasserstoff steht,
- G: für Wasserstoff (a) oder für die Gruppen 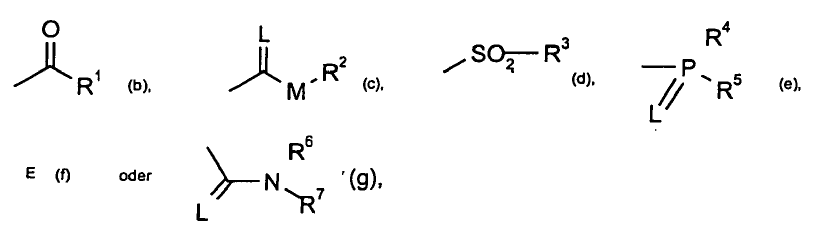 steht,
in welchen
E für ein Metallionäquivalent oder ein Ammoniumion steht und
L und M jeweils für Sauerstoff oder Schwefel stehen.
- R¹: für jeweils gegebenenfalls durch Fluor und/oder Chlor substituiertes C₁-C₁₄-Alkyl, C₂-C₁₄-Alkenyl; C₁-C₄-Alkoxy-C₁-C₆-alkyl, C₁-C₄-Alkylthio-C₁-C₆-alkyl, C₁-C₄-Polyalkoxy-C₂-C₄-alkyl oder Cycloalkyl mit 3 bis 6 Ringatomen, das durch 1 bis 2 Sauerstoff und/oder Schwefelatome unterbrochen sein kann,
für gegebenenfalls durch Fluor, Chlor, Brom, Methyl, Ethyl, Propyl, i-Propyl, Methoxy, Ethoxy, Trifluormethyl, Trifluormethoxy, Methylthio, Ethylthio, Methylsulfonyl, Ethylsulfonyl und/oder Nitro substituiertes Phenyl,
für gegebenenfalls durch Fluor, Chlor, Brom, Methyl, Ethyl, Propyl, i-Propyl, Methoxy, Ethoxy, Trifluormethyl und/oder Trifluormethoxy substituiertes Phenyl-C₁-C₃-alkyl steht,
für gegebenenfalls durch Fluor, Chlor, Brom, Methyl und/oder Ethyl substituiertes Thienyl, Furanyl, Pyridyl, Pyrimidyl, Thiazolyl oder Pyrazolyl,
für gegebenenfalls durch Fluor, Chlor, Methyl, und/oder Ethyl-substituiertes Phenoxy-C₁-C₄-alkyl, oder
für jeweils gegebenenfalls durch Fluor, Chlor, Amino, Methyl und/oder Ethyl substituiertes Pyridyloxy-C₁-C₄-alkyl, Pyrimidyloxy-C₁-C₄-alkyl oder Thiazolyloxy-C₁-C₄-alkyl steht,
- R²: für jeweils gegebenenfalls durch Fluor und/oder Chlor substituiertes C₁-C₁₄-Alkyl, C₃-C₁₄-Alkenyl, C₁-C₄-Alkoxy-C₂-C₆-alkyl, C₁-C₄-Poly-alkoxy-C₂-C₆-alkyl,
für gegebenenfalls durch Fluor, Chlor, Methyl, Ethyl, Propyl, iso-Propyl und/oder Methoxy substituiertes C₃-C₆-Cycloalkyl,
oder für jeweils gegebenenfalls durch Fluor, Chlor, Nitro, Methyl, Ethyl, Propyl, i-Propyl, Methoxy, Ethoxy, und/oder Trifluormethyl substituiertes Phenyl oder Benzyl steht,
- R³, R⁴ und R⁵: unabhängig voneinander für jeweils gegebenenfalls durch Fluor und/oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für jeweils gegebenenfälls durch Fluor, Chlor, Brom, Nitro, Cyano, C₁-C₂-Akoxy, C₁-C₂-Fluoralkoxy, C₁-C₂-Alkylthio, C₁-C₂-Fluoralkylthio und/oder C₁-C₃-Alkyl substituiertes Phenyl, Phenoxy oder Phenylthio stehen und
- R⁶ und R⁷: unabhängig voneinander für Wasserstoff, für jeweils gegebenenfalls durch Fluor, Chlor und/oder Brom substituiertes C₁-C₄-Alkyl, C₃-C₆-Cycloalkyl, C₁-C₄-Alkoxy, C₃-C₄-Alkenyl, C₁-C₄-Alkoxy-C₂-C₄-alkyl, für gegebenenfalls durch Fluor, Chlor, Brom, C₁-C₄-Halogenalkyl, C₁-C₄-Alkyl und/oder C₁-C₄-Alkoxy substituiertes Phenyl, für gegebenenfalls durch Fluor, Chlor, Brom, C₁-C₄-Alkyl, C₁-C₄-Halogen-alkyl und/oder C₁-C₄-Alkoxy substituiertes Benzyl, oder gemeinsam mit dem N-Atom, an das sie gebunden sind, für einen gegebenenfalls durch Sauerstoff oder Schwefel unterbrochenen C₃-C₆-Alkylenring stehen.

Unter Einbeziehung der verschiedenen Bedeutungen (a), (b), (c), (d), (e), (f) und (g) der Gruppe G der allgemeinen Formel (I) ergeben sich folgende hauptsächlichen Strukturen (Ia) bis (Ig): 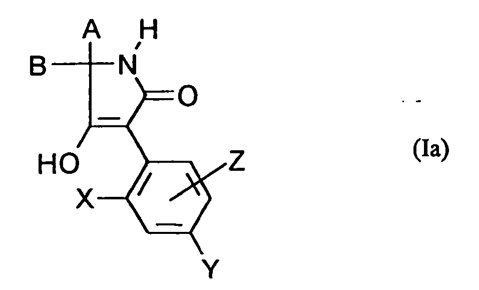 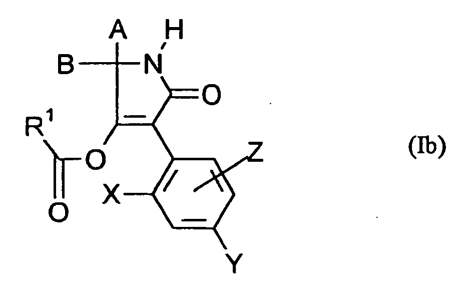 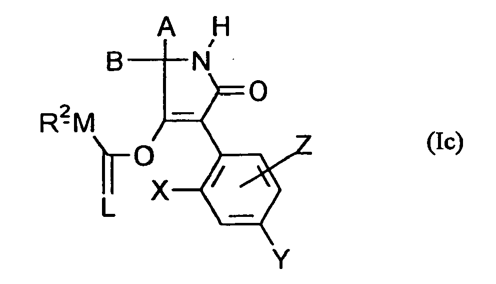 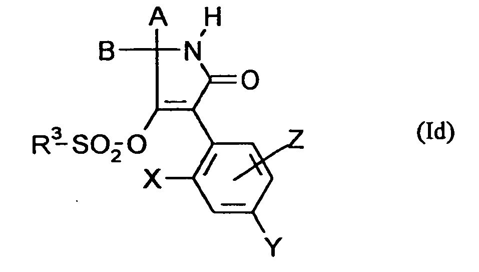 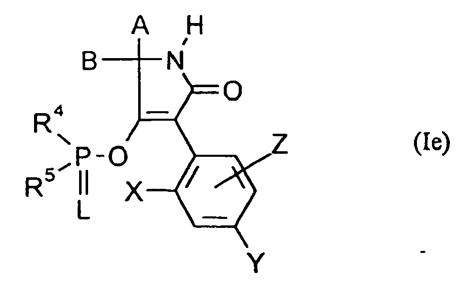 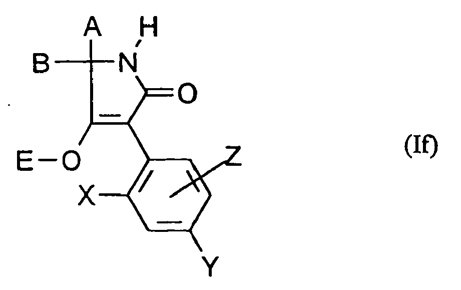 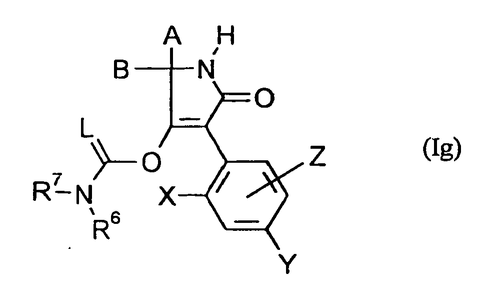 worin
A, B, E, L, M, X, Y, Z, R¹, R², R³, R⁴, R⁵, R⁶ und R⁷ die oben angegebenen Bedeutungen besitzen.

Aufgrund eines oder mehrerer Chiralitätszentren fallen die Verbindungen der Formel (Ia) - (Ig) im allgemeinen als Stereoisomerengemisch an die gegebenenfalls in üblicher Art und Weise getrennt werden können. Sie können sowohl in Form ihrer Diastereomerengemische als auch als reine Diastereomere oder Enantiomere verwendet werden. Im folgenden wird der Einfachheit halber stets von Verbindungen der Formel (Ia) bis (Ig) gesprochen, obwohl sowohl die reinen

Verbindungen, als auch die Gemische mit unterschiedlichen Anteilen an isomeren, enantiomeren und stereomeren Verbindungen gemeint sind.

Weiterhin wurde gefunden, daß man die neuen substituierten 1-H-3-Aryl-pyrrolidin-2,4-dion-Derivate der Formel (I) nach einem der im folgenden beschriebenen Verfahren erhält.
(A) Man erhält 1-H-3-Aryl-pyrrolidin-2,4-dione bzw. deren Enole der Formel (Ia) 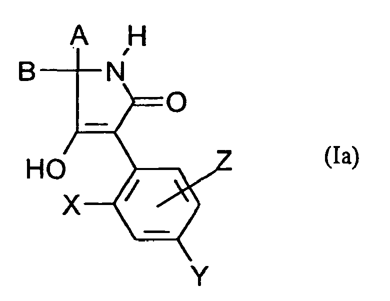 in welcher
   A, B, X, Y und Z die oben angegebene Bedeutung haben, wenn man
   α) N-Acylaminosäureester der Formel (II) 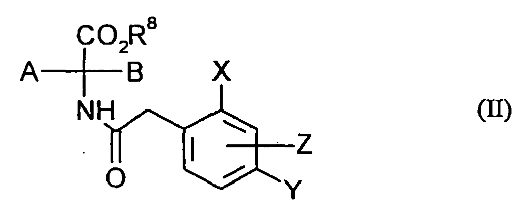 in welcher
      A, B, X, Y und Z die oben angegebene Bedeutung haben,
      und
      R⁸ für Alkyl steht,
      in Gegenwart eines Verdünnungsmittels und in Gegenwart einer Base intramolekular kondensiert;
      oder
   β) man erhält 1-H-3-Aryl-pyrrolidin-2,4-dione bzw. deren Enole der Formel (Iaa) 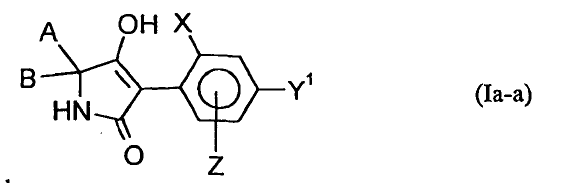 in welcher
      A, B, X und Z die oben angegebenen Bedeutungen haben, und
      Y¹ für -OR⁸ steht, wobei
      R⁸ für Alkyl steht,
      wenn man N-Acylaminosäureester der Formel (IIa) 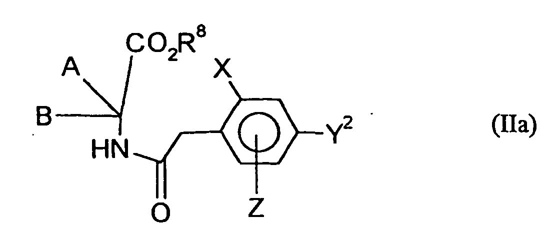 in welcher
      A, B, X und Z die oben angegebenen Bedeutungen haben,
      Y² für Fluor steht und
      R⁸ für Alkyl, vorzugsweise C₁-C₈-Alkyl, steht,
      in Gegenwart eines Verdünnungsmittels und in Gegenwart einer Base intramolekular kondensiert;
   oder
(B) man erhält Verbindungen der Formel (Ib) 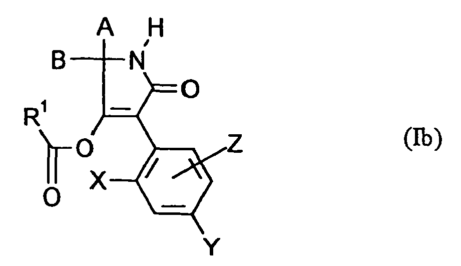 in welcher
   A, B, X, Y, Z und R¹ die oben angegebene Bedeutung haben,
   wenn man Verbindungen der Formel (Ia), 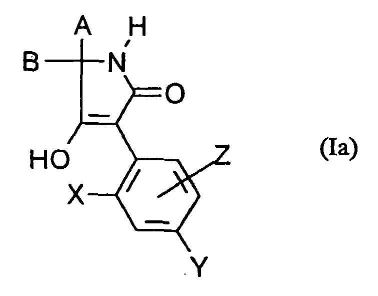 in welcher
   A, B, X, Y und Z die oben angegebene Bedeutung haben,
   α) mit Säurehalogeniden der allgemeinen Formel (III) 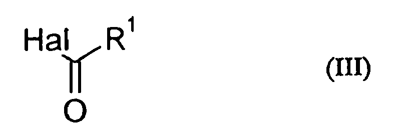 in welcher
      R¹ die oben angegebene Bedeutung hat und
      Hal für Halogen, insbesondere Chlor oder Brom steht,
      gegebenenfalls in Gegenwart eines Verdünnungsmittels und gegebenenfalls in Gegenwart eines Säurebindemittels umsetzt
      oder
   β) mit Carbonsäureanhydriden der allgemeinen Formel (IV)

      R¹-CO-O-CO-R¹ (IV)

      in welcher
      R¹ die oben angegebene Bedeutung hat,
      gegebenenfalls in Gegenwart eines Verdünnungsmittels und gegebenenfalls in Gegenwart eines Säurebindemittels,
   umsetzt;
   oder
(C) man erhält Verbindungen der Formel (Ic-a) 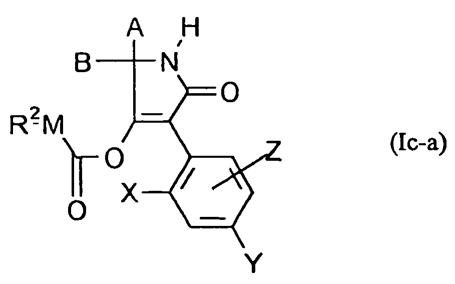 in welcher
   A, B, X, Y, Z und R² die oben angegebene Bedeutung haben,
   und
   M für Sauerstoff oder Schwefel steht, wenn man Verbindungen der Formel (Ia) 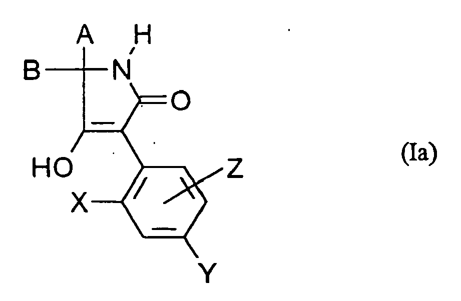 in welcher
   A, B, X, Y und Z die oben angegebene Bedeutung haben,
   mit Chlorameisensäureester oder Chlorameisensäurethiolester der allgemeinen Formel (V)

   R²-M-CO-Cl (V)

   in welcher
   R² und M die oben angegebene Bedeutung haben,
   gegebenenfalls in Gegenwart eines Verdünnungsmittels und gegebenenfalls in Gegenwart eines Säurebindemittels umsetzt;
   oder
(D) man erhält Verbindungen der Formel (Ic-b) 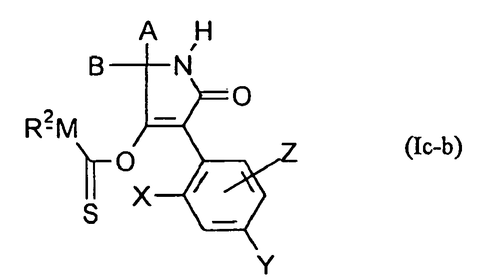 in welcher
   A, B, R², X, Y und Z die oben angegebene Bedeutung haben
   und
   M für Sauerstoff oder Schwefel steht,
   wenn man Verbindungen der Formel (Ia) 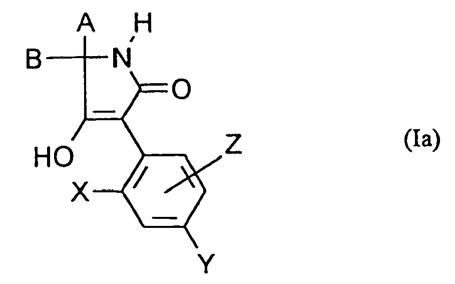 in welcher
   A, B, X, Y und Z die oben angegebene Bedeutung haben,
   α) mit Chlormonothioameisensäureestem oder Chlordithioanleisensäureestem der allgemeinen Formel (VI) 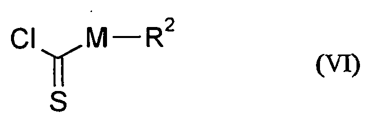 in welcher
      M und R² die oben angegebene Bedeutung haben,
      gegebenenfalls in Gegenwart eines Verdünnungsmittels und gegebenenfalls in Gegenwart eines Säurebindemittels umsetzt,
      oder
   β) mit Schwefelkohlenstoff und anschließend mit Alkylhalogeniden der allgemeinen Formel (VII)

      R²-Hal (VII)

      in welcher
      R² die oben angegebene Bedeutung hat
      und
      Hal für Chlor, Brom oder Iod steht,
      umsetzt;
      oder
(E) man erhält Verbindungen der Formel (Id) 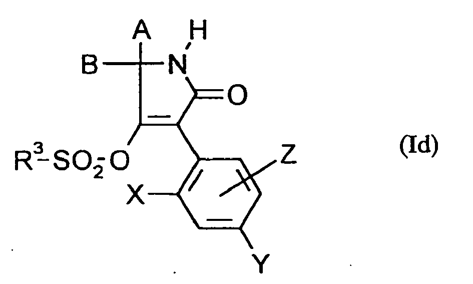 in welcher
   A, B, X, Y, Z und R³ die oben angegebene Bedeutung haben,
   wenn man Verbindungen der Formel (Ia) 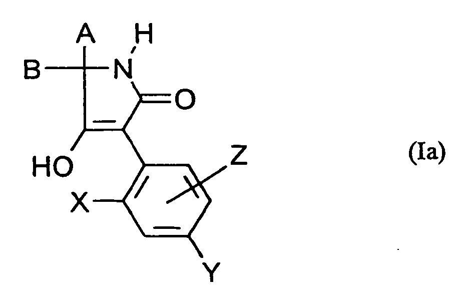 in welcher
   A, B, X, Y und Z die oben angegebene Bedeutung haben,
   mit Sulfonsäurechloriden der allgemeinen Formel (VIII)

   R³-SO₂-Cl (VIII)

   in welcher
   R³ die oben angegebene Bedeutung hat,
   gegebenenfalls in Gegenwart eines Verdünnungsmittels und gegebenenfalls in Gegenwart eines Säurebindemittels,
   umsetzt;
   oder
(F) man erhält 3-Aryl-pyrrolidin-2,4-dione der Formel (Ie) 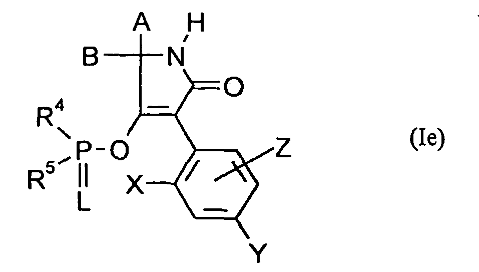 in welcher
   A, B, L, X, Y, Z, R⁴ und R⁵ die oben angegebene Bedeutung haben,
   wenn man
   1-H-3-Aryl-pyrrolidin-2,4-dione der Formel (Ia) bzw. deren Enole 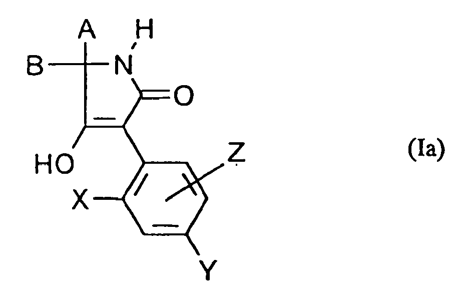 in welcher
   A, B, X, Y und Z die oben angegebene Bedeutung haben,
   mit Phosphorverbindungen der allgemeinen Formel (IX) 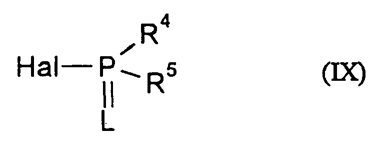 in welcher
   L, R⁴ und R⁵ die oben angegebene Bedeutung haben
   und
   Hal für Halogen, insbesondere Chlor oder Brom steht,
   umsetzt;
   oder
(G) man erhält Verbindungen der Formel (If) 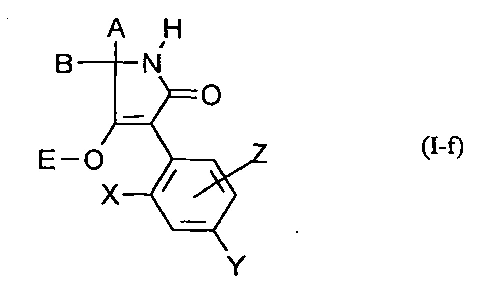 in welcher
   A, B, X, Y und Z die oben angegebene Bedeutung haben,
   und
   E für ein Metallionäquivalent oder für ein Ammoniumion steht,
   wenn man Verbindungen der Formel (Ia) 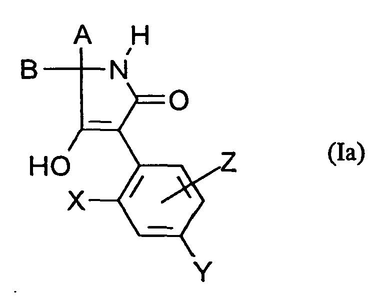 in welcher
   A, B, X, Y und Z die oben angegebene Bedeutung haben,
   mit Metallverbindungen oder Aminen der allgemeinen Formeln (X) und (XI)
   Me(OR⁹)ₜ (X) 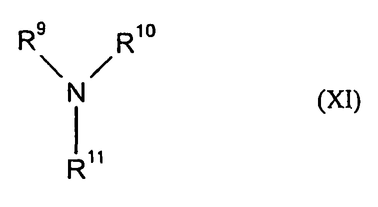 in welchen
   Me für ein- oder zweiwertige Metallionen,
   t für die Zahl 1 oder 2 und
   R⁹, R¹⁰ und R¹¹ unabhängig voneinander für Wasserstoff und/oder Alkyl stehen,
   gegebenenfalls in Gegenwart eines Verdünnungsmittels, umsetzt.
(H) Ferner wurde gefunden, daß man Verbindungen der Formel (Ig) 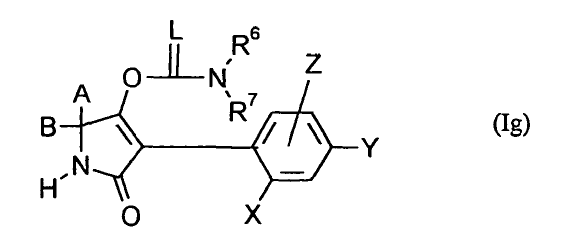 in welcher
   A, B, L, X, Y, Z, R⁶ und R⁷ die oben angegebene Bedeutung haben,
   erhält, wenn man Verbindungen der Formel (Ia) 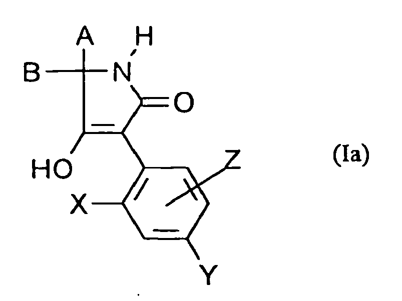 in welcher
   A, B, X, Y und Z die oben angegebene Bedeutung haben,
   α) mit Isocyanaten oder Isothiocyanaten der allgemeinen Formel (XII)

      R⁶-N=C=L (XII)

      in welcher
      R⁶ die oben angegebene Bedeutung hat
      gegebenenfalls in Gegenwart eines Verdünnungsmittels und gegebenenfalls in Gegenwart eines Katalysators
      oder
   β) mit Carbamidsäurechloriden oder Thiocarbamidsäurechloriden der allgemeinen Formel (XIII) 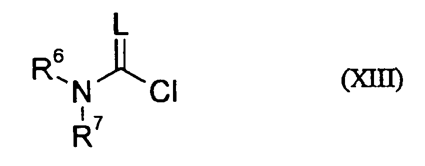 in welcher
      L, R⁶ und R⁷ die oben angegebene Bedeutung haben
      gegebenenfalls in Gegenwart eines Verdünnungsmittels und gegebenenfalls in Gegenwart eines Säurebindemittels,
      umsetzt.
      Weiterhin wurde gefunden, daß sich die neuen 1-H-3-Arylpyrrolidin-2,4-dion-Derivate der Formel (I) durch hervorragende Insektizide und akarizide Wirkungen auszeichnen. Weiterhin haben Verbindungen der Formel (I) herbizide und fungizide Nebenwirkungen.

Wenn nichts anderes angegeben ist, sind Alkylreste, auch in Verbindung mit Heteroatomen wie z.. in Alkoxy, soweit möglich jeweils geradkettig oder verzweigt.

Im einzelnen seien außer bei den bei Herstellungsbeispielen genannten Verbindungen die folgenden Verbindungen der Formel (Ia) genannt:

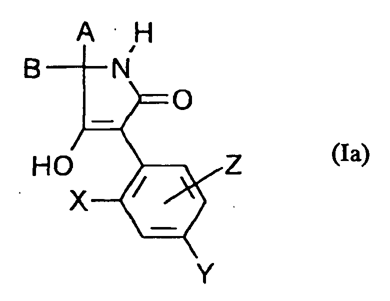

| X | Y | Z | A | B |
|---|---|---|---|---|
| Cl | OCH₃ | H | CH₃ | H |
| Cl | OCH₃ | H | C₂H₅ | H |
| Cl | OCH₃ | H | C₃H₇ | H |
| Cl | OCH₃ | H | i-C₃H₇ | H |
| Cl | OCH₃ | H | C₄H₉ | H |
| Cl | OCH₃ | H | i-C₄H₉ | H |
| Cl | OCH₃ | H | s-C₄H₉ | H |
| Cl | OCH₃ | H | t-C₄H₉ | H |
| Cl | OCH₃ | H | CH₃ | CH₃ |
| Cl | OCH₃ | H | C₂H₅ | CH₃ |
| Cl | OCH₃ | H | C₃H₇ | CH₃ |
| Cl | OCH₃ | H | i-C₃H₇ | CH₃ |
| Cl | OCH₃ | H | C₄F₉ | CH₃ |
| Cl | OCH₃ | H | i-C₄H₉ | CH₃ |
| Cl | OCH₃ | H | s-C₄H₉ | CH |
| Cl | OCH₃ | H | t-C₄H₉ | CH₃ |
| Cl | OCH₃ | H | C₂H₅ | C₂H₅ |
| Cl | OCH₃ | H | C₃H₇ | C₃H₇ |
| Cl | OCH₃ | H | 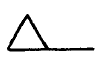 | CH₃ |
| Cl | OCH₃ | H | 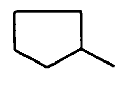 | CH₃ |
| Cl | OCH₃ | H | 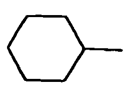 | CH₃ |
| Cl | H | 6-OCH₃ | CH₃ | H |
| Cl | H | 6-OCH₃ | C₂H₅ | H |
| Cl | H | 6-OCH₃ | C₃H₇ | H |
| Cl | H | 6-OCH₃ | i-C₃H₇ | H |
| Cl | H | 6-OCH₃ | C₄H₉ | H |
| Cl | H | 6-OCH₃ | i-C₄H₉ | H |
| Cl | H | 6-OCH₃ | s-C₄H₉ | H |
| Cl | H | 6-OCH₃ | t-C₄H₉ | H |
| Cl | H | 6-OCH₃ | CH₃ | CH₃ |
| Cl | H | 6-OCH₃ | C₂H₅ | CH₃ |
| Cl | H | 6-OCH₃ | C₃H₇ | CH₃ |
| Cl | H | 6-OCH₃ | i-C₃H₇ | CH₃ |
| Cl | H | 6-OCH₃ | C₄H₉ | CH₃ |
| Cl | H | 6-OCH₃ | i-C₄H₉ | CH₃ |
| Cl | H | 6-OCH₃ | s-C₄H₉ | CH₃ |
| Cl | H | 6-OCH₃ | t-C₄H₉ | CH₃ |
| Cl | H | 6-OCH₃ | C₂H₅ | C₂H₅ |
| Cl | H | 6-OCH₃ | C₃H₇ | C₃H₇ |
| Cl | H | 6-OCH₃ | 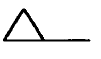 | CH₃ |
| Cl | H | 6-OCH₃ | 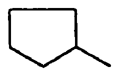 | CH₃ |
| Cl | H | 6-OCH₃ | 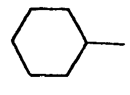 | CH₃ |
| OCH₃ | Cl | H | CH₃ | H |
| OCH₃ | Cl | H | C₂H₅ | H |
| OCH₃ | Cl | H | C₃H₇ | H |
| OCH₃ | Cl | H | i-C₃H₇ | H |
| OCH₃ | Cl | H | C₄H₉ | H |
| OCH₃ | Cl | H | i-C₄H₉ | H |
| OCH₃ | Cl | H | s-C₄H₉ | H |
| OCH₃ | Cl | H | t-C₄H₉ | H |
| OCH₃ | Cl | H | CH₃ | CH₃ |
| OCH₃ | Cl | H | C₂H₅ | CH₃ |
| OCH₃ | Cl | H | C₃H₇ | CH₃ |
| OCH₃ | Cl | H | i-C₃H₇ | CH₃ |
| OCH₃ | Cl | H | C₄H₉ | CH₃ |
| OCH₃ | Cl | H | i-C₄H₉ | CH₃ |
| OCH₃ | Cl | H | s-C₄H₉ | CH₃ |
| OCH₃ | Cl | H | t-C₄H₉ | CH₃ |
| OCH₃ | Cl | H | C₂H₅ | C₂H₅ |
| OCH₃ | Cl | H | C₃H₇ | C₃H₇ |
| OCH₃ | Cl | H | 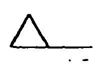 | CH₃ |
| OCH₃ | Cl | H | 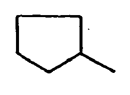 | CH₃ |
| OCH₃ | Cl | H | 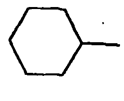 | CH₃ |
| Cl | OCH₃ | H | -(CH₂)₂- | |
| Cl | OCH₃ | H | -(CH₂)₄- | |
| Cl | OCH₃ | H | -(CH₂)₅- | |
| Cl | OCH₃ | H | -(CH₂)₆- | |
| Cl | OCH₃ | H | -(CH₂)₇- | |
| Cl | OCH₃ | H | -(CH₂)₂-O-(CH₂)₂- | |
| Cl | OCH₃ | H | -(CH₂)₂-S-(CH₂)₂- | |
| Cl | OCH₃ | H | -CH₂-CHCH₃-(CH₂)₃- | |
| Cl | OCH₃ | H | -(CH₂)₂-CHCH₃-(CH₂)₂- | |
| Cl | OCH₃ | H | -(CH₂)₂-CHC₂H₅(CH₂)₂- | |
| Cl | OCH₃ | H | -(CH₂)₂-CHC₃H₇-(CH₂)₂- | |
| Cl | OCH₃ | H | -(CH₂)₂-CHi-C₃H₇-(CH₂)₂- | |
| Cl | OCH₃ | H | -(CH₂)₂-CHOCH₃-(CH₂)₂- | |
| Cl | OCH₃ | H | -(CH₂)₂-CHOC₂H₅-(CH₂)₂- | |
| Cl | OCH₃ | H | -(CH₂)₂-CHOC₃H₇-(CH₂)₂- | |
| Cl | OCH₃ | H | -(CH₂)₂-CHi-OC₃H₇-(CH₂)₂- | |
| Cl | OCH₃ | H | -(CH₂)₂-C(CH₃)₂₋(CH₂)₂- | |
| Cl | OCH₃ | H | -CH₂-(CHCH₃)₂-(CH₂)₂- | |
| Cl | OCH₃ | H | 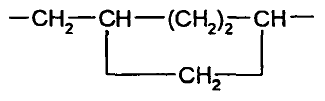 | |
| Cl | OCH₃ | H | 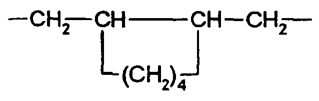 | |
| Cl | OCH₃ | H | 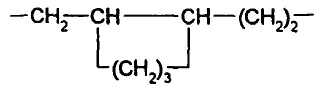 | |
| Cl | H | 6-OCH₃ | -(CH₂)₂- | |
| Cl | H | 6-OCH₃ | -(CH₂)₄- | |
| Cl | H | 6-OCH₃ | -(CH₂)₅- | |
| Cl | H | 6-OCH₃ | -(CH₂)₆- | |
| Cl | H | 6-OCH₃ | -(CH₂)₇- | |
| Cl | H | 6-OCH₃ | -(CH₂)₂-O-(CH₂)₂- | |
| Cl | H | 6-OCH₃ | -(CH₂)₂-S-(CH₂)₂- | |
| Cl | H | 6-OCH₃ | -CH₂-CHCH₃-(CH₂)₃- | |
| Cl | H | 6-OCH₃ | -(CH₂)₂-CHCH₃-(CH₂)₂- | |
| Cl | H | 6-OCH₃ | -(CH₂)₂-CHC₂H₅-(CH₂)₂- | |
| Cl | H | 6-OCH₃ | -(CH₂)₂-CHC₃H₇-(CH₂)₂- | |
| Cl | H | 6-OCH₃ | -(CH₂)₂-CHi-C₃H₇-(CH₂)₂- | |
| Cl | H | 6-OCH₃ | -(CH₂)₂-CHOCH₃-(CH₂)₂- | |
| Cl | H | 6-OCH₃ | -(CH₂)₂-CHOC₂H_{S}-(CH₂)₂- | |
| Cl | H | 6-OCH₃ | -(CH₂)₂-CHOC₃H₇-(CH₂)₂- | |
| Cl | H | 6-OCH₃ | -(CH₂)₂-CHi-OC₃H₇-(CH₂)₂- | |
| Cl | H | 6-OCH₃ | -(CH₂)₂-C(CH₃)₂-(CH₂)₂- | |
| Cl | H | 6-OCH₃ | -CH₂-(CHCH₃)₂-(CH₂)₂- | |
| Cl | H | 6-OCH₃ | 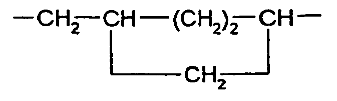 | |
| Cl | H | 6-OCH₃ | 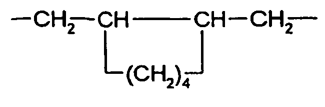 | |
| Cl | H | 6-OCH₃ | 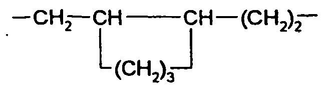 | |
| OCH₃ | Cl | H | -(CH₂)₂- | |
| OCH₃ | Cl | H | -(CH₂)₄- | |
| OCH₃ | Cl | H | -(CH₂)₅- | |
| OCH₃ | Cl | H | -(CH₂)₆- | |
| OCH₃ | Cl | H | -(CH₂)₇- | |
| OCH₃ | Cl | H | -(CH₂)₂-O-(CH₂)₂- | |
| OCH₃ | Cl | H | -(CH₂)₂-S-(CH₂)₂- | |
| OCH₃ | Cl | H | -CH₂-CHCH₃-(CH₂)₃- | |
| OCH₃ | Cl | H | -(CH₂)₂-CHCH₃-(CH₂)₂- | |
| OCH₃ | Cl | H | -(CH₂)₂-CHC₂H₅-(CH₂)₂- | |
| OCH₃ | Cl | H | -(CH₂)₂-CHC₃H₇-(CH₂)₂- | |
| OCH₃ | Cl | H | -(CH₂)₂-CHi-C₃H₇-(CH₂)₂- | |
| OCH₃ | Cl | H | -(CH₂)₂-CHOCH₃-(CH₂)₂- | |
| OCH₃ | Cl | H | -(CH₂)₂-CHOC₂H₅-(CH₂)₂- | |
| OCH₃ | Cl | H | -(CH₂)₂-CHOC₃H₇-(CH₂)₂- | |
| OCH₃ | Cl | H | -(CH₂)₂-CHi-OC₃H₁-(CH₂)₂- | |
| OCH₃ | Cl | H | -(CH₂)₂=C(CH₃)₂-(CH₂)₂- | |
| OCH₃ | Cl | H | -CH₂-(CHCH₃)₂-(CH₂)₂- | |
| OCH₃ | Cl | H | 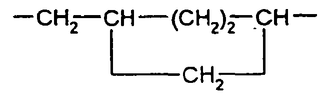 | |
| OCH₃ | Cl | H | 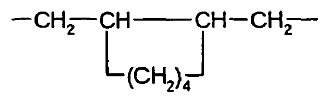 | |
| OCH₃ | Cl | H | 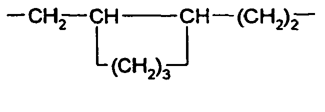 | |

Im einzelnen seien außer den bei den Herstellungsbeispielen genannten Verbindungen die folgenden Verbindungen der Formel (Ib) genannt:

**Tabelle 2:**

| | | | | | |
|---|---|---|---|---|---|
| 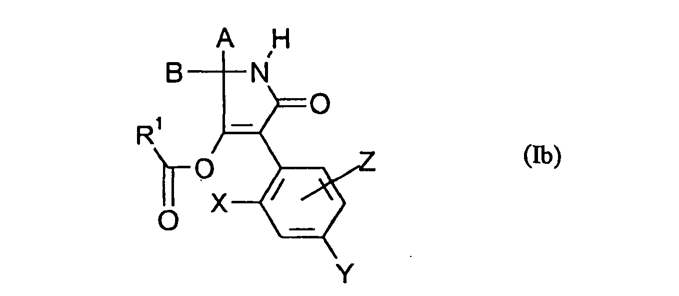 | | | | | |

| X | Y | Z | A | B | R¹ |
|---|---|---|---|---|---|
| Cl | OCH₃ | H | CH₃ | H | CH₃ |
| Cl | OCH₃ | H | C₂H₅ | H | CH₃ |
| Cl | OCH₃ | H | C₃H₇ | H | CH₃ |
| Cl | OCH₃ | H | i-C₃H₇ | H | CH₃ |
| Cl | OCH₃ | H | C₄H₉ | H | CH₃ |
| Cl | OCH₃ | H | i-C₄H₉ | H | CH₃ |
| Cl | OCH₃ | H | s-C₄H₉ | H | CH₃ |
| Cl | OCH₃ | H | t-C₄H₉ | H | CH₃ |
| Cl | OCH₃ | H | CH₃ | CH₃ | CH₃ |
| Cl | OCH₃ | H | C₂H₅ | CH₃ | CH₃ |
| Cl | OCH₃ | H | C₃H₇ | CH₃ | CH₃ |
| Cl | OCH₃ | H | i-C₃H₇ | CH₃ | CH₃ |
| Cl | OCH₃ | H | C₄H₉ | CH₃ | CH₃ |
| Cl | OCH₃ | H | i-C₄H₉ | CH₃ | CH₃ |
| Cl | OCH₃ | H | s-C₄H₉ | CH₃ | CH₃ |
| Cl | OCH₃ | H | t-C₄H₉ | CH₃ | CH₃ |
| Cl | OCH₃ | H | C₂H₅ | C₂H₅ | CH₃ |
| Cl | OCH₃ | H | C₃H₇ | C₃H₇ | CH₃ |
| Cl | OCH₃ | H | 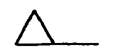 | CH₃ | CH₃ |
| Cl | OCH₃ | H | 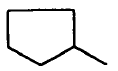 | CH₃ | CH₃ |
| Cl | OCH₃ | H | 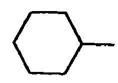 | CH₃ | CH₃ |
| Cl | H | 6-OCH₃ | CH₃ | H | CH₃ |
| Cl | H | 6-OCH₃ | C₂H₅ | H | CH₃ |
| Cl | H | 6-OCH₃ | C₃H₇ | H | CH₃ |
| Cl | H | 6-OCH₃ | i-C₃H₇ | H | CH₃ |
| Cl | H | 6-OCH₃ | C₄H₉ | H | CH₃ |
| Cl | H | 6-OCH₃ | i-C₄H₉ | H | CH₃ |
| Cl | H | 6-OCH₃ | s-C₄H₉ | H | CH₃ |
| Cl | H | 6-OCH₃ | t-C₄H₉ | H | CH₃ |
| Cl | H | 6-OCH₃ | CH₃ | CH₃ | CH₃ |
| Cl | H | 6-OCH₃ | C₂H₅ | CH₃ | CH₃ |
| Cl | H | 6-OCH₃ | C₃H₇ | CH₃ | CH₃ |
| Cl | H | 6-OCH₃ | i-C₃H₇ | CH₃ | CH₃ |
| Cl | H | 6-OCH₃ | C₄H₉ | CH₃ | CH₃ |
| Cl | H | 6-OCH₃ | i-C₄H₉ | CH₃ | CH₃ |
| Cl | H | 6-OCH | s-C₄H₉ | CH₃ | CH₃ |
| Cl | H | 6-OCH₃ | t-C₄H₉ | CH₃ | CH₃ |
| Cl | H | 6-OCH₃ | C₂H₅ | C₂H₅ | CH₃ |
| Cl | H | 6-OCH₃ | C₃H₇ | C₃H₇ | CH3 |
| Cl | H | 6-OCH₃ | 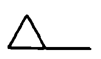 | CH₃ | CH₃ |
| Cl | H | 6-OCH₃ | 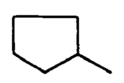 | CH₃ | CH₃ |
| Cl | H | 6-OCH₃ | 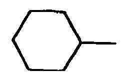 | CH₃ | CH₃ |
| OCH₃ | Cl | H | CH₃ | H | CH₃ |
| OCH₃ | Cl | H | C₂H₅ | H | CH₃ |
| OCH₃ | Cl | H | C₃H₇ | H | CH₃ |
| OCH₃ | Cl | H | i-C₃H₇ | H | CH₃ |
| OCH₃ | Cl | H | C₄H₉ | H | CH₃ |
| OCH₃ | Cl | H | i-C₄H₉ | H | CH₃ |
| OCH₃ | Cl | H | s-C₄H₉ | H | CH₃ |
| OCH₃ | Cl | H | t-C₄H₉ | H | CH₃ |
| OCH₃ | Cl | H | CH₃ | CH₃ | CH₃ |
| OCH₃ | Cl | H | C₂H₅ | CH₃ | CH₃ |
| OCH₃ | Cl | H | C₃H₇ | CH₃ | CH₃ |
| OCH₃ | Cl | H | i-C₃H₇ | CH₃ | CH₃ |
| OCH₃ | Cl | H | C₄H₉ | CH₃ | CH₃ |
| OCH₃ | Cl | H | i-C₄H₉ | CH₃ | CH₃ |
| OCH₃ | Cl | H | s-C₄H₉ | CH₃ | CH₃ |
| OCH₃ | Cl | H | t-C₄H₉ | CH₃ | CH₃ |
| OCH₃ | Cl | H | C₂H₅ | C₂H₅ | CH₃ |
| OCH₃ | Cl | H | C₃H₇ | C₃H₇ | CH₃ |
| OCH₃ | Cl | H | 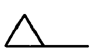 | CH₃ | CH₃ |
| OCH₃ | Cl | H | 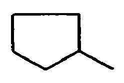 | CH₃ | CH₃ |
| OCH₃ | Cl | H | 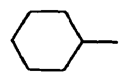 | CH₃ | CH₃ |
| Cl | OCH₃ | H | -(CH₂)₂- | | CH₃ |
| Cl | OCH₃ | H | -(CH₂)₄- | | CH₃ |
| Cl | OCH₃ | H | -(CH₂)₅- | | CH₃ |
| Cl | OCH₃ | H | -(CH₂)₆- | | CH₃ |
| Cl | OCH₃ | H | -(CH₂)₇- | | CH₃ |
| Cl | OCH₃ | H | -(CH₂)₂-O-(CH₂)₂- | | CH₃ |
| Cl | OCH₃ | H | -(CH₂)₂-S-(CH₂)₂- | | CH₃ |
| Cl | OCH₃ | H | -CH₂-CHCH₃-(CH₂)₃- | | CH₃ |
| Cl | OCH₃ | H | -(CH₂)₂-CHCH₃-(CH₂)₂- | | CH₃ |
| Cl | OCH₃ | H | -(CH₂)₂-CHC₂H₅-(CH₂)₂- | | CH₃ |
| Cl | OCH₃ | H | -(CH₂)₂-CHC₃H₇-(CH₂)₂- | | CH₃ |
| Cl | OCH₃ | H | -(CH₂)₂-CHi-C₃H₇-(CH₂)₂- | | CH₃ |
| Cl | OCH₃ | H | -(CH₂)₂-CHOCH₃-(CH₂)₂- | | CH₃ |
| Cl | OCH₃ | H | -(CH₂)₂-CHOC₂H₅-(CH₂)₂- | | CH₃ |
| Cl | OCH₃ | H | -(CH₂)₂-CHOC₃H₇-(CH₂)₂- | | CH₃ |
| Cl | OCH₃ | H | -(CH₂)₂-CHi-OC₃H₇-(CH₂)₂- | | CH₃ |
| Cl | OCH₃ | H | -(CH₂)₂-C(CH₃)₂-(CH₂)₂- | | CH₃ |
| Cl | OCH₃ | H | -CH₂-(CHCH₃)₂-(CH₂)₂- | | CH₃ |
| Cl | OCH₃ | H | 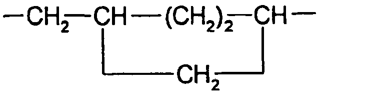 | | CH₃ |
| Cl | OCH₃ | H | 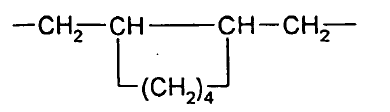 | | CH₃ |
| Cl | OCH₃ | H | 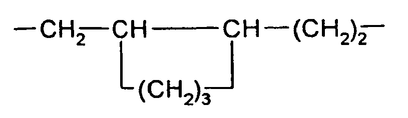 | | CH₃ |
| Cl | H | 6-OCH₃ | -(CH₂)₂- | | CH₃ |
| Cl | H | 6-OCH₃ | -(CH₂)₄- | | CH₃ |
| Cl | H | 6-OCH₃ | -(CH₂)₅- | | CH₃ |
| Cl | H | 6-OCH₃ | -(CH₂)₆- | | CH₃ |
| Cl | H | 6-OCH₃ | -(CH₂)₇- | | CH₃ |
| Cl | H | 6-OCH₃ | -(CH₂)₂-O-(CH₂)₂- | | CH₃ |
| Cl | H | 6-OCH₃ | -(CH₂)₂-S-(CH₂)₂- | | CH₂ |
| Cl | H | 6-OCH₃ | -CH₂-CHCH₃-(CH₂)₃- | | CH₃ |
| Cl | H | 6-OCH₃ | -(CH₂)₂-CHCH₃-(CH₂)₂- | | CH₂ |
| Cl | H | 6-OCH₃ | -(CH₂)₂-CHC₂H₅-(CH₂)₂- | | CH₃ |
| Cl | H | 6-OCH₃ | -(CH₂)₂-CHC₃H₇-(CH₂)₂- | | CH₃ |
| Cl | H | 6-OCH₃ | -(CH₂)₂-CHi-C₃H₇-(CH₂)₂- | | CH₃ |
| Cl | H | 6-OCH₃ | -(CH₂)₂-CHOCH₃-(CH₂)₂- | | CH₃ |
| Cl | H | 6-OCH₃ | -(CH₂)₂-CHOC₂H₅-(CH₂)₂- | | CH₃ |
| Cl | H | 6-OCH₃ | -(CH₂)₂-CHOC₃H₇-(CH₂)₂- | | CH₃ |
| Cl | H | 6-OCH₃ | -(CH₂)₂-CHi-OC₃H₇-(CH₂)₂- | | CH₃ |
| Cl | H | 6-OCH₃ | -(CH₂)₂-C(CH₃)₂-(CH₂)₂- | | CH₃ |
| Cl | H | 6-OCH₃ | -CH₂-(CHCH₃)₂-(CH₂)₂- | | CH₃ |
| Cl | H | 6-OCH₃ | 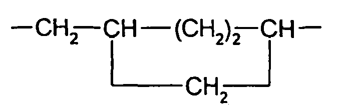 | | CH₃ |
| Cl | H | 6-OCH₃ | 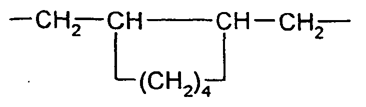 | | CH₃ |
| Cl | H | 6-OCH₃ | 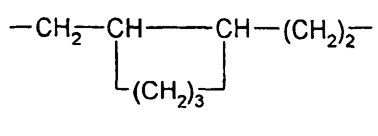 | | CH₃ |
| OCH₃ | Cl | H | -(CH₂)₂- | | CH₃ |
| OCH₃ | Cl | H | -(CH₂)₄- | | CH₃ |
| OCH₃ | Cl | H | -(CH₂)₅- | | CH₃ |
| OCH₃ | Cl | H | -(CH₂)₆- | | CH₃ |
| OCH₃ | Cl | H | -(CH₂)₇- | | CH₃ |
| OCH₃ | Cl | H | -(CH₂)₂-O-(CH₂)₂- | | CH₃ |
| OCH₃ | Cl | H | -(CH₂)₂-S-(CH₂)₂- | | CH₃ |
| OCH₃ | Cl | H | -CH₂-CHCH₃-(CH₂)₃- | | CH₃ |
| OCH₃ | Cl | H | -(CH₂)₂-CHCH₃-(CH₂)₂- | | CH₃ |
| OCH₃ | Cl | H | -(CH₂)₂-CHC₂H₅-(CH₂)₂- | | CH₃ |
| OCH₃ | Cl | H | -(CH₂)₂-CHC₃H₇-(CH₂)₂- | | CH₃ |
| OCH₃ | Cl | H | -(CH₂)₂-CHi-C₃H₇-(CH₂)₂- | | CH₃ |
| OCH₃ | Cl | H | -(CH₂)₂-CHOCH₃-(CH₂)₂- | | CH₃ |
| OCH₃ | Cl | H | -(CH₂)₂-CHOC₂H₅-(CH₂)₂- | | CH₃ |
| OCH₃ | Cl | H | -(CH₂)₂-CHOC₃H₇-(CH₂)₂- | | CH₃ |
| OCH₃ | Cl | H | -(CH₂)₂-CHi-OC₃H₇-(CH₂)₂- | | CH₃ |
| OCH₃ | Cl | H | -(CH₂)₂-C(CH₃)₂-(CH₂)₂- | | CH₃ |
| OCH₃ | Cl | H | -CH₂-(CHCH₃)₂-(CH₂)₂- | | CH₃ |
| OCH₃ | Cl | H | 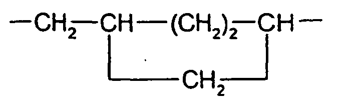 | | CH₃ |
| OCH₃ | Cl | H | 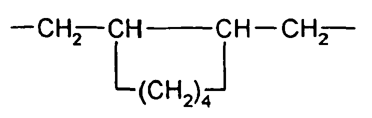 | | CH₃ |
| OCH₃ | Cl | H | 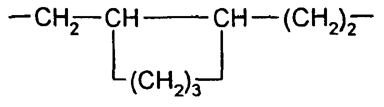 | | CH₃ |
| Cl | OCH₃ | H | CH₃ | H | i-C₃H₇ |
| Cl | OCH₃ | H | C₂H₅ | H | i-C₃H₇ |
| Cl | OCH₃ | H | C₃H₇ | H | i-C₃H₇ |
| Cl | OCH₃ | H | i-C₃H₇ | H | i-C₃H₇ |
| Cl | OCH₃ | H | C₄H₉ | H | i-C₃H₇ |
| Cl | OCH₃ | H | i-C₄H₉ | H | i-C₃H₇ |
| Cl | OCH₃ | H | s-C₄H₉ | H | i-C₃H₇ |
| Cl | OCH₃ | H | t-C₄H₉ | H | i-C₃H₇ |
| Cl | OCH₃ | H | CH₃ | CH₃ | i-C₃H₇ |
| Cl | OCH₃ | H | C₂H₅ | CH₃ | i-C₃H₇ |
| Cl | OCH₃ | H | C₃H₇ | CH₃ | i-C₃H₇ |
| Cl | OCH₃ | H | i-C₃H₇ | CH₃ | i-C₃H₇ |
| Cl | OCH₃ | H | C₄H₉ | CH₃ | i-C₃H₇ |
| Cl | OCH₃ | H | i-C₄H₉ | CH₃ | i-C₃H₇ |
| Cl | OCH₃ | H | s-C₄H₉ | CH₃ | i-C₃H₇ |
| Cl | OCH₃ | H | t-C₄H₉ | CH₃ | i-C₃H₇ |
| Cl | OCH₃ | H | C₂H₅ | C₂H₅ | i-C₃H₇ |
| Cl | OCH₃ | H | C₃H₇ | C₃H₇ | i-C₃H₇ |
| Cl | OCH₃ | H | 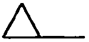 | CH₃ | i-C₃H₇ |
| Cl | OCH₃ | H | 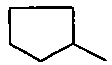 | CH₃ | i-C₃H₇ |
| Cl | OCH₃ | H | 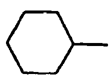 | CH₃ | i-C₃H₇ |
| Cl | H | 6-OCH₃ | CH₃ | H | i-C₃H₇ |
| Cl | H | 6-OCH₃ | C₂H₅ | H | i-C₃H₇ |
| Cl | H | 6-OCH₃ | C₃H₇ | H | i-C₃H₇ |
| Cl | H | 6-OCH₃ | i-C₃H₇ | H | i-C₃H₇ |
| Cl | H | 6-OCH₃ | C₄H₉ | H | i-C₃H₇ |
| Cl | H | 6-OCH₃ | i-C₄H₉ | H | i-C₃H₇ |
| Cl | H | 6-OCH₃ | s-C₄H₉ | H | i-C₃H₇ |
| Cl | H | 6-OCH₃ | t-C₄H₉ | H | i-C₃H₇ |
| Cl | H | 6-OCH₃ | CH₃ | CH₃ | i-C₃H₇ |
| Cl | H | 6-OCH₃ | C₂H₅ | CH₃ | i-C₃H₇ |
| Cl | H | 6-OCH₃ | C₃H₇ | CH₃ | i-C₃H₇ |
| Cl | H | 6-OCH₃ | i-C₃H₇ | CH₃ | i-C₃H₇ |
| Cl | H | 6-OCH₃ | C₄H₉ | CH₃ | i-C₃H₇ |
| Cl | H | 6-OCH₃ | i-C₄H₉ | CH₃ | i-C₃H₇ |
| Cl | H | 6-OCH₃ | s-C₄H₉ | CH₃ | i-C₃H₇ |
| Cl | H | 6-OCH₃ | t-C₄H₉ | CH₃ | i-C₃H₇ |
| Cl | H | 6-OCH₃ | C₂H₅ | C₂H₅ | i-C₃H₇ |
| Cl | H | 6-OCH₃ | C₃H₇ | C₃H₇ | i-C₃H₇ |
| Cl | H | 6-OCH₃ | 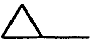 | CH₃ | i-C₃H₇ |
| Cl | H | 6-OCH₃ | 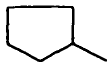 | CH₃ | i-C₃H₇ |
| Cl | H | 6-OCH₃ | 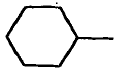 | CH₃ | i-C₃H₇ |
| OCH₃ | Cl | H | CH₃ | H | i-C₃H₇ |
| OCH₃ | Cl | H | C₂H₅ | H | i-C₃H₇ |
| OCH₃ | Cl | H | C₃H₇ | H | i-C₃H₇ |
| OCH₃ | Cl | H | i-C₃H₇ | H | i-C₃H₇ |
| OCH₃ | Cl | H | C₄H₉ | H | i-C₃H₇ |
| OCH₃ | Cl | H | i-C₄H₉ | H | i-C₃H₇ |
| OCH₃ | Cl | H | s-C₄H₉ | H | i-C₃H₇ |
| OCH₃ | Cl | H | t-C₄H₉ | H | i-C₃H₇ |
| OCH₃ | Cl | H | CH₃ | CH₃ | i-C₃H₇ |
| OCH₃ | Cl | H | C₂H₅ | CH₃ | i-C₃H₇ |
| OCH₃ | Cl | H | C₃H₇ | CH₃ | i-C₃H₇ |
| OCH₃ | Cl | H | i-C₃H₇ | CH₃ | i-C₃H₇ |
| OCH₃ | Cl | H | C₄H₉ | CH₃ | i-C₃H₇ |
| OCH₃ | Cl | H | i-C₄H₉ | CH₃ | i-C₃H₇ |
| OCH₃ | Cl | H | s-C₄H₉ | CH₃ | i-C₃H₇ |
| OCH₃ | Cl | H | t-C₄H₉ | CH₃ | i-C₃H₇ |
| OCH₃ | Cl | H | C₂H₅ | C₂H₅ | i-C₃H₇ |
| OCH₃ | Cl | H | C₃H₇ | C₃H₇ | i-C₃H₇ |
| OCH₃ | Cl | H | 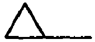 | CH₃ | i-C₃H₇ |
| OCH₃ | Cl | H | 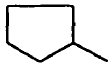 | CH₃ | i-C₃H₇ |
| OCH₃ | Cl | H | 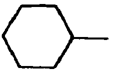 | CH₃ | i-C₃H₇ |
| Cl | OCH₃ | H | -(CH₂)₂- | | i-C₃H₇ |
| Cl | OCH₃ | H | -(CH₂)₄- | | i-C₃H₇ |
| Cl | OCH₃ | H | -(CH₂)₅- | | i-C₃H₇ |
| Cl | OCH₃ | H | -(CH₂)₆- | | i-C₃H₇ |
| Cl | OCH₃ | H | -(CH₂)₇- | | i-C₃H₇ |
| Cl | OCH₃ | H | -(CH₂)₂-O-(CH₂)₂- | | i-C₃H₇ |
| Cl | OCH₃ | H | -(CH₂)₂-S-(CH₂)₂- | | i-C₃H₇ |
| Cl | OCH₃ | H | -CH₂-CHCH₃-(CH₂)₃- | | i-C₃H₇ |
| Cl | OCH₃ | H | -(CH₂)₂-CHCH₃-(CH₂)₂- | | i-C₃H₇ |
| Cl | OCH₃ | H | -(CH₂)₂-CHC₂H₅-(CH₂)-₇- | | i-C₃H₇ |
| Cl | OCH₃ | H | -(CH₂)₂-CHC₃H₇-(CH₂)₂- | | i-C₃H₇ |
| Cl | OCH₃ | H | -(CH₂)₂-CHi-C₃H₇-(CH₂)₂- | | i-C₃H₇ |
| Cl | OCH₃ | H | -(CH₂)₂-CHOCH₃-(CH₂)₂- | | i-C₃H₇ |
| Cl | OCH₃ | H | -(CH₂)₂-CHOC₂H₅-(CH₂)₂- | | i-C₃H₇ |
| Cl | OCH₃ | H | -(CH₂)₂-CHOC₃H₇-(CH₂)₂- | | i-C₃H₇ |
| Cl | OCH₃ | H | -(CH₂)₂-CHi-OC₃H₇-(CH₂)₂- | | i-C₃H₇ |
| Cl | OCH₃ | H | -(CH₂)₂-C(CH₃)₂-(CH₂)₂- | | i-C₃H₇ |
| Cl | OCH₃ | H | -CH₂-(CHCH₃)₂-(CH₂)₂- | | i-C₃H₇ |
| Cl | OCH₃ | H | 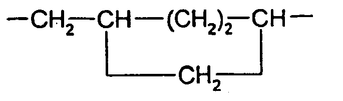 | | i-C₃H₇ |
| Cl | OCH₃ | H | 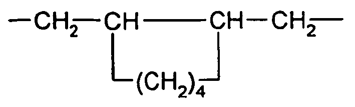 | | i-C₃H₇ |
| Cl | OCH₃ | H | 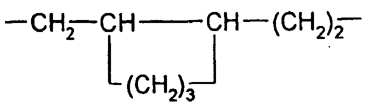 | | i-C₃H₇ |
| Cl | H | 6-OCH₃ | -(CH₂)₂- | | i-C₃H₇ |
| Cl | H | 6-OCH₃ | -(CH₂)₄- | | i-C₃H₇ |
| Cl | H | 6-OCH₃ | -(CH₂)₅- | | i-C₃H₇ |
| Cl | H | 6-OCH₃ | -(CH₂)₆- | | i-C₃H₇ |
| Cl | H | 6-OCH₃ | -(CH₂)₇- | | i-C₃H₇ |
| Cl | H | 6-OCH₃ | -(CH₂)₂-O-(CH₂)₂- | | i-C₃H₇ |
| Cl | H | 6-OCH₃ | -(CH₂)₂-S-(CH₂)₂- | | i-C₃H₇ |
| Cl | H | 6-OCH₃ | -CH₂-CHCH₃-(CH₂)₃- | | i-C₃H₇ |
| Cl | H | 6-OCH₃ | -(CH₂)₂-CHCH₃-(CH₂)₂- | | i-C₃H₇ |
| Cl | H | 6-OCH₃ | -(CH₂)₂-CHC₂H₅-(CH₂)₂- | | i-C₃H₇ |
| Cl | H | 6-OCH₃ | -(CH₂)₂-CHC₃H₇-(CH₂)₂- | | i-C₃H₇ |
| Cl | H | 6-OCH₃ | -(CH₂)₂-CHi-C₃H₇-(CH₂)₂- | | i-C₃H₇ |
| Cl | H | 6-OCH₃ | -(CH₂)₂-CHOCH₃-(CH₂)₂- | | i-C₃H₇ |
| Cl | H | 6-OCH₃ | -(CH₂)₂-CHOC₂H₅-(CH₂)₂- | | i-C₃H₇ |
| Cl | H | 6-OCH₃ | -(CH₂)₂-CHOC₃H₇-(CH₂)₂- | | i-C₃H₇ |
| Cl | H | 6-OCH₃ | -(CH₂)₂-CHi-OC₃H₇-(CH₂)₂- | | i-C₃H₇ |
| Cl | H | 6-OCH₃ | -(CH₂)₂-C(CH₃)₂-(CH₂)₂- | | i-C₃H₇ |
| Cl | H | 6-OCH₃ | -CH₂-(CHCH₃)₂-(CH₂)₂- | | i-C₃H₇ |
| Cl | H | 6-OCH₃ | 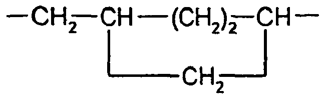 | | i-C₃H₇ |
| Cl | H | 6-OCH₃ | 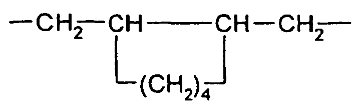 | | i-C₃H₇ |
| Cl | H | 6-OCH₃ | 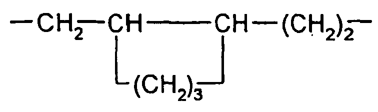 | | i-C₃H₇ |
| OCH₃ | Cl | H | -(CH₂)₂- | | i-C₃H₇ |
| OCH₃ | Cl | H | -(CH₂)₄- | | i-C₃H₇ |
| OCH₃ | Cl | H | -(CH₂)₅- | | i-C₃H₇ |
| OCH₃ | Cl | H | -(CH₂)₆- | | i-C₃H₇ |
| OCH₃ | Cl | H | -(CH₂)₇- | | i-C₃H₇ |
| OCH₃ | Cl | H | -(CH₂)₂-O-(CH₂)₂- | | i-C₃H₇ |
| OCH₃ | Cl | H | -(CH₂)₂-S-(CH₂)₂- | | i-C₃H₇ |
| OCH₃ | Cl | H | -CH₂-CHCH₃-(CH₂)₃- | | i-C₃H₇ |
| OCH₃ | Cl | H | -(CH₂)₂-CHCH₃-(CH₂)₂- | | i-C₃H₇ |
| OCH₃ | Cl | H | -(CH₂)₂-CHC₂H₅-(CH₂)₂- | | i-C₃H₇ |
| OCH₃ | Cl | H | -(CH₂)₂-CHC₃H₇-(CH₂)₂- | | i-C₃H₇ |
| OCH₃ | Cl | H | -(CH₂)₂-CHi-C₃H₇-(CH₂)₂- | | i-C₃H₇ |
| OCH₃ | Cl | H | -(CH₂)₂-CHOCH₃-(CH₂)₂- | | i-C₃H₇ |
| OCH₃ | Cl | H | -(CH₂)₂-CHOC₂H₅-(CH₂)₂- | | i-C₃H₇ |
| OCH₃ | Cl | H | -(CH₂)₂-CHOC₃H₇-(CH₂)₂- | | i-C₃H₇ |
| OCH₃ | Cl | H | -(CH₂)₂-CHi-OC₃H₇-(CH₂)₂- | | i-C₃H₇ |
| OCH₃ | Cl | H | -(CH₂)₂-C(CH₃)₂-(CH₂)₂- | | i-C₃H₇ |
| OCH₃ | Cl | H | -CH₂-(CHCH₃)₂-(CH₂)₂- | | i-C₃H₇ |
| OCH₃ | Cl | H | 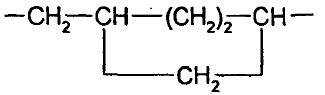 | | i-C₃H₇ |
| OCH₃ | Cl | H | 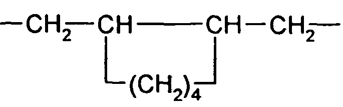 | | i-C₃H₇ |
| OCH₃ | Cl | H | 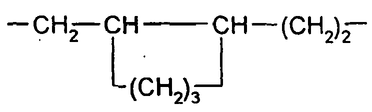 | | i-C₃H₇ |
| Cl | OCH₃ | H | CH₃ | H | t-C₄H₉ |
| Cl | OCH₃ | H | C₂H₅ | H | t-C₄H₉ |
| Cl | OCH₃ | H | C₃H₇ | H | t-C₄H₉ |
| Cl | OCH₃ | H | i-C₃H₇ | H | t-C₄H₉ |
| Cl | OCH₃ | H | C₄H₄ | H | t-C₄H₉ |
| Cl | OCH₃ | H | i-C₄H₉ | H | t-C₄H₉ |
| Cl | OCH₃ | H | s-C₄H₉ | H | t-C₄H₉ |
| Cl | OCH₃ | H | t-C₄H₉ | H | t-C₄H₉ |
| Cl | OCH₃ | H | CH₃ | CH₃ | t-C₄H₉ |
| Cl | OCH₃ | H | C₂H₅ | CH₃ | t-C₄H₉ |
| Cl | OCH₃ | H | C₃H₇ | CH₃ | t-C₄H₉ |
| Cl | OCH₃ | H | i-C₃H₇ | CH₃ | t-C₄H₉ |
| Cl | OCH₃ | H | C₄H₉ | CH₃ | t-C₄H₉ |
| Cl | OCH₃ | H | i-C₄H₉ | CH₃ | t-C₄H₉ |
| Cl | OCH₃ | H | s-C₄H₉ | CH₃ | t-C₄H₉ |
| Cl | OCH₃ | H | t-C₄H₉ | CH₃ | t-C₄H₉ |
| Cl | OCH₃ | H | C₂H₅ | C₂H₅ | t-C₄H₉ |
| Cl | OCH₃ | H | C₃H₇ | C₃H₇ | t-C₄H₉ |
| Cl | OCH₃ | H | 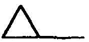 | CH₃ | t-C₄H₉ |
| Cl | OCH₃ | H | 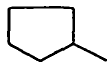 | CH₃ | t-C₄H₉ |
| Cl | OCH₃ | H | 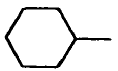 | CH₃ | t-C₄H₉ |
| Cl | H | 6-OCH₃ | CH₃ | H | t-C₄H₉ |
| Cl | H | 6-OCH₃ | C₂H₅ | H | t-C₄H₉ |
| Cl | H | 6-OCH₃ | C₃H₇ | H | t-C₄H₉ |
| Cl | H | 6-OCH₃ | i-C₃H₇ | H | t-C₄H₉ |
| Cl | H | 6-OCH₃ | C₄H₉ | H | t-C₄H₉ |
| Cl | H | 6-OCH₃ | i-C₄H₉ | H | t-C₄H₉ |
| Cl | H | 6-OCH₃ | s-C₄H₉ | H | t-C₄H₉ |
| Cl | H | 6-OCH₃ | t-C₄H₉ | H | t-C₄H₉ |
| Cl | H | 6-OCH₃ | CH₃ | CH₃ | t-C₄H₉ |
| Cl | H | 6-OCH₃ | C₂H₅ | CH₃ | t-C₄H₉ |
| Cl | H | 6-OCH₃ | C₃H₇ | CH₃ | t-C₄H₉ |
| Cl | H | 6-OCH₃ | i-C₃H₇ | CH₃ | t-C₄H₉ |
| Cl | H | 6-OCH₃ | C₄H₉ | CH₃ | t-C₄H₉ |
| Cl | H | 6-OCH₃ | i-C₄H₉ | CH₃ | t-C₄H₉ |
| Cl | H | 6-OCH₃ | s-C₄H₉ | CH₃ | t-C₄H₉ |
| Cl | H | 6-OCH₃ | t-C₄H₉ | CH₃ | t-C₄H₉ |
| Cl | H | 6-OCH₃ | C₂H₅ | C₂H₅ | t-C₄H₉ |
| Cl | H | 6-OCH₃ | C₃H₇ | C₃H₇ | t-C₄H₉ |
| Cl | H | 6-OCH₃ | 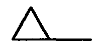 | CH₃ | t-C₄H₉ |
| Cl | H | 6-OCH₃ | 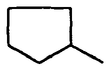 | CH₃ | t-C₄H₉ |
| Cl | H | 6-OCH₃ | 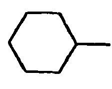 | CH₃ | t-C₄H₉ |
| OCH₃ | Cl | H | CH₃ | H | t-C₄H₉ |
| OCH₃ | Cl | H | C₂H₅ | H | t-C₄H₉ |
| OCH₃ | Cl | H | C₃H₇ | H | t-C₄H₉ |
| OCH₃ | Cl | H | i-C₃H₇ | H | t-C₄H₉ |
| OCH₃ | Cl | H | C₄H₉ | H | t-C₄H₉ |
| OCH₃ | Cl | H | i-C₄H₉ | H | t-C₄H₉ |
| OCH₃ | Cl | H | s-C₄H₉ | H | t-C₄H₉ |
| OCH₃ | Cl | H | t-C₄H₉ | H | t-C₄H₉ |
| OCH₃ | Cl | H | CH₃ | CH₃ | t-C₄H₉ |
| OCH₃ | Cl | H | C₂H₅ | CH₃ | t-C₄H₉ |
| OCH₃ | Cl | H | C₃H₇ | CH₃ | t-C₄H₉ |
| OCH₃ | Cl | H | i-C₃H₇ | CH₃ | t-C₄H₉ |
| OCH₃ | Cl | H | C₄H₉ | CH₃ | t-C₄H₉ |
| OCH₃ | Cl | H | i-C₄H₉ | CH₃ | t-C₄H₉ |
| OCH₃ | Cl | H | s-C₄H₉ | CH₃ | t-C₄H₉ |
| OCH₃ | Cl | H | t-C₄H₉ | CH₃ | t-C₄H₉ |
| OCH₃ | Cl | H | C₂H₅ | C₂H₅ | t-C₄H₉ |
| OCH₃ | Cl | H | C₃H₇ | C₃H₇ | t-C₄H₉ |
| OCH₃ | Cl | H | 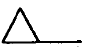 | CH₃ | t-C₄H₉ |
| OCH₃ | Cl | H | 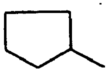 | CH₃ | t-C₄H₉ |
| OCH₃ | Cl | H | 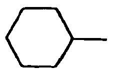 | CH₃ | t-C₄H₉ |
| Cl | OCH₃ | H | -(CH₂)₂- | | t-C₄H₄ |
| Cl | OCH₃ | H | -(CH₂)₄- | | t-C₄H₉ |
| Cl | OCH₃ | H | -(CH₂)₅- | | t-C₄H₄ |
| Cl | OCH₃ | H | -(CH₂)₆- | | t-C₄H₉ |
| Cl | OCH₃ | H | -(CH₂)₇- | | t-C₄H₉ |
| Cl | OCH₃ | H | -(CH₂)₂-O-(CH₂)₂- | | t-C₄H₉ |
| Cl | OCH₃ | H | -(CH₂)₂-S-(CH₂)₂- | | t-C₄H₉ |
| Cl | OCH₃ | H | -CH₂-CHCH₃-(CH₂)₃- | | t-C₄H₉ |
| Cl | OCH₃ | H | -(CH₂)₂-CHCH₃-(CH₂)-₂- | | t-C₄H₉ |
| Cl | OCH₃ | H | -(CH₂)₂-CHC₂H₅-(CH₂)₂- | | t-C₄H₉ |
| Cl | OCH₃ | H | -(CH₂)₂-CHC₃H₇-(CH₂)₂- | | t-C₄H₉ |
| Cl | OCH₃ | H | -(CH₂)₂-CHi-C₃H₇-(CH₂)₂- | | t-C₄H₄ |
| Cl | OCH₃ | H | -(CH₂)₂-CHOCH₃-(CH₂)₂- | | t-C₄H₉ |
| Cl | OCH₃ | H | -(CH₂)₂-CHOC₂H₅-(CH₂)₂- | | t-C₄H₉ |
| Cl | OCH₃ | H | -(CH₂)₂-CHOC₃H₇-(CH₂)₂- | | t-C₄H₉ |
| Cl | OCH₃ | H | -(CH₂)₂-CHi-OC₃H₇-(CH₂)₂- | | t-C₄H₉ |
| Cl | OCH₃ | H | -(CH₂)-C(CH₃)₂(CH₂)₂- | | t-C₄H₉ |
| Cl | OCH₃ | H | -CH₂-(CHCH)₂-(CH₂)₂- | | t-C₄H₉ |
| Cl | OCH₃ | H | 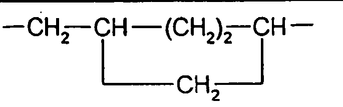 | | t-C₄H₉ |
| Cl | OCH₃ | H | 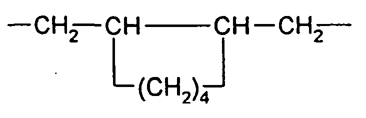 | | t-C₄H₉ |
| Cl | OCH₃ | H | 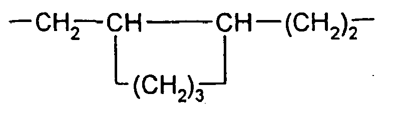 | | t-C₄H₉ |
| Cl | H | 6-OCH₃ | -(CH₂)₂- | | t-C₄H₉ |
| Cl | H | 6-OCH₃ | -(CH₂)₄- | | t-C₄H₉ |
| Cl | H | 6-OCH₃ | -(CH₂)₅- | | t-C₄H₉ |
| Cl | H | 6-OCH₃ | -(CH₂)₆- | | t-C₄H₉ |
| Cl | H | 6-OCH₃ | -(CH₂)₇- | | t-C₄H₉ |
| Cl | H | 6-OCH₃ | -(CH₂)₂-O-(CH₂)₂- | | t-C₄H₉ |
| Cl | H | 6-OCH₃ | -(CH₂)₂-S-(CH₂)₂- | | t-C₄H₉ |
| Cl | H | 6-OCH₃ | -CH₂-CHCH₃-(CH₂)₃- | | t-C₄H₉ |
| Cl | H | 6-OCH₃ | -(CH₂)₂-CHCH₃-(CH₂)₂- | | t-C₄H₉ |
| Cl | H | 6-OCH₃ | -(CH₂)₂-CHC₂H₅-(CH₂)₂- | | t-C₄H₉ |
| Cl | H | 6-OCH₃ | -(CH₂)₂-CHC₃H₇-(CH₂)₂- | | t-C₄H₉ |
| Cl | H | 6-OCH₃ | -(CH₂)₂-CHi-C₃H₇-(CH₂)₂- | | t-C₄H₉ |
| Cl | H | 6-OCH₃ | -(CH₂)₂-CHOCH₃-(CH₂)₂- | | t-C₄H₉ |
| Cl | H | 6-OCH₃ | -(CH₂)₂-CHOC₂H₅-(CH₂)₂- | | t-C₄H₉ |
| Cl | H | 6-OCH₃ | -(CH₂)₂-CHOC₃H₇-(CH₂)₂- | | t-C₄H₉ |
| Cl | H | 6-OCH₃ | -(CH₂)₂-CHi-OC₃H₇-(CH₂)₂- | | t-C₄H₉ |
| Cl | H | 6-OCH₃ | -(CH₂)₂-C(CH₃)₂-(CH₂)₂- | | t-C₄H₉ |
| Cl | H | 6-OCH₃ | -CH₂-(CHCH₃)₂-(CH₂)₂- | | t-C₄H₉ |
| Cl | H | 6-OCH₃ | 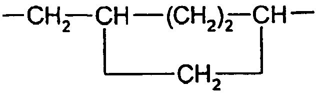 | | t-C₄H₉ |
| Cl | H | 6-OCH₃ | 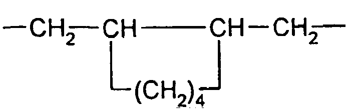 | | t-C₄H₉ |
| Cl | H | 6-OCH₃ | 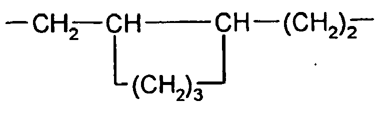 | | t-C₄H₉ |
| OCH₃ | Cl | H | -(CH₂)₂- | | t-C₄H₉ |
| OCH₃ | Cl | H | -(CH₂)₄- | | t-C₄H₉ |
| OCH₃ | Cl | H | -(CH₂)₅- | | t-C₄H₉ |
| OCH₃ | Cl | H | -(CH₂)₆- | | t-C₄H₉ |
| OCH₃ | Cl | H | -(CH₂)₇- | | t-C₄H₉ |
| OCH₃ | Cl | H | -(CH₂)₇-O-(CH₂)₂- | | t-C₄H₉ |
| OCH₃ | Cl | H | -(CH₂)₂-S-(CH₂)₂- | | t-C₄H₉ |
| OCH₃ | Cl | H | -CH₂-CHCH₃-(CH₂)₃- | | t-C₄H₉ |
| O-CH₃ | Cl | H | -(CH₂)₂-CHCH₃-(CH₂)₂- | | t-C₄H₉ |
| OCH₃ | Cl | H | -(CH₂)₂-CHC₂H₅-(CH₂)₂- | | t-C₄H₉ |
| OCH₃ | Cl | H | -(CH₂)₂-CHC₃H₇-(CH₂)₂- | | t-C₄H₉ |
| OCH₃ | Cl | H | -(CH₂)₂-CHi-C₃H₇-(CH₂)₂- | | t-C₄H₉ |
| OCH₃ | Cl | H | -(CH₂)₂-CHOCH₃-(CH₂)₂- | | t-C₄H₉ |
| OCH₃ | Cl | H | -(CH₂)₂CHOC₂H₅-(CH₂)₂- | | t-C₄H₉ |
| OCH₃ | Cl | H | -(CH₂)₂-CHOC₃H₇-(CH₂)₇- | | t-C₄H₉ |
| OCH₃ | Cl | H | -(CH₂)₂-CHi-OC₃H₇-(CH₂)₂- | | t-C₄H₉ |
| OCH₃ | Cl | H | -(CH₂)₂-C(CH₃)₂-(CH₂)₂- | | t-C₄H₉ |
| OCH₃ | Cl | H | -CH₂-(CHCH₃)₂-(CH₂)₂- | | t-C₄H₉ |
| OCH₃ | Cl | H | 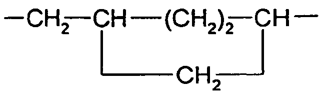 | | t-C₄H₉ |
| OCH₃ | Cl | H | 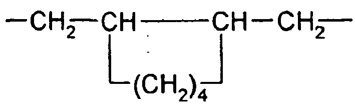 | | t-C₄H₉ |
| OCH₃ | Cl | H | 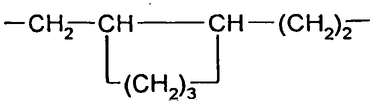 | | t-C₄H₉ |

Im einzelnen seien außer den bei den Herstellungsbeispielen genannten Verbindungen die folgenden Verbindungen der Formel (Ic) genannt:

**Tabelle 3:**

| | | | | | | | |
|---|---|---|---|---|---|---|---|
| 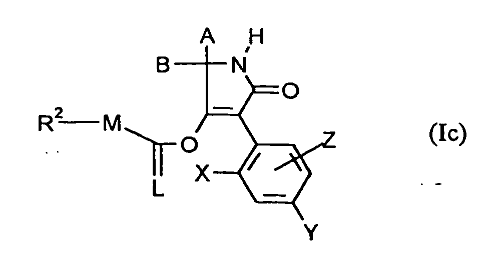 | | | | | | | |

| X | Y | Z | A | B | L | M | R² |
|---|---|---|---|---|---|---|---|
| Cl | OCH₃ | H | CH₃ | H | O | O | C₂H₅ |
| Cl | OCH₃ | H | C₂H₅ | H | O | O | C₂H₅ |
| Cl | OCH₃ | H | C₃H₇ | H | O | O | C₂H₅ |
| Cl | OCH₃ | H | i-C₃H₇ | H | O | O | C₂H₅ |
| Cl | OCH₃ | H | C₄H₉ | H | O | O | C₂H₅ |
| Cl | OCH₃ | H | i-C₄H₉ | H | O | O | C₂H₅ |
| Cl | OCH₃ | H | s-C₄H₉ | H | O | O | C₂H₅ |
| Cl | OCH₃ | H | t-C₄H₉ | H | O | O | C₂H₅ |
| Cl | OCH₃ | H | CH₃ | CH₃ | O | O | C₂H₅ |
| Cl | OCH₃ | H | C₂H₅ | CH₃ | O | O | C₂H₅ |
| Cl | OCH₃ | H | C₃H₇ | CH₃ | O | O | C₂H₅ |
| Cl | OCH₃ | H | i-C₃H₇ | CH₃ | O | O | C₂H₅ |
| Cl | OCH₃ | H | C₄H₉ | CH₃ | O | O | C₂H₅ |
| Cl | OCH₃ | H | i-C₄H₉ | CH₃ | O | O | C₂H₅ |
| Cl | OCH₃ | H | s-C₄H₉ | CH₃ | O | O | C₂H₅ |
| Cl | OCH₃ | H | t-C₄H₉ | CH₃ | O | O | C₂H₅ |
| Cl | OCH₃ | H | C₂H₅ | C₂H₅ | O | O | C₂H₅ |
| Cl | OCH₃ | H | C₃H₇ | C₃H₇ | O | O | C₂H₅ |
| Cl | OCH₃ | H | 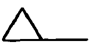 | CH₃ | O | O | C₂H₅ |
| Cl | OCH₃ | H | 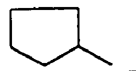 | CH₃ | O | O | C₂H₅ |
| Cl | OCH₃ | H | 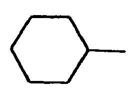 | CH₃ | O | O | C₂H₅ |
| Cl | H | 6-OCH₃ | CH₃ | H | O | O | C₂H₅ |
| Cl | H | 6-OCH₃ | C₂H₅ | H | O | O | C₂H₅ |
| Cl | H | 6-OCH₃ | C₃H₇ | H | O | O | C₂H₅ |
| Cl | H | 6-OCH₃ | i-C₃H₇ | H | O | O | C₂H₅ |
| Cl | H | 6-OCH₃ | C₄H₉ | H | O | O | C₂H₅ |
| Cl | H | 6-OCH₃ | i-C₄H₉ | H | O | O | C₂H₅ |
| Cl | H | 6-OCH₃ | s-C₄H₉ | H | O | O | C₂H₅ |
| Cl | H | 6-OCH₃ | t-C₄H₉ | H | O | O | C₂H₅ |
| Cl | H | 6-OCH₃ | CH₃ | CH₃ | O | O | C₂H₅ |
| Cl | H | 6-OCH₃ | C₂H₅ | CH₃ | O | O | C₂H₅ |
| Cl | H | 6-OCH₃ | C₃H₇ | CH₃ | O | O | C₂H₅ |
| Cl | H | 6-OCH₃ | i-C₃H₇ | CH₃ | O | O | C₂H₅ |
| Cl | H | 6-OCH₃ | C₄H₉ | CH₃ | O | O | C₂H₅ |
| Cl | H | 6-OCH₃ | i-C₄H₉ | CH₃ | O | O | C₂H₅ |
| Cl | H | 6-OCH₃ | s-C₄H₉ | CH₃ | O | O | C₂H₅ |
| Cl | H | 6-OCH₃ | t-C₄H₉ | CH₃ | O | O | C₂H₅ |
| Cl | H | 6-OCH₃ | C₂H₅ | C₂H₅ | O | O | C₂H₅ |
| Cl | H | 6-OCH₃ | C₃H₇ | C₃H₇ | O | O | C₂H₅ |
| Cl | H | 6-OCH₃ | 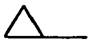 | CH₃ | O | O | C₂H₅ |
| Cl | H | 6-OCH₃ | 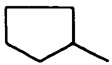 | CH₃ | O | O | C₂H₅ |
| Cl | H | 6-OCH₃ | 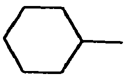 | CH₃ | O | O | C₂H₅ |
| OCH₃ | Cl | H | CH₃ | H | O | O | C₂H₅ |
| OCH₃ | Cl | H | C₂H₅ | H | O | O | C₂H₅ |
| OCH₃ | Cl | H | C₃H₇ | H | O | O | C₂H₅ |
| OCH₃ | Cl | H | i-C₃H₇ | H | O | O | C₂H₅ |
| OCH₃ | Cl | H | C₄H₉ | H | O | O | C₂H₅ |
| OCH₃ | Cl | H | i-C₄H₉ | H | O | O | C₂H₅ |
| OCH₃ | Cl | H | s-C₄H₉ | H | O | O | C₂H₅ |
| OCH₃ | Cl | H | t-C₄H₉ | H | O | O | C₂H₅ |
| OCH₃ | Cl | H | CH₃ | CH₃ | O | O | C₂H₅ |
| OCH₃ | Cl | H | C₂H₅ | CH₃ | O | O | C₂H₅ |
| OCH₃ | Cl | H | C₃H₇ | CH₃ | O | O | C₂H₅ |
| OCH₃ | Cl | H | i-C₃H₇ | CH₃ | O | O | C₂H₅ |
| OCH₃ | Cl | H | C₄H₉ | CH₃ | O | O | C₂H₅ |
| OCH₃ | Cl | H | i-C₄H₉ | CH₃ | O | O | C₂H₅ |
| OCH₃ | Cl | H | S-C₄H₉ | CH₃ | O | O | C₂H₅ |
| OCH₃ | Cl | H | t-C₄H₉ | CH₃ | O | O | C₂H₅ |
| OCH₃ | Cl | H | C₂H₅ | C₂H₅ | O | O | C₂H₅ |
| OCH₃ | Cl | H | C₃H₇ | C₃H₇ | O | O | C₂H₅ |
| OCH₃ | Cl | H | 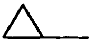 | CH₃ | O | O | C₂H₅ |
| OCH₃ | Cl | H | 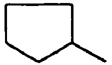 | CH₃ | O | O | C₂H₅ |
| OCH₃ | Cl | H | 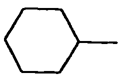 | CH₃ | O | O | C₂H₅ |
| Cl | OCH₃ | H | -(CH₂)₂- | | O | O | C₂H₅ |
| Cl | OCH₃ | H | -(CH₂)₄- | | O | O | C₂H₅ |
| Cl | OCH₃ | H | -(CH₂)₅- | | O | O | C₂H₅ |
| Cl | OCH₃ | H | -(CH₂)₆- | | O | O | C₂H₅ |
| Cl | OCH₃ | H | -(CH₂)₇- | | O | O | C₂H₅ |
| Cl | OCH₃ | H | -(CH₂)₂-O-(CH₂)₂- | | O | O | C₂H₅ |
| Cl | OCH₃ | H | -(CH₂)₂-S-(CH₂)₂- | | O | O | C₂H₅ |
| Cl | OCH₃ | H | -CH₂-CHCH₃-(CH₂)₃- | | O | O | C₂H₅ |
| Cl | OCH₃ | H | -(CH₂)₂-CHCH₃-(CH₂)₂- | | O | O | C₂H₅ |
| Cl | OCH₃ | H | -(CH₂)₂-CHC₂H₅-(CH₂)₂- | | O | O | C₂H₅ |
| Cl | OCH₃ | H | -(CH₂)₂-CHC₃H₇-(CH₇)₂- | | O | O | C₂H₅ |
| Cl | OCH₃ | H | -(CH₂)₂-CHi-C₃H₇-(CH₂)₂- | | O | O | C₂H₅ |
| Cl | OCH₃ | H | -(CH₂)₂-CHOCH₃-(CH₂)₂- | | O | O | C₂H₅ |
| Cl | OCH₃ | H | -(CH₂)₂-CHOC₂H₅-(CH₂)₂- | | O | O | C₂H₅ |
| Cl | OCH₃ | H | -(CH₂)₂-CHOC₃H₇-(CH₂)₂- | | O | O | C₂H₅ |
| Cl | OCH₃ | H | -(CH₂)₂-CHi-OC₃H₇-(CH₂)₂- | | O | O | C₂H₅ |
| Cl | OCH₃ | H | -(CH₂)₂-C(CH₃)₂-(CH₂)₂- | | O | O | C₂H₅ |
| Cl | OCH₃ | H | -CH₂-(CHCH₃)₂-(CH₂)₂- | | O | O | C₂H₅ |
| Cl | OCH₃ | H | 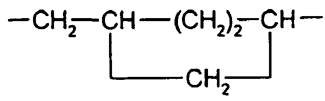 | | O | O | C₂H₅ |
| Cl | OCH₃ | H | 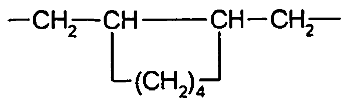 | | O | O | C₂H₅ |
| Cl | OCH₃ | H | 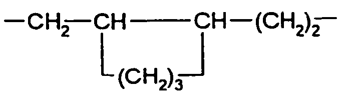 | | O | O | C₂H₅ |
| Cl | H | 6-OCH₃ | -(CH₂)₂- | | O | O | C₂H₅ |
| Cl | H | 6-OCH₃ | -(CH₂)₄- | | O | O | C₂H₅ |
| Cl | H | 6-OCH₃ | -(CH₂)₅- | | O | O | C₂H₅ |
| Cl | H | 6-OCH₃ | -(CH₂)₆- | | O | O | C₂H₅ |
| Cl | H | 6-OCH₃ | -(CH₂)₇- | | O | O | C₂H₅ |
| Cl | H | 6-OCH₃ | -(CH₂)₂-O-(CH₂)₂- | | O | O | C₂H₅ |
| Cl | H | 6-OCH₃ | -(CH₂)₂-S-(CH₂)₂- | | O | O | C₂H₅ |
| Cl | H | 6-OCH₃ | -CH₂-CHCH₃-(CH₂)₃- | | O | O | C₂H₅ |
| Cl | H | 6-OCH₃ | -(CH₂)₂-CHCH₃-(CH₂)₂- | | O | O | C₂H₅ |
| Cl | H | 6-OCH₃ | -(CH₂)₂-CHC₂H₅-(CH₂)₂- | | O | O | C₂H₅ |
| Cl | H | 6-OCH₃ | -(CH₂)₂-CHC₃H₇-(CH₂)₂- | | O | O | C₂H₅ |
| Cl | H | 6-OCH₃ | -(CH₂)₂-CHi-C₃H₇-(CH₂)₂- | | O | O | C₂H₅ |
| Cl | H | 6-OCH₃ | -(CH₂)₂-CHOCH₃-(CH₂)₂- | | O | O | C₂H₅ |
| Cl | H | 6-OCH₃ | -(CH₂)₂-CHOC₂H₅-(CH₂)₂- | | O | O | C₂H₅ |
| Cl | H | 6-OCH₃ | -(CH₂)₂-CHOC₃H₇-(CH₂)₂- | | O | O | C₂H₅ |
| Cl | H | 6-OCH₃ | -(CH₂)₂-CHi-OC₃H₇-(CH₂)₂- | | O | O | C₂H₅ |
| Cl | H | 6-OCH₃ | -(CH₂)₂-C(CH₃)₂-(CH₂)₂- | | O | O | C₂H₅ |
| Cl | H | 6-OCH₃ | -CH₂-(CHCH₃)₂-(CH₂)₂- | | O | O | C₂H₅ |
| Cl | H | 6-OCH₃ | 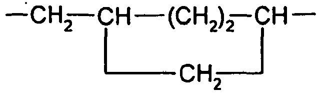 | | O | O | C₂H₅ |
| Cl | H | 6-OCH₃ | 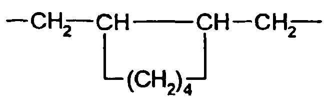 | | O | O | C₂H₅ |
| Cl | H | 6-OCH₃ | 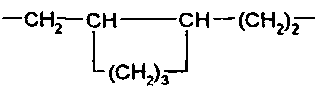 | | O | O | C₂H₅ |
| OCH₃ | Cl | H | -(CH₂)₂- | | O | O | C₂H₅ |
| OCH₃ | Cl | H | -(CH₂)₄- | | O | O | C₂H₅ |
| OCH₃ | Cl | H | -(CH₂)₅- | | O | O | C₂H₅ |
| OCH₃ | Cl | H | -(CH₂)₆- | | O | O | C₂H₅ |
| OCH₃ | Cl | H | -(CH₂)₇- | | O | O | C₂H₅ |
| OCH₃ | Cl | H | -(CH₂)₂-O-(CH₂)₂- | | O | O | C₂H₅ |
| OCH₃ | Cl | H | -(CH₂)₂-S-(CH₂)₂- | | O | O | C₂H₅ |
| OCH₃ | Cl | H | -CH₂-CHCH₃-(CH₂)₃- | | O | O | C₂H₉ |
| OCH₃ | Cl | H | -(CH₂)₂-CHCH₃-(CH₂)₂- | | O | O | C₂H₅ |
| OCH₃ | Cl | H | -(CH₂)₂-CHC₂H₅-(CH₇)₇- | | O | O | C₂H₅ |
| OCH₃ | Cl | H | -(CH₂)₂-CHC₃H₇-(CH₂)₂- | | O | O | C₂H₅ |
| OCH₃ | Cl | H | -(CH₂)₂-CHi-C₃H₇-(CH₂)₂- | | O | O | C₂H₅ |
| OCH₃ | Cl | H | -(CH₂)₂-CHOCH₃-(CH₂)₂- | | O | O | C₂H₅ |
| OCH₃ | Cl | H | -(CH₂)₂-CHOC₂H₅-(CH₂)₂- | | O | O | C₂H₅ |
| OCH₃ | Cl | H | -(CH₂)₂-CHOC₃H₇-(CH₂)₂- | | O | O | C₂H₅ |
| OCH₃ | Cl | H | -(CH₂)₂-CHi-OC₃H₇-(CH₂)- | | O | O | C₂H₅ |
| OCH₃ | Cl | H | -(CH₂)₂-C(CH₃)₂-(CH₂)₂- | | O | O | C₂H₅ |
| OCH₃ | Cl | H | -CH₂-(CHCH₃)₂-(CH₂)₂- | | O | O | C₂H₅ |
| OCH₃ | Cl | H | 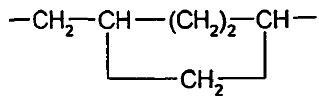 | | O | O | C₂H₅ |
| OCH₃ | Cl | H | 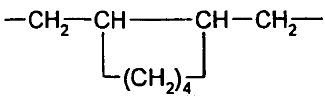 | | O | O | C₂H₅ |
| OCH₃ | Cl | H | 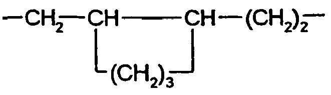 | | O | O | C₂H₅ |
| Cl | OCH₃ | H | CH₃ | H | O | O | i-C₃H₇ |
| Cl | OCH₃ | H | C₂H₅ | H | O | O | i-C₃H₇ |
| Cl | OCH₃ | H | C₃H₇ | H | O | O | -C₃H₇ |
| Cl | OCH₃ | H | i-C₃H₇ | H | O | O | i-C₃H₇ |
| Cl | OCH₃ | H | C₄H₉ | H | O | O | i-C₃H₇ |
| Cl | OCH₃ | H | i-C₄H₉ | H | O | O | i-C₃H₇ |
| Cl | OCH₃ | H | s-C₄H₉ | H | O | O | i-C₃H₇ |
| Cl | OCH₃ | H | t-C₄H₉ | H | O | O | i-C₃H₇ |
| Cl | OCH₃ | H | CH₃ | CH₃ | O | O | i-C₃H₇ |
| Cl | OCH₃ | H | C₂H₅ | CH₃ | O | O | i-C₃H₇ |
| Cl | OCH₃ | H | C₃H₇ | CH₃ | O | O | i-C₃H₇ |
| Cl | OCH₃ | H | i-C₃H₇ | CH₃ | O | O | i-C₃H₇ |
| Cl | OCH₃ | H | C₄H₉ | CH₃ | O | O | i-C₃H₇ |
| Cl | OCH₃ | H | i-C₄H₉ | CH₃ | O | O | i-C₃H₇ |
| Cl | OCH₃ | H | s-C₄H₉ | CH₃ | O | O | i-C₃H₇ |
| Cl | OCH₃ | H | t-C₄H₉ | CH₃ | O | O | -C₃H₇ |
| Cl | OCH₃ | H | C₂H₅ | C₂H₅ | O | O | i-C₃H₇ |
| Cl | OCH₃ | H | C₃H₇ | C₃H₇ | O | O | i-C₃H₇ |
| Cl | OCH₃ | H | 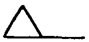 | CH₃ | O | O | i-C₃H₇ |
| Cl | OCH₃ | H | 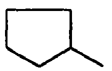 | CH₃ | O | O | i-C₃H₇ |
| Cl | OCH₃ | H | 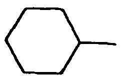 | CH₃ | O | O | i-C₃H₇ |
| Cl | H | 6-OCH₃ | CH₃ | H | O | O | i-C₃H₇ |
| Cl | H | 6-OCH₃ | C₂H₅ | H | O | O | i-C₃H₇ |
| Cl | H | 6-OCH₃ | C₃H₇ | H | O | O | i-C₃H₇ |
| Cl | H | 6-OCH₃ | i-C₃H₇ | H | O | O | i-C₃H₇ |
| Cl | H | 6-OCH₃ | C₄H₉ | H | O | O | i-C₃H₇ |
| Cl | H | 6-OCH₃ | i-C₄H₉ | H | O | O | i-C₃H₇ |
| Cl | H | 6-OCH₃ | s-C₄H₉ | H | O | O | i-C₃H₇ |
| Cl | H | 6-OCH₃ | t-C₄H₉ | H | O | O | -i-C₃H₇ |
| Cl | H | 6-OCH₃ | CH₃ | CH₃ | O | O | i-C₃H₇ |
| Cl | H | 6-OCH₃ | C₂H₅ | CH₃ | O | O | i-C₃H₇ |
| Cl | H | 6-OCH₃ | C₃H₇ | CH₃ | O | O | i-C₃H₇ |
| Cl | H | 6-OCH₃ | i-C₃H₇ | CH₃ | O | O | i-C₃H₇ |
| Cl | H | 6-OCH₃ | C₄H₉ | CH₃ | O | O | i-C₃H₇ |
| Cl | H | 6-OCH₃ | i-C₄H₉ | CH₃ | O | O | i-C₃H₇ |
| Cl | H | 6-OCH₃ | S-C₄H₉ | CH₃ | O | O | i-C₃H₇ |
| Cl | H | 6-OCH₃ | t-C₄H₉ | CH₃ | O | O | i-C₃H₇ |
| Cl | H | 6-OCH₃ | C₂H₅ | C₂H₅ | O | O | i-C₃H₇ |
| Cl | H | 6-OCH₃ | C₃H₇ | C₃H₇ | O | O | i-C₃H₇ |
| Cl | H | 6-OCH₃ | 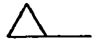 | CH₃ | O | O | i-C₃H₇ |
| Cl | H | 6-OCH₃ | 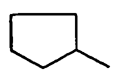 | CH₃ | O | O | i-C₃H₇ |
| Cl | H | 6-OCH₃ | 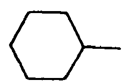 | CH₃ | O | O | i-C₃H₇ |
| OCH₃ | Cl | H | CH₃ | H | O | O | i-C₃H₇ |
| OCH₃ | Cl | H | C₂H₅ | H | O | O | i-C₃H₇ |
| OCH₃ | Cl | H | C₃H₇ | H | O | O | i-C₃H₇ |
| OCH₃ | Cl | H | i-C₃H₇ | H | O | O | i-C₃H₇ |
| OCH₃ | Cl | H | C₄H₉ | H | O | O | i-C₃H₇ |
| OCH₃ | Cl | H | i-C₄H₉ | H | O | O | i-C₃H₇ |
| OCH₃ | Cl | H | s-C₄H₉ | H | O | O | i-C₃H₇ |
| OCH₃ | Cl | H | t-C₄H₉ | H | O | O | i-C₃H₇ |
| OCH₃ | Cl | H | CH₃ | CH₃ | O | O | i-C₃H₇ |
| OCH₃ | Cl | H | C₂H₅ | CH₃ | O | O | i-C₃H₇ |
| OCH₃ | Cl | H | C₃H₇ | CH₃ | O | O | i-C₃H₇ |
| OCH₃ | Cl | H | i-C₃H₇ | CH₃ | O | O | i-C₃H₇ |
| OCH₃ | Cl | H | C₄H₉ | CH₃ | O | O | i-C₃H₇ |
| OCH₃ | Cl | H | i-C₄H₉ | CH₃ | O | O | i-C₃H₇ |
| OCH₃ | Cl | H | s-C₄H₉ | CH₃ | O | O | i-C₃H₇ |
| OCH₃ | Cl | H | t-C₄H₉ | CH₃ | O | O | i-C₃H₇ |
| OCH₃ | Cl | H | C₂H₅ | C₂H₅ | O | O | i-C₃H₇ |
| OCH₃ | Cl | H | C₃H₇ | C₃H₇ | O | O | i-C₃H₇ |
| OCH₃ | Cl | H | 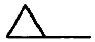 | CH₃ | O | O | i-C₃H₇ |
| OCH₃ | Cl | H | 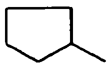 | CH₃ | O | O | i-C₃H₇ |
| OCH₃ | Cl | H | 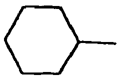 | CH₃ | O | O | i-C₃H₇ |
| Cl | OCH₃ | H | -(CH₂)₂- | | O | O | i-C₃H₇ |
| Cl | OCH₃ | H | -(CH₂)₄- | | O | O | i-C₃H₇ |
| Cl | OCH₃ | H | -(CH₂)₅- | | O | O | i-C₃H₇ |
| Cl | OCH₃ | H | -(CH₂)₆- | | O | O | i-C₃H₇ |
| Cl | OCH₃ | H | -(CH₂)₇- | | O | O | i-C₃H₇ |
| Cl | OCH₃ | H | -(CH₂)₂-O-(CH₂)₂- | | O | O | i-C₃H₇ |
| Cl | OCH₃ | H | -(CH₂)₂-S-(CH₂)₂- | | O | O | i-C₃H₇ |
| Cl | OCH₃ | H | -CH₂-CHCH₃-(CH₂)₃- | | O | O | i-C₃H₇ |
| Cl | OCH₃ | H | -(CH₂)₂-CHCH₃-(CH₂)₂- | | O | O | i-C₃H₇ |
| Cl | OCH₃ | H | -(CH₂)₂-CHC₂H₅-(CH₂)₂- | | O | O | i-C₃H₇ |
| Cl | OCH₃ | H | -(CH₂)₂-CHC₃H₇-(CH₂)₂- | | O | O | i-C₃H₇ |
| Cl | OCH₃ | H | -(CH₂)₂-CHi-C₃H₇-(CH₂)₂- | | O | O | i-C₃H₇ |
| Cl | OCH₃ | H | -(CH₂)₂-CHOCH₃-(CH₂)₂- | | O | O | i-C₃H₇ |
| Cl | OCH₃ | H | -(CH₂)₂-CHOC₂H₅-(CH₂)₂- | | O | O | i-C₃H₇ |
| Cl | OCH₃ | H | -(CH₂)₂-CHOC₃H₇-(CH₂)₂- | | O | O | i-C₃H₇ |
| Cl | OCH₃ | H | -(CH₂)₂-CHi-OC₃H₇-(CH₂)₂- | | O | O | i-C₃H₇ |
| Cl | OCH₃ | H | -(CH₂)₂-C(CH₃)₂-(CH₂)₂- | | O | O | i-C₃H₇ |
| Cl | OCH₃ | H | -CH₂-(CHCH₂)₂-(CH₂)₂- | | O | O | i-C₃H₇ |
| Cl | OCH₃ | H | 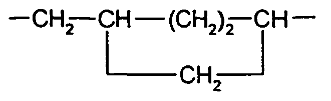 | | O | O | i-C₃H₇ |
| Cl | OCH₃ | H | 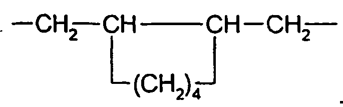 | | O | O | i-C₃H₇ |
| Cl | OCH₃ | H | 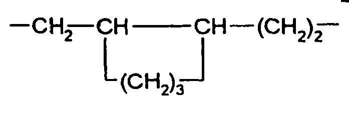 | | O | O | i-C₃H₇ |
| Cl | H | 6-OCH₃ | -(CH₂)₂- | | O | O | i-C₃H₇ |
| Cl | H | 6-OCH₃ | -(CH₂)₄- | | O | O | i-C₃H₇ |
| Cl | H | 6-OCH₃ | -(CH₂)₅- | | O | O | i-C₃H₇ |
| Cl | H | 6-OCH₃ | -(CH₂)₆- | | O | O | i-C₃H₇ |
| Cl | H | 6-OCH₃ | -(CH₂)₇- | | O | O | i-C₃H₇ |
| Cl | H | 6-OCH₃ | -(CH₂)₂-O-(CH₂)₂- | | O | O | i-C₃H₇ |
| Cl | H | 6-OCH₃ | -(CH₂)₂-S-(CH₂)₂- | | O | O | i-C₃H₇ |
| Cl | H | 6-OCH₃ | -CH₂-CHCH₃-(CH₂)₃- | | O | O | i-C₃H₇ |
| Cl | H | 6-OCH₃ | -(CH₂)₂-CHCH₃-(CH₂)₂- | | O | O | i-C₃H₇ |
| Cl | H | 6-OCH₃ | -(CH₂)₂-CHC₂H₅-(CH₂)₂- | | O | O | i-C₃H₇ |
| Cl | H | 6-OCH₃ | -(CH₂)₂-CHC₃H₇-(CH₂)₂- | | O | O | i-C₃H₇ |
| Cl | H | 6-OCH₃ | -(CH₂)₂-CHi-C₃H₇-(CH₂)₂- | | O | O | i-C₃H₇ |
| Cl | H | 6-OCH₃ | -(CH₂)₂-CHOCH₃-(CH₂)₂- | | O | O | i-C₃H₇ |
| Cl | H | 6-OCH₃ | -(CH₂)₂-CHOC₂H₅-(CH₂)₂- | | O | O | i-C₃H₇ |
| Cl | H | 6-OCH₃ | -(CH₂)₂-CHOC₃H₇-(CH₂)₂- | | O | O | i-C₃H₇ |
| Cl | H | 6-OCH₃ | -(CH₂)₂-CHi-OC₃H₇-(CH₂)₂- | | O | O | i-C₃H₇ |
| Cl | H | 6-OCH₃ | -(CH₂)₂-C(CH₃)₂-(CH₂)₂- | | O | O | i-C₃H₇ |
| Cl | H | 6-OCH₃ | -CH₂-(CHCH₃)₂-(CH₂)₂- | | O | O | i-C₃H₇ |
| Cl | H | 6-OCH₃ | 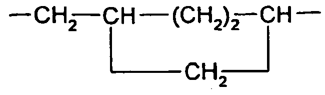 | | O | O | i-C₃H₇ |
| Cl | H | 6-OCH₃ | 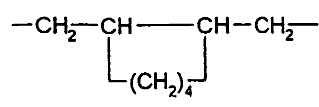 | | O | O | i-C₃H₇ |
| Cl | H | 6-OCH₃ | 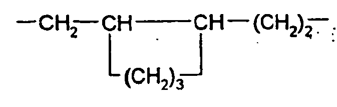 | | O | O | i-C₃H₇ |
| OCH₃ | Cl | H | -(CH₂)₂- | | O | O | i-C₃H₇ |
| OCH₃ | Cl | H | -(CH₂)₄- | | O | O | i-C₃H₇ |
| OCH₃ | Cl | H | -(CH₂)₅- | | O | O | i-C₃H₇ |
| OCH₃ | Cl | H | -(CH₂)₆- | | O | O | i-C₃H₇ |
| OCH₃ | Cl | H | -(CH₂)₇- | | O | O | i-C₃H₇ |
| OCH₃ | Cl | H | -(CH₂)₂-O-(CH₂)₂- | | O | O | i-C₃H₇ |
| OCH₃ | Cl | H | -(CH₂)₂-S-(CH₂)₂- | | O | O | i-C₃H₇ |
| OCH₃ | Cl | H | -CH₂-CHCH₃-(CH₂)₃- | | O | O | i-C₃H₇ |
| OCH₃ | Cl | H | -(CH₂)₂-CHCH₃-(CH₂)₂- | | O | O | i-C₃H₇ |
| OCH₃ | Cl | H | -(CH₂)₂-CHC₂H₅-(CH₂)₂- | | O | O | i-C₃H₇ |
| OCH₃ | Cl | H | -(CH₂)₂-CHC₃H₇-(CH₂)₂- | | O | O | i-C₃H₇ |
| OCH₃ | Cl | H | -(CH₂)₂-CHi-C₃H₇-(CH₂)₂- | | O | O | i-C₃H₇ |
| OCH₃ | Cl | H | -(CH₂)₂-CHOCH₃-(CH₂)₂- | | O | O | i-C₃H₇ |
| OCH₃ | Cl | H | -(CH₂)₂-CHOC₂H₅-(CH₂)₂- | | O | O | i-C₃H₇ |
| OCH₃ | Cl | H | -(CH₂)₂-CHOC₃H₇-(CH₂)₂- | | O | O | i-C₃H₇ |
| OCH₃ | Cl | H | -(CH₂)₇-CHi-OC₃H₇-(CH₂)₂- | | O | O | i-C₃H₇ |
| OCH₃ | Cl | H | -(CH₂)₂-C(CH₃)₂-(CH₂)₂- | | O | O | i-C₃H₇ |
| OCH₃ | Cl | H | -CH₂-(CHCH₃)₂-(CH₂)₂- | | O | O | i-C₃H₇ |
| OCH₃ | Cl | H | 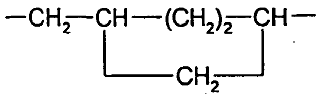 | | O | O | i-C₃H₇ |
| OCH₃ | Cl | H | 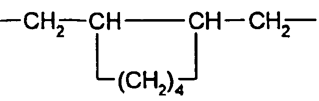 | | O | O | i-C₃H₇ |
| OCH₃ | Cl | H | 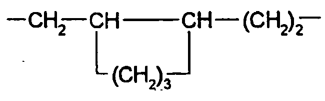 | | O | O | i-C₃H₇ |
| Cl | OCH₃ | H | CH₃ | H | O | S | i-C₃H₇ |
| Cl | OCH₃ | H | C₂H₅ | H | O | S | i-C₃H₇ |
| Cl | OCH₃ | H | C₃H₇ | H | O | S | i-C₃H₇ |
| Cl | OCH₃ | H | i-C₃H₇ | H | O | S | i-C₃H₇ |
| Cl | OCH₃ | H | C₄H₉ | H | O | S | i-C₃H₇ |
| Cl | OCH₃ | H | i-C₄H₉ | H | O | S | i-C₃H₇ |
| Cl | OCH₃ | H | S-C₄H₉ | H | O | S | i-C₃H₇ |
| Cl | OCH₃ | H | t-C₄H₉ | H | O | S | i-C₃H₇ |
| Cl | OCH₃ | H | CH₃ | CH₃ | O | S | i-C₃H₇ |
| Cl | OCH₃ | H | C₂H₅ | CH₃ | O | S | i-C₃H₇ |
| Cl | OCH₃ | H | C₃H₇ | CH₃ | O | S | i-C₃H₇ |
| Cl | OCH₃ | H | i-C₃H₇ | CH₃ | O | S | i-C₃H₇ |
| Cl | OCH₃ | H | C₄H₉ | CH₃ | O | S | i-C₃H₇ |
| Cl | OCH₃ | H | i-C₄H₉ | CH₃ | O | S | i-C₃H₇ |
| Cl | OCH₃ | H | s-C₄H₉ | CH₃ | O | S | i-C₃H₇ |
| Cl | OCH₃ | H | t-C₄H₉ | CH₃ | O | S | i-C₃H₇ |
| Cl | OCH₃ | H | C₂H₅ | C₂H₅ | O | S | i-C₃H₇ |
| Cl | OCH₃ | H | C₃H₇ | C₃H₇ | O | S | i-C₃H₇ |
| Cl | OCH₃ | H | 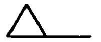 | CH₃ | O | S | i-C₃H₇ |
| Cl | OCH₃ | H | 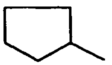 | CH₃ | O | S | i-C₃H₇ |
| Cl | OCH₃ | H | 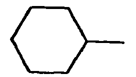 | CH₃ | O | S | i-C₃H₇ |
| Cl | H | 6-OCH₃ | CH₃ | H | O | S | i-C₃H₇ |
| Cl | H | 6-OCH₃ | C₂H₅ | H | O | S | i-C₃H₇ |
| Cl | H | 6-OCH₃ | C₃H₇ | H | O | S | i-C₃H₇ |
| Cl | H | 6-OCH₃ | i-C₃H₇ | H | O | S | i-C₃H₇ |
| Cl | H | 6-OCH₃ | C₄H₉ | H | O | S | i-C₃H₇ |
| Cl | H | 6-OCH₃ | i-C₄H₉ | H | O | S | i-C₃H₇ |
| Cl | H | 6-OCH₃ | s-C₄H₉ | H | O | S | i-C₃H₇ |
| Cl | H | 6-OCH₃ | t-C₄H₉ | H | O | S | i-C₃H₇ |
| Cl | H | 6-OCH₃ | CH₃ | CH₃ | O | S | i-C₃H₇ |
| Cl | H | 6-OCH₃ | C₂H₅ | CH₃ | O | S | i-C₃H₇ |
| Cl | H | 6-OCH₃ | C₃H₇ | CH₃ | O | S | i-C₃H₇ |
| Cl | H | 6-OCH₃ | i-C₃H₇ | CH₃ | O | S | i-C₃H₇ |
| Cl | H | 6-OCH₃ | C₄H₉ | CH₃ | O | S | i-C₃H₇ |
| Cl | H | 6-OCH₃ | i-C₄H₉ | CH₃ | O | S | i-C₃H₇ |
| Cl | H | 6-OCH₃ | s-C₄H₉ | CH₃ | O | S | i-C₃H₇ |
| Cl | H | 6-OCH₃ | t-C₄H₉ | CH₂ | O | S | i-C₃H₇ |
| Cl | H | 6-OCH₃ | C₂H₅ | C₂H₅ | O | S | i-C₃H₇ |
| Cl | H | 6-OCH₃ | C₃H₇ | C₃H₇ | O | S | i-C₃H₇ |
| Cl | H | 6-OCH₃ | 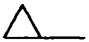 | CH₃ | O | S | i-C₃H₇ |
| Cl | H | 6-OCH₃ | 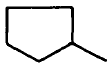 | CH₃ | O | S | i-C₃H₇ |
| Cl | H | 6-OCH₃ | 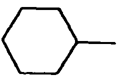 | CH₃ | O | S | i-C₃H₇ |
| OCH₃ | Cl | H | CH₃ | H | O | S | i-C₃H₇ |
| OCH₃ | Cl | H | C₂H₅ | H | O | S | i-C₃H₇ |
| OCH₃ | Cl | H | C₃H₇ | H | O | S | i-C₃H₇ |
| OCH₃ | Cl | H | i-C₃H₇ | H | O | S | i-C₃H₇ |
| OCH₃ | Cl | H | C₄H₉ | H | O | S | i-C₃H₇ |
| OCH₃ | Cl | H | i-C₄H₉ | H | O | S | i-C₃H₇ |
| OCH₃ | Cl | H | s-C₄H₉ | H | O | S | i-C₃H₇ |
| OCH₃ | Cl | H | t-C₄H₉ | H | O | S | i-C₃H₇ |
| OCH₃ | Cl | H | CH₃ | CH₃ | O | S | i-C₃H₇ |
| OCH₃ | Cl | H | C₂H₅ | CH₃ | O | S | i-C₃H₇ |
| OCH₃ | Cl | H | C₃H₇ | CH₃ | O | S | i-C₃H₇ |
| OCH₃ | Cl | H | i-C₃H₇ | CH₃ | O | S | i-C₃H₇ |
| OCH₃ | Cl | H | C₄H₉ | CH₃ | O | S | i-C₃H₇ |
| OCH₃ | Cl | H | i-C₄H₉ | CH₃ | O | S | i-C₃H₇ |
| OCH₃ | Cl | H | s-C₄H₉ | CH₃ | O | S | i-C₃H₇ |
| OCH₃ | Cl | H | t-C₄H₉ | CH₃ | O | S | i-C₃H₇ |
| OCH₃ | Cl | H | C₂H₅ | C₂H₅ | O | S | i-C₃H₇ |
| OCH₃ | Cl | H | C₃H₇ | C₃H₇ | O | S | i-C₃H₇ |
| OCH₃ | Cl | H | 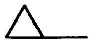 | CH₃ | O | S | i-C₃H₇ |
| OCH₃ | Cl | H | 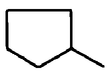 | CH₃ | O | S | i-C₃H₇ |
| OCH₃ | Cl | H | 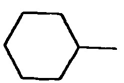 | CH₃ | O | S | i-C₃H₇ |
| Cl | OCH₃ | H | -(CH₂)₂- | | O | S | i-C₃H₇ |
| Cl | OCH₃ | H | -(CH₂)₄- | | O | S | i-C₃H₇ |
| Cl | OCH₃ | H | -(CH₂)₅- | | O | S | i-C₃H₇ |
| Cl | OCH₃ | H | -(CH₂)₆- | | O | S | i-C₃H₇ |
| Cl | OCH₃ | H | -(CH₂)₇- | | O | S | i-C₃H₇ |
| Cl | OCH₃ | H | -(CH₂)₂-O-(CH₂)₂- | | O | S | i-C₃H₇ |
| Cl | OCH₃ | H | -(CH₂)₂-S-(CH₂)₂- | | O | S | i-C₃H₇ |
| Cl | OCH₃ | H | -CH₂-CHCH₃-(CH₂)₃- | | O | S | i-C₃H₇ |
| Cl | OCH₃ | H | -(CH₂)₂-CHCH₃-(CH₂)₂- | | O | S | i-C₃H₇ |
| Cl | OCH₃ | H | -(CH₂)₂-CHC₂H₅-(CH₂)₂- | | O | S | i-C₃H₇ |
| Cl | OCH₃ | H | -(CH₂)₂-CHC₃H₇-(CH₂)₂- | | O | S | i-C₃H₇ |
| Cl | OCH₃ | H | -(CH₂)₂-CHi-C₃H₇-(CH₂)₂- | | O | S | i-C₃H₇ |
| Cl | OCH₃ | H | -(CH₂)₂-CHOCH₃-(CH₂)₂ | | O | S | i-C₃H₇ |
| Cl | OCH₃ | H | -(CH₂)₂-CHOC₂H₅-(CH₂)₂- | | O | S | i-C₃H₇ |
| Cl | OCH₃ | H | -(CH₂)₂-CHOC₃H₇-(CH₂)₂- | | O | S | i-C₃H₇ |
| Cl | OCH₃ | H | -(CH₂)₂-CHi-OC₃H₇-(CH₂)₂- | | O | S | i-C₃H₇ |
| Cl | OCH₃ | H | -(CH₂)₂-C(CH₃)₂-(CH₂)₂- | | O | S | i-C₃H₇ |
| Cl | OCH₃ | H | -CH₂-(CHCH₃)₂-(CH₂)₂- | | O | S | i-C₃H₇ |
| Cl | OCH₃ | H | 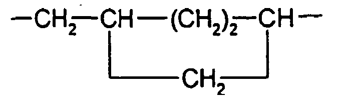 | | O | S | i-C₃H₇ |
| Cl | OCH₃ | H | 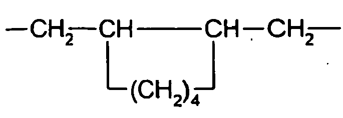 | | O | S | i-C₃H₇ |
| Cl | OCH₃ | H | 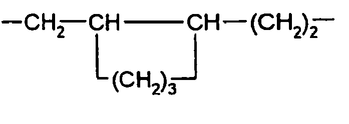 | | O | S | i-C₃H₇ |
| Cl | H | 6-OCH₃ | -(CH₂)₂- | | O | S | i-C₃H₇ |
| Cl | H | 6-OCH₃ | -(CH₂)₄- | | O | S | i-C₃H₇ |
| Cl | H | 6-OCH₃ | -(CH₂)₅- | | O | S | i-C₃H₇ |
| Cl | H | 6-OCH₋₃ | -(CH₂)₆- | | O | S | i-C₃H₇ |
| Cl | H | 6-OCH-₃ | -(CH₂)₇- | | O | S | i-C₃H₇ |
| Cl | H | 6-OCH₃ | -(CH₂)₂-O-(CH₂)₂- | | O | S | i-C₃H₇ |
| Cl | H | 6-OCH₃ | -(CH₂)₂-S-(CH₂)₂- | | O | S | i-C₃H₇ |
| Cl | H | 6-OCH₃ | -CH₂-CHCH₃-(CH₂)₃- | | O | S | i-C₃H₇ |
| Cl | H | 6-OCH₃ | -(CH₂)₂-CHCH₃-(CH₂)₂- | | O | S | i-C₃H₇ |
| Cl | H | 6-OCH₃ | -(CH₂)₂-CHC₂H₅-(CH₂)₂- | | O | S | i-C₃H₇ |
| Cl | H | 6-OCH₃ | -(CH₂)₂-CHC₃H₇-(CH₂)₂- | | O | S | i-C₃H₇ |
| Cl | H | 6-OCH₃ | -(CH₂)₂-CHi-C₃H₇-(CH₂)₂- | | O | S | i-C₃H₇ |
| Cl | H | 6-OCH₃ | -(CH₂)₂-CHOCH₃-(CH₂)₂- | | O | S | i-C₃H₇ |
| Cl | H | 6-OCH₃ | -(CH₂)₂-CHOC₂H₅-(CH₂)₂- | | O | S | i-C₃H₇ |
| Cl | H | 6-OCH₃ | -(CH₂)₂-CHOC₃H₇-(CH₂)₂- | | O | S | i-C₃H₇ |
| Cl | H | 6-OCH₃ | -(CH₂)₂-CHi-OC₃H₇-(CH₂)₂- | | O | S | i-C₃H₇ |
| Cl | H | 6-OCH₃ | -(CH₂)₂-C(CH₃)₂-(CH₂)₂- | | O | S | i-C₃H₇ |
| Cl | H | 6-OCH₃ | -CH₂-(CHCH₃)₂-(CH₂)₂- | | O | S | i-C₃H₇ |
| Cl | H | 6-OCH₃ | 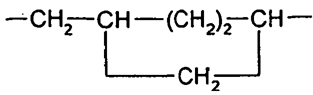 | | O | S | i-C₃H₇ |
| Cl | H | 6-OCH₃ | 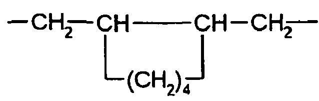 | | O | S | i-C₃H₇ |
| Cl | H | 6-OCH₃ | 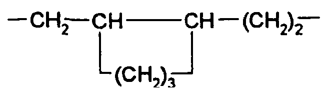 | | O | S | i-C₃H₇ |
| OCH₃ | Cl | H | -(CH₂)₂- | | O | S | i-C₃H₇ |
| OCH₃ | Cl | H | -(CH₂)₄- | | O | S | i-C₃H₇ |
| OCH₃ | Cl | H | -(CH₂)₅- | | O | S | i-C₃H₇ |
| OCH₃ | Cl | H | -(CH₂)₆- | | O | S | i-C₃H₇ |
| OCH₃ | Cl | H | -(CH₂)₇- | | O | S | i-C₃H₇ |
| OCH₃ | Cl | H | -(CH₂)₂-O-(CH₂)₂- | | O | S | i-C₃H₇ |
| OCH₃ | Cl | H | -(CH₂)₂-S-(CH₂)₂- | | O | S | i-C₃H₇ |
| OCH₃ | Cl | H | -CH₂-CHCH₃-(CH₂)₃- | | O | S | i-C₃H₇ |
| OCH₃ | Cl | H | -(CH₂)₂-CHCH₃-(CH₂)₂- | | O | S | i-C₃H₇ |
| OCH₃ | Cl | H | -(CH₂)₂-CHC₂H₅-(CH₂)₂- | | O | S | i-C₃H₇ |
| OCH₃ | Cl | H | -(CH₂)₂-CHC₃H₇-(CH₂)₂- | | O | S | i-C₃H₇ |
| OCH₃ | Cl | H | -(CH₂)₂-CHi-C₃H₇-(CH₂)₂- | | O | S | i-C₃H₇ |
| OCH₃ | Cl | H | -(CH₂)₂-CHOCH₃-(CH₂)₂- | | O | S | i-C₃H₇ |
| OCH₃ | Cl | H | -(CH₂)₂-CHOC₂H₅-(CH₂)₂- | | O | S | i-C₃H₇ |
| OCH₃ | Cl | H | -(CH₂)₂-CHOC₃H₇-(CH₂)₂- | | O | S | i-C₃H₇ |
| OCH₃ | Cl | H | -(CH₂)₂-CHi-OC₃H₇-(CH₂)₂- | | O | S | i-C₃H₇ |
| OCH₃ | Cl | H | -(CH₂)₂-C(CH₃)₂-(CH₂)₂- | | O | S | i-C₃H₇ |
| OCH₃ | Cl | H | -CH₂-(CHCH₃)₂-(CH₂)₂- | | O | S | i-C₃H₇ |
| OCH₃ | Cl | H | 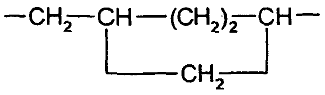 | | O | S | i-C₃H₇ |
| OCH₃ | Cl | H | 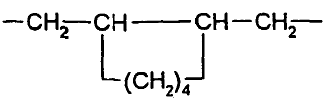 | | O | S | i-C₃H₇ |
| OCH₃ | Cl | H | 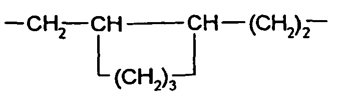 | | O | S | i-C₃H₇ |
| Cl | OCH₃ | H | CH₃ | H | O | O | s-C₄H₉ |
| Cl | OCH₃ | H | C₂H₅ | H | O | O | s-C₄H₉ |
| Cl | OCH₃ | H | C₃H₇ | H | O | O | s-C₄H₉ |
| Cl | OCH₃ | H | i-C₃H₇ | H | O | O | s-C₄H₉ |
| Cl | OCH₃ | H | C₄H₉ | H | O | O | s-C₄H₉ |
| Cl | OCH₃ | H | i-C₄H₉ | H | O | O | s-C₄H₉ |
| Cl | OCH₃ | H | s-C₄H₉ | H | O | O | s-C₄H₉ |
| Cl | OCH₃ | H | t-C₄H₉ | H | O | O | s-C₄H₉ |
| Cl | OCH₃ | H | CH₃ | CH₃ | O | O | s-C₄H₉ |
| Cl | OCH₃ | H | C₂H₅ | CH₃ | O | O | s-C₄H₉ |
| Cl | OCH₃ | H | C₃H₇ | CH₃ | O | O | sC₄H₉ |
| Cl | OCH₃ | H | i-C₃H₇ | CH₃ | O | O | sC₄H₉ |
| Cl | OCH₃ | H | C₄H₉ | CH₃ | O | O | s-C₄H₉ |
| Cl | OCH₃ | H | i-C₄H₉ | CH₃ | O | O | s-C₄H₉ |
| Cl | OCH₃ | H | s-C₄H₉ | CH₃ | O | O | sC₄H₉ |
| Cl | OCH₃ | H | t-C₄H₉ | CH₃ | O | O | sC₄H₉ |
| Cl | OCH₃ | H | C₂H₅ | C₂H₅ | O | O | sC₄H₉ |
| Cl | OCH₃ | H | C₃H₇ | C₃H₇ | O | O | s-C₄H₉ |
| Cl | OCH₃ | H | 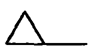 | CH₃ | O | O | s-C₄H₉ |
| Cl | OCH₃ | H | 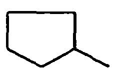 | CH₃ | O | O | s-C₄H₉ |
| Cl | OCH₃ | H | 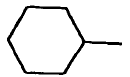 | CH₃ | O | O | s-C₄H₉ |
| Cl | H | 6-OCH₃ | CH₃ | H | O | O | s-C₄H₉ |
| Cl | H | 6-OCH₃ | C₂H₅ | H | O | O | s-C₄H₉ |
| Cl | H | 6-OCH₃ | C₃H₇ | H | O | O | s-C₄H₉ |
| Cl | H | 6-OCH₃ | i-C₃H₇ | H | O | O | s-C₄H₉ |
| Cl | H | 6-OCH₃ | C₄H₉ | H | O | O | s-C₄H₉ |
| Cl | H | 6-OCH₃ | i-C₄H₉ | H | O | O | s-C₄H₉ |
| Cl | H | 6-OCH₃ | s-C₄H₉ | H | O | O | s-C₄H₉ |
| Cl | H | 6-OCH₃ | t-C₄H₉- | H | O | O | s-C₄H₉ |
| Cl | H | 6-OCH₃ | CH₃ | CH₃ | O | O | s-C₄H₉ |
| Cl | H | 6-OCH₃ | C₂H₅ | CH₃ | O | O | s-C₄H₉ |
| Cl | H | 6-OH₃ | C₃H₇ | CH₃ | O | O | s-C₄H₉ |
| Cl | H | 6-OCH₃ | i-C₃H₇ | CH₃ | O | O | s-C₄H₉ |
| Cl | H | 6-OCH₃ | C₄H₉ | CH₃ | O | O | s-C₄H₉ |
| Cl | H | 6-OCH₃ | i-C₄H₉ | CH₃ | O | O | s-C₄H₉ |
| Cl | H | 6-OCH₃ | s-C₄H₉ | CH₃ | O | O | s-C₄H₉ |
| Cl | H | 6-OCH₃ | t-C₄H₉ | CH₃ | O | O | s-C₄H₉ |
| Cl | H | 6-OCH₃ | C₂H₅ | C₂H₅ | O | O | s-C₄H₉ |
| Cl | H | 6-OCH₃ | C₃H₇ | C₃H₇ | O | O | s-C₄H₉ |
| Cl | H | 6-OCH₃ | 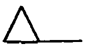 | CH₃ | O | O | s-C₄H₉ |
| Cl | H | 6-OCH₃ | 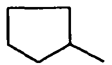 | CH₃ | O | O | s-C₄H₉ |
| Cl | H | 6-OCH₃ | 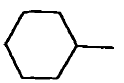 | CH₃ | O | O | s-C₄H₉ |
| OCH₃ | Cl | H | CH₃ | H | O | O | s-C₄H₉ |
| OCH₃ | Cl | H | C₂H₅ | H | O | O | s-C₄H₉ |
| OCH₃ | Cl | H | C₃H₇ | H | O | O | s-C₄H₉ |
| OCH₃ | Cl | H | i-C₃H₇ | H | O | O | s-C₄H₉ |
| OCH₃ | Cl | H | C₄H₉ | H | O | O | s-C₄H₉ |
| OCH₃ | Cl | H | i-C₄H₉ | H | O | O | s-C₄H₉ |
| OCH₃ | Cl | H | s-C₄H₉ | H | O | 0 | s-C₄H₉ |
| OCH₃ | Cl | H | t-C₄H₉ | H | O | O | s-C₄H₉ |
| OCH₃ | Cl | H | CH₃ | CH₃ | O | 0 | s-C₄H₉ |
| OCH₃ | Cl | H | C₂Hµ₅ | CH₃ | O | O | s-C₄H₉ |
| OCH₃ | Cl | H | C₃H₇ | CH₃ | O | O | s-C₄H₉ |
| OCH₃ | Cl | H | i-C₃H₇ | CH₃ | O | O | ₛ-C₄H₉ |
| OCH₃ | Cl | H | C₄H₉ | CH₃ | O | O | s-C₄H₉ |
| OCH₃ | Cl | H | i-C₄H₉ | CH₃ | O | O | s-C₄H₉ |
| OCH₃ | Cl | H | s-C₄H₉ | CH₃. | O | O | s-C₄H₉ |
| OCH₃ | Cl | H | t-C₄H₉ | CH₃ | O | O | s-C₄H₉ |
| OCH₃ | Cl | H | C₂H₅ | C₂H₅ | O | O | s-C₄H₉ |
| OCH₃ | Cl | H | C₃H₇ | C₃H₇ | O | O | s-C₄H₉ |
| OCH₃ | Cl | H | 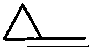 | CH₃ | O | O | s-C₄H₉ |
| OCH₃ | Cl | H | 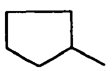 | CH₃ | O | O | s-C₄H₉ |
| OCH₃ | Cl | H | 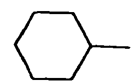 | CH₃ | O | O | s-C₄H₉ |
| Cl | OCH₃ | H | -(CH₂)₂- | | O | O | s-C₄H₉ |
| Cl | OCH₃ | H | -(CH₂)₄- | | O | O | s-C₄H₉ |
| Cl | OCH₃ | H | -(CH₂)₅- | | O | O | s-C₄H₉ |
| Cl | OCH₃ | H | -(CH₂)₆- | | O | O | s-C₄H₉ |
| Cl | OCH₃ | H | -(CH₂)₇- | | O | O | s-C₄H₉ |
| Cl | OCH₃ | H | -(CH₂)₂-O-(CH₂)₂- | | O | O | s-C₄H₉ |
| Cl | OCH₃ | H | -(CH₂)₂-S-(CH₂)₂- | | O | O | s-C₄H₉ |
| Cl | OCH₃ | H | -CH₂-CHCH₃-(CH₂)₃- | | O | O | s-C₄H₉ |
| Cl | OCH₃ | H | -(CH₂)₂-CHCH₃-(CH₂)₂- | | O | O | s-C₄H₉ |
| Cl | OCH₃ | H | -(CH₂)₂-CHC₂H₅-(CH₂)₂- | | O | O | s-C₄H₉ |
| Cl | OCH₃ | H | -(CH₂)₂-CHC₃H₇-(CH₂)₂- | | O | O | s-C₄H₉ |
| Cl | OCH₃ | H | -(CH₂)₂-CHi-C₃H₇-(CH₂)₂- | | O | O | s-C₄H₉ |
| Cl | OCH₃ | H | -(CH₂)₂-CHOCH₃-(CH₂)₂- | | O | O | s-C₄H₉ |
| Cl | OCH₃ | H | -(CH₂)₂-CHOC₂H₅-(CH₂)₂- | | O | O | s-C₄H₉ |
| Cl | OCH₃ | H | -(CH₂)₂-CHOC₃H₇-(CH₂)₂- | | O | 0 | s-C₄H₉ |
| Cl | OCH₃ | H | -(CH₂)₂-CHi-OC₃H₇-(CH₂)₂- | | O | O | s-C₄H₉ |
| Cl | OCH₃ | H | -(CH₂)₂-C(CH₃)₂-(CH₂)₂- | | O | O | s-C₄H₉ |
| Cl | OCH₃ | H | -CH₂-(CHCH₃)₂-(CH₂)₂- | | O | O | s-C₄H₉ |
| Cl | OCH₃ | H | 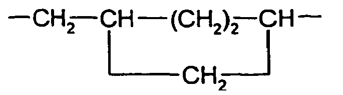 | | O | O | s-C₄H₉ |
| Cl | OCH₃ | H | 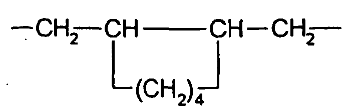 | | O | O | s-C₄H₉ |
| Cl | OCH₃ | H | 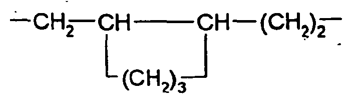 | | O | O | s-C₄H₉ |
| Cl | H | 6-OCH₃ | -(CH₂)₂- | | O | O | s-C₄H₉ |
| Cl | H | 6-OCH₃ | -(CH₂)₄- | | O | O | s-C₄H₉ |
| Cl | H | 6-OCH₃ | -(CH₂)₅- | | O | O | s-C₄H₉ |
| Cl | H | 6-OCH₃ | -(CH₂)₆- | | O | O | s-C₄H₉ |
| Cl | H | 6-OCH₃ | -(CH₂)₇- | | O | O | s-C₄H₉ |
| Cl | H | 6-OCH₃ | -(CH₂)₂-O-(CH₂)₂)- | | O | O | s-C₄H₉ |
| Cl | H | 6-OCH₃ | -(CH₂)₂-S-(CH₂)₂- | | O | O | s-C₄H₉ |
| Cl | H | 6-OCH₃ | -CH₂-CHCH₃-(CH₂)₃- | | O | O | s-C₄H₉ |
| Cl | H | 6-OCH₃ | -(CH₂)₂CHCH₃-(CH₂)₂- | | O | O | s-C₄H₉ |
| Cl | H | 6-OCH₃ | -(CH₂)₂-CHC₂H₅-(CH₂)₂- | | O | O | s-C₄H₉ |
| Cl | H | 6-OCH₃ | -(CH₂)₂-CHC₃H₇-(CH₂)₂- | | O | O | s-C₄H₉ |
| Cl | H | 6-OCH₃ | -(CH₂)₂-CHi-C₃H₇-(CH₂)₂- | | O | O | s-C₄H₉ |
| Cl | H | 6-OCH₃ | -(CH₂)₂-CHOCH₃-(CH₂)₂- | | O | O | s-C₄H₉ |
| Cl | H | 6-OCH₃ | -(CH₂)₂-CHOC₂H₅-(CH₂)₂- | | O | O | s-C₄H₉ |
| Cl | H | 6-OCH₃ | -(CH₂)₂-CHOC₃H₇-(CH₂)₂- | | O | O | s-C₄H₉ |
| Cl | H | 6-OCH₃ | -(CH₂)₂-CHi-OC₃H₇-(CH₂)₂- | | O | O | s-C₄H₉ |
| Cl | H | 6-OCH₃ | -(CH₂)₂-C(CH₃)₂-(CH₂)₂- | | O | O | s-C₄H₉ |
| Cl | H | 6-OCH₃ | -CH₂-(CHCH₃)₂-(CH₂)₂- | | O | O | s-C₄H₉ |
| Cl | H | 6-OCH₃ | 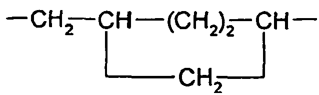 | | O | O | s-C₄H₉ |
| Cl | H | 6-OCH₃ | 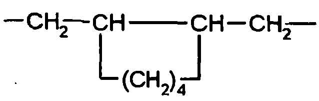 | | O | O | s-C₄H₉ |
| Cl | H | 6-OCH₃ | 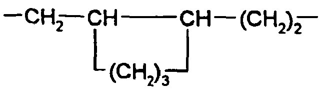 | | O | O | s-C₄H₉ |
| OCH₃ | Cl | H | -(CH₂)₂- | | O | O | s-C₄H₉ |
| OCH₃ | Cl | H | -(CH₂)₄- | | O | O | s-C₄H₉ |
| OCH₃ | Cl | H | -(CH₂)₅- | | O | O | s-C₄H₉ |
| OCH₃ | Cl | H | -(CH₂)₆- | | O | O | s-C₄H₉ |
| OCH₃ | Cl | H | -(CH₂)₇- | | O | O | s-C₄H₉ |
| OCH₃ | Cl | H | -(CH₂)₂-O-(CH₂)₂- | | O | O | s-C₄H₉ |
| OCH₃ | Cl | H | -(CH₂)₂-S-(CH₂)₂- | | O | O | s-C₄H₉ |
| OCH₃ | Cl | H | -CH₂-CHCH₃-(CH₂)₃- | | O | O | s-C₄H₉ |
| OCH₃ | Cl | H | -(CH₂)₂-CHCH₃-(CH₂)₂- | | O | O | s-C₄H₉ |
| OCH₃ | Cl | H | -(CH₂)₂-CHC₂H₅-(CH₂)₂- | | O | O | s-C₄H₉ |
| OCH₃ | Cl | H | -(CH₂)₂-CHC₃H₇-(CH₂)₂- | | O | O | s-C₄H₉ |
| OCH₃ | Cl | H | -(CH₂)₂-CHi-C₃H₇-(CH₂)₂- | | O | O | s-C₄H₉ |
| OCH₃ | Cl | H | -(CH₂)₂-CHOCH₃-(CH₂)₂- | | O | O | s-C₄H₉ |
| OCH₃ | Cl | H | -(CH₂)₂-CHOC₂CH₅-(CH₂)₂- | | O | O | s-C₄H₉ |
| OCH₃ | Cl | H | -(CH₂)₂-CHOC₃H₇-(CH₂)₂- | | O | O | s-C₄H₉ |
| OCH-₄ | Cl | H | -(CH₂)₂-CHi-OC₃H₇-(CH₂)₂- | | O | O | s-C₄H₉ |
| OCH₃ | Cl | H | -(CH₂)₂-C(CH₃)₂-(CH₂)₂- | | O | O | s-C4H₉ |
| OCH₃ | Cl | H | -CH₂-(CHCH₃)₂-(CH₂)₂- | | O | O | s-C₄H₉ |
| OCH₃ | Cl | H | 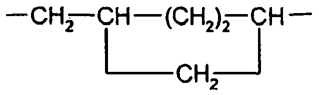 | | O | O | s-C₄H₉ |
| OCH₃ | Cl | H | 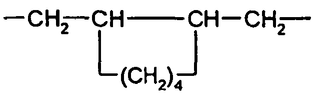 | | O | O | s-C₄H₉ |
| OCH₃ | Cl | H | 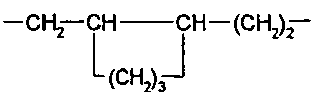 | | O | O | s-C₄H₉ |

Im einzelnen seien außer den bei den Herstellungsbeispielen genannten Verbindungen die folgenden Verbindungen der Formel (Id) genannt:

**Tabelle 4:**

| | | | | | |
|---|---|---|---|---|---|
| 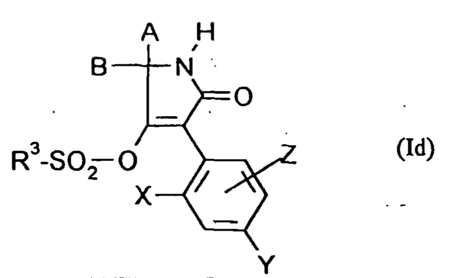 | | | | | |

| | Y | Z | A | B | R³ |
|---|---|---|---|---|---|
| Cl | OCH₃ | H | CH₃ | H | CH₃ |
| Cl | OCH₃ | H | C₂H₅ | H | CH₃ |
| Cl | OCH₃ | H | C₃H₇ | H | CH₃ |
| Cl | OCH₃ | H | i-C₃H₇ | H | CH₃ |
| Cl | OCH₃ | H | C₄H₉ | H | CH₃ |
| Cl | OCH₃ | H | i-C₄H₉ | H | CH₃ |
| Cl | OCH₃ | H | s-C₄H₉ | H | CH₃ |
| Cl | OCH₃ | H | t-C₄H₉ | H | CH₃ |
| Cl | OCH₃ | H | CH₃ | CH₃ | CH₃ |
| Cl | OCH₃ | H | C₂H₅ | CH₃ | CH₃ |
| Cl | OCH₃ | H | C₃H₇ | CH₃ | CH₃ |
| Cl | OCH₃ | H | i-C₃H₇ | CH₃ | CH₃ |
| Cl | OCH₃ | H | C₄H₉ | CH₃ | CH₃ |
| Cl | OCH₃ | H | i-C₄H₉ | CH₃ | CH₃ |
| Cl | OCH₃ | H | s-C₄H₉ | CH₃ | CH₃ |
| Cl | OCH₃ | H | t-C₄H₉ | CH₃ | CH₃ |
| Cl | OCH₃ | H | C₂H₅ | C₂H₅ | CH₃ |
| Cl | OCH₃ | H | C₃H₇ | C₃H₇ | CH₃ |
| Cl | OCH₃ | H | 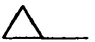 | CH₃ | CH₃ |
| Cl | OCH₃ | H | 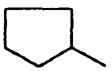 | CH₃ | CH₃ |
| Cl | OCH₃ | H | 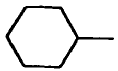 | CH₃ | CH₃ |
| Cl | H | 6-OCH₃ | CH₃ | H | CH₃ |
| Cl | H | 6-OCH₃ | C₂H₅ | H | CH₃ |
| Cl | H | 6-OCH | C₃H₇ | H | CH₃ |
| Cl | H | 6-OCH₃ | i-C₃H₇ | H | CH₃ |
| Cl | H | 6-OCH₃ | C₄H₉ | H | CH₃ |
| Cl | H | 6-OCH₃ | i-C₄H₉ | H | CH₃ |
| Cl | H | 6-OCH₃ | s-C₄H₉ | H | CH₃ |
| Cl | H | 6-OCH₃ | t-C₄H₉ | H | CH₃ |
| Cl | H | 6-OCH₃ | CH₃ | CH₃ | CH₃ |
| Cl | H | 6-OCH₃ | C₂H₅ | CH₃ | CH₃ |
| Cl | H | 6-OCH₃ | C₃H₇ | CH₃ | CH₃ |
| Cl | H | 6-OCH₃ | i-C₃H₇ | CH₃ | CH₃ |
| Cl | H | 6-OCH₃ | C₄H₉ | CH₃ | CH₃ |
| Cl | H | 6-OCH₃ | i-C₄H₉ | CH₃ | CH₃ |
| Cl | H | 6-OCH₃ | s-C₄H₉ | CH₃ | CH₃ |
| Cl | H | 6-OCH₃ | t-C₄H₉ | CH₃ | CH₃ |
| Cl | H | 6-OCH₃ | C₂H₅ | C₂H₅ | CH₃ |
| Cl | H | 6-OCH₃ | C₃H₇ | C₃H₇ | CH₃ |
| Cl | H | 6-OCH₃ | 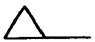 | CH₃ | CH₃ |
| Cl | H | 6-OCH₃ | 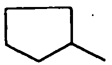 | CH₃ | CH₃ |
| Cl | H | 6-OCH₃ | 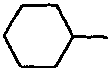 | CH₃ | CH₃ |
| OCH₃ | Cl | H | CH₃ | H | CH₃ |
| OCH₃ | Cl | H | C₂H₅ | H | CH₃ |
| OCH₃ | Cl | H | C₃H₇ | H | CH₃ |
| OCH₃ | Cl | H | i-C₃H₇ | H | CH₃ |
| OCH₃ | Cl | H | C₄H₉ | H | CH₃ |
| OCH₃ | Cl | H | i-C₄H₉ | H | CH₃ |
| OCH₃ | Cl | H | s-C₄H₉ | H | CH₃ |
| OCH₃ | Cl | H | t-C₄H₉ | H | CH₃ |
| OCH₃ | Cl | H | CH₃ | CH₃ | CH₃ |
| OCH₃ | Cl | H | C₂H₅ | CH₃ | CH₃ |
| OCH₃ | Cl | H | C₃H₇ | CH₃ | CH₃ |
| OCH₃ | Cl | H | i-C₃H₇ | CH₃ | CH₃ |
| OCH₃ | Cl | H | C₄H₉ | CH₃ | CH₃ |
| OCH₃ | Cl | H | i-C₄H₉ | CH₃ | CH₃ |
| OCH₃ | Cl | H | s-C₄H₉ | CH₃ | CH₃ |
| OCH₃ | Cl | H | t-C₄H₉ | CH₃ | CH₃ |
| .OCH₃ | Cl | H | C₂H₅ | C₂H₅ | CH₃ |
| OCH₃- | Cl | H | C₃H₇ | C₃H₇ | CH₃ |
| OCH₃ | Cl | H | 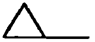 | CH₃ | CH₃ |
| OCH₃ | Cl | H | 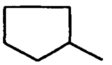 | CH₃ | CH₃ |
| OCH₃ | Cl | H | 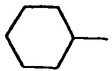 | CH₃ | CH₃ |
| Cl | OCH₃ | H | -(CH₂)₂- | | CH₃ |
| Cl | OCH₃ | H | -(CH₂)₄- | | CH₃ |
| Cl | OCH₃ | H | -(CH₂)₅- | | CH₃ |
| Cl | OCH₃ | H | -(CH₂)₆- | | CH₃ |
| Cl | OCH₃ | H | -(CH₂)₇- | | CH₃ |
| Cl | OCH₃ | H | -(CH₂)₂-O-(CH₂)₂- | | CH₃ |
| Cl | OCH₃ | H | -(CH₂)₂-S-(CH₂)₂- | | CH₃ |
| Cl | OCH₃ | H | -CH₂-CHCH₃-(CH₂)₃- | | CH₃ |
| Cl | OCH₃ | H | -(CH₂)₂-CHCH₃-(CH₂)₂- | | CH₃ |
| Cl | OCH₃ | H | -(CH₂)₂-CHC₂H₅-(CH₂)₂- | | CH₃ |
| Cl | OCH₃ | H | -(CH₂)₂-CHC₃H₇-(CH₂)₂- | | CH₃ |
| Cl | OCH₃ | H | -(CH₂)₂-CHi-C₃H₇-(CH₂)₂- | | CH₃ |
| Cl | OCH₃ | H | -(CH₂)₂-CHOCH₃-(CH₂)₂- | | CH₃ |
| Cl | OCH₃ | H | -(CH₂)₂-CHOC₂H₅-(CH₂)₂- | | CH₃ |
| Cl | OCH₃ | H | -(CH₂)₂-CHOC₃H₇-(CH₂)₂- | | CH₃ |
| Cl | OCH₃ | H | -(CH₂)₂-CHi-OC₃H₇-(CH₂)₂- | | CH₃ |
| Cl | OCH₃ | H | -(CH₂)₂-C(CH₃)₂-(CH-₂)₂- | | CH₃ |
| Cl | OCH₃ | H | -CH₂-(CHCH₃)₂-(CH₂)₂- | | CH₃ |
| Cl | OCH₃ | H | 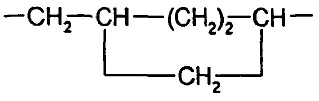 | | CH₃ |
| Cl | OCH₃ | H | 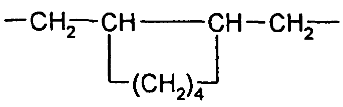 | | CH₃ |
| Cl | OCH₃ | H | 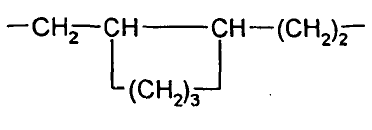 | | CH₃ |
| Cl | H | 6-OCH | -(CH₂)₂- | | CH₃ |
| Cl | H | 6-OCH₃ | -(CH₂)₄- | | CH₃ |
| Cl | H | 6-OCH₃ | -(CH₂)₅- | | CH₃ |
| Cl | H | 6-OCH₃ | -(CH₂)₆- | | CH₃ |
| Cl | H | 6-OCH₃ | -(CH₂)₇- | | CH₃ |
| Cl | H | 6-OCH₃ | -(CH₂)₂-O-(CH₇)₂- | | CH₃ |
| Cl | H | 6-OCH₃ | -(CH₂)₂-S-(CH₂)₂- | | CH₃ |
| Cl | H | 6-OCH₃ | -CH₂-CHCH₃-(CH₂)₃- | | CH₃ |
| Cl | H | 6-OCH₃ | -(CH₂)₂-CHCH₃-(CH₂)₂- | | CH₃ |
| Cl | H | 6-OCH₃ | -(CH₂)₂-CHC₂H₅-(CH₂)₂- | | CH₃ |
| Cl | H | 6-OCH₃ | -(CH₂)₂-CHC₃H₇-(CH₂)₂- | | CH₃ |
| Cl | H | 6-OCH₃ | -(CH₂)₂-CHi-C₃H₇-(CH₂)₂- | | CH₃ |
| Cl | H | 6-OCH₃ | -(CH₂)₂-CHOCH₃-(CH₂)₂- | | CH₃ |
| Cl | H | 6-OCH₃ | -(CH₂)₂-CHOC₂H₅-(CH₂)₂- | | CH₃ |
| Cl | H | 6-OCH₃ | -(CH₂)₂-CHOC₃H₇-(CH₂)₂- | | CH₃ |
| Cl | H | 6-OCH₃ | -(CH₂)₂-CHi-OC₃H₇-(CH₂)₂- | | CH₃ |
| Cl | H | 6-OCH₃ | -(CH₂)₂-C(CH₃)₂-(CH₂)₂- | | CH₃ |
| Cl | H | 6-OCH₃ | -CH₂-(CHCH₃)₂-(CH₂)₂- | | CH₃ |
| Cl | H | 6-OCH₃ | 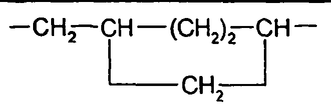 | | CH₃ |
| Cl | H | 6-OCH₃ | 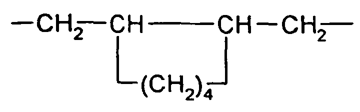 | | CH₃ |
| Cl | H | 6-OCH₃ | 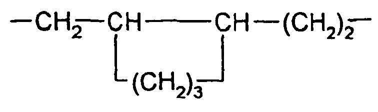 | | CH₃ |
| OCH₃ | Cl | H | -(CH₂)₂- | | CH₃ |
| OCH₃ | Cl | H | -(CH₂)₄- | | CH₃ |
| OCH₃ | Cl | H | -(CH₂)₅- | | CH₃ |
| OCH₃ | Cl | H | -(CH₂)₆- | | CH₃ |
| OCH₃ | Cl | H | -(CH₂)₇- | | CH₃ |
| OCH₃ | Cl | H | -(CH₂)₂-O-(CH₂)₂- | | CH₃ |
| OCH₃ | Cl | H | -(CH₂)₂-S-(CH₂)₂- | | CH₃ |
| OCH₃ | Cl | H | -CH₂-CHCH₃-(CH₂)₃- | | CH₃ |
| OCH₃ | Cl | H | -(CH₂)₂-CHCH₃-(CH₂)₂- | | CH₃ |
| OCH₃ | Cl | H | -(CH₂)₂-CHC₂H₅-(CH₂)₂- | | CH₃ |
| OCH₃ | Cl | H | -(CH₂)₂-CHC₃H₇-(CH₂)₂- | | CH₃ |
| OCH₃ | Cl | H | -(CH₂)₂-CHi-C₃H₇-(CH₂)₂- | | CH₃ |
| OCH₃ | Cl | H | -(CH₂)₂-CHOCH₃-(CH₂)₂- | | CH₃ |
| OCH₃ | Cl | H | -(CH₂)₂-CHOC₂H₅-(CH₂)₂- | | CH₃ |
| OCH₃ | Cl | H | -(CH₂)₂-CHOC₃H₇-(CH₂)₂- | | CH₃ |
| OCH₃ | Cl | H | -(CH₂)₂-CHi-OC₃H₇-(CH₂)₂- | | CH₃ |
| OCH₃ | Cl | H | -(CH₂)₂-C(CH₃)₂-(CH₂)₂- | | CH₃ |
| OCH₃ | Cl | H | -CH₂-(CHCH₃)₂-(CH₂)- | | CH₃ |
| OCH₃ | Cl | H | 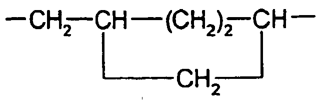 | | CH₃ |
| OCH₃ | Cl | H | 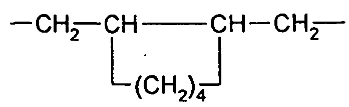 | | CH₃ |
| OCH₃ | Cl | H | 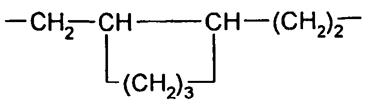 | | CH₃ |

Im einzelnen seien außer den bei den Herstellungsbeispielen genannten Verbindungen die folgenden Verbindungen der Formel (Ie) genannt:

**Tabelle 5:**

| | | | | | | | |
|---|---|---|---|---|---|---|---|
| 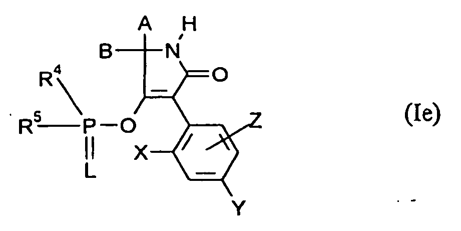 | | | | | | | |

| X | Y | Z | A | B | L | R⁴ | R⁵ |
|---|---|---|---|---|---|---|---|
| Cl | OCH₃ | H | CH₃ | H | S | CH₃ | i-C₃H₇-S- |
| Cl | OCH₃ | H | C₂H₅ | H | S | CH₃ | i-C₃H₇-S- |
| Cl | OCH₃ | H | C₃H₇ | H | S | CH₃ | i-C₃H₇-S- |
| Cl | OCH₃ | H | i-C₃H₇ | H | S | CH₃ | i-C₃H₇-S- |
| Cl | OCH₃ | H | C₄H₉ | H | S | CH₃ | i-C₃H₇-S- |
| Cl | OCH₃ | H | i-C₄H₉ | H | S | CH₃ | i-C₃H₇-S- |
| Cl | OCH₃ | H | s-C₄H₉ | H | S | CH₃ | i-C-₃H₇-S- |
| Cl | OCH₃ | H | t-C₄H₉ | H | S | CH₃ | i-C₃H₇-S- |
| Cl | OCH₃ | H | CH₃ | CH₃ | S | CH₃ | i-C₃H₇-S- |
| Cl | OCH₃ | H | C₂H₅ | CH₃ | S | CH₃ | i-C₃H₇-S- |
| Cl | OCH₃ | H | C₃H₇ | CH₃ | S | CH₃ | i-C₃H₇-S- |
| Cl | OCH₃ | H | i-C₃H₇ | CH₃ | S | CH₃ | i-C₃H₇-S- |
| Cl | OCH₃ | H | C₄H₉ | CH₃ | S | CH₃ | i-C₃H₇-S- |
| Cl | OCH₃ | H | i-C₄H₉ | CH₃ | S | CH₃ | i-C₃H₇-S- |
| Cl | OCH₃ | H | s-C₄H₉ | CH₃ | S | CH₃ | i-C₃H₇-S- |
| Cl | OCH₃ | H | t-C₄H₉ | CH₃ | S | CH₃ | i-C₃H₇-S- |
| Cl | OCH₃ | H | C₂H₅ | C₂H₅ | S | CH₃ | i-C₃H₇-S- |
| Cl | OCH₃ | H | C₃H₇ | C₃H₇ | S | CH₃ | i-C₃H₇-S- |
| Cl | OCH₃ | H | 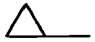 | CH₃ | S | CH₃ | i-C₃H₇-S- |
| Cl | OCH₃ | H | 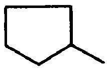 | CH₃ | S | CH₃ | i-C₃H₇-S- |
| Cl | OCH₃ | H | 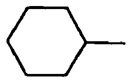 | CH₃ | S | CH₃ | i-C₃H₇-S- |
| Cl | H | 6-OCH₃ | CH₃ | H | S | CH₃ | i-C₃H₇-S- |
| Cl | H | 6-OCH₃ | C₂H₅ | H | S | CH₃ | i-C₃H₇-S- |
| Cl | H | 6-OCH₃ | C₃H₇ | H | S | CH₃ | i-C₃H₇-S- |
| Cl | H | 6-OCH₃ | i-C₃H₇ | H | S | CH₃ | i-C₃H₇-S- |
| Cl | H | 6-OCH₃ | C₄H₉ | H | S | CH₃ | i-C₃H₇-S- |
| Cl | H | 6-OCH₃ | i-C₄H₉ | H | S | CH₃ | i-C₃H₇-S- |
| Cl | H | 6-OCH₃ | s-C₄H₉ | H | S | CH₃ | i-C₃H₇-S- |
| Cl | H | 6-OCH₃ | t-C₄H₉ | H | S | CH₃ | i-C₃H₇-S- |
| Cl | H | 6-OCH₃ | CH₃ | CH₃ | S | CH₃ | i-C₃H₇-S- |
| Cl | H | 6-OCH₃ | C₂H₅ | CH₃ | S | CH₃ | i-C₃H₇-S- |
| Cl | H | 6-OCH₃ | C₃H₇ | CH₃ | S | CH₃ | i-C₃H₇-S- |
| Cl | H | 6-OCH₃ | i-C₃H₇ | CH₃ | S | CH₃ | i-C₃H₇-S- |
| Cl | H | 6-OCH₃ | C₄H₉ | CH₃ | S | CH₃ | i-C₃H₇-S- |
| Cl | H | 6-OCH₃ | i-C₄H₉ | CH₃ | S | CH₃ | i-C₃H₇-S- |
| Cl | H | 6-OCH₃ | s-C₄H₉ | CH₃ | S | CH₃ | i-C₃H₇-S- |
| Cl | H | 6-OCH₃ | t-C₄H₉ | CH₃ | S | CH₃ | i-C₃H₇-S- |
| Cl | H | 6-OCH₃ | C₂H₅ | C₂H₅ | S | CH₃ | i-C₃H₇-S- |
| Cl | H | 6-OCH₃ | C₃H₇ | C₃H₇ | S | CH₃ | i-C₃H₇-S- |
| Cl | H | 6-OCH₃ | 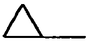 | CH₃ | S | CH₃ | i-C₃H₇-S- |
| Cl | H | 6-OCH₃ | 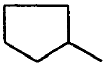 | CH₃ | S | CH₃ | i-C₃H₇-S- |
| Cl | H | 6-OCH₃ | 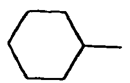 | CH₃ | S | CH₃ | i-C₃H₇-S- |
| OCH₃ | Cl | H | CH₃ | H | S | CH₃ | i-C₃H₇-S- |
| OCH₃ | Cl | H | C₂H₅ | H | S | CH₃ | i-C₃H₇-S- |
| OCH₃ | Cl | H | C₃H₇ | H | S | CH₃ | i-C₃H₇-S- |
| OCH₃ | Cl | H | i-C₃H₇ | H | S | CH₃ | i-C₃H₇-S- |
| OCH₃ | Cl | H | C₄H₉ | H | S | CH₃ | i-C₃H₇-S- |
| OCH₃ | Cl | H | i-C₄H₉ | H | S | CH₃ | i-C₃H₇S- |
| OCH₃ | Cl | H | s-C₄H₉ | H | S | CH₃ | i-C₃H₇-S- |
| OCH₃ | Cl | H | t-C₄H₉ | H | S | CH₃ | i-C₃H₇-S- |
| OCH₃ | Cl | H | CH₃ | CH₃ | S | CH₃ | i-C₃H₇-S- |
| OCH₃ | Cl | H | C₂H₅ | CH₃ | S | CH₃ | i-C₃H₇-S- |
| OCH₃ | Cl | H | C₃H₇ | CH₃ | S | CH₃ | i-C₃H₇-S- |
| OCH₃ | Cl | H | i-C₃H₇ | CH₃ | S | CH₃ | i-C₃H₇S- |
| OCH₃ | Cl | H | C₄H₉ | CH₃ | S | CH₃ | i-C₃H₇-S- |
| OCH₃ | Cl | H | i-C₄H₉ | CH₃ | S | CH₃ | i-C₃H₇-S- |
| OCH₃ | Cl | H | s-C₄H₉ | CH₃ | S | CH₃ | i-C₃H₇-S- |
| OCH₃ | Cl | H | t-C₄H₉ | CH₃ | S | CH₃ | i-C₃H₇-S- |
| OCH₃ | Cl | H | C₂H₅ | C₂H₅ | S | CH₃ | i-C₃H₇-S- |
| OCH₃ | Cl | H | C₃H₇ | C₃H₇ | S | CH₃ | i-C₃H₇-S- |
| OCH₃ | Cl | H | 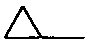 | CH₃ | S | CH₃ | i-C₃H₇-S- |
| OCH₃ | Cl | H | 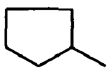 | CH₃ | S | CH₃ | i-C₃H₇-S- |
| OCH₃ | Cl | H | 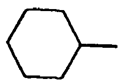 | CH₃ | S | CH₃ | i-C₃H₇-S- |
| Cl | OCH₃ | H | -(CH₂)₂- | | S | CH₃ | i-C₃H₇-S- |
| Cl | OCH₃ | H | -(CH₂)₄- | | S | CH₃ | i-C₃H₇-S- |
| Cl | OCH₃ | H | -(CH₂)₅- | | S | CH₃ | i-C₃H₇-S- |
| Cl | OCH₃ | H | -(CH₂)₆- | | S | CH₃ | i-C₃H₇-S- |
| Cl | OCH₃ | H | -(CH₂)₇- | | S | CH₃ | i-C₃H₇-S- |
| Cl | OCH₃ | H | -(CH₂)₂-O-(CH₂)₂- | | S | CH₃ | i-C₃H₇-S- |
| Cl | OCH₃ | H | -(CH₂)₂-S-(CH₂)₂- | | S | CH₃ | i-C₃H₇-S- |
| Cl | OCH₃ | H | -CH₂-CHCH₃-(CH₂)₃- | | S | CH₃ | i-C₃H₇-S- |
| Cl | OCH₃ | H | -(CH₂)₂-CHCH₃-(CH₂)₂- | | S | CH₃ | i-C₃H₇-S- |
| Cl | OCH₃ | H | -(CH₂)₂-CHC₂H₅-(CH₂)₂- | | S | CH₃ | i-C₃H₇-S- |
| Cl | OCH₃ | H | -(CH₂)₂-CHC₃H₇-(CH₂)-₂ | | S | CH₃ | i-C₃H₇-S- |
| Cl | OCH₃ | H | -(CH₂)₂-CHi-C₃H₇-(CH₂)₂- | | S | CH₃ | i-C₃H₇-S- |
| Cl | OCH₃ | H | -(CH₂)₂-CHOCH₃-(CH₂)₂- | | S | CH₃ | i-C₃H₇-S- |
| Cl | OCH₃ | H | -(CH₂)₂-CHOC₂H₅-(CH₂)₂- | | S | CH₃ | i-C₃H₇-S- |
| Cl | OCH₃ | H | -(CH₂)₂-CHOC₃H₇-(CH₂)₂- | | S | CH₃ | i-C₃H₇-S- |
| Cl | OCH₃ | H | -(CH₂)₂-CHi-OC₃H₇-(CH₂)₂- | | S | CH₃ | i-C₃H₇-S- |
| Cl | OCH₃ | H | -(CH₂)₂-C(CH₃)₂-(CH₂)₂- | | S | CH₃ | i-C₃H₇-S- |
| Cl | OCH₃ | H | -CH₂-(CHCH₃)₂-(CH₂)₂- | | S | CH₃ | i-C₃H₇-S- |
| Cl | OCH₃ | H | 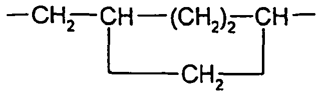 | | S | CH₃ | i-C₃H₇S- |
| Cl | OCH₃ | H | 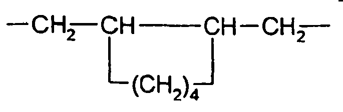 | | S | CH₃ | i-C₃H₇-S- |
| Cl | OCH₃ | H | 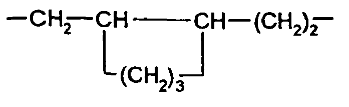 | | S | CH₃ | i-C₃H₇-S- |
| Cl | H | 6-OCH₃ | -(CH₂)₂- | | S | CH₃ | i-C₃H₇-S- |
| Cl | H | 6-OCH₃ | -(CH₂)₄- | | S | CH₃ | i-C₃H₇-S- |
| Cl | H | 6-OCH₃ | -(CH₂)₅- | | S | CH₃ | i-C₃H₇-S- |
| Cl | H | 6-OCH₃ | -(CH₂)₆- | | S | CH₃ | i-C₃H₇-S- |
| Cl | H | 6-OCH₃ | -(CH₂)₇- | | S | CH₃ | i-C₃H₇-S- |
| Cl | H | 6-OCH₃ | -(CH₂)₂-O-(CH₂)₂- | | S | CH₃ | i-C₃H₇-S- |
| Cl | H | 6-OCH₃ | -(CH₂)₂-S-(CH₂)₂- | | S | CH₃ | i-C₃H₇-S- |
| Cl | H | 6-OCH₃ | -CH₂-CHCH₃-(CH₂)₃- | | S | CH₃ | i-C₃H₇-S- |
| Cl | H | 6-OCH₃ | -(CH₂)₂-CHCH₃-(CH₂)₂- | | S | CH₃ | i-C₃H₇-S- |
| Cl | H | 6-OCH₃ | -(CH₂)₂-CHC₂H₅-(CH₂)₂- | | S | CH₃ | i-C₃H₇-S- |
| Cl | H | 6-OCH₃ | -(CH₂)₂-CHC₃H₇-(CH₂)₂- | | S | CH₃ | i-C₃H₇-S- |
| Cl | H | 6-OCH₃ | -(CH₂)₂-CHi-C₃H₇-(CH₂)₂- | | S | CH₃ | i-C₃H₇-S- |
| Cl | H | 6-OCH₃ | -(CH₂)₂-CHOCH₃-(CH₂)₂- | | S | CH₃ | i-C₃H₇-S- |
| Cl | H | 6-OCH₃ | -(CH₂)₂-CHOC₂H₅-(CH₂)₂- | | S | CH₃ | i-C₃H₇-S- |
| Cl | H | 6-OCH₃ | -(CH₂)₂-CHOC₃H₇-(CH₂)₂- | | S | CH₃ | i-C₃H₇-S- |
| Cl | H | 6-OCH₃ | -(CH₂)₂-CHi-OC₃H₇-(CH₂)₂- | | S | CH₃ | i-C₃H₇-S- |
| Cl | H | 6-OCH₃ | -(CH₂)₂-C(CH₃)₂-(CH₂)₂- | | S | CH₃ | i-C₃H₇-S- |
| Cl | H | 6-OCH₃ | -CH₂-(CHCH₃)₂-(CH₂)₂- | | S | CH₃ | i-C₃H₇-S- |
| Cl | H | 6-OCH₃ | 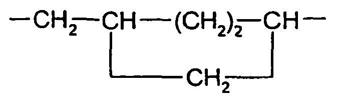 | | S | CH₃ | i-C₃H₇-S- |
| Cl | H | 6-OCH₃ | 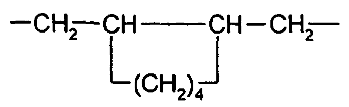 | | S | CH₃ | i-C₃H₇-S- |
| Cl | H | 6-OCH₃ | 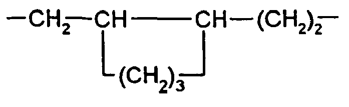 | | S | CH₃ | i-C₃H₇-S- |
| OCH₃ | Cl | H | -(CH₂)₂- | | S | CH₃ | i-C₃H₇-S- |
| OCH₃ | Cl | H | -(CH₂)₄- | | S | CH₃ | i-C₃H₇-S- |
| OCH₃ | Cl | H | -(CH₂)₅- | | S | CH₃ | i-C₃H₇-S- |
| OCH₃ | Cl | H | -(CH₂)₆- | | S | CH₃ | i-C₃H₇-S- |
| OCH₃ | Cl | H | -(CH₂)₇- | | S | CH₃ | i-C₃H₇-S- |
| OCH₃ | Cl | H | -(CH₂)₂-O-(CH₂)₂- | | S | CH₃ | i-C₃H₇-S- |
| OCH₃ | Cl | H | -(CH₂)₂-S-(CH₂)₂- | | S | CH₃ | i-C₃H₇-S- |
| OCH₃ | Cl | H | -CH₂-CHCH₃-(CH₂)₃- | | S | CH₃ | i-C₃H₇-S- |
| OCH₃ | Cl | H | -(CH₂)₂-CHCH₃-(CH₂)₂- | | S | CH₃ | i-C₃H₇-S- |
| OCH₃ | Cl | H | -(CH₂)₂-CHC₂H₅-(CH₂)₂- | | S | CH₃ | i-C₃H₇-S- |
| OCH₃ | Cl | H | -(CH₂)₂-CH₃H₇-(CH₂)₂- | | S | CH₃ | i-C₃H₇-S- |
| OCH₃ | Cl | H | -(CH₂)₂-CHi-C₃H₇-(CH₂)₂- | | S | CH₃ | i-C₃H₇-S- |
| OCH₃ | Cl | H | -(CH₂)₂-CHOCH₃-(CH₂)₂- | | S | CH₃ | i-C₃H₇-S- |
| OCH₃ | Cl | H | -(CH₂)₂-CHOC₂H₅-(CH₂)₂- | | S | CH₃ | i-C₃H₇-S- |
| OCH₃ | Cl | H | -(CH₂)₂-CHOC₃H₇-(CH₂)₂- | | S | CH₃ | i-C₃H₇-S- |
| OCH₃ | Cl | H | -(CH₂)₂-CHi-OC₃H₇-(CH₂)₂- | | S | CH₃ | i-C₃H₇-S- |
| OCH₃ | Cl | H | -(CH₂)₂-C(CH₃)₂-(CH₂)₂- | | S | CH₃ | i-C₃H₇-S- |
| OCH₃ | Cl | H | -CH₂-(CHCH₃)₂-(CH₂)₂- | | S | CH₃ | i-C₃H₇-S- |
| OCH₃ | Cl | H | 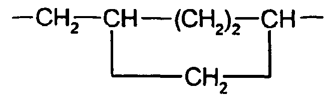 | | S | CH₃ | i-C₃H₇-S- |
| OCH₃ | Cl | H | 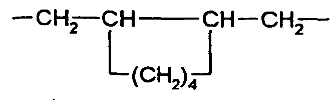 | | S | CH₃ | i-C₃H₇-S- |
| OCH₃ | Cl | H | 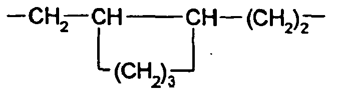 | | S | CH₃ | i-C₃H₇-S- |
| Cl | OCH₃ | H | CH₃ | H | S | C₂H₅ | i-C₃H₇-S- |
| Cl | OCH₃ | H | C₂H₅ | H | S | C₂H₅ | i-C₃H₇-S- |
| Cl | -OCH₃ | H | C₃H₇ | H | S | C₂H₅ | i-C₃H₇S- |
| Cl | OCH₃ | H | i-C₃H₇ | H | S | C₂H₅ | i-C₃H₇-S- |
| Cl | OCH₃ | H | C₄H₉ | H | S | C₂H₅ | i-C₃H₇-S- |
| Cl | OCH₃ | H | i-C₄H₉ | H | S | C₂H₅ | i-C₃H₇-S- |
| Cl | OCH₃ | H | s-C₄H₉ | H | S | C₂H₅ | i-C₃H₇-S- |
| Cl | OCH₃ | H | t-C₄H₉ | H | S | C₂H₅ | i-C₃H₇-S- |
| Cl | OCH₃ | H | CH₃ | CH₃ | S | C₂H₅ | i-C₃H₇-S- |
| Cl | OCH₃ | H | C₂H₅ | CH₃ | S | C₂H₅ | i-C₃H₇-S- |
| Cl | OCH₃ | H | C₃H₇ | CH₃ | S | C₂H₅ | i-C₃H₇-S- |
| Cl | OCH₃ | H | i-C₃H₇ | CH₃ | S | C₂H₅ | i-C₃H₇-S- |
| Cl | OCH₃ | H | C₄H₉ | CH₃ | S | C₂H₅ | i-C₃H₇-S- |
| Cl | OCH₃ | H | i-C₄H₉ | CH₃ | S | C₂H₅ | i-C₃H₇-S- |
| Cl | OCH₃ | H | s-C₄H₉ | CH₃ | S | C₂H₅ | i-C₃H₇-S- |
| Cl | OCH₃ | H | t-C₄H₉ | CH₃ | S | C₂H₅ | i-C₃H₇-S- |
| Cl | OCH₃ | H | C₂H₅ | C₂H₅ | S | C₂H₅ | i-C₃H₇-S- |
| Cl | OCH₃ | H | C₃H₇ | C₃H₇ | S | C₂H₅ | i-C₃H₇-S- |
| Cl | OCH₃ | H | 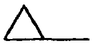 | CH₃ | S | C₂H₅ | i-C₃H₇-S- |
| Cl | OCH₃ | H | 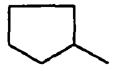 | CH₃ | S | C₂H₅ | i-C₃H₇-S- |
| Cl | OCH₃ | H | 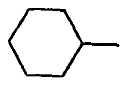 | CH₃ | S | C₂H₅ | i-C₃H₇-S- |
| Cl | H | 6-OCH₃ | CH₃ | H | S | C₂H₅ | i-C₃H₇-S- |
| Cl | H | 6-OCH₃ | C₂H₅ | H | S | C₂H₅ | i-C₃H₇-S- |
| Cl | H | 6-OCH₃ | C₃H₇ | H | S | C₂H₅ | i-C₃H₇-S- |
| Cl | H | 6-OCH₃ | i-C₃H₇ | H | S | C₂H₅ | i-C₃H₇-S- |
| Cl | H | 6-OCH₃ | C₄H₉ | H | S | C₂H₅ | i-C₃H₇-S- |
| Cl | H | 6-OCH₃ | i-C₄H₉ | H | S | C₂H₅ | i-C₃H₇-S- |
| Cl | H | 6-OCH₃ | s-C₄H₉ | H | S | C₂H₅ | i-C₃H₇-S- |
| Cl | H | 6-OCH₃ | t-C₄H₉ | H | S | C₂H₅ | i-C₃H₇-S- |
| Cl | H | 6-OCH₃ | CH₃ | CH₃ | S | C₂H₅ | i-C₃H₇-S- |
| Cl | H | 6-OCH₃ | C₂H₅ | CH₃ | S | C₂H₅ | i-C₃H₇-S- |
| Cl | H | 6-OCH₃ | C₃H₇ | CH₃ | S | C₂H₅ | i-C₃H₇-S- |
| Cl | H | 6-OCH₃ | i-C₃H₇ | CH₃ | S | C₂H₅ | i-C₃H₇-S- |
| Cl | H | 6-OCH₃ | C₄H₉ | CH₃ | S | C₂H₅ | i-C₃H₇-S- |
| Cl | H | 6-OCH₃ | i-C₄H₉ | CH₃ | S | C₂H₅ | i-C₃H₇-S- |
| Cl | H | 6-OCH₃ | s-C₄H₉ | CH₃ | S | C₂H₅ | i-C₃H₇-S- |
| Cl | H | 6-OCH₃ | t-C₄H₉ | CH₃ | S | C₂H₅ | i-C₃H₇-S- |
| Cl | H | 6-OCH₃ | C₂H₅ | C₂H₅ | S | C₂H₅ | i-C₃H₇-S- |
| Cl | H | 6-OCH₃ | C₃H₇ | C₃H₇ | S | C₂H₅ | i-C₃H₇-S- |
| Cl | H | 6-OCH₃ | 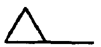 | CH₃ | S | C₂H₅ | i-C₃H₇-S- |
| Cl | H | 6-OCH₃ | 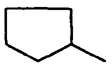 | CH₃ | S | C₂H₅ | i-C₃H₇-S- |
| Cl | H | 6-OCH₃ | 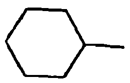 | CH₃ | S | C₂H₅ | i-C₃H₇-S- |
| OCH₃ | Cl | H | CH₃ | H | S | C₂H₅ | i-C₃H₇-S- |
| OCH₃ | Cl | H | C₂H₅ | H | S | C₂H₅ | i-C₃H₇-S- |
| OCH₃ | Cl | H | C₃H₇ | H | S | C₂H₅ | i-C₃H₇-S- |
| OCH₃ | Cl | H | i-C₃H₇ | H | S | C₂H₅ | i-C₃H₇-S- |
| OCH₃ | Cl | H | C₄H₉ | H | S | C₂H₅ | i-C₃H₇-S- |
| OCH₃ | Cl | H | i-C₄H₉ | H | S | C₂H₅ | i-C₃H₇-S- |
| OCH₃ | Cl | H | s-C₄H₉ | H | S | C₂H₅ | i-C₃H₇-S- |
| OCH₃ | Cl | H | t-C₄H₉ | H | S | C₂H₅ | i-C₃H₇-S- |
| OCH₃ | Cl | H | CH₃ | CH₃ | S | C₂H₅ | i-C₃H₇-S- |
| OCH₃ | Cl | H | C₂H₅ | CH₃ | S | C₂H₅ | i-C₃H₇-S- |
| OCH₃ | Cl | H | C₃H₇ | CH₃ | S | C₂H₅ | i-C₃H₇-S- |
| OCH₃ | Cl | H | i-C₃H₇ | CH₃ | S | C₂H₅ | i-C₃H₇-S- |
| OCH₃ | Cl | H | C₄H₉ | CH₃ | S | C₂H₅ | i-C₃H₇-S- |
| OCH₃ | Cl | H | i-C₄H₉ | CH₃ | S | C₂H₅ | i-C₃H₇-S- |
| OCH₃ | Cl | H | s-C₄H₉ | CH₃ | S | C₂H₅ | i-C₃H₇-S- |
| OCH₃ | Cl | H | t-C₄H₉ | CH₃ | S | C₂H₅ | i-C₃H₇-S- |
| OCH₃ | Cl | H | C₂H₅ | C₂H₅ | S | C₂H₅ | i-C₃H₇-S- |
| OCH₃ | Cl | H | C₃H₇ | C₃H₇ | S | C₂H₅ | i-C₃H₇-S- |
| OCH₃ | Cl | H | 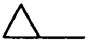 | CH₃ | S | C₂H₅ | i-C₃H₇-S- |
| OCH₃ | Cl | H | 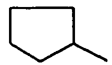 | CH₃ | S | C₂H₅ | i-C₃H₇-S- |
| OCH₃ | Cl | H | 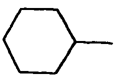 | CH₃ | S | C₂H₅ | i-C₃H₇-S- |
| Cl | OCH₃ | H | -(CH₂)₂- | | S | C₂H₅ | i-C₃H₇-S- |
| Cl | OCH₃ | H | -(CH₂)₄- | | S | C₂H₅ | i-C₃H₇-S- |
| Cl | OCH₃ | H | -(CH₂)₅- | | S | C₂H₅ | i-C₃H₇-S- |
| Cl | OCH₃ | H | -(CH₂)₆- | | S | C₂H₅ | i-C₃H₇-S- |
| Cl | OCH₃ | H | -(CH₂)₇- | | S | C₂H₅ | i-C₃H₇-S- |
| Cl | OCH₃ | H | -(CH₂)₂-O-(CH₂)₂- | | S | C₂H₅ | i-C₃H₇-S- |
| Cl | OCH₃ | H | -(CH₂)₂-S-(CH₂)₂- | | S | C₂H₅ | i-C₃H₇-S- |
| Cl | OCH₃ | H | -CH₂-CHCH₃-(CH₂)₃- | | S | C₂H₅ | i-C₃H₇-S- |
| Cl | OCH₃ | H | -(CH₂)₂-CHCH₃-(CH₂)₂- | | S | C₂H₅ | i-C₃H₇-S- |
| Cl | OCH₃ | H | -(CH₂)₂-CHC₂H₅-(CH₂)₂- | | S | C₂H₅ | i-C₃H₇-S- |
| Cl | OCH₃ | H | -(CH₂)₂-CHC₃H₇-(CH₂)₂- | | S | C₂H₅ | i-C₃H₇-S- |
| Cl | OCH₃ | H | -(CH₂)₂-CHi-C₃H₇-(CH₂)₂- | | S | C₂H₅ | i-C₃H₇-S- |
| Cl | OCH₃ | H | -(CH₂)₂-CHOCH₃-(CH₂)₂- | | S | C₂H₅ | i-C₃H₇-S- |
| Cl | OCH₃ | H | -(CH₂)₂-CHOC₂H₅-(CH₂)₂- | | S | C₂H₅ | i-C₃H₇-S- |
| Cl | OCH₃ | H | -(CH₂)₂-CHOC₃H₇-(CH₂)₂- | | S | C₂H₅ | i-C₃H₇-S- |
| Cl | OCH₃ | H | -(CH₂)₂-CHi-OC₃H₇-(CH₂)₂- | | S | C₂H₅ | i-C₃H₇-S- |
| Cl | OCH₃ | H | -(CH₂)₂-C(CH₂)₂-(CH₂)₂- | | S | C₂H₅ | i-C₃H₇-S- |
| Cl | OCH₃ | H | -CH₂-(CHCH₃)₂-(CH₂)₂- | | S | C₂H₅ | i-C₃H₇-S- |
| Cl | OCH₃ | H | 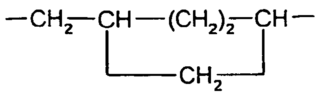 | | S | C₂H₅ | i-C₃H₇-S- |
| Cl | OCH₃ | H | 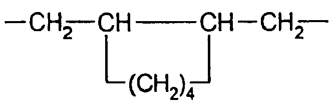 | | S | C₂H₅ | i-C₃H₇-S- |
| Cl | OCH₃ | H | 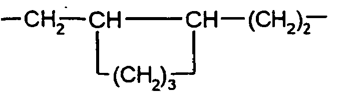 | | S | C₂H₅ | i-C₃H₇-S- |
| Cl | H | 6-OCH₃ | -(CH₂)₂- | | S | C₂H₅ | i-C₃H₇-S- |
| Cl | H | 6-OCH₃ | -(CH₂)₄- | | S | C₂H₅ | i-C₃H₇-S- |
| Cl | H | 6-OCH₃ | -(CH₂)₅- | | S | C₂H₅ | i-C₃H₇-S- |
| Cl | H | 6-OCH₃ | -(CH₂)₆- | | S | C₂H₅ | i-C₃H₇-S- |
| Cl | H | 6-OCH₃ | -(CH₂)₇- | | S | C₂H₅ | i-C₃H₇-S- |
| Cl | H | 6-OCH₃ | -(CH₂)₂-O-(CH₂)₂- | | S | C₂H₅ | i-C₃H₇-S- |
| Cl | H | 6-OCH₃ | -(CH₂)₂-S-(CH₂)₂- | | S | C₂H₅ | i-C₃H₇-S- |
| Cl | H | 6-OCH₃ | -CH₂-CHCH₃-(CH₂)₃- | | S | C₂H₅ | i-C₃H₇-S- |
| Cl | H | 6-OCH₃ | -(CH₂)₂-CHCH₃-(CH₂)₂- | | S | C₂H₅ | i-C₃H₇-S- |
| Cl | H | 6-OCH₃ | -(CH₂)₂-CHC₂H₅-(CH₂)₂- | | S | C₂H₅ | i-C₃H₇-S- |
| Cl | H | 6-OCH₃ | -(CH₂)₂-CHC₃H₇-(CH₂)₂- | | S | C₂H₅ | i-C₃H₇-S- |
| Cl | H | 6-OCH₃ | -(CH₂)₂-CHi-C₃H₇-(CH₂)₂- | | S | C₂H₅ | i-C₃H₇-S- |
| Cl | H | 6-OCH₃ | -(CH₂)₂-CHOCH₃-(CH₂)₂- | | S | C₂H₅ | i-C₃H₇-S- |
| Cl | H | 6-OCH₃ | -(CH₂)₂-CHOC₂H₅-(CH₂)₂- | | S | CH₅ | i-C₃H₇-S- |
| Cl | H | 6-OCH₃ | -(CH₂)₂-CHOC₃H₇-(CH₂)₂- | | S | CH₅ | i-C₃H₇-S- |
| Cl | H | 6-OCH₃ | -(CH₂)₂-CHi-OC₃H₇-(CH₂)₂- | | S | C₂H₅ | i-C₃H₇-S- |
| Cl | H | 6-OCH₃ | -(CH₂)₂-C(CH₃)₂-(CH₂)₂- | | S | C₂H₅ | i-C₃H₇-S- |
| Cl | H | 6-OCH₃ | -CH₂-(CHCH₃)₂-(CH₂)₂- | | S | C₂H₅ | i-C₃H₇-S- |
| Cl | H | 6-OCH₃ | 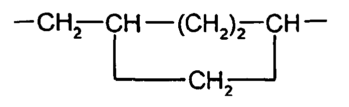 | | S | C₂H₅ | i-C₃H₇-S- |
| Cl | H | 6-OCH₃ | 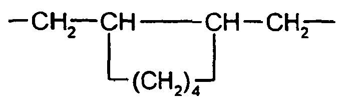 | | S | C₂H₅ | i-C₃H₇-S- |
| Cl | H | 6-OCH₃ | 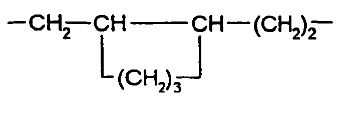 | | S | C₂H₅ | i-C₃H₇-S- |
| OCH₃ | Cl | H | -(CH₂)₂- | | S | C₂H₅ | i-C₃H₇-S- |
| OCH₃ | Cl | H | -(CH₂)₄- | | S | C₇H₅ | i-C₃H₇-S- |
| OCH₃ | Cl | H | -(CH₂)₅- | | S | C₂H₅ | i-C₃H₇-S- |
| OCH₃ | Cl | H | -(CH₂)₆- | | S | C₂H₅ | i-C₃H₇-S- |
| OCH₃ | Cl | H | -(CH₂)₇- | | S | C₂H₅ | i-C₃H₇-S- |
| OCH₃ | Cl | H | -(CH₂)₂-O-(CH₂)₂- | | S | C₂H₅ | i-C₃H₇-S- |
| OCH₃ | Cl | H | -(CH₂)₂-S-(CH₂)₂- | | S | C₂H₅ | i-C₃H₇-S- |
| OCH₃ | Cl | H | -CH₂-CHCH₃-(CH₂)₃- | | S | C₂H₅ | i-C₃H₇-S- |
| OCH₃ | Cl | H | -(CH₂)₂-CHCH₃-(CH₂)₂- | | S | C₂H₅ | i-C₃H₇-S- |
| OCH₃ | Cl | H | -(CH₂)₂-CHC₂H₅-(CH₂)₂- | | S | C₂H₅ | i-C₃H₇-S- |
| OCH₃ | Cl | H | -(CH₂)₂-CHC₃H₇-(CH₂)₂- | | S | C₂H₅ | i-C₃H₇-S- |
| OCH₃ | Cl | H | -(CH₂)₂-CHi-C₃H₇-(CH₂)₂- | | S | C₂H₅ | i-C₃H₇-S- |
| OCH₃ | Cl | H | -(CH₂)₂-CHOCH₃-(CH₂)₂- | | S | C₂H₅ | i-C₃H₇-S- |
| OCH₃ | Cl | H | -(CH₂)₂-CHOC₂H₅-(CH₂)₂- | | S | C₂H₅ | i-C₃H₇-S- |
| OCH₃ | Cl | H | -(CH₂)₂-CHOC₃H₇-(CH₂)₂- | | S | C₂H₅ | i-C₃H₇-S- |
| OCH₃ | Cl | H | -(CH₂)₂-CHi-OC₃H₇-(CH₂)₂- | | S | C₂H₅ | i-C₃H₇-S- |
| OCH₃ | Cl | H | -(CH₂)₂-C(CH₃)₂-(CH₂)₂- | | S | C₂H₅ | i-C₃H₇-S- |
| OCH₃ | Cl | H | -CH₂-(CHCH₃)₂-(CH₂)₂- | | S | C₂H₅ | i-C₃H₇-S- |
| OCH₃ | Cl | H | 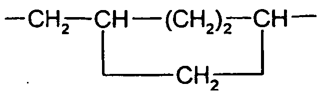 | | S | C₂H₅ | i-C₃H₇-S- |
| OCH₃ | Cl | H | 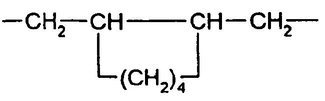 | | S | C₂H₅ | i-C₃H₇-S- |
| OCH₃ | Cl | H | 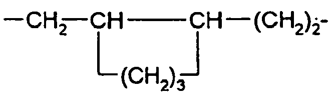 | | S | C₂H₅ | i-C₃H₇-S- |
| Cl | OCH₃ | H | CH₃ | H | O | CH₃ | i-C₃H₇-S- |
| Cl | OCH₃ | H | C₂H₅ | H | O | CH₃ | i-C₃H₇-S- |
| Cl | OCH₃ | H | C₃H₇ | H | O | CH₃ | i-C₃H₇-S- |
| Cl | OCH₃ | H | i-C₃H₇ | H | O | CH₃ | i-C₃H₇-S- |
| Cl | OCH₃ | H | C₄H₉ | H | O | CH₃ | i-C₃H₇-S- |
| Cl | OCH₃ | H | i-C₄H₉ | H | O | CH₃ | i-C₃H₇-S- |
| Cl | OCH₃ | H | s-C₄H₉ | H | O | CH₃ | i-C₃H₇-S- |
| Cl | OCH₃ | H | t-C₄H₉ | H | O | CH₃ | i-C₃H₇-S- |
| Cl | OCH₃ | H | CH₃ | CH₃ | O | CH₃ | i-C₃H₇-S- |
| Cl | OCH₃ | H | C₂H₅ | CH₃ | O | CH₃ | i-C₃H₇-S- |
| Cl | OCH₃ | H | C₃H₇ | CH₃ | O | CH₃ | i-C₃H₇-S- |
| Cl | OCH₃ | H | i-C₃H₇ | CH₃ | O | CH₃ | i-C₃H₇-S- |
| Cl | OCH₃ | H | C₄H₉ | CH₃ | O | CH₃ | i-C₃H₇-S- |
| Cl | OCH₃ | H | i-C₄H₉ | CH₃ | O | CH₃ | i-C₃H₇-S- |
| Cl | OCH₃ | H | s-C₄H₉ | CH₃ | O | CH₃ | i-C₃H₇-S- |
| Cl | OCH₃ | H | t-C₄H₉ | CH₃ | O | CH₃ | i-C₃H₇-S- |
| Cl | OCH₃ | H | C₂H₅ | C₂H₅ | O | CH₃ | i-C₃H₇-S- |
| Cl | OCH₃ | H | C₃H₇ | C₃H₇ | O | CH₃ | i-C₃H₇-S- |
| Cl | OCH₃ | H | 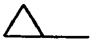 | CH₃ | O | CH₃ | i-C₃H₇-S- |
| Cl | OCH₃ | H | 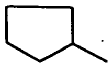 | CH₃ | O | CH₃ | i-C₃H₇-S- |
| Cl | OCH₃ | H | 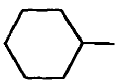 | CH₃ | O | CH₃ | i-C₃H₇-S- |
| Cl | H | 6-OCH₃ | CH₃ | H | O | CH₃ | i-C₃H₇-S- |
| Cl | H | 6-OCH₃ | C₂H₅ | H | O | CH₃ | i-C₃H₇-S- |
| Cl | H | 6-OCH₃ | C₃H₇ | H | O | CH₃ | i-C₃H₇-S- |
| Cl | H | 6-OCH₃ | i-C₃H₇ | H | O | CH₃ | i-C₃H₇-S- |
| Cl | H | 6-OCH₃ | C₄H₉ | H | O | CH₃ | i-C₃H₇-S- |
| Cl | H | 6-OCH₃ | i-C₄H₉ | H | O | CH₃ | i-C₃H₇-S- |
| Cl | H | 6-OCH₃ | s-C₄H₉ | H | O | CH₃ | i-C₃H₇-S- |
| Cl | H | 6-OCH₃ | t-C₄H₉ | H | O | CH₃ | i-C₃H₇-S- |
| Cl | H | 6-OCH₃ | CH₃ | CH₃ | O | CH₃ | i-C₃H₇-S- |
| Cl | H | 6-OCH₃ | C₂H₅ | CH₃ | O | CH₃ | i-C₃H₇-S- |
| Cl | H | 6-OCH₃ | C₃H₇ | CH₃ | O | CH₃ | i-C₃H₇-S- |
| Cl | H | 6-OCH₃ | i-C₃H₇ | CH₃ | O | CH₃ | i-C₃H₇-S- |
| Cl | H | 6-OCH₃ | C₄H₉ | CH₃ | O | CH₃ | i-C₃H₇-S- |
| Cl | H | 6-OCH₃ | i-C₄H₉ | CH₃ | O | CH₃ | i-C₃H₇-S- |
| Cl | H | 6-OCH₃ | s-C₄H₉ | CH₃ | O | CH₃ | i-C₃H₇-S- |
| Cl | H | 6-OCH₃ | t-C₄H₉ | CH₃ | O | CH₃ | i-C₃H₇-S- |
| Cl | H | 6-OCH₃ | C₂H₅ | C₂H₅ | O | CH₃ | i-C₃H₇-S- |
| Cl | H | 6-OCH₃ | C₃H₇ | C₃H₇ | O | CH₃ | i-C₃H₇-S- |
| Cl | H | 6-OCH₃ | 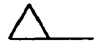 | CH₃ | O | CH₃ | i-C₃H₇-S- |
| Cl | H | 6-OCH₃ | 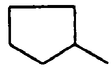 | CH₃ | O | CH₃ | i-C₃H₇-S- |
| Cl | H | 6-OCH₃ | 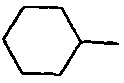 | CH₃ | O | CH₃ | i-C₃H₇-S- |
| OCH₃ | Cl | H | CH₃ | H | O | CH₃ | i-C₃H₇-S- |
| OCH₃ | Cl | H | C₂H₅ | H | O | CH₃ | i-C₃H₇-S- |
| OCH₃ | Cl | H | C₃H₇ | H | O | CH₃ | i-C₃H₇-S- |
| OCH₃ | Cl | H | i-C₃H₇ | H | O | CH₃ | i-C₃H₇-S- |
| OCH₃ | Cl | H | C₄H₉ | H | O | CH₃ | i-C₃H₇-S- |
| OCH₃ | Cl | H | i-C₄H₉ | H | O | CH₃ | i-C₃H₇-S- |
| OCH₃ | Cl | H | s-C₄H₉ | H | O | CH₃ | i-C₃H₇-S- |
| OCH₃ | Cl | H | t-C₄H₉ | H | O | CH₃ | i-C₃H₇-S- |
| OCH₃ | Cl | H | CH₃ | CH₃ | O | CH₃ | i-C₃H₇-S- |
| OCH₃ | Cl | H | C₂H₅ | CH₃ | O | CH₃ | i-C₃H₇-S- |
| OCH₃ | Cl | H | C₃H₇ | CH₃ | O | CH₂ | i-C₃H₇-S- |
| OCH₃ | Cl | H | i-C₃H₇ | CH₃ | O | CH₃ | i-C₃H₇-S- |
| OCH₃ | Cl | H | C₄H₉ | CH₃ | O | CH₃ | i-C₃H₇-S- |
| OCH₃ | Cl | H | i-C₄H₉ | CH₃ | O | CH₃ | i-C₃H₇-S- |
| OCH₃ | Cl | H | s-C₄H₉ | CH₃ | O | CH₃ | i-C₃H₇-S- |
| OCH₃ | Cl | H | t-C₄H₉ | CH₃ | O | CH₃ | i-C₃H₇-S- |
| OCH₃ | Cl | H | C₂H₅ | C₂H₅ | O | CH₃ | i-C₃H₇-S- |
| OCH₃ | Cl | H | C₃H₇ | C₃H₇ | O | CH₃ | i-C₃H₇-S- |
| OCH₃ | Cl | H | 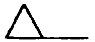 | CH₃ | O | CH₃ | i-C₃H₇-S- |
| OCH₃ | Cl | H | 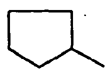 | CH₃ | O | CH₃ | i-C₃H₇-S- |
| OCH₃ | Cl | H | 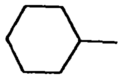 | CH₃ | O | CH₃ | i-C₃H₇-S- |
| Cl | OCH₃ | H | -(CH₂)₂- | | O | CH₃ | i-C₃H₇-S- |
| Cl | OCH₃ | H | -(CH₂)₄- | | O | CH₃ | i-C₃H₇-S- |
| Cl | OCH₃ | H | -(CH₂)₅- | | O | CH₃ | i-C₃H₇-S- |
| Cl | OCH₃ | H | -(CH₂)₆- | | O | CH₃ | i-C₃H₇-S- |
| Cl | OCH₃ | H | -(CH₂)₇- | | O | CH₃ | i-C₃H₇-S- |
| Cl | OCH₃ | H | -(CH₂)₂-O-(CH₂)₂- | | O | CH₃ | i-C₃H₇-S- |
| Cl | OCH₃ | H | -(CH₂)₂-S-(CH₂)₂- | | O | CH₃ | i-C₃H₇-S- |
| Cl | OCH₃ | H | -CH₂-CHCH₃-(CH₂)₃- | | O | CH₃ | i-C₃H₇-S- |
| Cl | OCH₃ | H | -(CH₂)₂-CHCH₃-(CH₂)₂- | | O | CH₃ | i-C₃H₇-S- |
| Cl | OCH₃ | H | -(CH₂)₂-CHC₂H₅-(CH₂)₂- | | O | CH₃ | i-C₃H₇-S- |
| Cl | OCH₃ | H | -(CH₂)₂-CHC₃H₇-(CH₂)₂- | | O | CH₃ | i-C₃H₇-S- |
| Cl | OCH₃ | H | -(CH₂)₂-CHi-C₃H₇-(CH₂)₂- | | O | CH₃ | i-C₃H₇-S- |
| Cl | OCH₃ | H | -(CH₂)₂-CHOCH₃-(CH₂)₂- | | O | CH₃ | i-C₃H₇-S- |
| Cl | OCH₃ | H | -(CH₂)₂-CHOC₂H₅-(CH₂)₂- | | O | CH₃ | i-C₃H₇-S- |
| Cl | OCH₃ | H | -(CH₂)₂-CHOC₃H₇-(CH₂)₂- | | O | CH₃ | i-C₃H₇-S- |
| Cl | OCH₃ | H | -(CH₂)₂-CHi-OC₃H₇-(CH₂)₂- | | O | CH₃ | i-C₃H₇-S- |
| Cl | OCH₃ | H | -(CH₂)₂-C(CH₃)₂-(CH₂)₂- | | O | CH₃ | i-C₃H₇-S- |
| Cl | OCH₃ | H | -CH₂-(CHCH₃)₂-(CH₂)₂- | | O | CH₃ | i-C₃H₇-S- |
| Cl | OCH₃ | H | 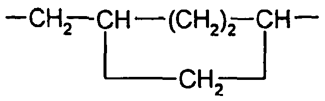 | | O | CH₃ | i-C₃H₇-S- |
| Cl | OCH₃ | H | 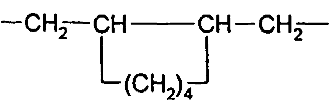 | | O | CH₃ | i-C₃H₇-S- |
| Cl | OCH₃ | H | 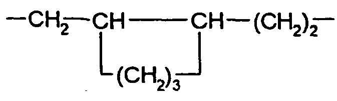 | | O | CH₃ | i-C₃H₇-S- |
| Cl | H | 6-OCH₃ | -(CH₂)₂- | | O | CH₃ | i-C₃H₇-S- |
| Cl | H | 6-OCH₃ | -(CH₂)₄- | | O | CH₃ | i-C₃H₇-S- |
| Cl | H | 6-OCH₃ | -(CH₂)₅- | | O | CH₃ | i-C₃H₇-S- |
| Cl | H | 6-OCH₃ | -(CH₂)₆- | | O | CH₃ | i-C₃H₇-S- |
| Cl | H | 6-OCH₃ | -(CH₂)₇- | | O | CH₃ | i-C₃H₇-S- |
| Cl | H | 6-OCH₃ | -(CH₂)₂-O-(CH₂)₂- | | O | CH₃ | i-C₃H₇-S- |
| Cl | H | 6-OCH₃ | -(CH₂)₂-S-(CH₂)₂- | | O | CH₃ | i-C₃H₇-S- |
| Cl | H | 6-OCH₃ | -CH₂-CHCH₃-(CH₂)₃- | | O | CH₃ | i-C₃H₇-S- |
| Cl | H | 6-OCH₃ | -(CH₂)₂-CHCH₃-(CH₂)₂- | | O | CH₃ | i-C₃H₇-S- |
| Cl | H | 6-OCH₃ | -(CH₂)₂-CHC₂H₅-(CH₂)₂- | | O | CH₃ | i-C₃H₇-S- |
| Cl | H | 6-OCH₃ | -(CH₂)₂-CHC₃H₇-(CH₂)₂- | | O | CH₃ | i-C₃H₇-S- |
| Cl | H | 6-OCH₃ | -(CH₂)₂-CHi-C₂H₇-(CH₂)₂- | | O | CH₃ | i-C₃H₇-S- |
| Cl | H | 6-OCH₃ | -(CH₂)₂-CHOCH₃-(CH₂)₂- | | O | CH₃ | i-C₃H₇-S- |
| Cl | H | 6-OCH₃ | -(CH₂)₂-CHOC₂H₅-(CH₂)₂- | | O | CH₃ | i-C₃H₇-S- |
| Cl | H | 6-OCH₃ | -(CH₂)₂-CHOC₃H₇-(CH₂)₂- | | O | CH₃ | i-C₃H₇-S- |
| Cl | H | 6-OCH₃ | -(CH₂)₂-CHi-OC₃H₇-(CH₂)₂- | | O | CH₃ | i-C₃H₇-S- |
| Cl | H | 6-OCH₃ | -(CH₂)₂-C(CH₃)₂-(CH₂)₂- | | O | CH₃ | i-C₃H₇-S- |
| Cl | H | 6-OCH₃ | -CH₂-(CHCH₃)₂-(CH₂)₂- | | O | CH₃ | i-C₃H₇-S- |
| Cl | H | 6-OCH₃ | 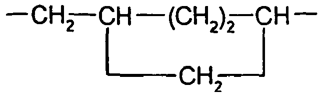 | | O | CH₃ | i-C₃H₇-S- |
| Cl | H | 6-OCH₃ | 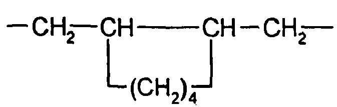 | | O | CH₃ | i-C₃H₇-S- |
| Cl | H | 6-OCH₃ | 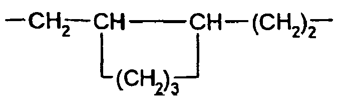 | | O | CH₃ | i-C₃H₇-S- |
| OCH₃ | Cl | H | -(CH₂)₂- | | O | CH₃ | i-C₃H₇-S- |
| OCH₃ | Cl | H | -(CH₂)₄- | | O | CH₃ | i-C₃H₇-S- |
| OCH₃ | Cl | H | -(CH₂)₅- | | O | CH₃ | i-C₃H₇-S- |
| OCH₃ | Cl | H | -(CH₂)₆- | | O | CH₂ | i-C₃H₇-S- |
| OCH₃ | Cl | H | -(CH₂)₇- | | O | CH₃ | i-C₃H₇-S- |
| OCH₃ | Cl | H | -(CH₂)₂-O-(CH₂)₂- | | O | CH₃ | i-C₃H₇-S- |
| OCH₃ | Cl | H | -(CH₂)₂-S-(CH₂)₂- | | O | CH₃ | i-C₃H₇-S- |
| OCH₃ | Cl | H | -CH₂-CHCH₃-(CH₂)₃- | | O | CH₃ | i-C₃H₇-S- |
| OCH₃ | Cl | H | -(CH₂)₂-CHCH₃-(CH₂)₂- | | O | CH₃ | i-C₃H₇-S- |
| OCH₃ | Cl | H | -(CH₂)₂-CHC₂H₅-(CH₂)₂- | | O | CH₃ | i-C₃H₇-S- |
| OCH₃ | Cl | H | -(CH₂)₂-CHC₃H₇-(CH₂)₂- | | O | CH₃ | i-C₃H₇-S- |
| OCH₃ | Cl | H | -(CH₂)₂-CHi-C₃H₇-(CH₂)₂- | | O | CH₃ | i-C₃H₇-S- |
| OCH₃ | Cl | H | -(CH₂)₂-CHOCH₃-(CH₂)₂- | | O | CH₃ | i-C₃H₇-S- |
| OCH₃ | Cl | H | -(CH₂)₂-CHOC₂H₅-(CH₂)₂- | | O | CH₃ | i-C₃H₇-S- |
| OCH₃ | Cl | H | -(CH₂)₂-CHOC₃H₇-(CH₂)₂- | | O | CH₃ | i-C₃H₇-S- |
| OCH₃ | Cl | H | -(CH₂)₂-CHi-OC₃H₇-(CH₂)₂- | | O | CH₃ | i-C₃H₇-S- |
| OCH₃ | Cl | H | -(CH₂)₂-C(CH₃)₂-(CH₂)₂- | | O | CH₃ | i-C₃H₇-S- |
| OCH₃ | Cl | H | -CH₂-(CHCH₃)₂-(CH₂)₂- | | O | CH₃ | i-C₃H₇-S- |
| OCH₃ | Cl | H | 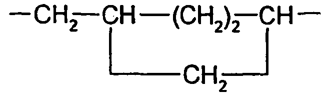 | | O | CH₃ | i-C₃H₇-S- |
| OCH₃ | Cl | H | 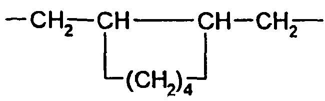 | | O | CH₃ | i-C₃H₇-S- |
| OCH₃ | Cl | H | 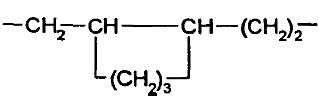 | | O | CH₃ | i-C₃H₇-S- |
| Cl | OCH₃ | H | CH₃ | H | O | C₂H₅ | i-C₃H₇-S- |
| Cl | OCH₃ | H | C₂H₅ | H | O | C₂H₅ | i-C₃H₇-S- |
| Cl | OCH₃ | H | C₃H₇ | H | O | C₂H₅ | i-C₃H₇-S- |
| Cl | OCH₃ | H | i-C₃H₇ | H | O | C₂H₅ | i-C₃H₇-S- |
| Cl | OCH₃ | H | C₄H₉ | H | O | C₂H₅ | i-C₃H₇-S- |
| Cl | OCH₃ | H | i-C₄H₉ | H | O | C₂H₅ | i-C₃H₇-S- |
| Cl | OCH₃ | H | s-C₄H₉ | H | O | C₂H₅ | i-C₃H₇-S- |
| Cl | OCH₃ | H | t-C₄H₉ | H | O | C₂H₅ | i-C₃H₇-S- |
| Cl | OCH₃ | H | CH₃ | CH₃ | O | C₂H₅ | -C₃H₇-S- |
| Cl | OCH₃ | H | C₂H₅ | CH₃ | O | C₂H₅ | i-C₃H₇-S- |
| Cl | OCH₃ | H | C₃H₇ | CH₃ | O | C₂H₅ | i-C₃H₇-S- |
| Cl | OCH₃ | H | i-C₃H₇ | CH₃ | O | C₂H₅ | -C₃H₇-S- |
| Cl | OCH₃ | H | C₄H₉ | CH₃ | O | C₂H₅ | i-C₃H₇-S- |
| Cl | OCH₃ | H | i-C₄H₉ | CH₃ | O | C₂H₅ | -C₃H₇-S- |
| Cl | OCH₃ | H | s-C₄H₉ | CH₃ | O | C₂H₅ | i-C₃H₇-S- |
| Cl | OCH₃ | H | t-C₄H₉ | CH₃ | O | C₂H₅ | i-C₃H₇-S- |
| Cl | OCH₃ | H | C₂H₅ | C₂H₅ | O | C₂H₅ | i-C₃H₇-S- |
| Cl | OCH₃ | H | C₃H₇ | C₃H₇ | O | C₂H₅ | i-C₃H₇-S- |
| Cl | OCH₃ | H | 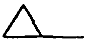 | CH₃ | O | C₂H₅ | i-C₃H₇-S- |
| Cl | OCH₃ | H | 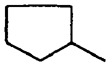 | CH₃ | O | C₂H₅ | i-C₃H₇-S- |
| Cl | OCH₃ | H | 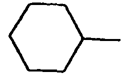 | CH₃ | O | C₂H₅ | i-C₃H₇-S- |
| Cl | H | 6-OCH₃ | CH₃ | H | O | C₂H₅ | i-C₃H₇-S- |
| Cl | H | 6-OCH₃ | C₂H₅ | H | O | C₂H₅ | i-C₃H₇-S- |
| Cl | H | 6-OCH₃ | C₃H₇ | H | O | C₂H₅ | i-C₃H₇-S- |
| Cl | H | 6-OCH₃ | i-C₃H₇ | H | O | C₂H₅ | i-C₃H₇-S- |
| Cl | H | 6-OCH₃ | C₄H₉ | H | O | C₂H₅ | i-C₃H₇-S- |
| Cl | H | 6-OCH₃ | i-C₄H₉ | H | O | C₂H₅ | i-C₃H₇-S- |
| Cl | H | 6-OCH₃ | s-C₄H₉ | H | O | C₂H₅ | i-C₃H₇-S- |
| Cl | H | 6-OCH₃ | t-C₄H₉ | H | O | C₂H₅ | i-C-₃H₇-S- |
| Cl | H | 6-OCH₃ | CH₃ | CH₃ | O | C₂H₅ | i-C₃H₇-S- |
| Cl | H | 6-OCH₃ | C₂H₅ | CH₃ | O | C₂H₅ | i-C₃H₇-S- |
| Cl | H | 6-OCH₃ | C₃H₇ | CH₃ | O | C₂H₅ | i-C₃H₇-S- |
| Cl | H | 6-OCH₃ | i-C₃H₇ | CH₃ | O | C₂H₅ | i-C₃H₇-S- |
| Cl | H | 6-OCH₃ | C₄H₉ | CH₃ | O | C₂H₅ | i-C₃H₇-S- |
| Cl | H | 6-OCH₃ | i-C₄H₉ | CH₃ | O | C₂H₅ | i-C₃H₇-S- |
| Cl | H | 6-OCH₃ | s-C₄H₉ | CH₃ | O | C₂H₅ | i-C₃H₇-S- |
| Cl | H | 6-OCH₃ | t-C₄H₉ | CH₃ | O | C₂H₅ | i-C₃H₇-S- |
| Cl | H | 6-OCH₃ | C₂H₅ | C₂H₅ | O | C₂H₅ | i-C₃H₇-S- |
| Cl | H | 6-OCH₃ | C₃H₇ | C₃H₇ | O | C₂H₅ | i-C₃H₇-S- |
| Cl | H | 6-OCH₃ | 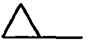 | CH₃ | O | C₂H₅ | i-C₃H₇-S- |
| Cl | H | 6-OCH₃ | 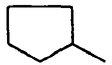 | CH₃ | O | C₂H₅ | i-C₃H₇-S- |
| Cl | H | 6-OCH₃ | 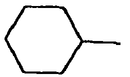 | CH₃ | O | C₂H₅ | i-C₃H₇-S- |
| OCH₃ | Cl | H | CH₃ | H | O | C₂H₅ | i-C₃H₇-S- |
| OCH₃ | Cl | H | C₂H₅ | H | O | C₂H₅ | i-C₃H₇-S- |
| OCH₃ | Cl | H | C₃H₇ | H | O | C₂H₅ | i-C₃H₇-S- |
| OCH₃ | Cl | H | i-C₃H₇ | H | O | C₂H₅ | i-C₃H₇-S- |
| OCH₃ | Cl | H | C₄H₉ | H | O | C₂H₅ | i-C₃H₇-S- |
| OCH₃ | Cl | H | i-C₄H₉ | H | O | C₂H₅ | i-C₃H₇-S- |
| OCH₃ | Cl | H | s-C₄H₉ | H | O | C₂H₅ | i-C₃H₇-S- |
| OCH₃ | Cl | H | t-C₄H₉ | H | O | C₂H₅ | i-C₃H₇-S- |
| OCH₃ | Cl | H | CH₃ | CH₃ | O | C₂H₅ | i-C₃H₇-S- |
| OCH₃ | Cl | H | C₂H₅ | CH₃ | O | C₂H₅ | i-C₃H₇-S- |
| OCH₃ | Cl | H | C₃H₇ | CH₃ | O | C₂H₅ | i-C₃H₇-S- |
| OCH₃ | Cl | H | i-C₃H₇ | CH₃ | O | C₂H₅ | i-C₃H₇-S- |
| OCH₃ | Cl | H | C₄H₉ | CH₃ | O | C₂H₅ | i-C₃H₇-S- |
| OCH₃ | Cl | H | i-C₄H₉ | CH₃ | O | C₂H₅ | i-C₃H₇-S- |
| OCH₃ | Cl | H | s-C₄H₉ | CH₃ | O | C₂H₅ | i-C₃H₇-S- |
| OCH₃ | Cl | H | t-C₄H₉ | CH₃ | O | C₂H₅ | i-C₃H₇-S- |
| OCH₃ | Cl | H | C₂H₅ | C₂H₅ | O | C₂H₅ | i-C₃H₇-S- |
| OCH₃ | Cl | H | C₃H₇ | C₃H₇ | O | C₂H₅ | i-C₃H₇-S- |
| OCH₃ | Cl | H | 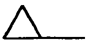 | CH₃ | O | C₂H₅ | i-C₃H₇-S- |
| OCH₃ | Cl | H | 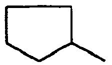 | CH₃ | O | C₂H₅ | i-C₃H₇-S- |
| OCH₃ | Cl | H | 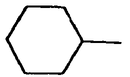 | CH₃ | O | C₂H₅ | i-C₃H₇-S- |
| Cl | OCH₃ | H | -(CH₂)₂- | | O | C₂H₅ | i-C₃H₇-S- |
| Cl | OCH₃ | H | -(CH₂)₄- | | O | C₂H₅ | i-C₃H₇-S- |
| Cl | OCH₃ | H | -(CH₂)₅- | | O | C₂H₅ | i-C₃H₇-S- |
| Cl | OCH₃ | H | -(CH₂)₆- | | O | C₂H₅ | i-C₃H₇-S- |
| Cl | OCH₃ | H | -(CH₂)₇- | | O | C₂H₅ | i-C₃H₇-S- |
| Cl | OCH₃ | H | -(CH₂)₂-O-(CH₂)₂- | | O | C₂H₅ | i-C₃H₇-S- |
| Cl | OCH₃ | H | -(CH₂)₂-S-(CH₂)₂- | | O | C₇H₅ | i-C₃H₇-S- |
| Cl | OCH₃ | H | -CH₂-CHCH₃-(CH₂)₃- | | O | C₂H₅ | i-C₃H₇-S- |
| Cl | OCH₃ | H | -(CH₂)₂-CHCH₃-(CH₂)₂- | | O | C₂H₅ | i-C₃H₇-S- |
| Cl | OCH₃ | H | -(CH₂)₂-CHC₂H₅-(CH₂)₂- | | O | C₂H₅ | i-C₃H₇-S- |
| Cl | OCH₃ | H | -(CH₂)₂-CHC₃H₇-(CH₂)₂- | | O | C₂H₅ | i-C₃H₇-S- |
| Cl | OCH₃ | H | -(CH₂)₂-CHi-C₃H₇-(CH₂)₂- | | O | C₂H₅ | i-C₃H₇-S- |
| Cl | OCH₃ | H | -(CH₂)₂-CHOCH₃-(CH₂)₂- | | O | C₂H₅ | i-C₃H₇-S- |
| Cl | OCH₃ | H | -(CH₂)₉-CHOC₂H₅-(CH₂)₂- | | O | C₂H₅ | i-C₃H₇-S- |
| Cl | OCH₃ | H | -(CH₂)₂-CHOC₃H₇-(CH₂)₂- | | O | C₂H₅ | i-C₃H₇-S- |
| Cl | OCH₃ | H | -(CH₂)₂-CHi-OC₃H₇-(CH₂)₂- | | O | C₂H₅ | i-C₃H₇-S- |
| Cl | OCH₃ | H | -(CH₂)₂-C(CH₃)₂-(CH₂)₂- | | O | C₂H₅ | i-C₃H₇-S- |
| Cl | OCH₃ | H | -CH₂-(CHCH₃)₂-(CH₂)₂- | | O | C₂H₅ | i-C₃H₇-S- |
| Cl | OCH₃ | H | 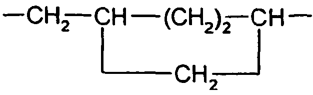 | | O | C₂H₅ | i-C₃H₇-S- |
| Cl | OCH₃ | H | 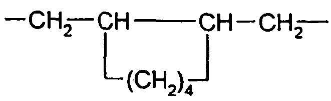 | | O | C₂H₅ | i-C₃H₇-S- |
| Cl | OCH₃ | H | 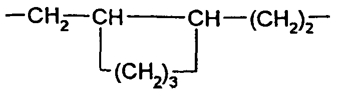 | | O | C₂H₅ | i-C₃H₇-S- |
| Cl | H | 6-OCH₃ | -(CH₂)₂- | | O | C₂H₅ | i-C₃H₇-S- |
| Cl | H | 6-OCH₃ | -(CH₂)₄- | | O | C₂H₅ | i-C₃H₇-S- |
| Cl | H | 6-OCH₃ | -(CH₂)₅- | | O | C₂H₅ | i-C₃H₇-S- |
| Cl | H | 6-OCH₃ | -(CH₂)₆- | | O | C₂H₅ | i-C₃H₇-S- |
| Cl | H | 6-OCH₃ | -(CH₂)₇- | | O | C₂H₅ | i-C₃H₇-S- |
| Cl | H | 6-OCH₃ | -(CH₂)₂-O-(CH₂)₂- | | O | C₂H₅ | i-C₃H₇-S- |
| Cl | H | 6-OCH₃ | -(CH₂)₂-S-(CH₂)₂- | | O | C₂H₅ | i-C₃H₇-S- |
| Cl | H | 6-OCH₃ | -CH₂-CHCH₃-(CH₂)₃- | | O | C₂H₅ | i-C₃H₇-S- |
| Cl | H | 6-OCH₃ | -(CH₂)₂-CHCH₃-(CH₂)₂- | | O | C₂H₅ | i-C₃H₇-S- |
| Cl | H | 6-OCH₃ | -(CH₂)₂-CHC₂H₅-(CH₂)₂- | | O | C₂H₅ | i-C₃H₇-S- |
| Cl | H | 6-OCH₃ | -(CH₂)₂-CHC₃H₇-(CH₂)₂- | | O | C₂H₅ | i-C₃H₇-S- |
| Cl | H | 6-OCH₃ | -(CH₂)₂-CHi-C₃H₇-(CH₂)₂- | | O | C₂H₅ | i-C₃H₇-S- |
| Cl | H | 6-OCH₃ | -(CH₂)₂-CHOCH₃-(CH₂)₂- | | O | C₂H₅ | i-C₃H₇-S- |
| Cl | H | 6-OCH₃ | -(CH₂)₂-CHOC₂H₅-(CH₂)₂- | | O | C₂H₅ | i-C₃H₇-S- |
| Cl | H | 6-OCH₃ | -(CH₂)₂-CHOC₃H₇-(CH₂)₂- | | O | C₂H₅ | i-C₃H₇-S- |
| Cl | H | 6-OCH₃ | -(CH₂)₂-CHi-OC₃H₇-(CH₂)₂- | | O | C₂H₅ | i-C₃H₇-S- |
| Cl | H | 6-OCH₃ | -(CH₂)₂-C(CH₃)₂-(CH₂)₂- | | O | C₂H₅ | i-C₃H₇-S- |
| Cl | H | 6-OCH₃ | -CH₂-(CHCH₃)₂-(CH₂)₂- | | O | C₂H₅ | i-C₃H₇-S- |
| Cl | H | 6-OCH₃ | 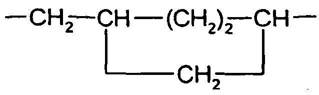 | | O | C₂H₅ | i-C₃H₇-S- |
| Cl | H | 6-OCH₃ | 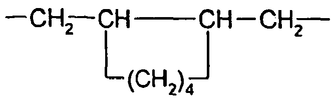 | | O | C₂H₅ | i-C₃H₇-S- |
| Cl | H | 6-OCH₃ | 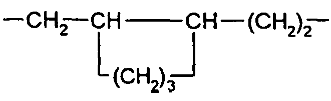 | | O | C₂H₅ | i-C₃H₇-S- |
| OCH₃ | Cl | H | -(CH₂)₂- | | O | C₂H₅ | i-C₃H₇-S- |
| OCH₃ | Cl | H | -(CH₂)₄- | | O | C₂H₅ | i-C₃H₇-S- |
| OCH₃ | Cl | H | -(CH₂)₅- | | O | C₂H₅ | i-C₃H₇-S- |
| OCH₃ | Cl | H | -(CH₂)₆- | | O | C₂H₅ | i-C₃H₇-S- |
| -OCH₃ | Cl | H | -(CH₂)₇- | | O | C₂H₅ | i-C₃H₇-S- |
| OCH₃ | Cl | H | -(CH₂)₂-O-(CH₂)₂- | | O | C₂H₅ | i-C₃H₇-S- |
| OCH₃ | Cl | H | -(CH₂)₂-S-(CH₂)₂- | | O | C₂H₅ | i-C₃H₇-S- |
| OCH₃ | Cl | H | -CH₂-CHCH₃-(CH₂)₃- | | O | C₂H₅ | i-C₃H₇-S- |
| OCH₃ | Cl | H | -(CH₂)₂-CHCH₃-(CH₂)₂- | | O | C₂H₅ | i-C₃H₇-S- |
| OCH₃ | Cl | H | -(CH₂)₂-CHC₂H₅-(CH₂)₂- | | O | C₂H₅ | i-C₃H₇-S- |
| OCH₃ | Cl | H | -(CH₂)₂-CHC₃H₇-(CH₂)₂- | | O | C₂H₅ | i-C₃H₇-S- |
| OCH₃ | Cl | H | -(CH₂)₂-CHi-C₃H₇-(CH₂)₂- | | O | C₂H₅ | i-C₃H₇-S- |
| OCH₃ | Cl | H | -(CH₂)₂-CHOCH₃-(CH₂)₂- | | O | C₂H₅ | i-C₃H₇-S- |
| OCH₃ | Cl | H | -(CH₂)₂-CHOC₂H₅-(CH₂)₂- | | O | C₂H₅ | i-C₃H₇-S- |
| OCH₃ | Cl | H | -(CH₂)₂-CHOC₂H₅-(CH₂)₂- | | O | C₂H₅ | i-C₃H₇-S- |
| OCH₃ | Cl | H | -(CH₂)₂-CHi-OC₃H₇-(CH₂)₂- | | O | C₂H₅ | i-C₃H₇-S- |
| OCH₃ | Cl | H | -(CH₂)₂-C(CH₃)₂-(CH₂)₂- | | O | C₂H₅ | i-C₃H₇-S- |
| OCH₃ | Cl | H | -CH₂-(CHCH₃)₂-(CH₂)₂- | | O | C₂H₅ | i-C₃H₇-S- |
| OCH₃ | Cl | H | 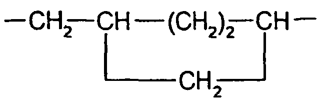 | | O | C₂H₅ | i-C₃H₇-S- |
| OCH₃ | Cl | H | 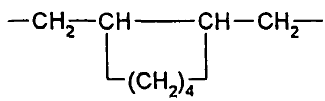 | | O | C₂H₅ | i-C₃H₇-S- |
| OCH₃ | Cl | H | 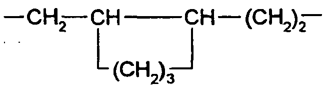 | | O | C₂H₅ | i-C₃H₇-S- |

Im einzelnen seien außer den bei den Herstellungsbeispielen genannten Verbindungen die folgenden Verbindungen der Formel (If-a) genannt:

**Tabelle 6a:**

| | | | | |
|---|---|---|---|---|
| 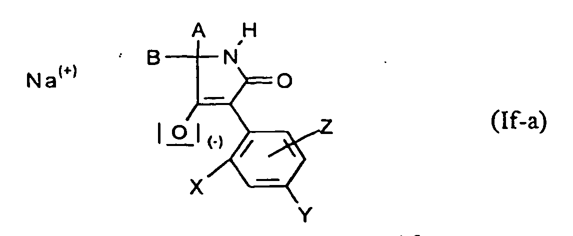 | | | | |

| X | Y | Z | A | B |
|---|---|---|---|---|
| Cl | OCH₃ | H | CH₃ | H |
| Cl | OCH₃ | H | C₂H₅ | H |
| Cl | OCH₃ | H | C₃H₇ | H |
| Cl | OCH₃ | H | i-C₃H₇ | H |
| Cl | OCH₃ | H | C₄H₉ | H |
| Cl | OCH₃ | H | i-C₄H₉ | H |
| Cl | OCH₃ | H | s-C₄H₉ | H |
| Cl | OCH₃ | H | t-C₄H₄ | H |
| Cl | OCH₃ | H | CH₃ | CH₃ |
| Cl | OCH₃ | H | C₂H₅ | CH₃ |
| Cl | OCH₃ | H | C₃H₇ | CH₃ |
| Cl | OCH₃ | H | i-C₃H₇ | CH₃ |
| Cl | OCH₃ | H | C₄H₉ | CH₃ |
| Cl | OCH₃ | H | i-C₄H₉ | CH₃ |
| Cl | OCH₃ | H | s-C₄H₉ | CH₃ |
| Cl | OCH₃ | H | t-C₄H₉ | CH₃ |
| Cl | OCH₃ | H | C₂H₅ | C₂H₅ |
| Cl | OCH₃ | H | C₃H₇ | C₃H₇ |
| Cl | OCH₃ | H | 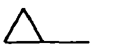 | CH₃ |
| Cl | OCH₃ | H | 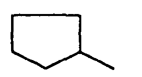 | CH₃ |
| Cl | OCH₃ | H | 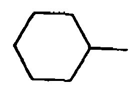 | CH₃ |
| Cl | H | 6-OCH₃ | CH₃ | H |
| Cl | H | 6-OCH₃ | C₂H₅ | H |
| Cl | H | 6-OCH₃ | C₃H₇ | H |
| Cl | H | 6-OCH₃ | i-C₃H₇ | H |
| Cl | H | 6-OCH₃ | C₄H₉ | H |
| Cl | H | 6-OCH₃ | i-C₄H₉ | H |
| Cl | H | 6-OCH₃ | s-C₄H₉ | H |
| Cl | H | 6-OCH₃ | t-C₄H₉ | H |
| Cl | H | 6-OCH₃ | CH₃ | CH₃ |
| Cl | H | 6-OCH₃ | C₂H₅ | CH₃ |
| Cl | H | 6-OCH₃ | C₃H₇ | CH₃ |
| Cl | H | 6-OCH₃ | i-C₃H₇ | CH₃ |
| Cl | H | 6-OCH₃ | C₄H₉ | CH₃ |
| Cl | H | 6-OCH₃ | i-C₄H₉ | CH₃ |
| Cl | H | 6-OCH₃ | s-C₄H₉ | CH₃ |
| Cl | H | 6-OCH₃ | t-C₄H₉ | CH₃ |
| Cl | H | 6-OCH₃ | C₂H₅ | C₂H₅ |
| Cl | H | 6-OCH₃ | C₃H₇ | C₃H₇ |
| Cl | H | 6-OCH₃ | 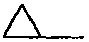 | CH₃ |
| Cl | H | 6-OCH₃ | 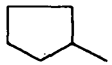 | CH₃ |
| Cl | H | 6-OCH₃ | 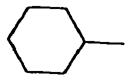 | CH₃ |
| OCH₃ | Cl | H | CH₃ | H |
| OCH₃ | Cl | H | C₂H₅ | H |
| OCH₃ | Cl | H | C₃H₇ | H |
| OCH₃ | Cl | H | i-C₃H₇ | H |
| OCH₃ | Cl | H | C₄H₉ | H |
| OCH₃ | Cl | H | i-C₄H₉ | H |
| OCH₃ | Cl | H | s-C₄H₉ | H |
| OCH₃ | Cl | H | t-C₄H₉ | H |
| OCH₃ | Cl | H | CH₃ | CH₃ |
| OCH₃ | Cl | H | C₂H₅ | CH₃ |
| OCH₃ | Cl | H | C₃H₇ | CH₃ |
| OCH₃ | Cl | H | i-C₃H₇ | CH₃ |
| OCH₃ | Cl | H | C₄H₉ | CH₃ |
| OCH₃ | Cl | H | i-C₄H₉ | CH₃ |
| OCH₃ | Cl | H | s-C₄H₉ | CH₃ |
| OCH₃ | Cl | H | t-C₄H₉ | CH₃ |
| OCH₃ | Cl | H | C₂H₅ | C₂H₅ |
| OCH₃ | Cl | H | C₃H₇ | C₃H₇ |
| OCH₃ | Cl | H | 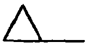 | CH₃ |
| OCH₃ | Cl | H | 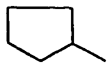 | CH₃ |
| OCH₃ | Cl | H | 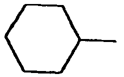 | CH₃ |
| Cl | OCH₃ | H | -(CH₂)₂- | |
| Cl | OCH₃ | H | -(CH₂)₄- | |
| Cl | OCH₃ | H | -(CH₂)₅- | |
| Cl | OCH₃ | H | -(CH₂)₆- | |
| Cl | OCH₃ | H | -(CH₂)₆- | |
| Cl | OCH₃ | H | -(CH₂)₂-O-(CH₂)₂- | |
| Cl | OCH₃ | H | -(CH₂)₂-S-(CH₂)₂- | |
| Cl | OCH₃ | H | -CH₂-CHCH₃-(CH₂)₃- | |
| Cl | OCH₃ | H | -(CH₂)₂-CHCH₃-(CH₂)₂- | |
| Cl | OCH₃ | H | -(CH₂)₂-CHC₂H₅-(CH₂)₂- | |
| Cl | OCH₃ | H | -(CH₂)₂-CHC₃H₇-(CH₂)₂- | |
| Cl | OCH₃ | H | -(CH₂)₂-CHi-C₃H₇-(CH₂)₂- | |
| Cl | OCH₃ | H | -(CH₂)₂-CHOCH₃-(CH₂)₂- | |
| Cl | OCH₃ | H | -(CH₂)₂-CHOC₂H₅-(CH₂)₂- | |
| Cl | OCH₃ | H | -(CH₂)₂-CHOC₃H₇-(CH₂)₂- | |
| Cl | OCH₃ | H | -(CH₂)₂-CHi-OC₃H₇-(CH₂)₂- | |
| Cl | OCH₃ | H | -(CH₂)₂-C(CH₃)₂-(CH₂)₂- | |
| Cl | OCH₃ | H | -CH₂-(CHCH₃)₂-(CH₂)₂- | |
| Cl | OCH₃ | H | 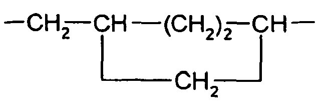 | |
| Cl | OCH₃ | H | 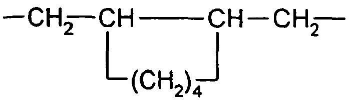 | |
| Cl | OCH₃ | H | 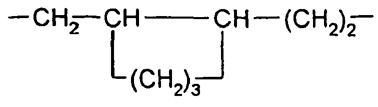 | |
| Cl | H | -OCH₃ | -(CH₂)₂- | |
| Cl | H | 6-OCH₃ | -(CH₂)₄- | |
| Cl | H | 6-OCH₃ | -(CH₂)₅- | |
| Cl | H | 6-OCH₃ | -(CH₂)₆- | |
| Cl | H | 6-OCH₃ | -(CH₂)₇- | |
| Cl | H | 6-OCH₃ | -(CH₂)₂-O-(CH₂)₂- | |
| Cl | H | 6-OCH₃ | -(CH₂)₂-S-(CH₂)₂- | |
| Cl | H | 6-OCH₃ | -CH₂-CHCH₃-(CH₂)₃- | |
| Cl- | H | 6-OCH₃ | -(CH₂)₂-CHCH₃-(CH₂)₂- | |
| Cl | H | 6-OCH₃ | -(CH₂)₂-CHC₂H₅-(CH₂)₂- | |
| Cl | H | 6-OCH₃ | -(CH₂)₂-CHC₃H₇-(CH₂)₂- | |
| Cl | H | 6-OCH₃ | -(CH₂)₂-CHi-C₃H₇-(CH₂)₂- | |
| Cl | H | 6-OCH₃ | -(CH₂)₂-CHOCH₃-(CH₂)₇- | |
| Cl | H | 6-OCH₃ | -(CH₂)₂-CHOC₂H₅-(CH₂)₂- | |
| Cl | H | 6-OCH₃ | -(CH₂)₂-CHOC₃H₇-(CH₂)₂- | |
| Cl | H | 6-OCH₃ | -(CH₂)₂-CHi-OC₃H₇-(CH₂)₂- | |
| Cl | H | 6-OCH₃ | -(CH₂)₂-C(CH₃)₂-(CH₂)₂- | |
| Cl | H | 6-OCH₃ | -CH₂-(CHCH₃)₂-(CH₂)₂- | |
| Cl | H | 6-OCH₃ | 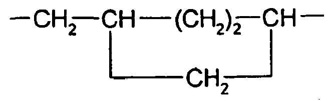 | |
| Cl | H | 6-OCH₃ | 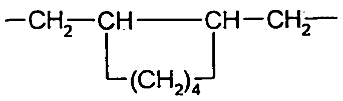 | |
| Cl | H | 6-OCH₃ | 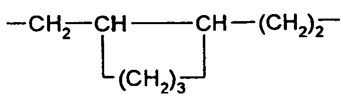 | |
| OCH₃ | Cl | H | -(CH₂)₂- | |
| OCH₃ | Cl | H | -(CH₂)₄- | |
| OCH₃ | Cl | H | -(CH₂)₅- | |
| OCH₃ | Cl | H | -(CH₂)₆- | |
| OCH₃ | Cl | H | -(CH₂)₇- | |
| OCH₃ | Cl | H | -(CH₂)₂-O-(CH₂)₂- | |
| OCH₃ | Cl | H | -(CH₂)₂-S-(CH₂)₂- | |
| OCH₃ | Cl | H | -CH₂-CHCH₃-(CH₂)₃- | |
| OCH₃ | Cl | H | -(CH₂)₂-CHCH₃-(CH₂)₂- | |
| OCH₃ | Cl | H | -(CH₂)₂-CHC₂H₅-(CH₂)₂- | |
| OCH₃ | Cl | H | -(CH₂)₂-CHC₃H₇-(CH₂)₂- | |
| OCH₃ | Cl | H | -(CH₂)₂-CHi-C₃H₇-(CH₂)₂- | |
| OCH₃ | Cl | H | -(CH₂)₂-CHOC₃-(CH₂)₂- | |
| OCH₃ | Cl | H | -(CH₂)₂-CHOC₂H₅-(CH₂)₂- | |
| OCH₃ | Cl | H | -(CH₂)₂-CHOC₃H₇-(CH₂)₂- | |
| OCH₃ | Cl | H | -(CH₂)₂-CHi-OC₃H₇-(CH₂)₂- | |
| OCH₃ | Cl | H | -(CH₂)₂-C(CH₃)₂-(CH₂)₂- | |
| OCH₃ | Cl | H | -CH₂-(CHCH₃)₂-(CH₂)₂- | |
| OCH₃ | Cl | H | 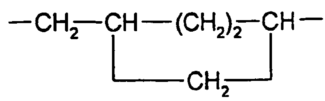 | |
| OCH₃ | Cl | H | 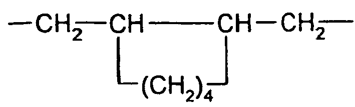 | |
| OCH₃ | Cl | H | 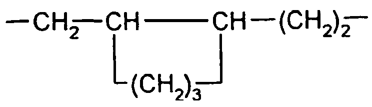 | |

Im einzelnen seien außer den bei den Herstellungsbeispielen genannten Verbindungen die folgenden Verbindungen der Formel (If-b) genannt:

**Tabelle 6b:**

| | | | | |
|---|---|---|---|---|
| 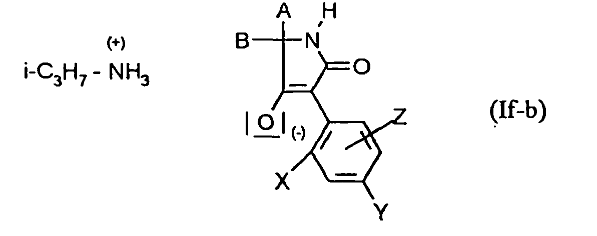 | | | | |

| X | Y | Z | A | B |
|---|---|---|---|---|
| Cl | OCH₃ | H | CH₃ | H |
| Cl | OCH₃ | H | C₂H₅ | H |
| Cl | OCH₃ | H | C₃H₇ | H |
| Cl | OCH₃ | H | i-C₃H₇ | H |
| Cl | OCH₃ | H | C₄H9 | H |
| Cl | OCH₃ | H | i-C₄H₉ | H |
| Cl | OCH₃ | H | s-C₄H₉ | H |
| Cl | OCH₃ | H | t-C₄H₉ | H |
| Cl | OCH₃ | H | CH₃ | CH₃ |
| Cl | OCH₃ | H | C₂H₅ | CH₃ |
| Cl | OCH₃ | H | C₃H₇ | CH₃ |
| Cl | OCH₃ | H | i-C₃H₇ | CH₃ |
| Cl | OCH₃ | H | C₄H₉ | CH₃ |
| Cl | OCH₃ | H | i-C₄H₉ | CH₃ |
| Cl | OCH₃ | H | s-C₄H₉ | CH₃ |
| Cl | OCH₃ | H | t-C₄H₉ | CH₃ |
| Cl | OCH₃ | H | C₂H₅ | C₂H₅ |
| Cl | OCH₃ | H | C₃H₇ | C₃H₇ |
| Cl | OCH₃ | H | 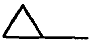 | CH₃ |
| Cl | OCH₃ | H | 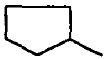 | CH₃ |
| Cl | OCH₃ | H | 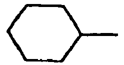 | CH₃ |
| Cl | H | 6-OCH₃ | CH₃ | H |
| Cl | H | 6-OCH₃ | C₂H₅ | H |
| Cl | H | 6-OCH₃ | C₃H₇ | H |
| Cl | H | 6-OCH₃ | i-C₃H₇ | H |
| Cl | H | 6-OCH₃ | C₄H₉ | H |
| Cl | H | 6-OCH₃ | i-C₄H₉ | H |
| Cl | H | 6-OCH₃ | s-C₄H₉ | H |
| Cl | H | 6-OCH₃ | t-C₄H₉ | H |
| Cl | H | 6-OCH₃ | CH₃ | CH₃ |
| Cl | H | 6-OCH₃ | C₂H₅ | CH₃ |
| Cl | H | 6-OCH₃ | C₃H₇ | CH₃ |
| Cl | H | 6-OCH₃ | i-C₃H₇ | CH₃ |
| Cl | H | 6-OCH₃ | C₄H₉ | CH₃ |
| Cl | H | 6-OCH₃ | i-C₄H₉ | CH₃ |
| Cl | H | 6-OCH₃ | s-C₄H₉ | CH₃ |
| Cl | H | 6-OCH₃ | t-C₄H₉ | CH₃ |
| Cl | H | 6-OCH₃ | C₂H₅ | C₂H₅ |
| Cl | H | 6-OCH₃ | C₃H₇ | C₃H₇ |
| Cl | H | 6-OCH₃ | 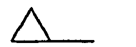 | CH₃ |
| Cl | H | 6-OCH₃ | 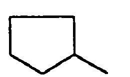 | CH₃ |
| Cl | H | 6-OCH₃ | 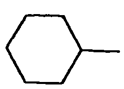 | CH₃ |
| OCH₃ | Cl | H | CH₃ | H |
| OCH₃ | Cl | H | C₂H₅ | H |
| OCH₃ | Cl | H | C₃H₇ | H |
| OCH₃ | Cl | H | i-C₃H₇ | H |
| OCH₃ | Cl | H | C₄H₉ | H |
| OCH₃ | Cl | H | i-C₄H₉ | H |
| OCH₃ | Cl | H | s-C₄H₉ | H |
| -OCH₃ | Cl | H | t-C₄H₉ | H |
| OCH₃ | Cl | H | CH₃ | CH₃ |
| OCH₃ | Cl | H | C₂H₅ | CH₃ |
| OCH₃ | Cl | H | C₃H₇ | CH₃ |
| OCH₃ | Cl | H | i-C₃H₇ | CH₃ |
| OCH₃ | Cl | H | C₄H₉ | CH₃ |
| OCH₃ | Cl | H | i-C₄H₉ | CH₃ |
| OCH₃ | Cl | H | s-C₄H₉ | CH₃ |
| OCH₃ | Cl | H | t-C₄H₃ | CH₃ |
| OCH₃ | Cl | H | C₂H₅ | C₂H₅ |
| OCH₃ | Cl | H | C₃H₇ | C₃H₇ |
| OCH₃ | Cl | H | 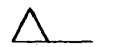 | CH₃ |
| OCH₃ | Cl | H | 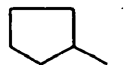 | CH₃ |
| OCH₃ | Cl | H | 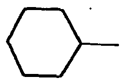 | CH₃ |
| Cl | OCH₃ | H | -(CH₂)₂- | |
| Cl | OCH₃ | H | -(CH₂)₄- | |
| Cl | OCH₃ | H | -(CH₂)₅- | |
| Cl | OCH₃ | H | -(CH₂)₆- | |
| Cl | OCH₃ | H | -(CH₂)₇- | |
| Cl | OCH₃ | H | -(CH₂)₂-O-(CH₂)₂- | |
| Cl | OCH₃ | H | -(CH₂)₂-S-(CH₂)₂- | |
| Cl | OCH₃ | H | -CH₂-CHCH₃-(CH₂)₃- | |
| Cl | OCH₃ | H | -(CH₂)₂-CHCH₃-(CH₂)₂- | |
| Cl | OCH₃ | H | -(CH₂)₂-CHC₂H₅-(CH₂)₂- | |
| Cl | OCH₃ | H | -(CH₂)₂-CHC₃H₇-(CH₂)₂- | |
| Cl | OCH₃ | H | -(CH₂)₂-CHi-C₃H₇-(CH₂)₂- | |
| Cl | OCH₃ | H | -(CH₂)₂-CHOCH₃-(CH₂)₂- | |
| Cl | OCH₃ | H | -(CH₂)₂-CHOC₂H₅-(CH₂)₂- | |
| Cl | OCH₃ | H | -(CH₂)₂-CHOC₃H₇-(CH₂)₂- | |
| Cl | OCH₃ | H | -(CH₂)₂-CHi-OC₃H₇-(CH₂)₂- | |
| Cl | OCH₃ | H | -(CH₂)₂-C(CH₃)₂-(CH₂)₂- | |
| Cl | OCH₃ | H | -CH₂-(CHCH₃)₂-(CH₂)₂- | |
| Cl | OCH₃ | H | 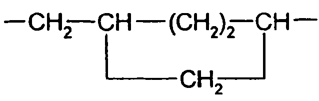 | |
| Cl | OCH₃ | H | 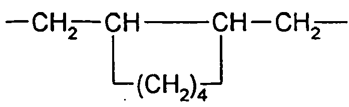 | |
| Cl | OCH₃ | H | 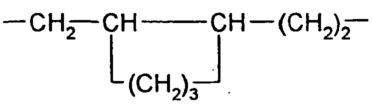 | |
| Cl | H | 6-OCH₃ | -(CH₂)₂- | |
| Cl | H | 6-OCH₃ | -(CH₂)₄- | |
| Cl | H | 6-OCH₃ | -(CH₂)₅- | |
| Cl | H | 6-OCH₃ | -(CH₂)₆- | |
| Cl | H | 6-OCH₃ | -(CH₂)₇- | |
| Cl | H | 6-OCH₃ | -(CH₂)₂-O-(CH₂)₂- | |
| Cl | H | 6-OCH₃ | -(CH₂)₂-S-(CH₂)₂- | |
| Cl | H | 6-OCH₃ | -CH₂-CHCH₃-(CH₂)₃- | |
| Cl | H | 6-OCH₃ | -(CH₂)₂-CHCH₃-(CH₂)₂- | |
| Cl | H | 6-OCH₃ | -(CH₂)₂-CHC₂H₅-(CH₂)₂- | |
| Cl | H | 6-OCH₃ | -(CH₂)₂-CHC₃H₇-(CH₂)₂- | |
| Cl | H | 6-OCH₃ | -(CH₂)₂-CHi-C₃H₇-(CH₂)₂- | |
| Cl | H | 6-OCH₃ | -(CH₂)₂-CHOCH₃-(CH₂)₂- | |
| Cl | H | 6-OCH₃ | -(CH₂)₂-CHOC₂H₅-(CH₂)₂- | |
| Cl | H | 6-OCH₃ | -(CH₂)₂-CHOC₃H₇-(CH₂)₂- | |
| Cl | H | 6-OCH₃ | -(CH₂)₂-CHi-OC₃H₇-(CH₂)₂- | |
| Cl | H | 6-OCH₃ | -(CH₂)₂-C(CH₃)₂-(CH₂)₂- | |
| Cl | H | 6-OCH₃ | -CH₂-(CHCH₃)₂-(CH₂)₂- | |
| Cl | H | 6-OCH₃ | 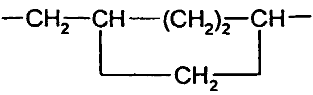 | |
| Cl | H | 6-OCH₃ | 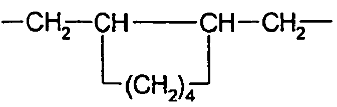 | |
| Cl | H | 6-OCH₃ | 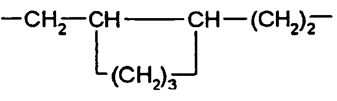 | |
| OCH₃ | Cl | H | -(CH₂)₂- | |
| OCH₃ | Cl | H | -(CH₂)₄- | |
| OCH₃ | Cl | H | -(CH₂)₅- | |
| OCH₃ | Cl | H | -(CH₂)₆- | |
| OCH₃ | Cl | H | -(CH₂)₇- | |
| OCH₃ | Cl | H | -(CH₂)₂-O-(CH₂)₂- | |
| OCH₃ | Cl | H | -(CH₂)-₂-S-(CH₂)₂- | |
| OCH₃ | Cl | H | -CH₂-CHCH₃-(CH₂)₃- | |
| OCH₃ | Cl | H | -(CH₂)₂-CHCH₃-(CH₂)₂- | |
| OCH₃ | Cl | H | -(CH₂)2-CHC₂H₅-(CH₂)₂- | |
| OCH₃ | Cl | H | -(CH₂)₂-CHC₃H₇-(CH₂)₂- | |
| OCH₃ | Cl | H | -(CH₂)₂-CHi-C₃H₇-(CH₂)₂- | |
| OCH₃ | Cl | H | -(CH₂)₂-CHOCH₃-(CH₂)₂- | |
| OCH₃ | Cl | H | -(CH₂)₂-CHOC₂H₅-(CH₂)₂- | |
| OCH₃ | Cl | H | -(CH₂)₂-CHOC₃H₇-(CH₂)₂- | |
| OCH₃ | Cl | H | -(CH₂)₂-CHi-OC₃H₇-(CH₂)₂- | |
| OCH₃ | Cl | H | -(CH₂)₂-C(CH₃)₂-(CH₂)₂- | |
| OCH₃ | Cl | H | -CH₂-(CHCH₃)₂-(CH₂)₂- | |
| OCH₃ | Cl | H | 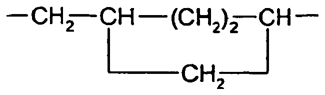 | |
| OCH₃ | Cl | H | 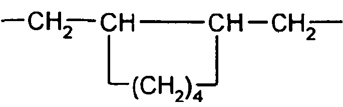 | |
| OCH₃ | Cl | H | 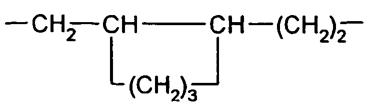 | |

Im einzelnen seien außer den bei den Herstellungsbeispielen genannten Verbindungen die folgenden Verbindungen der Formel (Ig-a) genannt:

**Tabelle 7a:**

| | | | | |
|---|---|---|---|---|
| 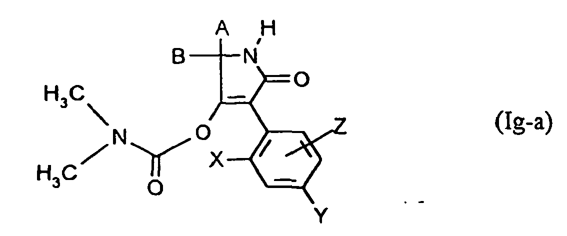 | | | | |

| X | Y | Z | A | B |
|---|---|---|---|---|
| Cl | OCH₃ | H | CH₃ | H |
| Cl | OCH₃ | H | C₃H₅ | H |
| Cl | OCH₃ | H | C₃H₇ | H |
| Cl | OCH₃ | H | i-C₃H₇ | H |
| Cl | OCH₃ | H | C₄H₉ | H |
| Cl | OCH₃ | H | i-C₄H₉ | H |
| Cl | OCH₃ | H | s-C₄H₉ | H |
| Cl | OCH₃ | H | t-C₄H₉ | H |
| Cl | OCH₃ | H | CH₃ | CH₃ |
| Cl | OCH₃ | H | C₂H₃ | CH₃ |
| Cl | OCH₃ | H | C₃H₇ | CH₃ |
| Cl | OCH₃ | H | i-C₃H₇ | CH₃ |
| Cl | OCH₃ | H | C₄H₉ | CH₃ |
| Cl | OCH₃ | H | i-C₃H₉ | CH₃ |
| Cl | OCH₃ | H | s-C₄H₉ | CH₃ |
| Cl | OCH₃ | H | t-C₄H₉ | CH₃ |
| Cl | OCH₃ | H | C₂H₅ | C₂H₅ |
| Cl | OCH₃ | H | C₃H₇ | C₃H₇ |
| Cl | OCH₃ | H | 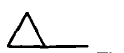 | CH₃ |
| Cl | OCH₃ | H | 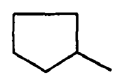 | CH₃ |
| Cl | OCH₃ | H | 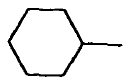 | CH₃ |
| Cl | H | 6-OCH₃ | CH₃ | H |
| Cl | H | 6-OCH₃ | C₂H₅ | H |
| Cl | H | 6-OCH₃ | C₃H₇ | H |
| Cl | H | 6-OCH₃ | i-C₃H₇ | H |
| Cl | H | 6-OCH₃ | C₄H₉ | H |
| Cl | H | 6-OCH₃ | i-C₄H₉ | H |
| Cl | H | 6-OCH₃ | s-C₄H₉ | H |
| Cl | H | 6-OCH₃ | t-C₄H₉ | H |
| Cl | H | 6-OCH₃ | CH₃ | CH₃ |
| Cl | H | 6-OCH₃ | C₂H₅ | CH₃ |
| Cl | H | 6-OCH₃ | C₃H₇ | CH₃ |
| Cl | H | 6-OCH₃ | i-C₃H₇ | CH₃ |
| Cl | H | 6-OCH₃ | C₄H₉ | CH₃ |
| Cl | H | 6-OCH₃ | i-C₄H₉ | CH₃ |
| Cl | H | 6-OCH₃ | s-C₄H₉ | CH₃ |
| Cl | H | 6-OCH₃ | t-C₄H₉ | CH₃ |
| Cl | H | 6-OCH₃ | C₂H₅ | C₂H₃ |
| Cl | H | 6-OCH₃ | C₃H₇ | C₃H₇ |
| Cl | H | 6-OCH₃ | 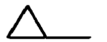 | CH₃ |
| Cl | H | 6-OCH₃ | 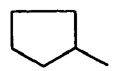 | CH₃ |
| Cl | H | 6-OCH₃ | 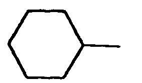 | CH₃ |
| OCH₃ | Cl | H | CH₃ | H |
| OCH₃ | Cl | H | C₂H₅ | H |
| OCH₃ | Cl | H | C₃H₇ | H |
| OCH₃ | Cl | H | i-C₃H₇ | H |
| OCH₃ | Cl | H | C₄H₉ | H |
| OCH₃ | Cl | H | i-C₄H₉ | H |
| OCH₃ | Cl | H | s-C₄H₉ | H |
| OCH₃ | Cl | H | t-C₄H₉ | H |
| OCH₃ | Cl | H | CH₃ | CH₃ |
| OCH₃ | Cl | H | C₂H₅ | CH₃ |
| OCH₃ | Cl | H | C₃H₇ | CH₃ |
| OCH₃ | Cl | H | i-C₃H₇ | CH₃ |
| OCH₃ | Cl | H | C₄H₉ | CH₃ |
| OCH₃ | Cl | H | i-C₄H₉ | CH₃ |
| OCH₃ | Cl | H | s-C₄H₉ | CH₃ |
| OCH₃ | Cl | H | t-C₄H₉ | CH₃ |
| OCH₃ | Cl | H | C₂H₅ | C₂H₅ |
| OCH₃ | Cl | H | C₃H₇ | C₃H₇ |
| OCH₃ | Cl | H | 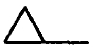 | CH₃ |
| OCH₃ | Cl | H | 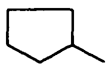 | CH₃ |
| OCH₃ | Cl | H | 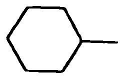 | CH₃ |
| Cl | OCH₃ | H | -(CH₂)₂- | |
| Cl | OCH₃ | H | -(CH₂)₄- | |
| Cl | OCH₃ | H | -(CH₂)₅- | |
| Cl | OCH₃ | H | -(CH₂)₆- | |
| Cl | OCH₃ | H | -(CH₂)₇- | |
| Cl | OCH₃ | H | -(CH₂)₂-O-(CH₂)₂- | |
| Cl | OCH₃ | H | -(CH₂)₂-S-(CH₂)₂- | |
| Cl | OCH₃ | H | -CH₂-CHCH₃-(CH₂)₃- | |
| Cl | OCH₃ | H | -(CH₂)₂-CHCH₃-(CH₂)₂- | |
| Cl | OCH₃ | H | -(CH₂)₂-CHC₂H₅-(CH₂)₂- | |
| Cl | OCH₃ | H | -(CH₂)₂-CHC₃H₇-(CH₂)₂- | |
| Cl | OCH₃ | H | -(CH₂)₂-CHi-C₃H₇-(CH₂)₂- | |
| Cl | OCH₃ | H | -(CH₂)₂-CHOCH₃-(CH₂)₂- | |
| Cl | OCH₃ | H | -(CH₂)₂-CHOC₂H₅-(CH₂)₂- | |
| Cl | OCH₃ | H | -(CH₂)₂-CHOC₃H₇-(CH₂)₂- | |
| Cl | OCH₃ | H | -(CH₂)₂-CHi-OC₃H₇-(CH₂)₂- | |
| Cl | OCH₃ | H | -(CH₂)₂-C(CH₃)₂-(CH₂)₂- | |
| Cl | OCH₃ | H | -CH₂-(CHCH₃)₂-(CH₂)₂- | |
| Cl | OCH₃ | H | 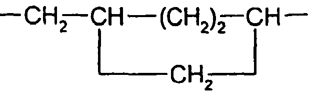 | |
| Cl | OCH₃ | H | 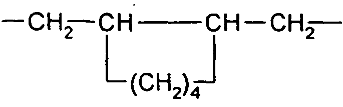 | |
| Cl | OCH₃ | H | 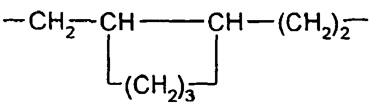 | |
| Cl | H | 6-OCH₃ | -(CH₂)₂- | |
| Cl | H | 6-OCH₃ | -(CH₂)₄- | |
| Cl | H | 6-OCH₃ | -(CH₂)₅- | |
| Cl | H | 6-OCH₃ | -(CH₂)₆- | |
| Cl | H | 6-OCH₃ | -(CH₂)₇- | |
| Cl | H | 6-OCH₃ | -(CH₂)₂-O-(CH₂)₂- | |
| Cl | H | 6-OCH₃ | -(CH₂)₂-S-(CH₂)₂- | |
| Cl | H | 6-OCH₃ | -CH₂-CHCH₃-(CH₂)₃- | |
| Cl | H | 6-OCH₃ | -(CH₂)₂-CHCH₃-(CH₂)₂- | |
| Cl | H | 6-OCH₃ | -(CH₂)₂-CHC₂H₅-(CH₂)₂- | |
| Cl | H | 6-OCH₃ | -(CH₂)₂-CHC₃H₇-(CH₂)_{2µ}- | |
| Cl | H | 6-OCH₃ | -(CH₂)₂-CHi-C₃H₇-(CH₂)₂- | |
| Cl | H | 6-OCH₃ | -(CH₂)₂-CHOCH₃-(CH₂)₂- | |
| Cl | H | 6-OCH₃ | -(CH₂)₂-CHOC₂H₅-(CH₂)2- | |
| Cl | H | 6-OCH₃ | -(CH₂)₂-CHOC₃H₇-(CH₂)₂- | |
| Cl | H | 6-OCH₃ | -(CH₂)₂-CHi-OC₃H₇-(CH₂)₂- | |
| Cl | H | 6-OCH₃ | -(CH₂)₂C(CH₃)₂-(CH₂)₂- | |
| Cl | H | 6-OCH₃ | -CH₂-(CHCH₃)₂-(CH₂)₂- | |
| Cl | H | 6-OCH₃ | 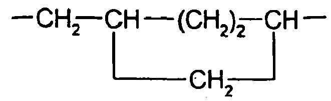 | |
| Cl | H | 6-OCH₃ | 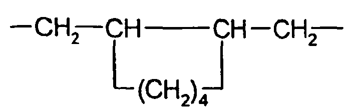 | |
| Cl | H | 6-OCH₃ | 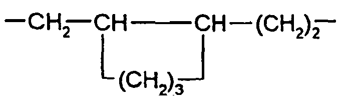 | |
| OCH₃ | Cl | H | -(CH₂)₂- | |
| OCH₃ | Cl | H | -(CH₂)₄- | |
| OCH₃ | Cl | H | -(CH₂)₅- | |
| OCH₃ | Cl | H | -(CH₂)₆- | |
| OCH₃ | Cl | H | -(CH₂)₇- | |
| OCH₃ | Cl | H | -(CH₂)₂-O-(CH₂)₂- | |
| OCH₃ | Cl | H | -(CH₂)₂-S-(CH₂)₂- | |
| OCH₃ | Cl | H | -CH₂-CHCH₃-(CH₂)₃- | |
| OCH₃ | Cl | H | -(CH₂)₂-CHCH₃-(CH₂)₂- | |
| OCH₃ | Cl | H | -(CH₂)₂-CHC₂H₅-(CH₂)₂- | |
| OCH₃ | Cl | H | -(CH₂)₂-CHC₃H₇-(CH₂)₂- | |
| OCH₃ | Cl | H | -(CH₂)₂-CHi-C₃H₇-(CH₂)₂- | |
| OCH₃ | Cl | H | -(CH₂)₂-CHOCH₃-(CH₂)₂- | |
| OCH₃ | Cl | H | -(CH₂)₂-CHOC₂H₅-(CH₂)₂- | |
| OCH₃ | Cl | H | -(CH₂)₂-CHOC₃H₇-(CH₂)₂- | |
| OCH₃ | Cl | H | -(CH₂)₂-CHi-OC₃H₇-(CH₂)₂- | |
| OCH₃ | Cl | H | -(CH₂)₂-C(CH₃)₂-(CH₂)₂- | |
| OCH₃ | Cl | H | -CH₂-(CHCH₃)₂-(CH₂)₂- | |
| OCH₃ | Cl | H | 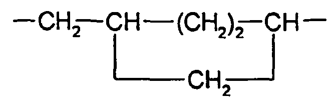 | |
| OCH₃ | Cl | H | 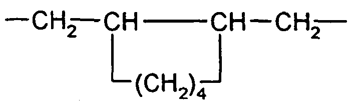 | |
| OCH₃ | Cl | H | 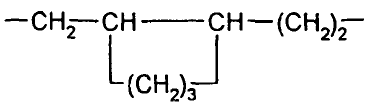 | |

Im einzelnen seien außer den bei den Herstellungsbeispielen genannten Verbindungen die folgenden Verbindungen der Formel (Ig-b) genannt:

**Tabelle 7b:**

| | | | | |
|---|---|---|---|---|
| 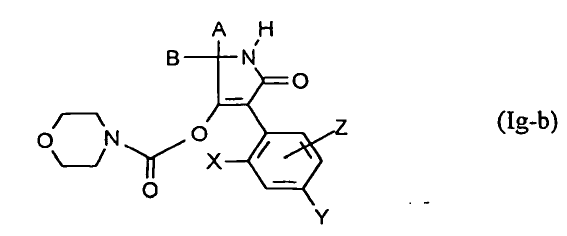 | | | | |

| X | Y | Z | A | B |
|---|---|---|---|---|
| Cl | OCH₃ | H | CH₃ | H |
| Cl | OCH₃ | H | C₂H₅ | H |
| Cl | OCH₃ | H | C₃H₇ | H |
| Cl | OCH₃ | H | i-C₃H₇ | H |
| Cl | OCH₃ | H | C₄H₉ | H |
| Cl | OCH₃ | H | i-C₄H₉ | H |
| Cl | OCH₃ | H | s-C₄H₉ | H |
| Cl | OCH₃ | H | t-C₄H₉ | H |
| Cl | OCH₃ | H | CH₃ | CH₃ |
| Cl | OCH₃ | H | C₂H₅ | CH₃ |
| Cl | OCH₃ | H | C₃H₇ | CH₃ |
| Cl | OCH₃ | H | i-C₃H₇ | CH₃ |
| Cl | OCH₃ | H | C₄H₉ | CH₃ |
| Cl | OCH₃ | H | i-C₄H₉ | CH₃ |
| Cl | OCH₃ | H | s-C₄H₉ | CH₃ |
| Cl | OCH₃ | H | t-C₄H₉ | CH₃ |
| Cl | OCH₃ | H | C₂H₅ | C₂H₅ |
| Cl | OCH₃ | H | C₃H₇ | C₃H₇ |
| Cl | OCH₃ | H | 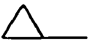 | CH₃ |
| Cl | OCH₃ | H | 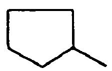 | CH₃ |
| Cl | OCH₃ | H | 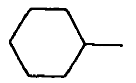 | CH₃ |
| Cl | H | 6-OCH₃ | CH₃ | H |
| Cl | H | 6-OCH₃ | C₇H₅ | H |
| Cl | H | 6-OCH₃ | C₃H₇ | H |
| Cl | H | 6-OCH₃ | i-C₃H₇ | H |
| Cl | H | 6-OCH₃ | C₄H₉ | H |
| Cl | H | 6-OCH₃ | i-C₄H₉ | H |
| Cl | H | 6-OCH₃ | s-C₄H₉ | H |
| Cl | H | 6-OCH₃ | t-C₄H₉ | H |
| Cl | H | 6-OCH₃ | CH₃ | CH₃ |
| Cl | H | 6-OCH₃ | C₂H₅ | CH₃ |
| Cl | H | 6-OCH₃ | C₃H₇ | CH₃ |
| Cl | H | 6-OCH₃ | i-C₃H₇ | CH₃ |
| Cl | H | 6-OCH₃ | C₄H₉ | CH₃ |
| Cl | H | 6-OCH₃ | i-C₄H₉ | CH₃ |
| Cl | H | 6-OCH₃ | s-C₄H₉ | CH₃ |
| Cl | H | 6-OCH₃ | t-C₄H₉ | CH₃ |
| Cl | H | 6-OCH₃ | C₂H₅ | C₂H₅ |
| Cl | H | 6-OCH₃ | C₃H₇ | C₃H₇ |
| Cl | H | 6-OCH₃ | 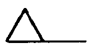 | CH₃ |
| Cl | H | 6-OCH₃ | 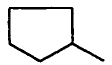 | CH₃ |
| Cl | H | 6-OCH₃ | 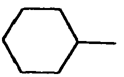 | CH₃ |
| OCH₃ | Cl | H | CH₃ | H |
| OCH₃ | Cl | H | C₂H₅ | H |
| OCH₃ | Cl | H | C₃H₇ | H |
| OCH₃ | Cl | H | i-C₃H₇ | H |
| OCH₃ | Cl | H | C₄H₉ | H |
| OCH₃ | Cl | H | i-C₄H₉ | H |
| OCH₃ | Cl | H | s-C₄H₉ | H |
| -OCH₃ | Cl | H | t-C₄H₉ | H |
| OCH₃ | Cl | H | CH₃ | CH₃ |
| OCH₃ | Cl | H | C₂H₅ | CH₃ |
| OCH₃ | Cl | H | C₃H₇ | CH₃ |
| OCH₃ | Cl | H | i-C₃H₇ | CH₃ |
| OCH₃ | Cl | H | C₄H₉ | CH₃ |
| OCH₃ | Cl | H | i-C₄H₉ | CH₃ |
| OCH₃ | Cl | H | s-C₄H₉ | CH₃ |
| OCH₃ | Cl | H | t-C₄H₉ | CH₃ |
| OCH₃ | Cl | H | C₂H₅ | C₂H₅ |
| OCH₃ | Cl | H | C₃H₇ | C₃H₇ |
| OCH₃ | Cl | H | 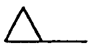 | CH₃ |
| OCH₃ | Cl | H | 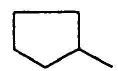 | CH₃ |
| OCH₃ | Cl | H | 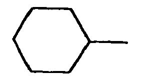 | CH₃ |
| Cl | OCH₃ | H | -(CH₂)₂- | |
| Cl | OCH₃ | H | -(CH₂)₄- | |
| Cl | OCH₃ | H | -(CH₂)₅- | |
| Cl | OCH₃ | H | -(CH₂)₆- | |
| Cl | OCH₃ | H | -(CH₂)₇- | |
| Cl | OCH₃ | H | -(CH₂)₂-O-(CH₂)₂- | |
| Cl | OCH₃ | H | -(CH₂)₂-S-(CH₂)₂- | |
| Cl | OCH₃ | H | -CH₂-CHCH₃-(CH₂)₃- | |
| Cl | OCH₃ | H | -(CH₂)₂-CHCH-₃-(CH₂)₂- | |
| Cl | OCH₃ | H | -(CH₂)₂-CHC₂H₅-(CH₂)₂- | |
| Cl | OCH₃ | H | -(CH₂)₂-CHC₃H₇-(CH₂)₂- | |
| Cl | OCH₃ | H | -(CH₂)₂-CHi-C₃H₇-(CH₂)₂- | |
| Cl | OCH₃ | H | -(CH₂)₂-CHOCH₃-(CH₂)₂- | |
| Cl | OCH₃ | H | -(CH₂)₂-CHOC₂H₅-(CH₂)₂- | |
| Cl | OCH₃ | H | -(CH₂)₂-CHOC₃H₇-(CH₂)₂- | |
| Cl | OCH₃ | H | -(CH₂)₂-CHi-OC₃H₇-(CH₂)₂- | |
| Cl | OCH₃ | H | -(CH₂)₂-C(CH₃)₂-(CH₂)₂- | |
| Cl | OCH₃ | H | -CH₂-(CHCH₃)₂-(CH₂)₂- | |
| Cl | OCH₃ | H | 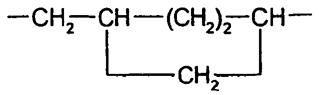 | |
| Cl | OCH₃ | H | 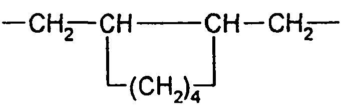 | |
| Cl | OCH₃ | H | 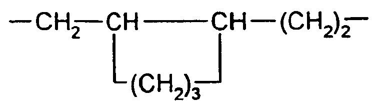 | |
| Cl | H | 6-OCH₃ | -(CH₂)₂- | |
| Cl | H | 6-OCH₃ | -(CH₂)₄- | |
| Cl | H | 6-OCH₃ | -(CH₂)₅- | |
| Cl | H | 6-OCH₃ | -(CH₂)₆- | |
| Cl | H | 6-OCH₃ | -(CH₂)₇- | |
| Cl | H | 6-OCH₃ | -(CH₂)₂-O-(CH₂)₂- | |
| Cl | H | 6-OCH₃ | -(CH₂)₂-S-(CH₂)₂- | |
| Cl | H | 6-OCH₃ | -CH₂-CHCH₃-(CH₂)₃- | |
| Cl | H | 6-OCH₃ | -(CH₂)₂-CHCH₃-(CH₂)₂- | |
| Cl | H | 6-OCH₃ | -(CH₂)₂-CHC₂H₅-(CH₂)₂- | |
| Cl | H | 6-OCH₃ | -(CH₂)₂-CHC₃H₇-(CH₂)₂- | |
| Cl | H | 6-OCH₃ | -(CH₂)₂-CHi-C₃H₇-(CH₂)₂- | |
| Cl | H | 6-OCH₃ | -(CH₂)₂-CHOCH₃-(CH₂)₂- | |
| Cl | H | 6-OCH₃ | -(CH₂)₂-CHOC₂H₅-(CH₂)₂- | |
| Cl | H | 6-OCH₃ | -(CH₂)₂-CHOC₃H₇-(CH₂)₂- | |
| Cl | H | 6-OCH₃ | -(CH₂)₂-CHi-OC₃H₇-(CH₂)₂- | |
| Cl | H | 6-OCH₃ | -(CH₂)₂-C(CH₃)₂-(CH₂)₂- | |
| Cl | H | 6-OCH₃ | -CH₂-(CHCH₃)₂-(CH₂)₂- | |
| Cl | H | 6-OCH₃ | 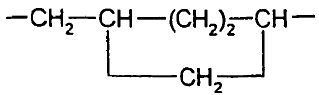 | |
| Cl | H | 6-OCH₃ | 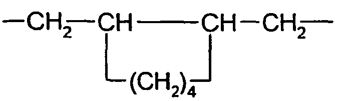 | |
| Cl | H | 6-OCH₃ | 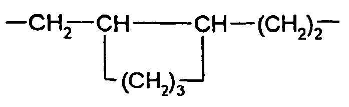 | |
| OCH₃ | Cl | H | -(CH₂)₂- | |
| OCH₃ | Cl | H | -(CH₂)₄- | |
| OCH₃ | Cl | H | -(CH₂)₅- | |
| OCH₃ | Cl | H | -(CH₂)₆- | |
| OCH₃ | Cl | H | -(CH₂)₇- | |
| OCH₃ | Cl | H | -(CH₂)₂-O-(CH₂)₂- | |
| OCH₃ | Cl | H | -(CH₂)₂-S-(CH₂)₂- | |
| OCH₃ | Cl | H | -CH₂-CHCH₄-(CH₂)₃- | |
| OCH₃ | Cl | H | 3-(CH₂)₂-CHCH₃-(CH₂)₂- | |
| OCH₃ | Cl | H | -(CH₂)₂-CHC₂H₅-(CH₂)₂- | |
| OCH₃ | Cl | H | -(CH₂)₂-CHC₃H₇-(CH₂)₂- | |
| OCH₃ | Cl | H | -(CH₂)₂-CHi-C₃H₇-(CH₂)₂- | |
| OCH₃ | Cl | H | -(CH₂)₂-CHOCH₃-(CH₂)₂- | |
| OCH₃ | Cl | H | -(CH₂)₂-CHOC₂H₅-(CH₂)₂- | |
| OCH₃ | Cl | H | -(CH₂)₂-CHOC₃H₇-(CH₂)₂- | |
| OCH₃ | Cl | H | -(CH₂)₂-CHi-OC₃H₇-(CH₂)₂- | |
| OCH₃ | Cl | H | -(CH₂)₂-C(CH₃)₂-(CH₂)₂- | |
| OCH₃ | Cl | H | -CH₂-(CHCH₃)₂-(CH₂)₂- | |
| OCH₃ | Cl | H | 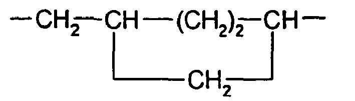 | |
| OCH₃ | Cl | H | 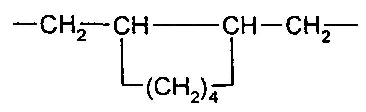 | |
| OCH₃ | Cl | H | 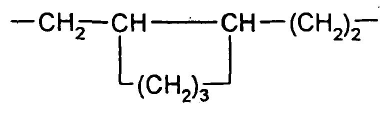 | |

Verwendet man gemäß Verfahren (A_{α}) N-(2-Chlor-4-methoxyphenylacetyl)-1-amino-4-ethyl-cyclohexan-carbonsäureethylester als Ausgangsstoff, so kann der Verlauf des erfindungsgemäßen Verfahrens durch folgendes Reaktionsschema wiedergegeben werden:

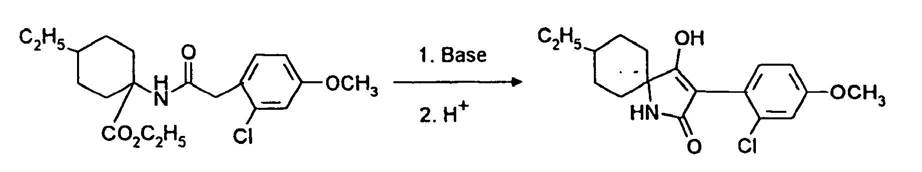

Verwendet man gemäß Verfahren (A_{β}) N-(2-Chlor-4-fluorphenylacetyl)-2-amino-2.3-dimethyl-buttersäuremethylester als Ausgangsstoff, so kann der Verlauf des erfindungsgemäßen Verfahrens durch folgendes Reaktionsschema wiedergegeben werden:

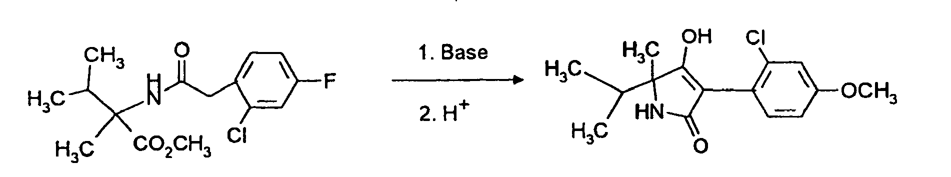

Verwendet man gemäß Verfahren (B_{α}) 3-(2-Chlor-6-methoxyphenyl)-5,5-dimethyl-pyrrolidin-2,4-dion und Pivaloylchlorid als Ausgangsstoffe, so kann der Verlauf des erfindungsgemäßen Verfahrens durch folgendes Reaktionsschema wiedergegeben werden:

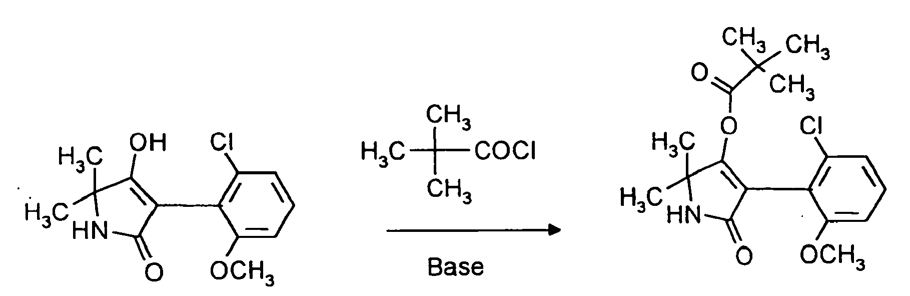

Verwendet man gemäß Verfahren (B_{β}) 3-(2-Brom-6-methoxyphenyl)-5-isopropyl-5-methyl-pyrrolidin-2,4-dion und Acetanhydrid als Ausgangsverbindungen, so kann der Verlauf des erfindungsgemäßen Verfahrens durch folgendes Reaktionsschema wiedergegeben werden:

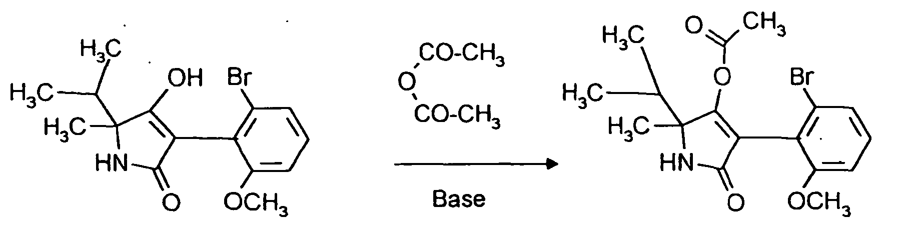

Verwendet man gemäß Verfahren (C) 3-(2-Methoxy-4-chlor-phenyl)-5,5-diethyl-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

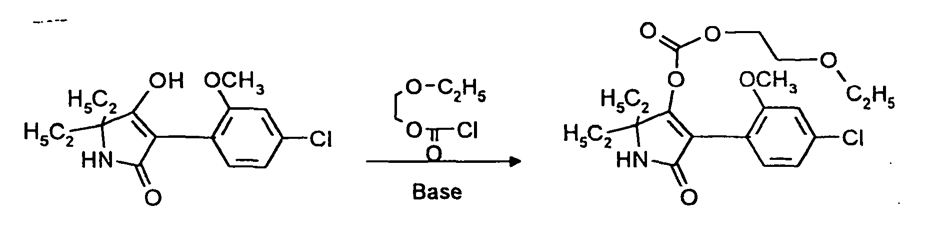

Verwendet man gemäß Verfahren (D_{α}) 3-(2-Chlor-4-methoxyphenyl)-5.5-pentamethylen-pyrrolidin-2,4-dion und Chlormonothioameisensäuremehylester als Ausgangsprodukte, so kann der Reaktionsverlauf wie folgt wiedergegeben werden:

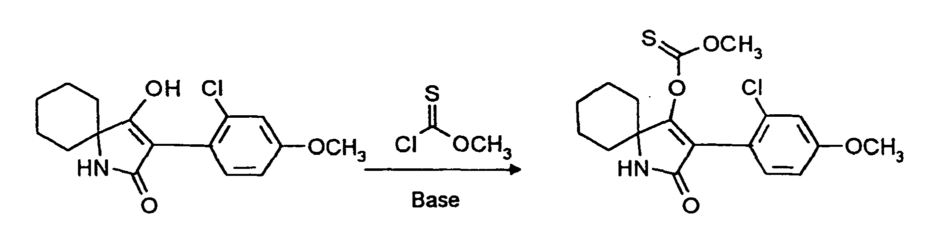

Verwendet man gemäß Verfahren (D_{β}) 3-(2-Brom-4-ethoxy-phenyl)-5,5-ethylmer-captoethyl-pyrrolidin-2,4-dion, Schwefelkohlenstoff und Methyliodid als Ausgangskomponenten, so kann der Reaktionsverlauf wie folgt wiedergegeben werden:

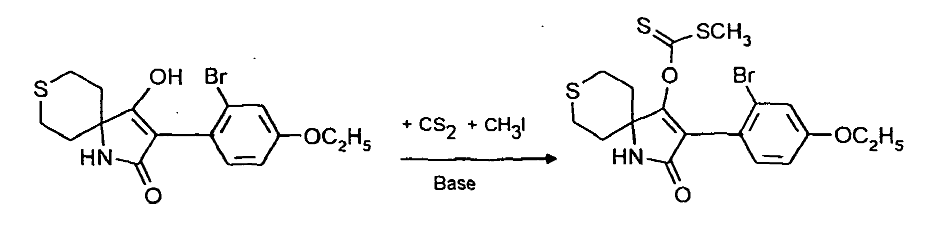

Verwendet man gemäß Verfahren (E) 3-(2-Chlor-4-isöpropoxy-phenyl)-5.5-(2-methyl)-pentamethylen-pyrrolidin-2,4-dion und Methansulfonsäurechlorid als Ausgangsprodukt, so kann der Reaktionsverlauf durch folgendes Reaktionsschema wiedergegeben werden:

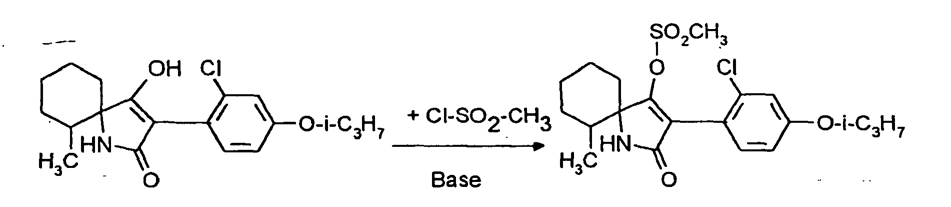

Verwendet man gemäß Verfahren (F) 3-(2-Methoxy-4-chlorphenyl)-5-isobutyl-5-methyl-pyrrolidin-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

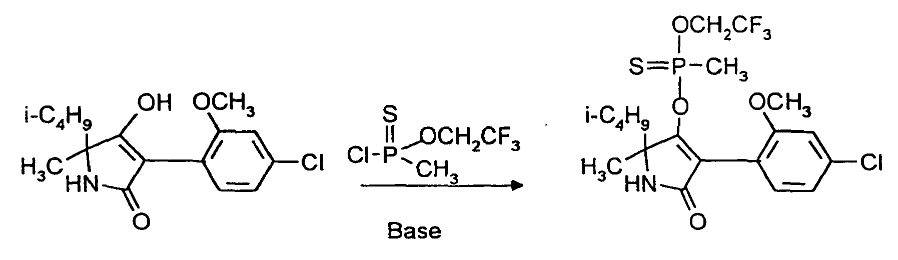

Verwendet man gemäß Verfahren (G) 3-(2,4-Dimethoxyphenyl)-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

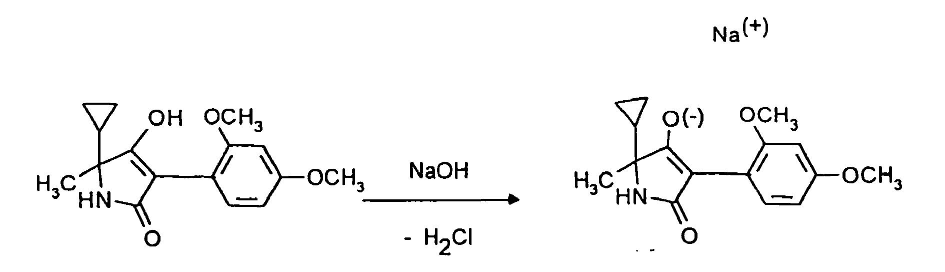

Verwendet man gemäß Verfahren (H_{α}) 3-(2-Chlor-4-ethoxyphenyl)-5,5-hexamethylen-pyrrolidin-2,4-dion und Ethylisocyanat als Ausgangsprodukte, so kann der Reaktionsverlauf durch folgendes Schema wiedergegeben werden:

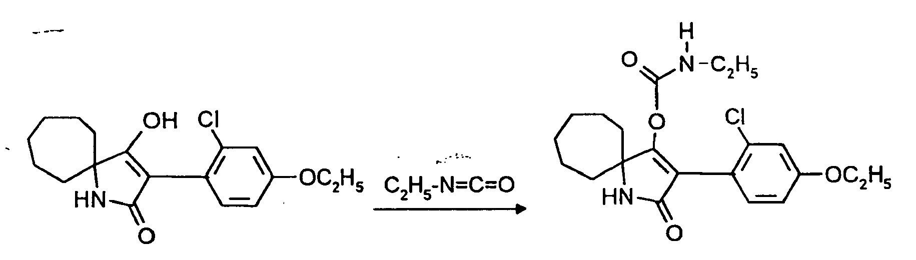

Verwendet man gemäß Verfahren (H_{β}) 3-(2-Methoxy-4-chlorphenyl)-5-methylpyrrolidin-2,4-dion und Dimethylcarbamidsäurechlorid als Ausgangsprodukte, so kann der Reaktionsverlauf durch folgendes Schema wiedergegeben werden:

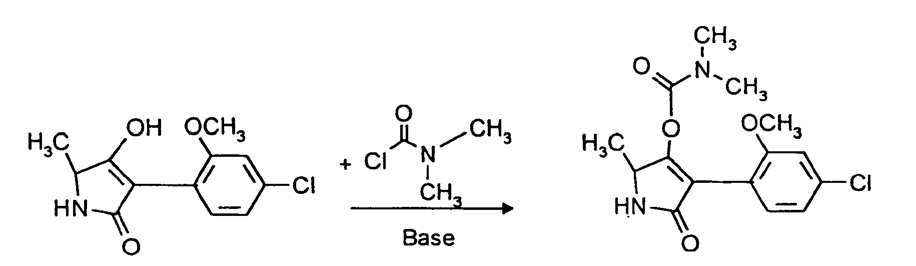

Die bei den erfindungsgemäßen Verfahren (A) als Ausgangsstoffe benötigten Verbindungen der Formel (II) 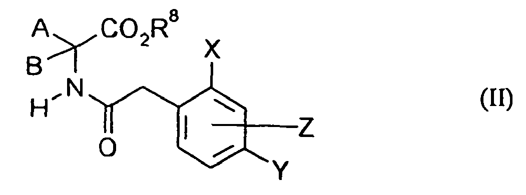 in welcher
A, B, X, Y, Z und R⁸ die oben angegebene Bedeutung haben,
sind neu.

Man erhält z.B. Acyl-aminosäureester der Formel (II), wenn man
Aminosäurederivate der Formel (XIV), 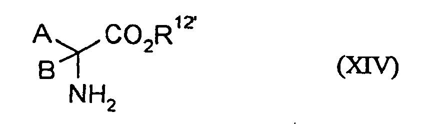 in welcher
R^{12'} für Wasserstoff (XIVa) oder Alkyl (XIVb) steht
und
A und B die oben angegebene Bedeutung haben,
mit Phenylessigsäurehalogeniden der Formel (XV) 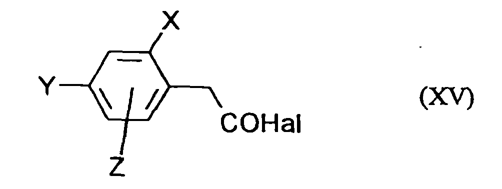 in welcher
X, Y und Z die oben angegebene Bedeutung haben und
Hal für Chlor oder Brom steht,
acyliert (Chem. Reviews 52, 237-416 (1953); Bhattacharya, Indian J. Chem. 6, 341-5, 1968) und die dabei für R^{12'} = Wasserstoff entstandenen
Acylaminosäuren der Formel (IIa), 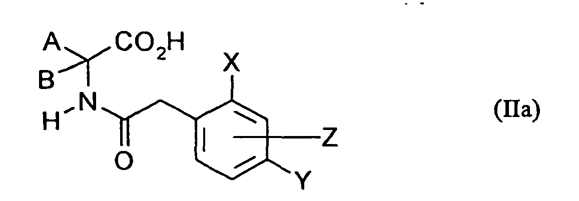 in welcher
A, B, X, Y und Z die oben angegebene Bedeutung haben,
verestert (Chem. Ind. (London) 1568 (1968)).

Die substituierten cyclischen Aminocarbonsäuren der Formel (XIVa) sind im allgemeinen nach der Bucherer-Bergs-Reaktion oder nach der Strecker-Synthese erhältlich und fallen dabei jeweils in unterschiedlichen Isomerenformen an. So erhält man nach den Bedingungen der Bucherer-Bergs-Reaktion vorwiegend die Isomeren (im folgenden der Einfachheit halber β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 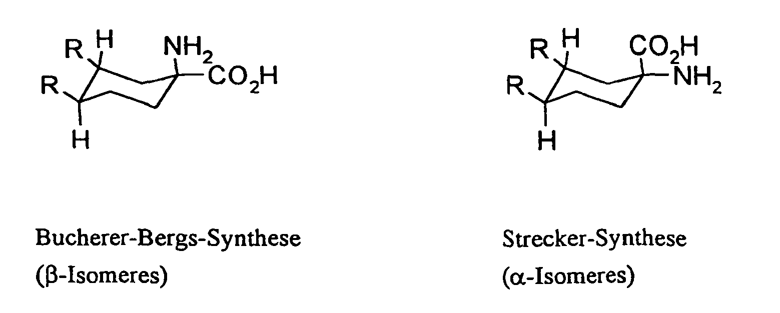 (L. Munday, J. Chem. Soc. 4372 (1961); J.T. Eward, C. Jitrangeri, Can. J. Chem. 53, 3339 (1975).

Weiterhin lassen sich die bei den obigen Verfahren (A) verwendeten Ausgangsstoffe der Formel (II) 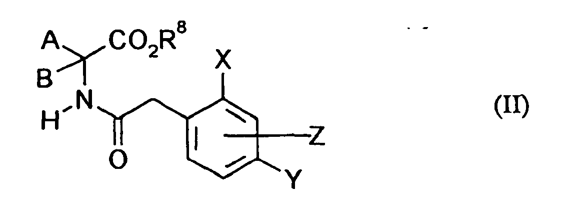 in welcher
A, B, X, Y, Z und R⁸ die oben angegebene Bedeutung haben,
herstellen, wenn man Aminonitrile der Formel (XVI) 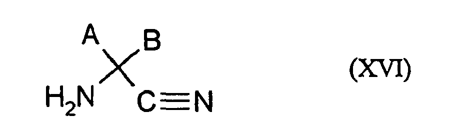 in welcher
A und B die oben angegebene Bedeutung haben,
mit Phenylessigsäurehalogeniden der Formel (XV) 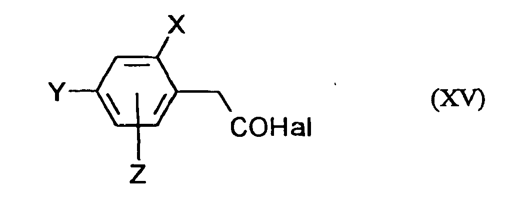 in welcher
X, Y und Z die oben angegebene Bedeutung haben und
Hal für Chlor oder Brom steht,
zu Verbindungen der Formel (XVII) 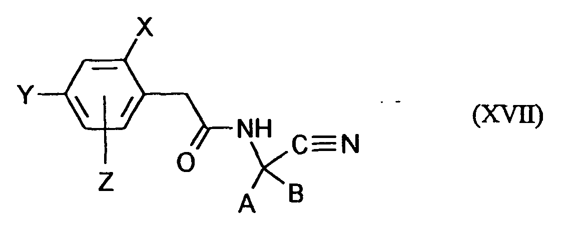 in welcher
A, B, X, Y und Z die oben angegebene Bedeutung haben,
umsetzt, und diese anschließend einer schwefelsauren Alkoholyse unterwirft.

Die Verbindungen der Formel (XVII) sind ebenfalls neu.

Beispielhaft aber nicht begrenzend seien außer den bei den Herstellungsbeispielen genannten Zwischenprodukten die folgenden Verbindungen der Formel (II) genannt:
N-(2-Chlor-4-methoxyphenylacetyl)-alanin-methylester
N-(2-Chlor-4-methoxyphenylacetyl)-leucin-methylester
N-(2-Chlor-4-methoxyphenylacetyl)-isoleucin-methylester
N-(2-Chlor-4-methoxyphenylacetyl)-valin-methylester
N-(2-Chlor-4-methoxyphenylacetyl)-aminoisobuttersäure-methylester
N-(2-Chlor-4-methoxyphenylacetyl)-2-ethyl-2-aminobuttersäure-methylester
N-(2-Chlor-4-methoxyphenylacetyl)-2-methyl-2-aminovaleriansäure-methylester
N-(2-Chlor-4-methoxyphenylacetyl)-2,3-dimethyl-2-aminovaleriansäure-methylester
N-(2-Chlor-4-methoxyphenylacetyl)-1-amino-cyclopentancarbonsäure-methylester
N-(2-Chlor-4-methoxyphenylacetyl)-1-amino-cyclohexancarbonsäure-methylester
N-(2-Chlor-4-methoxyphenylacetyl)-1-amino-cycloheptancarbonsäure-methylester
N-(2-Chlor-4-methoxyphenylacetyl)-1-amino-cyclooktancarbonsäure-methylester
N-(4-Chlor-2-methoxyphenylacetyl)-alanin-methylester
N-(4-Chlor-2-methoxyphenylacetyl)-leucin-methylester
N-(4-Chlor-2-methoxyphenylacetyl)-isoleucin-methylester
N-(4-Chlor-2-methoxyphenylacetyl)-valin-methylester
N-(4-Chlor-2-methoxyphenylacetyl)-aminoisobuttersäure-methylester
N-(4-Chlor-2-methoxyphenylacetyl)-2-ethyl-2-aminobuttersäure-methylester
N-(4-Chlor-2-methoxyphenylacetyl)-2-methyl-2-aminovaleriansäure-methylester
N-(4-Chlor-2-methoxyphenylacetyl)-2,3-dimethyl-2-aminovaleriansäure-methylester
N-(4-Chlor-2-methoxyphenylacetyl)-1-amino-cyclopentancarbonsäure-methylester
N-(4-Chlor-2-methoxyphenylacetyl)-1-amino-cyclohexancarbonsäure-methylester
N-(4-Chlor-2-methoxyphenylacetyl)-1-amino-cycloheptancarbonsäure-methylester
N-(4-Chlor-2-metboxyphenylacetyl)-1-amino-cyclooktancarbonsäure-methylester
N-(2-Chlor-6-methoxyphenylacetyl)-alanin-methylester
N-(2-Chlor-6-methoxyphenylacetyl)-leucin-methylester
N-(2-Chlor-6-methoxyphenylacetyl)-isoleucin-methylester
N-(2-Chlor-6-methoxyphenylacetyl)-valin-methylester
N-(2-Chlor-6-methoxyphenylacetyl)-aminoisobuttersäure-methylester
N-(2-Chlor-6-methoxyphenylacetyl)-2-ethyl-2-aminobuttersäure-methylester
N-(2-Chlor-6-methoxyphenylacetyl)-2-methyl-2-aminovaleriansäure-methylester
N-(2-Chlor-6-methoxyphenylacetyl)-2,3-dimethyl-2-aminovaleriansäure-methylester
N-(2-Chlor-6-methoxyphenylacetyl)-1-amino-cyclopentancarbonsäure-methylester
N-(2-Chlor-6-methoxyphenylacetyl)-1-amino-cyclohexancarbonsäure-methylester
N-(2-Chlor-6-methoxyphenylacetyl)-1-amino-cycloheptancarbonsäure-methylester
N-(2-Chlor-6-methoxyphenylacetyl)-1-amino-cyclooktancarbonsäure-methylester
N-(2-Chlor-4-methoxy-phenylacetyl)-1-amino-2-methyl-cyclohexancarbonsäure-methylester,
N-(2-Chlor-4-methoxy-phenylacetyl)-1-amino-3-methyl-cyclohexancarbonsäure-methylester,
N-(2-Chlor-4-methoxy-phenylacetyl)-1-amino-4-methyl-cyclohexancarbonsäure-methylester,
N-(2-Chlor-4-methoxy-phenylacetyl)-1-amino-3,4-dimethyl-cyclohexancarbonsäure-methylester,
N-(2-Chlor-4-methoxy-phenylacetyl)-1-amino-4-ethyl-cyclohexancarbonsäure-methylester,
N-(2-Chlor-4-methoxy-phenylacetyl)-1-amino-4-isopropyl-cyclohexancarbonsäure-methylester,
N-(2-Chlor-4-methoxy-phenylacetyl)-1-amino-4-tert.-butyl-cyclohexancarbonsäure-methylester,
N-(2-Chlor-4-methoxy-phenylacetyl)-1-amino-4-methoxy-cyclohexancarbonsäure-methylester,
N-(2-Chlor-6-methoxy-phenylacetyl)-1-amino-2-methyl-cyclohexancarbonsäure-methylester,
N-(2-Chlor-6-methoxy-phenylacetyl)-1-amino-3-methyl-cyclohexancarbonsäure-methylester,
N-(2-Chlor-6-methoxy-phenylacetyl)-1-amino-4-methyl-cyclohexancarbonsäure-methylester,
N-(2-Chlor-6-methoxy-phenylacetyl)-1-amino-3,4-dimethyl-cyclohexancarbonsäure-methyl ester,
N-(2-Chlor-6-methoxy-phenylacetyl)-1-amino-4-ethyl-cyclohexancarbonsäure-methylester,
N-(2-Chlor-6-methoxy-phenylacetyl)-1-amino-4-isopropyl-cyclohexancarbonsäure-methylester,
N-(2-Chlor-6-methoxy-phenylacetyl)-1-amino-4-tert.-butyl-cyclohexancarbonsäure-methylester,
N-(2-Chlor-6-methoxy-phenylacetyl)-1-amino-4-methoxy-cyclohexancarbonsäure-methylester,
N-(4-Chlor-2-methoxy-phenylacetyl)-1-amino-2-methyl-cyclohexancarbonsäure-methylester,
N-(4-Chlor-2-methoxy-phenylacetyl)-1-amino-3-methyl-cyclohexancarbonsäure-methylester,
N-(4-Chlor-2-methoxy-phenylacetyl)-1-amino-4-methyl-cyclohexancarbonsäure-methylester,
N-(4-Chlor-2-methoxy-phenylacetyl)-1-amino-3,4-dimethyl-cyclohexancarbonsäure-methylester,
N-(4-Chlor-2-methoxy-phenylacetyl)-1-amino-4-ethyl-cyclohexancarbonsäure-methylester,
N-(4-Chlor-2-methoxy-phenylacetyl)-1-amino-4-isopropyl-cyclohexancarbonsäure-methylester,
N-(4-Chlor-2-methoxy-phenylacetyl)-1-amino-4-tert.-butyl-cyclohexancarbonsäure-methylester,
N-(4-Chlor-2-methoxy-phenylacetyl)-1-amino-4-methoxy-cyclohexancarbonsäure-methylester,

Beispielhaft, aber nicht begrenzend, seien außer den bei den Herstellungsbeispielen genannten Zwischenprodukten die folgenden Verbindungen der Formel (IIa) genannt:
N-(2-Chlor-4-methoxyphenylacetyl)-alanin
N-(2-Chlor-4-methoxyphenylacetyl)-leucin
N-(2-Chlor-4-methoxyphenylacetyl)-isoleucin
N-(2-Chlor-4-methoxyphenylacetyl)-valin
N-(2-Chlor-4-methoxyphenylacetyl)-aminoisobuttersäure
N-(2-Chlor-4-methoxyphenylacetyl)-2-ethyl-2-aminobuttersäure
N-(2-Chlor-4-methoxyphenylacetyl)-2-methyl-2-aminovaleriansäure
N-(2-Chlor-4-methoxyphenylacetyl)-2,3-dimethyl-2-aminovaleriansäure
N-(2-Chlor-4-methoxyphenylacetyl)-1-amino-cyclopentancarbonsäure
N-(2-Chlor-4-methoxyphenylacetyl)-1-amino-cyclohexancarbonsäure
N-(2-Chlor-4-methoxyphenylacetyl)-1-amino-cycloheptancarbonsäure
N-(2-Chlor-4-methoxyphenylacetyl)-1-amino-cyclooktancarbonsäure
N-(4-Chlor-2-methoxyphenylacetyl)-alanin
N-(4-Chlor-2-methoxyphenylacetyl)-leucin
N-(4-Chlor-2-methoxyphenylacetyl)-isoleucin
N-(4-Chlor-2-methoxyphenylacetyl)-valin
N-(4-Chlor-2-methoxyphenylacetyl)-aminoisobuttersäure
N-(4-Chlor-2-methoxyphenylacetyl)-2-ethyl-2-aminobuttersäure
N-(4-Chlor-2-methoxyphenylacetyl)-2-methyl-2-aminovaleriansäure
N-(4-Chlor-2-methoxyphenylacetyl)-2,3-dimethyl-2-aminovaleriansäure
N-(4-Chlor-2-methoxyphenylacetyl)-1-amino-cyclopentancarbonsäure
N-(4-Chlor-2-methoxyphenylacetyl)-1-amino-cyclohexancarbonsäure
N-(4-Chlor-2-methoxyphenylacetyl)-1-amino-cycloheptancarbonsäure
N-(4-Chlor-2-methoxyphenylacetyl)-1-amino-cyclooktancarbonsäure
N-(2-Chlor-6-methoxyphenylacetyl)-alanin
N-(2-Chlor-6-methoxyphenylacetyl)-leucin
N-(2-Chlor-6-methoxyphenylacetyl)-isoleucin
N-(2-Chlor-6-methoxyphenylacetyl)-valin
N-(2-Chlor-6-methoxyphenylacetyl)-aminoisobuttersäure
N-(2-Chlor-6-methoxyphenylacetyl)-2-ethyl-2-aminobuttersäure
N-(2-Chlor-6-methoxyphenylacetyl)-2-methyl-2-aminovaleriansäure
N-(2-Chlor-6-methoxyphenylacetyl)-2,3-dimethyl-2-aminovaleriansäure
N-(2-Chlor-6-methoxyphenylacetyl)-1-amino-cyclopentancarbonsäure
N-(2-Chlor-6-methoxyphenylacetyl)-1-amino-cyclohexancarbonsäure
N-(2-Chlor-6-methoxyphenylacetyl)-1-amino-cycloheptancarbonsäure
N-(2-Chlor-6-methoxyphenylacetyl)-1-amino-cyclooktancarbonsäure
N-(2-Chlor-4-methoxy-phenylacetyl)-1-amino-2-methyl-cyclohexancarbonsäure
N-(2-Chlor-4-methoxy-phenylacetyl)-1-amino-3-methyl-cyclohexancarbonsäure
N-(2-Chlor-4-methoxy-phenylacetyl)-1-amino-4-methyl-cyclohexancarbonsäure
N-(2-Chlor-4-methoxy-phenylacetyl)-1-amino-3,4-dimethyl-cyclohexancarbonsäure
N-(2-Chlor-4-methoxy-phenylacetyl)-1-amino-4-ethyl-cyclohexancarbonsäure
N-(2-Chlor-4-methoxy-phenylacetyl)-1-amino-4-isopropyl-cyclohexancarbonsäure
N-(2-Chlor-4-methoxy-phenylacetyl)-1-amino-4-tert.-butyl-cyclohexancarbonsäure
N-(2-Chlor-4-methoxy-phenylacetyl)-1-amino-4-methoxy-cyclohexancarbonsäure
N-(4-Chlor-2-methoxy-phenylacetyl)-1-amino-2-methyl-cyclohexancarbonsäure
N-(4-Chlor-2-methoxy-phenylacetyl)-1-amino-3-methyl-cyclohexancarbonsäure
N-(4-Chlor-2-methoxy-phenylacetyl)-1-amino-4-methyl-cyclohexancarbonsäure
N-(4-Chlor-2-methoxy-phenylacetyl)-1-amino-3,4-dimethyl-cyclohexancarbonsäure
N-(4-Chlor-2-methoxy-phenylacetyl)-1-amino-4-ethyl-cyclohexancarbonsäure
N-(4-Chlor-2-methoxy-phenylacetyl)-1-amino-4-isopropyl-cyclohexancarbonsäure
N-(4-Chlor-2-methoxy-phenylacetyl)-1-amino-4-tert.-butyl-cyclohexancarbonsäure
N-(4-Chlor-2-methoxy-phenylacetyl)-1-amino-4-methoxy-cyclohexancarbonsäure
N-(2-Chlor-6-methoxy-phenylacetyl)-1-amino-2-methyl-cyclohexancarbonsäure
N-(2-Chlor-6-methoxy-phenylacetyl)-1-amino-3 -methyl-cyclohexancarbonsäure
N-(2-Chlor-6-methoxy-phenylacetyl)-1-amino-4-methyl-cyclohexancarbonsäure
N-(2-Chlor-6-methoxy-phenylacetyl)-1-amino-3,4-dimethyl-cyclohexancarbonsäure
N-(2-Chlor-6-methoxy-phenylacetyl)-1-amino-4-ethyl-cyclohexancarbonsäure
N-(2-Chlor-6-methoxy-phenylacetyl)-1-amino-4-isopropyl-cyclohexancarbonsäure
N-(2-Chlor-6-methoxy-phenylacetyl)-1-amino-4-tert.-butyl-cyclohexancarbonsäure
N-(2-Chlor-6-methoxy-phenylacetyl)-1-amino-4-methoxy-cyclohexancarbonsäure

Verbindungen der Formel (IIa) sind beispielsweise aus den Phenylessigsäure-halogeniden der Formel (XV) und Aminosäuren der Formel (XIVa) nach Schotten-Baumann (Organikum, 9. Auflage, 446 (1970) VEB Deutscher Verlag der Wissenschaften, Berlin) erhältlich.

Die Phenylessigsäurehalogenide der Formel (XV) 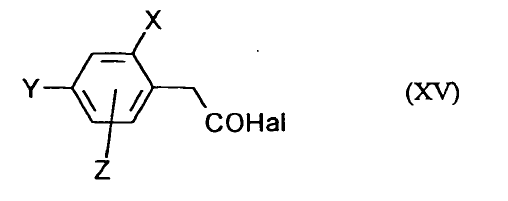 in welcher
X, Y und Z die oben angegebene Bedeutung haben und
Hal für Brom oder Chlor steht,
sind neu, mit Ausnahme der Verbindung 2-Chlor-4-methoxyphenylessigsäurechlo-rid.
(vgl. J. Polym. Sci., Part A, Polym. Chem. 30, 997 (1992), FR-2 054 532)

Man erhält die neuen Phenylessigsäurehalogenide der Formel (XV), wenn man Phenylessigsäuren der Formel (XVIII) 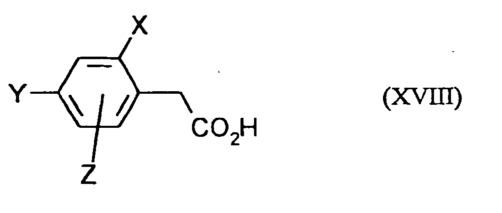 in welcher
X, Y und Z die oben angegebene Bedeutung haben,
mit Halogenierungsmitteln, wie beispielsweise Phosgen, Phosphortri- oder -pentachlorid oder -bromid oder Thionylchlorid, gegebenenfalls in Gegenwart eines inerten Verdünnungsmittels, wie Kohlenwasserstoffen oder halogenierten Kohlenwasserstoffen, bei Temperaturen zwischen -30°C und 150°C, bevorzugt zwischen -20°C und 100°C, umsetzt.

Die Phenylessigsäuren der Formel (XVII) sind zum Teil bekannt und/oder lassen sich z.B. nach folgendem Formelschema herstellen:

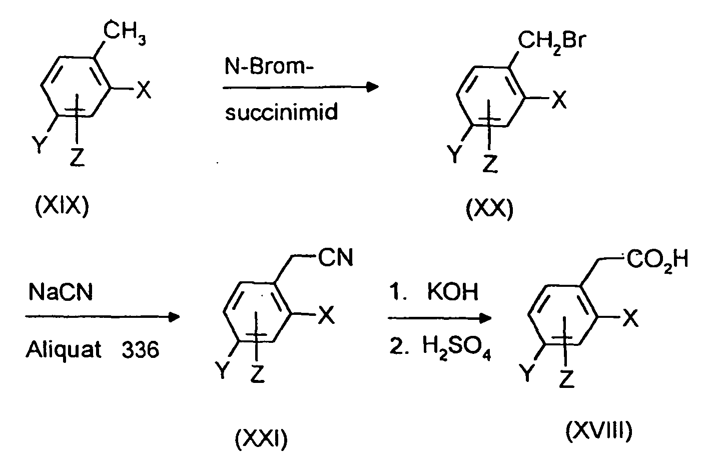

In den Formeln (XIX), (XX), (XXI) und (XVIII) haben X, Y und Z die oben angegebene Bedeutung.

Die Toluole der Formel (XIX) sind allgemein bekannte Verbindungen der organischen Chemie.

Die zur Durchführung der erfindungsgemäßen Verfahren (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Alkylhalogenide der Formel (VII), Sulfonsäurechloride der Formel (VIII), Phosphorverbindungen der Formel (IX) und Metallhydroxide oder Amine der Formel (X) und (XI) und Isocyanate, Isothiocyanate oder Carbamidsäurechloride der Formel (XIII) sind allgemein bekannte Verbindungen der organischen bzw. anorganischen Chemie.

Das Verfahren (A) ist dadurch gekennzeichnet, daß man Verbindungen der Formel (II) in welcher A, B, X, Y, Z und R⁸ die oben angegebene Bedeutung haben, in Gegenwart von Basen einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Isopropanol, Butanol, iso-Butanol und tert.-Butanol.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und - hydride, wie Natriumamid, Natriumhydrid und Calciumhydrid, und außerdem auch Alkalimetallalkoholate, wie Natrium-methylat, Natriumethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung der erfindungsgemäßen Verfahren (A_{α}) und (A_{β}) setzt man die Reaktionskomponenten der Formeln (II) und (IIa) und die deprotonierenden Basen im allgemeinen in etwa doppeltäquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (Bα) bei der Verwendung von Carbonsäurehalogeniden innerhalb eines größeren Bereiches variiert werden. Im allgemeinen arbeitet man bei Temperaturen zwischen - 20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Die Reaktionstemperaturen können bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der- Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.
Das Verfahren (C) ist dadurch gekennzeichnet, daß man Verbindungen der Formel (Ia) mit Chlorameisensäureestern oder Chlorameisensäurethiolestern der Formel (V) umsetzt.

Verwendet man die entsprechenden Chlorameisensäureester bzw. Chlorameisensäurethiolester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkali-metallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) bei Verwendung der Chlorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester bzw. Chlorameisensäurethiolester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren (D_{α}) setzt man pro Mol Ausgangsverbindung der Formel (Ia) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (D_{β}) setzt man pro Mol Ausgangsverbindung der Formel (Ia) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus der Verbindung der Formel (Ia) durch Zusatz eines Deprotonierungsmittels (wie z.B. Kaliumtertiärbutylat oder Natriumhydrid) das entsprechende Salz herzustellen. Man setzt die Verbindung (Ia) solange mit Schwefelkohlenstoff um, bis die Bildung der Zwischenverbindung abgeschlossen ist, z.B. nach mehrstündigem Rühren bei Raumtemperatur.

Die weitere Umsetzung mit dem Alkylhalogenid der Formel (VII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren (E) setzt man pro Mol Ausgangsverbindung der Formel (Ia) ca. 1 Mol Sulfonsäurechlorid (VIII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Dimethylsulfid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.
Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (F) setzt man zum Erhalt von Verbindungen der Struktur (Ie) auf 1 Mol der Verbindung (Ia), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Als gegebenenfalls zugesetzte Verdünnungsmittel kommen aller inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (G) ist dadurch gekennzeichnet, daß man Verbindungen der Formel (Ia) mit Metallverbindungen (X) oder Aminen (XI) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (G) wird im allgemeinen unter Normaldruck durchgeführt. Die Reaktionstemperaturen liegen im allgemeinen zwischen -20°C und 100°C, vorzugsweise zwischen 0°C und 50°C.

Bei Herstellungsverfahren (H_{α}) setzt man pro Mol Ausgangsverbindung der Formel (Ia) ca. 1 Mol Isocyanat der Formel (XII) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (H_{β}) setzt man pro Mol Ausgangsverbindung der Formel (Ia) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzusweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.
Die Reaktion kann bei Normaldruck oder unter erhöhtem Druck durchgeführt werdern, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius,
Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp: Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp.. Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae), gegen die Larven der grünen Reiszikade (Nephotettix cincticeps) oder gegen die Raupen der Kohlschabe (Plutella maculipennis) einsetzen.

Die erfindungsgemäßen Verbindungen zeigen auch eine gute Wirkung gegen Venturia inaequalis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline; Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Genannt seien die folgenden Verbindungen:
Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zetamethrin,
Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb,
Acephate, Azinphos A, Azinphos M, Bromophos A, Cadusafos, Carbophenothion, Chlorfenvinphos, Chlormephos, Chlorpyrifos, Chlorpyrifos M, Cyanophos, Demeton M, Demeton-S-methyl, Demeton S, Diazinon, Dichlorvos, Dicliphos, Dichlofenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemethon M, Oxydeprofos, Parathion A, Parathion M, Phenthoate, Phorate, Phosalone, Phosmet, Phosphamdon, Phoxim, Pirimiphos. A, Pirimiphos M, Propaphos, Prothiophos, Prothoate, Pyraclophos, Pyridaphention, Quinalphos, Salithion, Sebufos, Sulfotep, Sulprofos, Tetrachlorvinphos, Temephos, Thiomethon, Thionazin, Trichlorfon, Triazophos, Vamidothion,
Buprofezin, Chlorfluazuron, Diflubenzuron, Flucycloxuron, Flufenoxuron, Hexaflumuron, Pyriproxifen, Tebufenozide, Teflubenzuron, Triflumuron,
Imidacloprid, Nitenpyram,
Abamectin, Amitraz, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron,
Aldicarb, Bendiocarb, Benfuracarb, Carbofuran, Carbosulfan, Chlorethoxyfos, Cloethocarb, Disulfoton, Ethoprophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Pröthiofos, Pyrachlofos, Sebufos, Silafluofen, Tebupirimphos, Tefluthrin, Terbufos, Thiodicarb, Thiafenox,
Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene,
die Verbindung der Formel 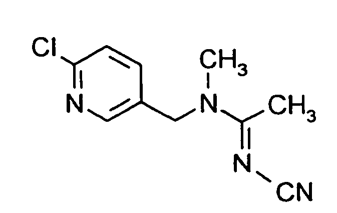 und die Verbindung der Formel

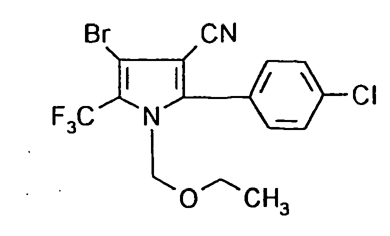

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die Herstellung und die Verwendung der erfindungsgemäßen Wirkstoffe geht aus den nachfolgenden Beispielen hervor.

### Herstellungsbeispiele:

### Beispiel (Ia-1):

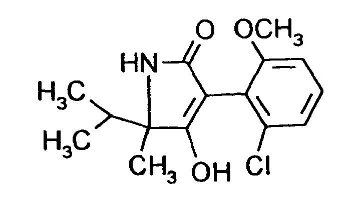

Zu einer Suspension von 18,06 g (0,16 Mol) Kalium-tert.-butylat in 50 ml absolutem Tetrahydrofuran werden in der Siedehitze 23,8 g (0,073 Mol) N-(2-Chlor-6-methoxyphenylacetyl)-2-amino-2,3-dimethyl-buttersäuremethylester, gelöst in 150 ml absolutem Toluol, zugetropft und 1,5 h bei Rückflußtemperatur erhitzt. Nach Abkühlen auf Raumtemperatur wird das Reaktionsgemisch mit 230 ml Wasser versetzt, die wäßrige Phase abgetrennt, die Toluolphase erneut mit 110 ml Wasser extrahiert und die vereinigten wäßrigen Phasen bei 15-20°C mit ca. 25 ml konzentrierter Salzsäure sauergestellt. Das Produkt wird abgesaugt und getrocknet. Man erhält 20,2 g (93 % d. Theorie) 3-(2-Chlor-6-methoxyphenyl)-5-isopropyl-5-methyl-pyrrolidin-2,4-dion vom Schmp. Fp.: 172-174°C.

### Beispiel (Ia-2)

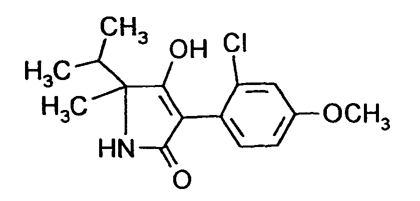

Zu einer Suspension von 56 g (0,498 Mol) Kalium-tert.-butylat in 150 ml absolutem Tetrahydrofuran, tropft man in der Siedehitze 71,4 g (0,226 Mol) N-(2-Chlor-4-fluorphenylacetyl)-2-amino-2,3-dimethyl-buttersäuremethylester gelöst in 450 ml absolutem Toluol und rührt 90 Min. nach bei Rückflußtemperatur. Nach Abkühlen auf Raumtemperatur gibt man 700 ml Wasser zum Reaktionsgemisch, trennt die organische Phase ab und wäscht diese erneut mit 340 ml Wasser. Die vereinigten wäßrigen Phase werden bei 15-20°C mit ca.. 80 ml konzentrierter Salzsäure sauergestellt und das Produkt abgesaugt. Nach dem Trocknen erhält man 57,1g (85 % d. Theorie) 3-(2-Chlor-4-methoxyphenyl)-5-isopropyl-5-methylpyrrolidin-2,4-dion vom Fp. 130 bis 140°C.
- ¹H-NMR (300 MHz, d₆-DMSO):: 0.78, 0.94 (2d, 6H, 5-CH (CH₃)₂); 1.3 (s, 3H, 5-CH₃; 1.93 (m, 1H, 5-CH (CH₃)₂); 3.77 (s, 3H, OCH₃); 6.8, 6.9 (dd, 1H, Ar 5-H); 7.03 (d, 1H, Ar 3-H); 7.11 (d, 1H, Ar 6-H); 7.46 (br, 1H, NH); 10.77 (s, 1H, OH).

Analog zu Beispiel (Ia-1) und gemäß den allgemeinen Angaben in der Beschreibung zu den erfindungsgemäßen Verfahren werden die nachfolgend in Tabelle 8 aufgeführten Endprodukte der Formel (Ia) erhalten.

**Tabelle 8**

| | | | | | | | |
|---|---|---|---|---|---|---|---|
| 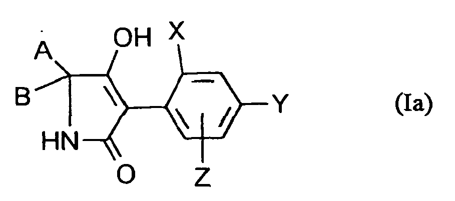 | | | | | | | |

| Bsp.Nr. | X | Y | Z | A | B | Isomer | Fp.°C |
|---|---|---|---|---|---|---|---|
| Ia-3 | OCH₃ | Cl | H | i-C₃H₇ | CH₃ | - | 204-206 |
| Ia-4 | Cl | OCH₃ | H | 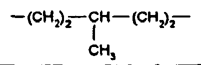 | | β | > 220 |
| Ia-5 | Cl | OCH₃ | H | 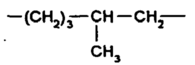 | | β | > 220 |
| Ia-6 | Cl | OCH₃ | H | -(CH₂)₅- | | - | 116 |
| Ia-7 | Cl | OCH₃ | H | CH₃ CH₃ | | - | 132 |
| Ia-8 | OCH₃ | Cl | H | 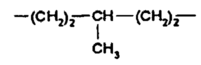 | | β | 212 |
| Ia-9 | Cl | H | 6-OCH₃ | 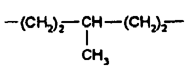 | | β | 196 |

### Beispiel (Ib-1)

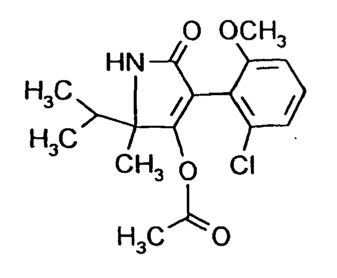

4.43 g (0,015 Mol) 3-(2-Chlor-6-methoxyphenyl)-5-isopropyl-5-methyl-pyrrolidin-2,4-dion werden in 70 ml absolutem Dichlormethan vorgelegt und mit 2,1 ml Triethylamin versetzt. Bei 0 bis 10°C werden 1,13 ml Acetylchlorid in 5 ml absolutem Dichlormethan zugegeben und der Ansatz bei Raumtemperatur weitergerührt. Das Ende der Reaktion wird dünnschichtchromatographisch ermittelt. Anschließend wird zweimal mit jeweils 100 ml 0,5 N Natronlauge gewaschen, die organische Phase über Magnesiumsulfat getrocknet und das Lösungsmittel im Vakuum abgezogen.

Man erhält 4,62 g (91 % der Theorie) 3-(2-Chlor-6-methoxyphenyl)-5-isopropyl-5-methyl-4-acetoxy-Δ3-pyrrolidin-2-on vom Schmelzpunkt Fp.: 70-72°C.

Analog zu Beispiel (Ib-1) und gemäß den allgemeinen Angaben in der Beschreibung zu den erfindungsgemäßen Verfahren werden die nachfolgend in Tabelle 9 aufgeführten Endprodukte der Formel (Ib) erhalten:

**Tabelle 9**

| | | | | | | | | |
|---|---|---|---|---|---|---|---|---|
| 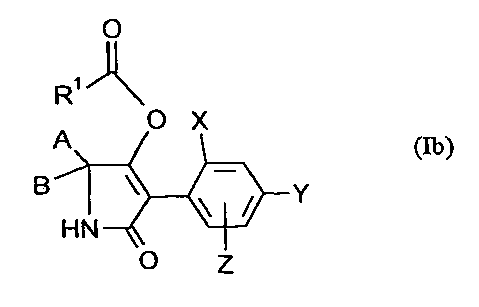 | | | | | | | | |

| Bsp.-Nr. | X | Y | Z | A | B | R¹ | Isomer | Fp.°C |
|---|---|---|---|---|---|---|---|---|
| Ib-2 | Cl | OCH₃ | H | i-C₃H₇ | CH₃ | CH₃ | - | 120 |
| Ib-3 | Cl | OCH₃ | H | i-C₃H₇ | CH₃ | t-C₄H₉ | - | 113 |
| Ib-4 | OCH₃ | Cl | H | i-C₃H₇ | CH₃ | CH₃ | - | 122-123 |
| Ib-5 | OCH₃ | Cl | H | i-C₃H₇ | CH₃ | t-C₄H₄ | - | 182 |
| Ib-6 | Cl | OCH₃ | H | 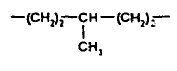 | | CH₃ | β | > 220 |
| Ib-7 | Cl | OCH₃ | H | 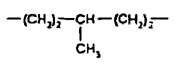 | | i-C₃H₇ | β | > 220 |
| Ib-8 | Cl | OCH₃ | H | 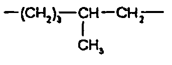 | | CH₃ | β | > 220 |
| Ib-9 | Cl | OCH₃ | H | 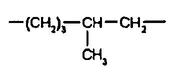 | | i-C₃H₇ | β | 207 |
| Ib-10 | OCH₃ | Cl | H | 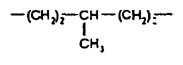 | | i-C₃H₇ | β | 211 |
| Ib-11 | Cl | H | 6-OCH₃ | 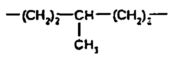 | | i-C₃H₇ | β | > 220 |

### Beispiel (Ic-1)

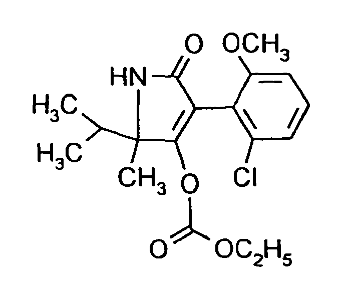

4,43 g (0,015 Mol) 3-(2-Chlor-6-methoxyphenyl)-5-isopropyl-5-methyl-pyrrolidin-2,4-dion werden in 70 ml absolutem Dichlormethan vorgelegt und mit 2,1 ml Triethylamin versetzt. Bei 0 bis 10°C werden 1,5 ml Chlorameisensäure-ethylester in 5 ml absolutem Dichlormethan zugegeben und der Ansatz bei Raumtemperatur weitergerührt. Das Ende der Reaktion wird dünnschichtchromatographisch ermittelt. Anschließend wird zweimal mit jeweils 100 ml 0,5 N Natronlauge gewaschen, die organische Phase über Magnesiumsulfat getrocknet und das Lösungsmittel im Vakuum abgezogen.

Man erhält 2,68 g (48 % der Theorie) Kohlensäure-O-(ethylester-O-[3-(2-chlor-6-methoxyphenyl)]-5-isopropyl-5-methyl-Δ3-pyrrolin-4-yl-2-on vom Schmelzpunkt Fp.: 104-106°C.

Analog zu Beispiel (Ic-1) und gemäß den allgemeinen Angaben in der Beschreibung zu den erfindungsgemäßen Verfahren werden die nachfolgend in Tabelle 10 aufgeführten Endprodukte der Formel (Ic) erhalten:

**Tabelle 10**

| | | | | | | | | |
|---|---|---|---|---|---|---|---|---|
| 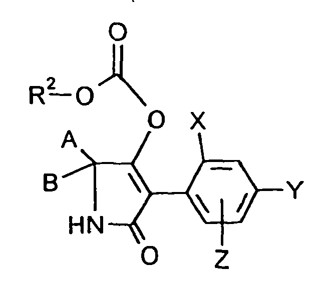 | | | | | | | | |

| Bsp.-Nr. | X | Y | Z | A | B | R² | Isomer | Fp.°C |
|---|---|---|---|---|---|---|---|---|
| -Io-2- | Cl | OCH₃ | H | i-C₃H₇ | CH₃ | C₂H₅ | - | 58-66 |
| Ic-3 | OCH₃ | Cl | H | i-C₃H₇ | CH₃ | C₂H₅ | - | 120 |
| Ic-4 | Cl | OCH₃ | H | 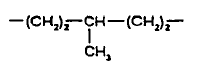 | | C₂H₅ | β | > 220 |
| Ic-5 | Cl | OCH₃ | H | 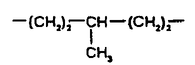 | | s-C₄H₉ | β | > 220 |
| Ic-6 | Cl | OCH₃ | H | 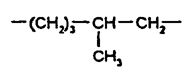 | | C₂H₅ | β | 182 |
| Ic-7 | Cl | OCH₃ | H | 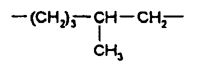 | | s-C₄H₉ | β | 174 |
| Ic-8 | OCH₃ | Cl | H | 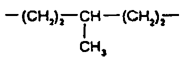 | | C₂H₅ | β | 201 |
| Ic-9 | Cl | H | 6-OCH₃ | 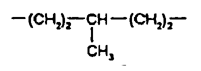 | | C₂H₅ | β | > 220 |

### Herstellung der Ausgangsverbindungen:

### Beispiel (II-1)

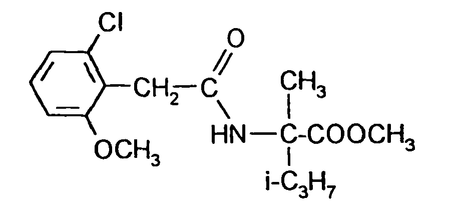

Zu 47 g (0,481 Mol) konzentrierter Schwefelsäure gibt man unter Rühren und Eiskühlung tropfenweise 29,9 g (0,101 Mol) 2-(2-Chlor-6-methoxyphenyl)-N-[1-cyano-2-(3-methyl)-butyl]-acetamid in 200 ml Dichlormethan gelöst, wobei sich die Temperatur der Reaktionsmischung auf 30°C bis 40°C erwärmt und rührt nach beendeter Zugabe weitere 2 Stunden bei 30°C bis 40°C bis die Dichlormethanphase der Reaktionsmischung farblos geworden ist. Anschließend gibt man ebenfalls tropfenweise unter Eiskühlung 66 ml absolutes Methanol zu, wobei sich die Reaktionsmischung abermals bis 40°C erwärmt. Anschließend rührt man weitere 6 Stunden bei 40°C bis 70°C. Zur Aufarbeitung gibt man die Reaktionsmischung unter Rühren in 460 g Eis, extrahiert mit Dichlormethan, wäscht die vereinigten organischen Phasen mit wäßriger Natriumhydrogencarbonat säurefrei, trocknet über Magnesiumsulfat und entfernt das Lösungsmittel im Vakuum.

Man erhält 23,8 g (72 % der Theorie) an 2-(2-Chlor-6-methoxyphenyl)-N-[1-methoxycarbonyl-2-(3-methyl)-butyl]-acetamid vom Schmelzpunkt 76-78°C.

### Beispiel (II-2)

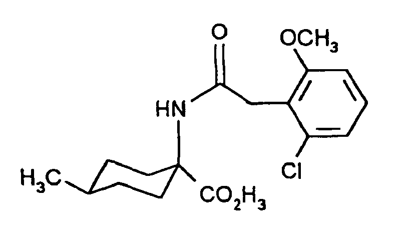

24,28 g (0,117 mol) 1-Amino-4-methyl-cyclohexancarbonsäuremethylester-Hydrochlorid werden in 220 ml absolutem Tetrahydrofuran mit 32,7 ml Triethylamin versetzt und bei 0 bis 10°C 25,6 g (0,117 mol) 2-Chlor-6-methoxy-phenylessigsäurechlorid in 25 ml absolutem Tetrahydrofuran zugetropft. Nach Beendigung der Reaktion wird der Niederschlag abgesaugt, das Filtrat eingeengt, der Rückstand in Methylenchlorid aufgenommen, mit verdünnter Salzsäure gewaschen, getrocknet und das Lösungsmittel im Vakuum abgedampft. Nach dem Umkristallisieren aus MTB-Ether/n-Hexan erhielt man 30,3 g (Δ 73 % der Theorie) cis-N-(2-Chlor-6-methoxy-phenylacetyl)-4-methyl-1-aminocyclohexancarbonsäuremethylestervom Fp. 159°C.

Analog zu den Verbindungen II-1 und II-2 und gemäß den allgemeinen Angaben zu den erfindungsgemäßen Verfahren werden die nachfolgend in Tabelle 11 aufgeführten Produkte der Formel (II) erhalten.

**Tabelle 11**

| | | | | | | | | |
|---|---|---|---|---|---|---|---|---|
| 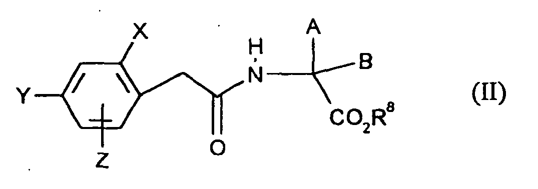 | | | | | | | | |

| Bsp.-Nr. | X | Y | Z | A | B | R⁸ | Isomer | Fp.°C |
|---|---|---|---|---|---|---|---|---|
| II-3 | OCH₃ | Cl | H | 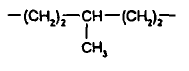 | | CH₃ | β | 131 |
| II-4 | OCH₃ | Cl | H | i-C₃H₇ | CH₃ | CH₃ | - | 75-76 |
| II-5 | Cl | OCH₃ | H | 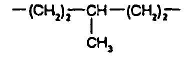 | | CH₃ | β | 133 |
| II-6 | Cl | OCH₃ | H | 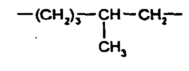 | | CH₃ | β | 125 |
| II-7 | Cl | OCH₃ | H | CH₃ | CH₃ | CH₃ | - | Öl |
| II-8 | Cl | OCH₃ | H | -(CH₂)₅- | | CH₃ | - | 106 |

### Beispiel (XVII-1)

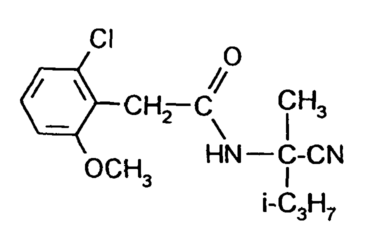

Zu 16,8 g (0,15 Mol) 2-Amino-2-methyl-isovaleronitril und 22,4 ml (0,16 Mol) Triethylamin in 250 ml absolutem Tetrahydrofuran gibt man bei 0°C bis 10°C tropfenweise unter Rühren 32,9 g (0,15 Mol) 2-Chlor-6-methoxyphenylessigsäurechlorid in 50 ml absolutem Tetrahydrofuran und rührt nach beendeter Zugabe bei Raumtemperatur, bis im Dünnschichtchromatogramm kein Ausgangsprodukt mehr nachweisbar ist. Zur Aufarbeitung gibt man die Reaktionsmischung unter Rühren in eine Mischung aus 600 ml Eiswasser und 200 ml 1N-Salzsäure, extrahiert 3 mal mit Dichlormethan, wäscht die organischen Phasen mit NatriumhydrogencarbonatLösung, trocknet die organische Phase über Magnesiumsulfat und entfernt das Lösungsmittel im Vakuum.

Man erhält 29,9 g (67 % der Theorie) an 2-(2-Chlor-6-methoxyphenyl)-N-[2-cyano-2-(3-methyl)-butyl]-acetamid vom Schmelzpunkt 104-106°C.

### Beispiel (XVII-2)

Analog erhält man 2-(4-Chlor-2-methoxyphenyl)-N-[2-cyano-2-(3-methyl)-butyl]-acetamid vom Schmelzpunkt 110-111°C.

### Beispiel (XVII-3)

Analog erhält man 2-(2-Chlor-4-fluorphenyl)-N-[2-cyano-2-(3-methyl)-butyl]-acetamid vom Schmp. 109-111°C.

### Beispiel (XX-1)

### 2-Chlor-6-methoxybenzylbromid

Es werden 442 g 2-Chlor-6-methoxytoluol in 2420 ml Tetrachlorkohlenstoff gelöst und mit 516 g N-Bromsuccinimid unter Zugabe von 5,8 g AIBN 2 h unter Rückfluß erhitzt. Der Feststoff wird abfiltriert, mit wenig Tetrachlorkohlenstoff gewaschen, das Filtrat eingeengt und der verbleibende Rückstand fraktioniert. Man erhält 483 g farbloses Öl (Kp. 100°C/1.4 mbar, F. 44-47°C).
- ¹H-NMR (CDCl₃):: 7.24 (t, 1H); 7.03 (dd, 1H); 6.83 (dd, 1H); 4.76 (s, 2H); 3.93 (s, 3H).

### Beispiel (XX-2)

### 4-Chlor-2-methoxybenzylbromid

Es werden 303 g 4-Chlor-2-methoxytoluol in 1665 ml Tetrachlorkohlenstoff gelöst und mit 355 g N-Bromsuccinimid unter Zugabe von 4,0 g AIBN 2 h unter Rückfluß erhitzt. Der Feststoff wird abfiltriert, mit wenig Tetrachlorkohlenstoff gewaschen, das Filtrat eingeengt und der verbleibende Rückstand fraktioniert. Man erhält 307 g farbloses Öl (Kp. 103°C/1,5 mbar, F. 42-45°C).
- ¹H-NMR (CDCl₃)_{:}: 7.29 (d, 1H); 6.95 (dd, 1H); 6.93 (dd, 1H); 4.95 (s, 2H); 3.93 (s, 3H).

### Beispiel (XX-3)

### 2-Chlor-4-methoxybenzylbromid

Es werden 186 g 2-Chlor-4-methoxytoluol in 1000 ml Tetrachlorkohlenstoff gelöst und mit 212 g N-Bromsuccinimid unter Zugabe von 2,4 g AIBN 2 h unter Rückfluß erhitzt. Der Feststoff wird abfiltriert, mit wenig Tetrachlorkohlenstoff gewaschen, das Filtrat eingeengt und der verbleibende Rückstand fraktioniert. Man erhält 252 g oranges Öl (Reinheit 82 %).
- ¹H-NMR (CDCl₃):: 7.40 (d, 1H); 7.23 (dd, 1H); 6.85 (dd, 1H); 4.43 (s, 2H); 3.88 (s, 3H).

### Allgemeine Arbeitsvorschrift zur Herstellung der Verbindungen der Formel (XXI):

Es werden 1,3 Mol Natriumcyanid in 65 ml Wasser vorgelegt und mit 1,7 g Phasentransferkatalysator Aliquat 336 versetzt. Die Mischung erwärmt man auf 70°C und tropft innerhalb von 30 Min. 1 Mol eines Benzylbromidderivats der Formel (XX), gelöst in 200 ml Toluol, zu. Es wird 3 bis 6 h auf 70°C erhitzt. Die abgekühlte Reaktionsmischung wird in 600 ml Toluol/600 ml Wasser gegeossen, die organische Phase abgetrennt und mehrfach mit Wasser gewaschen. Man trocknet, engt ein und destilliert den Rückstand.

### Beispiel (XXI-1)

### (2-Chlor-6-methoxyphenyl)acetonitril

Es werden 483 g 2-Chlor-6-methoxybenzylbromid in 410 ml Toluol mit 131 g Natriumcyanid in 132 ml Wasser und 3,28 g Aliquat 336 umgesetzt. Man erhält 325 g farbloses Öl (Kp. 103°C/0,08 mbar).
- ¹H-NMR (CDCl₃):: 7.25 (t, 1H); 7.03 (dd, 1H); 6.82 (dd, 1H); 3.89 (s, 3H), 3.83 (s; 2H).

### Beispiel (XXI-2)

### (4-Chlor-2-methoxyphenyl)acetonitril

Es werden 269 g 4-Chlor-2-methoxybenzylbromid in 214 ml Toluol mit 68,2 g Natriumcyanid in 69 ml Wasser und 1,8 g Aliquat 336 umgesetzt. Man erhält 141. g farbloses Öl (Kp. 107°C/0,08 mbar).
- ¹H-NMR (CDCl₃):: 7.30 (d, 1H); 6.98 (dd, 1H); 6.90 (d, 1H); 3.89 (s, 3H); 3.66 (s,2H).

### Beispiel (XXI-3),

### (2-Chlor-4-methoxyphenyl)acetonitril

Es werden 212 g (Reinheit 82 %) 2-Chlor-4-methoxybenzylbromid in 900 ml Dichlormethan mit 176 g Kaliumcyanid in 675 ml Wasser und 15,5 g Tetrabutylammoniumhydrogensulfat innerhalb von 4 h bei 40°C umgesetzt. Nach der Aufarbeitung erhält man 157 g bräunliches Öl (Reinheit 79 %).
- ¹H-NMR (CDCl₃):: 7.33 (d, 1H); 7.19 (dd, 1H); 6,91 (d, 1H); 3.87 (s, 3H); 3.67 (s, 2H).

### Allgemeine Arbeitsvorschrift zur Herstellung der Verbindungen der Formel (XVIII):

Es werden 2 Mol Kaliumhydroxid in 400 ml Ethylenglykol gelöst und mit 1 Mol eines Arylacetonitrils der Formel (XXI) versetzt. Man erwärmt 5 h auf 100°C, kühlt dann auf Raumtemperatur und verdünnt mit 800 ml Wasser. Die Lösung wird mit 20 %iger Schwefelsäure auf ca. pH 1 angesäuert. Man saugt die Säure ab, wäscht mit Wasser nach und trocknet.

### Beispiel (XVIII-1)

### (2-Chlor-6-methoxyphenyl)essigsäure

Es werden 325 g 2-Chlor-6-methoxyphenylacetonitril mit 171 g Kaliumhydroxid in 690 ml Ethylenglykol umgesetzt. Man erhält 331 g Feststoff (Fp. 164-165°C).
- ¹H-NMR (CDCl₃):: 7.19 (t, 1H); 7.00 (dd, 1H); 6.80 (dd, 1H); 3.90 (s, 2H); 3.83 (s, 3H).

### Beispiel (XVIII-2)

### (4-Chlor-2-methoxyphenyl)essigsäure

Es werden 141 g 4-Chlor-2-methoxyphenylacetonitril mit 83,2 g Kaliumhydroxid in 280 ml Ethylenglykol umgesetzt. Man erhält 148 g Feststoff (Fp: 100°C).
- ¹H-NMR (CDCl₃):: 8.40-8.90 (m br, 1H); 7.10 (d, 1H); 6.86 (dd, 1H); 6.89 (d, 1H); 3.80 (s, 3H); 3.60 (s, 2H).

### Beispiel (XVIII-3)

### (2-Chlor-4-methoxyphenyl)essigsäure

Es werden 657 g (Reinheit 75 %) 2-Chlor-4-methoxyphenylacetonitril mit 303 g Kaliumhydroxid in 1308 ml Ethylenglykol umgesetzt. Man erhielt 430 g Feststoff (Fp. 83-86°C).
- ¹H-NMR (CDCl₃):: 8.10-8.60 (m br, 1H); 7.32 (d, 1H); 7.16 (dd, 1H); 6.89. (d, 1H); 3.87 (s, 3H); 3.53 (s, 2H).

### Beispiel (XV-1)

### (2-Chlor-4-methoxyphenyl)essigsäurechlorid

Es werden 160 g 2-Chlor-4-methoxyphenylessigsäure in 210 ml Toluol suspendiert und mit 96 ml Thionylchlorid bei 80°C bis zum Ende der Gasentwicklung gerührt. Die flüchtigen Bestandteile werden abgezogen und der Rückstand destilliert. Man erhält 90 g eines orangen Öls (Dünnschichter 170°C, 0,08 mbar).
- ¹H-NMR (CDCl₃):: 7.29 (d, 1H); 7.14 (dd, 1H); 6.91 (d, 1H); 4.05 (s, 2H); 3.90 (s, 3H).

### Beispiel (XV-2)

Analog zu Beispiel (XV-1) erhält man (2-Chlor-6-methoxyphenyl)essigsäurechlorid vom Kp. 106°C/1,1 mbar.

### Beispiel (XV-3)

Analog zu Beispiel (XV-1) erhält man (4-Chlor-2-methoxyphenyl)essigsäurechlorid vom Kp. 111°C/1,1 mbar.

In den folgenden Anwendungsbeispielen wurden die nachstehend aufgeführten Verbindungen als Vergleichssubstanzen eingesetzt: 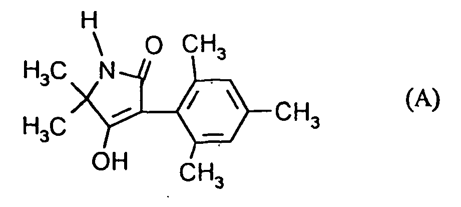 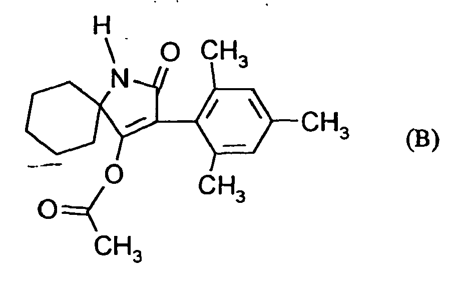 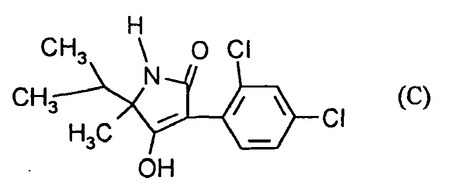 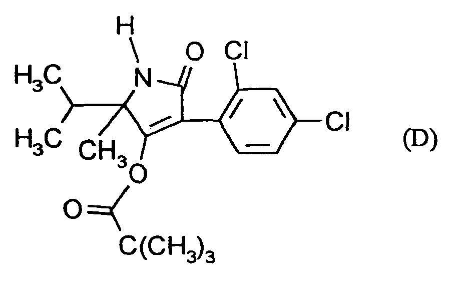 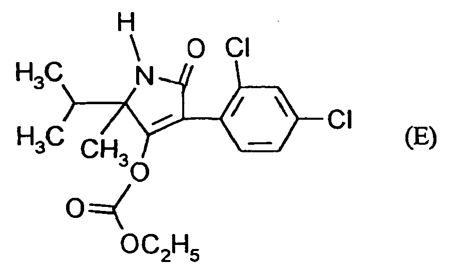 alle bekannt aus EP 0 456 063.

### Beispiel A

### Plutella-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica olearacea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test bewirkten z.B. die Verbindungen gemäß den Herstellungsbeispielen Ia-1, Ia-2 und Ib-2 bei einer beispielhaften Wirkstoffkonzentration von 0,001 % eine Abtötung von mindestens 90 % nach 7 Tagen, während die aus dem Stand der Technik bekannten Verbidungen (A) und (B) eine Abtötung von nur 10 % bewirkten.

### Beispiel B

### Tetranychus-Test (OP-resistent)

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe (Tetranychus urticae) befallen sind, werden mit einer Wirkstoffzubereitung der gewünschten Konzentration gespritzt.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test bewirkten z.B. die Verbindungen gemäß den Herstellungsbeispielen Ib-3 und Ic-2 bei einer beispielhaften Wirkstoffkonzentration von 0,00016 % eine Abtötung von mindestens 80 % nach 7 Tagen, während die aus dem Stand der Technik bekannten Verbindungen (D) und (E) keine Abtötung bewirkten.

## Patentansprüche

1. 1-H-3-Aryl-pyrrolidin-2,4-dion-Derivate der Formel (I) 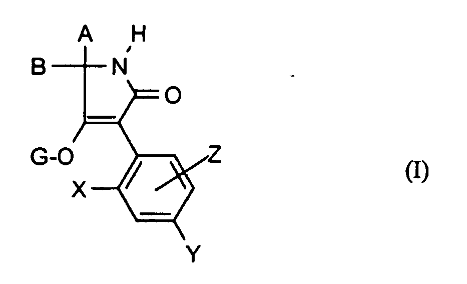 in welcher
A für Wasserstoff, für jeweils gegebenenfalls durch Halogen substituiertes C₁-C₈-Alkyl, C₃-C₄-Alkenyl, C₁-C₆-Alkoxy-C₂-C₄-alkyl, C₁-C₄-Polyalkoxy-C₂-C₄-alkyl, C₁-C₆-Alkylthio-C₂-C₄-alkyl, Cycloalkyl mit 3 bis 6 Ringatomen, das durch 1 bis 2 Sauerstoff- und/oder Schwefel-atome unterbrochen sein kann oder für jeweils gegebenenfalls durch Fluor, Chlor, Brom, Methyl, Ethyl, Propyl, iso-Propyl, Methoxy, Ethoxy, Trifluormethyl und/oder Nitro substituiertes Phenyl, Thienyl, Pyridyl, Imidazolyl, Pyrazolyl, Triazolyl, Indolyl, Thiazol oder Phenyl-C₁-C₃-alkyl steht,
B für Wasserstoff, C₁-C₈-Alkyl oder C₁-C₄-Alkoxyalkyl steht oder
A, B und das Kohlenstoffatom an das sie gebunden sind, für einen gesättigten oder ungesättigten C₃-C₈-Spirocyclus stehen, der gegebenenfalls einfach oder mehrfach durch Methyl, Ethyl, Propyl, Isopropyl, Butyl, iso-Butyl, sec.-Butyl, tert,-Butyl, Cyclohexyl, Trifluormethyl, Methoxy, Ethoxy, Propoxy, iso-Propoxy, Butoxy, iso-Butoxy, sek.-Butoxy, tert.-Butoxy, Methylthio, Ethylthio, Fluor, Chlor oder Phenyl substituiert und gegebenenfalls durch Sauerstoff oder Schwefel unterbrochen ist oder
A, B und das Kohlenstoffatom, an das sie gebunden sind, für einen C₃-C₆-Spirocyclus stehen, der durch eine gegebenenfalls durch ein Sauerstoff- oder Schwefelatom unterbrochene Alkylendiyl- oder durch eine Alkylendioxyl-Gruppe substituiert ist, die mit dem Kohlenstoffatom, an das sie gebunden ist, einen weiteren fünf- bis siebengliedrigen Spirocyclus bildet oder
A,B und das Kohlenstoffatom, an das sie gebunden sind, für einen C₃-C₆-Spirocyclus stehen, bei dem zwei Substituenten gemeinsam für einen gesättigten oder ungesättigten fünf- oder sechsgliedrigen Cyclus stehen, der durch Sauerstoff oder Schwefel unterbrochen sein kann,
X für Chlor steht,
Y für Methoxy steht,
Z für Wasserstoff steht,
oder
X für Chlor steht,
Y für Wasserstoff steht,
Z für Methoxy in der 6-Possition steht,
oder
X für Methoxy steht,
Y für Chlor steht,
Z für Wasserstoff steht,
G für Wasserstoff (a) oder für die Gruppen 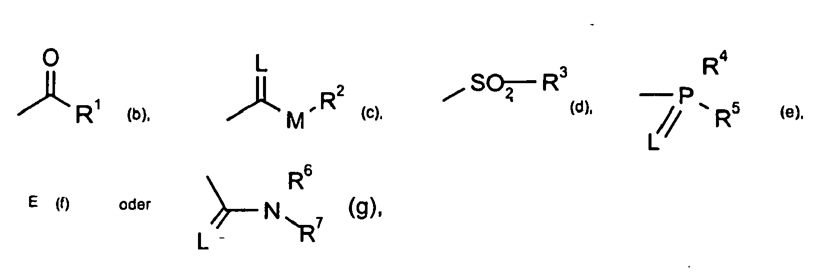 steht,
in welchen
E für ein Metallionäquivalent oder ein Ammoniumion steht und
L und M jeweils für Sauerstoff oder Schwefel stehen.
R¹ für jeweils gegebenenfalls durch Fluor und/oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₂-C₄-alkyl oder Cycloalkyl mit 3 bis 6 Ringatomen, das durch 1 bis 2 Sauerstoff- und/oder Schwefelatome unterbrochen sein kann,
für gegebenenfalls durch Fluor, Chlor, Brom, Methyl, Ethyl, Propyl, i-Propyl, Methoxy, Ethoxy, Trifluormethyl, Trifluormethoxy, Methylthio, Ethylthio, Methylsulfonyl, Ethylsulfonyl und/oder Nitro substituiertes Phenyl,
für gegebenenfalls durch Fluor, Chlor, Brom, Methyl, Ethyl, Propyl, i-Propyl, Methoxy, Ethoxy, Trifluormethyl und/oder Trifluormethoxy substituiertes Phenyl-_{C1}-C₃-alkyl steht,
für gegebenenfalls durch Fluor, Chlor, Brom, Methyl und/oder Ethyl substituiertes Thienyl, Furanyl, Pyridyl, Pyrimidyl, Thiazolyl oder Pyrazolyl,
für gegebenenfalls durch Fluor, Chlor, Methyl, und/oder Ethyl-substituiertes Phenoxy-C₁-C₄-alkyl, oder
für jeweils gegebenenfalls durch Fluor, Chlor, Amino, Methyl und/oder Ethyl substituiertes Pyridyloxy-C₁-C₄-alkyl, Pyrimidyloxy-C₁-C₄-alkyl oder Thiazolyloxy-C₁-C₄-alkyl steht,
R² für jeweils gegebenenfalls durch Fluor und/oder Chlor substituiertes C₁-C₁₄-Alkyl, C₃-C₁₄-Alkenyl, C₁-C₄-Alkoxy-C₂-C₆-alkyl, C₁-C₄-Polyalkoxy-C₂-C₆-alkyl,
für gegebenenfalls durch Fluor, Chlor, Methyl, Ethyl, Propyl, iso-Propyl und/oder Methoxy substituiertes C₃-C₆-Cycloalkyl,
oder für jeweils gegebenenfalls durch Fluor, Chlor, Nitro, Methyl, Ethyl, Propyl, i-Propyl, Methoxy, Ethoxy, und/oder Trifluormethyl substituiertes Phenyl oder Benzyl steht,
R³, R⁴ und R⁵ unabhängig voneinander für jeweils gegebenenfalls durch Fluor und/oder Chlor substituiertes C₁-C₄-Alkyl, C₁-C₄-Alkoxy, C₁-C₄-Alkylamino, Di-(C₁-C₄)-alkylamino, C₁-C₄-Alkylthio, für jeweils gegebenenfalls durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio und/oder C₁-C₃-Alkyl substituiertes Phenyl, Phenoxy oder Phenylthio stehen und
R⁶ und R⁷ unabhängig voneinander für Wasserstoff, für jeweils gegebenenfalls durch Fluor, Chlor und/oder Brom substituiertes C₁-C₄-Alkyl, C₃-C₆-Cycloallcyl, C₁-C₄-Alkoxy, C₃-C₄-Alkenyl, C₁-C₄-Alkoxy-C₂-C₄-alkyl, für gegebenenfalls durch Fluor, Chlor, Brom, C₁-C₄-Halogenalkyl, C₁-C₄-Alkyl und/oder C₁-C₄-Alkoxy substituiertes Phenyl, für gegebenenfalls durch Fluor, Chlor, Brom, C₁-C₄-Alkyl, C₁-C₄-Halogenalkyl und/oder C₁-C₄-Alkoxy substituiertes Benzyl, oder gemeinsam mit dem N-Atom, an das sie gebunden sind, für einen gegebenenfalls durch Sauerstoff oder Schwefel unterbrochenen C₃-C₆-Alkylenring stehen.

2. Verfahren zur Herstellung der 1-H-3-Aryl-pyrrolidin-2,4-dion-Derivate der Formel (I) gemäß Anspruch 1, **dadurch gekennzeichnet, dass** man
(A) zum Erhalt von 1-H-3-Aryl-pyrrolidin-2,4-dionen bzw. deren Enolen der Formel (Ia) 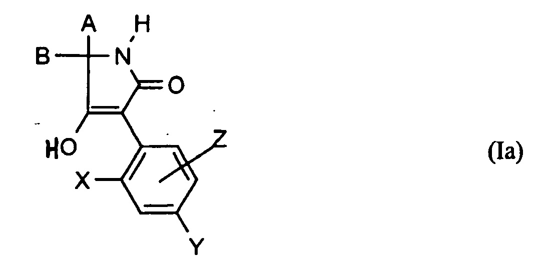 in welcher
A, B, X, Y und Z die in Anspruch 1 angegebene Bedeutung haben,
α) N-Acylaminosäureester der Formel (II) 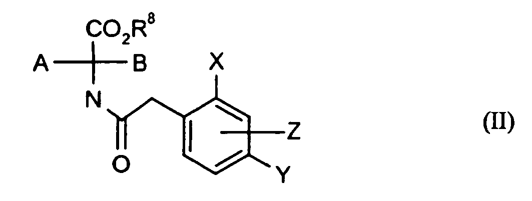 m welcher
A, B, X, Y und Z die oben angegebene Bedeutung haben,
und
R⁸ für Alkyl steht,
in Gegenwart eines Verdünnungsmittels und in Gegenwart einer Base intramolekular kondensiert;
oder
β) zum Erhalt von 1-H-3-Aryl-pyrrolidin-2,4-dionen bzw. deren Enolen der Formel (Ia-a) 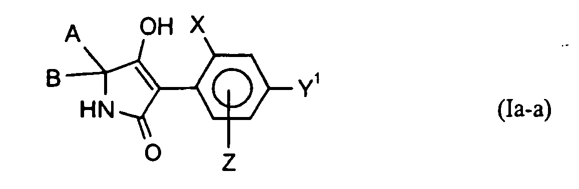 in welcher
A, B, X und Z die oben angegebenen Bedeutungen haben,
und
Y¹ für -OR⁸ steht, wobei
R⁸ für Alkyl steht,
N-Acylaminosäureester der Formel (IIa) 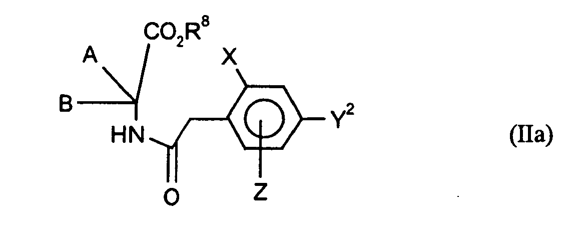 in welcher
A, B, X und Z die oben angegebenen Bedeutungen haben,
Y² für Fluor steht und
R⁸ für Alkyl steht,
in Gegenwart eines Verdünnungsmittels und in Gegenwart einer Base intramolekular kondensiert;
oder
(B) zum Erhalt von Verbindungen der Formel (Ib) 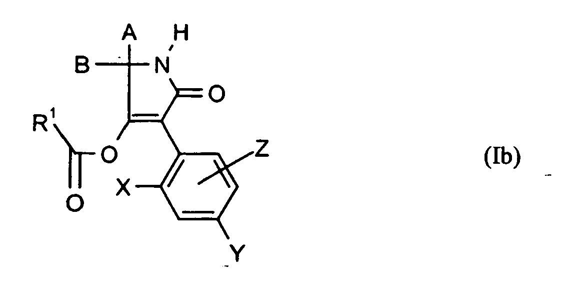 in welcher
A, B, X, Y, Z und R¹ die in Anspruch 1 angegebene Bedeutung haben,
Verbindungen der Formel (Ia), 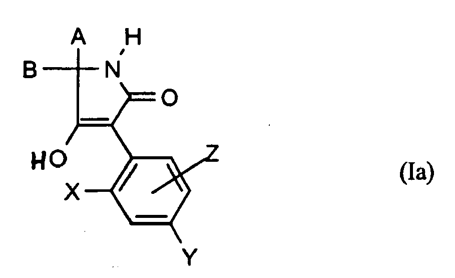 in welcher
A, B, X, Y und Z die oben angegebene Bedeutung haben,
α) mit Säurehalogeniden der allgemeinen Formel (III) 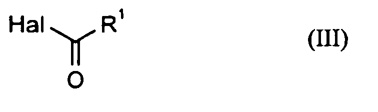 in welcher
R¹ die oben angegebene Bedeutung hat und
Hal für Halogen steht,
gegebenenfalls in Gegenwart eines Verdünnungsmittels und gegebenenfalls in Gegenwart eines Säurebindemittels umsetzt
oder
β) mit Carbonsäureanhydriden der allgemeinen Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt;
oder
(C) zum Erhalt von Verbindungen der Formel (Ic-a) 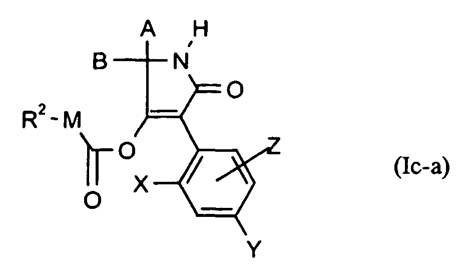 in welcher
A, B, X, Y, Z und R² die in Anspruch 1 angegebene Bedeutung haben,
und
M für Sauerstoff oder Schwefel steht,
Verbindungen der Formel (Ia) 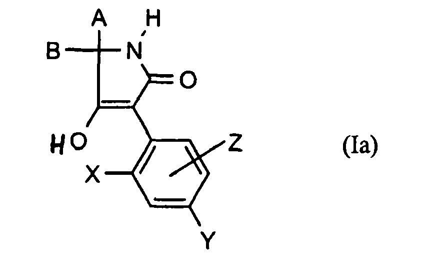 in welcher
A, B, X, Y und Z die oben angegebene Bedeutung haben,
mit Chlorameisensäureester oder Chlorameisensäurethiolester der allgemeinen Formel (V)
R² -M-CO-Cl (V)
in welcher
R² und M die oben angegebene Bedeutung haben,
gegebenenfalls in Gegenwart eines Verdünnungsmittels und gegebenenfalls in Gegenwart eines Säurebindemittels umsetzt;
oder
(D) zum Erhalt von Verbindungen der Formel (Ic-b) 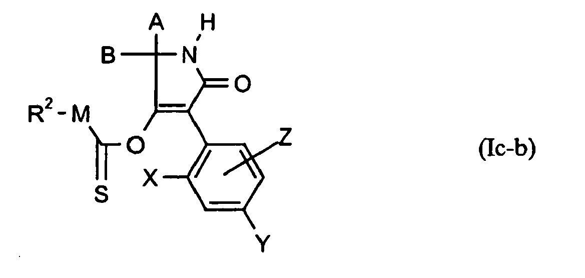 in welcher
A, B, R², X, Y und Z die oben angegebene Bedeutung haben
und
M für Sauerstoff oder Schwefel steht,
Verbindungen der Formel (Ia) 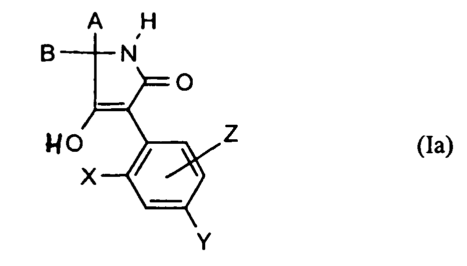 in welcher
A, B, X, Y und Z die oben angegebene Bedeutung haben,
α) mit Chlormonothioameisensäureestern oder Chlordithioameisensäureestern der allgemeinen Formel (VI) 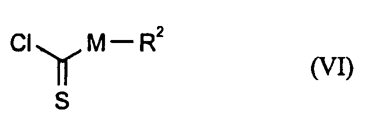 in welcher
M und R² die oben angegebene Bedeutung haben,
gegebenenfalls in Gegenwart eines Verdünnungsmittels und gegebenenfalls in Gegenwart eines Säurebindemittels umsetzt,
oder
β) mit Schwefelkohlenstoff und anschließend mit Alkylhalogeniden der allgemeinen Formel (VII)
R²-Hal (VII)
in welcher
R² die oben angegebene Bedeutung hat
und
Hal für Chlor, Brom oder Iod steht,
umsetzt;
oder
(E) zum Erhalt von Verbindungen der Formel (Id) 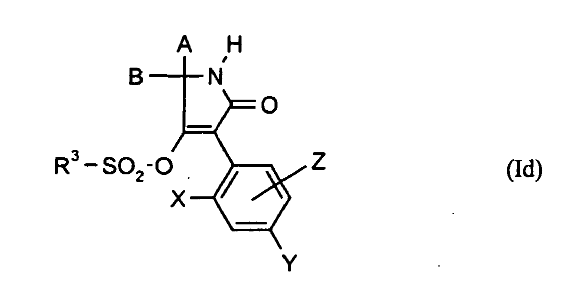 in welcher
A, B, X, Y, Z und R³ die in Anspruch 1 angegebene Bedeutung haben,
Verbindungen der Formel (Ia) 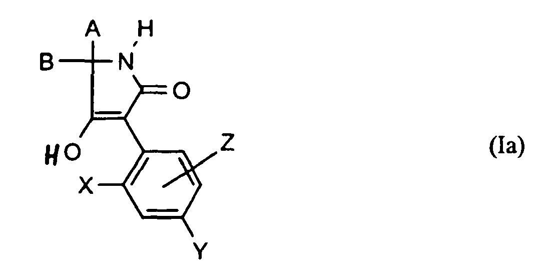 in welcher
A, B, X, Y und Z die oben angegebene Bedeutung haben,
mit Sulfonsäurechloriden der allgemeinen Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels,
umsetzt;
oder
(F) zum Erhalt von 3-Aryl-pyrrolidin-2,4-dionen der Formel (Ie) 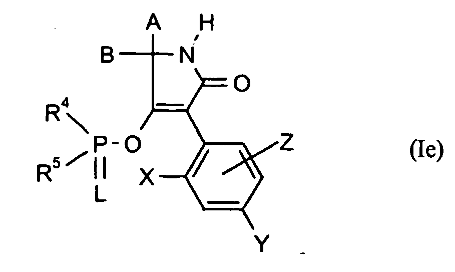 in welcher
A, B, L, X, Y, Z, R⁴ und R⁵ die in Anspruch 1 angegebene Bedeutung haben,
1-H-3-Aryl-pyrrolidin-2,4-dione der Formel (Ia) bzw. deren Enole 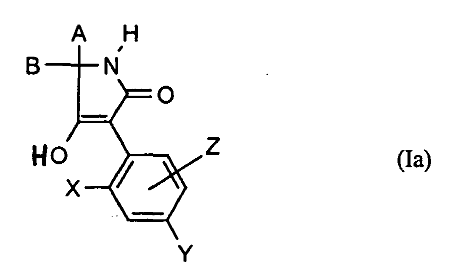 in welcher
A, B, X, Y und Z die oben angegebene Bedeutung haben,
mit Phosphorverbindungen der allgemeinen Formel (IX) 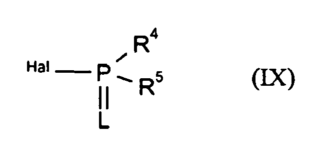 in welcher
L, R⁴ und R⁵ die oben angegebene Bedeutung haben
und
Hal für Halogen steht,
umsetzt;
oder
(G) zum Erhalt von Verbindungen der Formel (If) 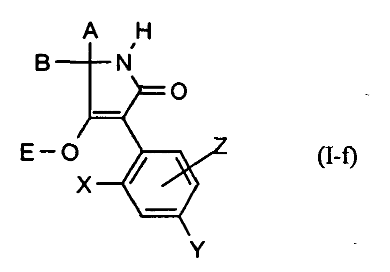 in welcher
A, B, X, Y und Z die oben angegebene Bedeutung haben,
und
E für ein Metallionäquivalent oder für ein Ammoniumion steht,
Verbindungen der Formel (Ia) 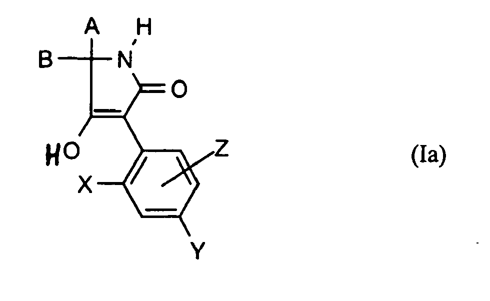 in welcher
A, B, X, Y und Z die oben angegebene Bedeutung haben,
mit Metallverbindungen oder Aminen der allgemeinen Formeln (X) und (XI)
Me(OR⁹)ₜ (X) 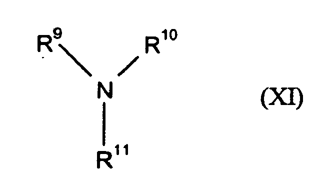
in welchen
Me für ein- oder zweiwertige Metallionen,
t für die Zahl 1 oder 2 und
R⁹, R¹⁰ und R¹¹ unabhängig voneinander für Wasserstoff und/oder Alkyl
stehen,
gegebenenfalls in Gegenwart eines Verdünnungsmittels, umsetzt,
oder
(H) zum Erhalt von Verbindungen der Formel (Ig) 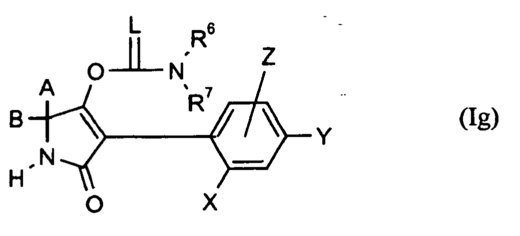 in welcher
A, B, L, X, Y, Z, R⁶ und R⁷ die in Anspruch 1 angegebene Bedeutung haben,
Verbindungen der Formel (Ia) 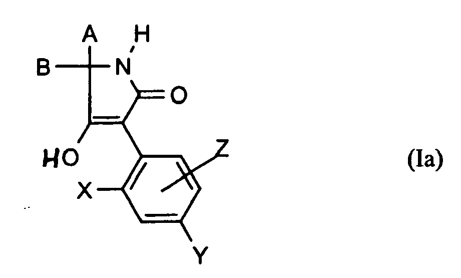 in welcher
A, B, X, Y und Z die oben angegebene Bedeutung haben,
α) mit Isocyanaten oder Isothiocyanaten der allgemeinen Formel (XII)
R⁶-N=C=L (XII)
in welcher
R⁶ die oben angegebene Bedeutung hat
gegebenenfalls in Gegenwart eines Verdünnungsmittels und gegebenenfalls in Gegenwart eines Katalysators
oder
b) mit Carbamidsäurechloriden oder Thiocarbamidsäurechloriden der allgemeinen Formel (XIII) 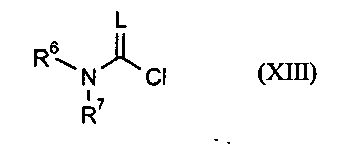 in welcher
L, R⁶ und R⁷ die oben angegebene Bedeutung haben
gegebenenfalls in Gegenwart eines Verdünnungsmittels und gegebenenfalls in Gegenwart eines Säurebindemittels,
umsetzt.

3. Verbindungen der Formel (II) 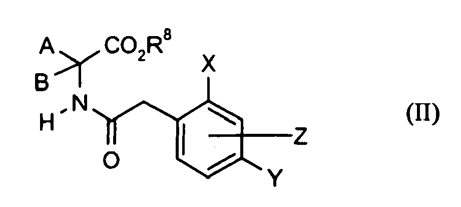 in welcher
A, B, X, Y und Z die in Anspruch 1 angegebene Bedeutung haben und R⁸ für Alkyl steht.

4. Verfahren zur Herstellung der Acyl-aminosäureester der Formel (II), gemäß Anspruch 7, **dadurch gekennzeichnet, dass** man Aminosäurederivate der Formel (XIV), 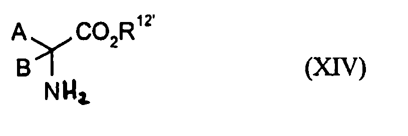 in welcher
R^{12'} für Wasserstoff (XIVa) oder Alkyl (XIVb) steht
und
A und B die in Anspruch 1 angegebene Bedeutung haben,
mit Phenylessigsäurehalogerüden der Formel (XV) 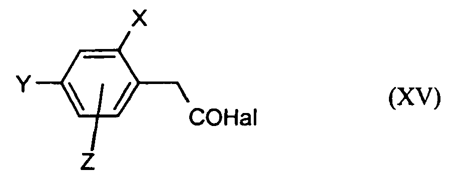 in welcher
X, Y und Z die oben angegebene Bedeutung haben und
Hal für Chlor oder Brom steht,
acyliert,
und die dabei für R¹²' = Wasserstoff entstandenen Acylaminosäuren der Formel (IIa), 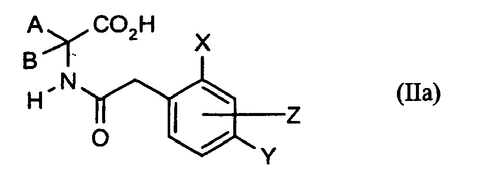 in welcher
A, B, X, Y und Z die oben angegebene Bedeutung haben,
verestert oder
dass man Aminonitrile der Formel (XVI) 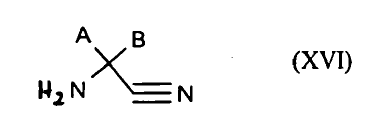 in welcher
A und B die oben angegebene Bedeutung haben,
mit Phenylessigsäurehalogeniden der Formel (XV) 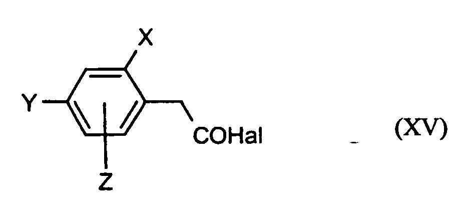 in welcher
X, Y und Z die oben angegebene Bedeutung haben und
Hal für Chlor oder Brom steht,
zu Verbindungen der Formel (XVII) 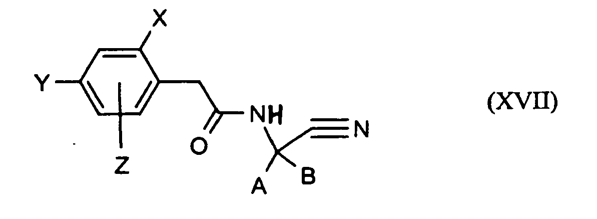 in welcher
A, B, X, Y und Z die oben angegebene Bedeutung haben,
umsetzt, und diese anschließend einer schwefelsauren Alkoholyse unterwirft.

5. Verbindungen der Formel (XVII) 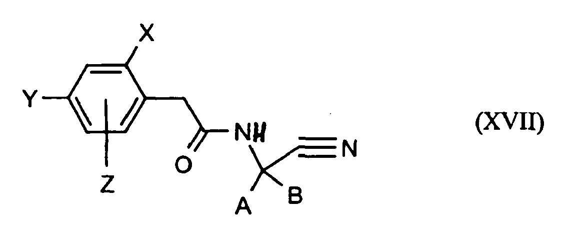 in welcher
A, B, X, Y und Z die in Anspruch 1 angegebene Bedeutung haben.

6. Verfahren zur Herstellung von Verbindungen der Formel (XVII) gemäß Anspruch 9, **dadurch gekennzeichnet, dass** man Aminonitrile der Formel (XVI) 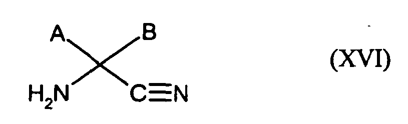 in welcher
A und B die in Anspruch 1 angegebene Bedeutung haben,
mit Phenylessigsäurehalogeniden der Formel (XV) 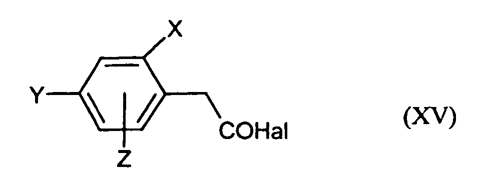 in welcher
X, Y und Z die in Anspruch 1 angegebene Bedeutung haben und
Hal für Chlor oder Brom steht,
umsetzt.

7. Phenylessigsäurehalogenide der Formel (XV) 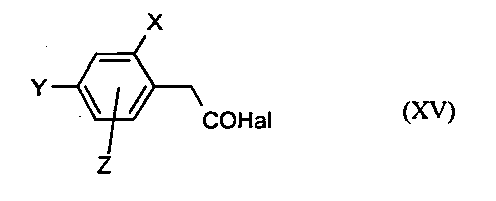 in welcher
X, Y und Z die oben angegebene Bedeutung haben und
Hal für Brom oder Chlor steht,
mit Ausnahme der Verbindung 2-Chlor-4-methoxyphenylessigsäurechlorid.

8. Schädlingsbekämpfungsmittel, **gekennzeichnet durch** einen Gehalt an mindestens einem 1-H-3-Aryl-pyrrolidin-2,4-dion-derivat der Formel (I) gemäß Anspruch 1.

9. Verwendung von 1-H-3-Aryl-pyrrolidin-2,4-dion-derivaten der Formel (I) gemäß Anspruch 1 zur Bekämpfung von Schädlingen.

10. Verfahren zur Bekämpfung von Schädlingen, **dadurch gekennzeichnet, dass** man 1-H-3-Aryl-pyrrolidin-2,4-dion-derivate der Formel (I) gemäß Anspruch 1 auf Schädlinge und/oder ihren Lebensraum einwirken lässt.

11. Verfahren zur Herstellung von Schädlingsbekämpfungsmitteln, **dadurch gekennzeichnet, dass** man 1-H-3-Aryl-pyrrolidin-2,4-dion-derivate der Formel (I) gemäß Anspruch 1 mit Streckmitteln und/oder oberflächenaktiven Mitteln vermischt.

12. Verbindungen der Formel (I) gemäß Anspruch 1, in welcher die Substituenten die in der Tabelle angegebenen Bedeutungen haben:
| **X** | **Y** | **Z** | **A** | **B** | **G** |
|---|---|---|---|---|---|
| Cl | H | 6-OCH₃ | i-C₃H₇ | CH₃ | H |
| Cl | OCH₃ | H | i-C₃H₇ | CH₃ | H |
| OCH₃ | Cl | H | i-C₃H₇ | CH₃ | H |
| Cl | OCH₃ | H | 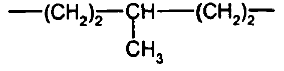 | | H |
| Cl | OCH₃ | H | 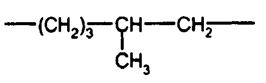 | | H |
| Cl | OCH₃ | H | -(CH₂)₅- | | H |
| Cl | OCH₃ | H | CH₃ | CH₃ | H |
| OCH₃ | Cl | H | 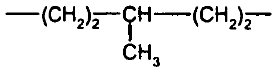 | | H |
| Cl | H | 6-OCH₃ | 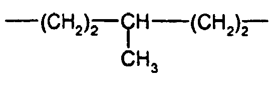 | | H |
| Cl | H | 6-OCH₃ | i-C₃H₇ | CH₃ | 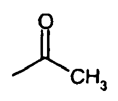 |
| Cl | OCH₃ | H | i-C₃H₇ | CH₃ | 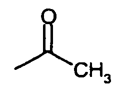 |
| Cl | OCH₃ | H | i-C₃H₇ | CH₃ | 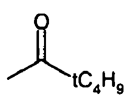 |
| OCH₃ | Cl | H | i-C₃H₇ | CH₃ | 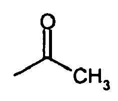 |
| OCH₃ | Cl | H | i-C₃H₇ | CH₃ | 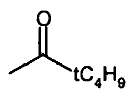 |
| Cl | OCH₃ | H | 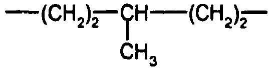 | | 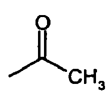 |
| Cl | OCH₃ | H | 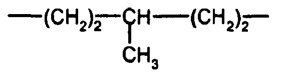 | | 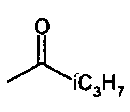 |
| Cl | OCH₃ | H | 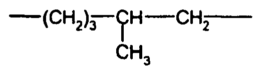 | | 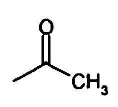 |
| Cl | OCH₃ | H | 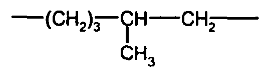 | | 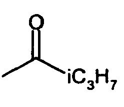 |
| OCH₃ | Cl | H | 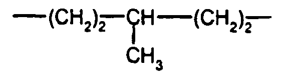 | | 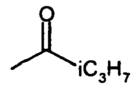 |
| Cl | H | 6-OCH₃ | 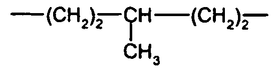 | | 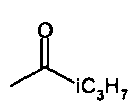 |
| Cl | OCH₃ | H | i-C₃H₇ | CH₃ | 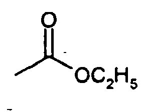 |
| OCH₃ | Cl | H | i-C₃H₇ | CH₃ | 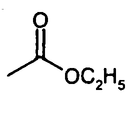 |
| Cl | OCH₃ | H | 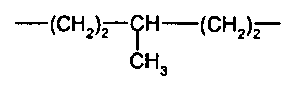 | | 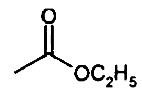 |
| Cl | OCH₃ | H | 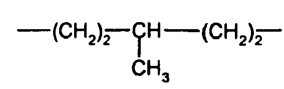 | | 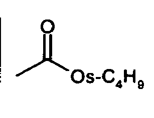 |
| Cl | OCH₃ | H | 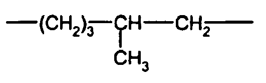 | | 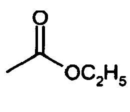 |
| Cl | OCH₃ | H | 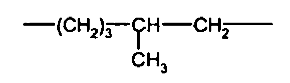 | | 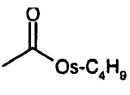 |
| OCH₃ | Cl | H | 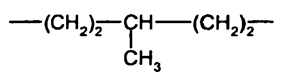 | | 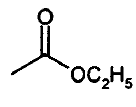 |
| Cl | H | 6-OCH₃ | 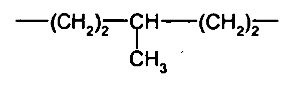 | | 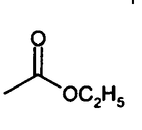 |
| Cl | H | 6-OCH₃ | i-C₃H₇ | CH₃ | 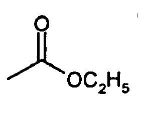 |

## Claims

1. 1-H-3-Aryl-pyrrolidine-2,4-dione derivatives of the formula (I) 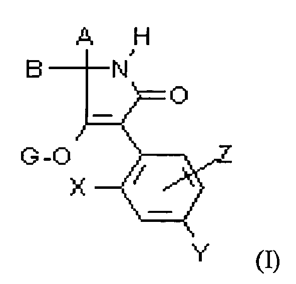 in which
A represents hydrogen, or represents C₁-C₈-alkyl, C₃-C₄-allcenyl, C₁-C₆-alkoxy-C₂-C₄-alkyl, C₁-C₄-polyalkoxy-C₂-C₄-alkyl, C₁-C₆-alkylthio-C₂-C₄-alkyl or cycloalkyl having 3 to 6 ring atoms which can be interrupted by 1 to 2 oxygen and/or sulphur atoms, in each case optionally substituted by halogen, or represents phenyl, thienyl, pyridyl, imidazolyl, pyrazolyl, triazolyl, indolyl, thiazolyl or phenyl-C₁-C₃-alkyl, in each case optionally substituted by fluorine, chlorine, bromine, methyl, ethyl, propyl, iso-propyl, methoxy, ethoxy, trifluoromethyl and/or nitro.
B represents hydrogen, C₁-C₈-alkyl or C₁-C₄-alkoxyalkyl, or
A, B and the carbon atom to which they are bonded represent a saturated or unsaturated C₃-C₈-spirocycle which is optionally monosubstituted or polysubstituted by methyl, ethyl, propyl, isopropyl, butyl, iso-butyl, sec-butyl, tert-butyl, cyclohexyl, trifluoromethyl, methoxy, ethoxy, propoxy, iso-propoxy, butoxy, iso-butoxy, sec-butoxy, tert-butoxy, methylthio, ethylthio, fluorine, chlorine or phenyl and optionally interrupted by oxygen or sulphur, or
A, B and the carbon atom to which they are bonded represent a C₃-C₆-spirocycle which is substituted by an alkylenediyl group which is optionally interrupted by an oxygen or sulphur atom or by an alkylenedioxy group, and this alkylenediyl or alkylenedioxy group together with the carbon atom to which it is bonded forms a further five- to seven-membered spirocycle, or
A, B and the carbon atom to which they are bonded represent a C₃-C₆-spirocycle in which two substituents together represent a saturated or unsaturated five- or six-membered cycle which can be interrupted by oxygen or sulphur,
X represents chlorine,
Y represents methoxy,
Z represents hydrogen,
or
X represents chlorine,
Y represents hydrogen,
Z represents methoxy in the 6-position,
or
X represents methoxy,
Y represents chlorine,
Z represents hydrogen,
G represents hydrogen (a) or the groups 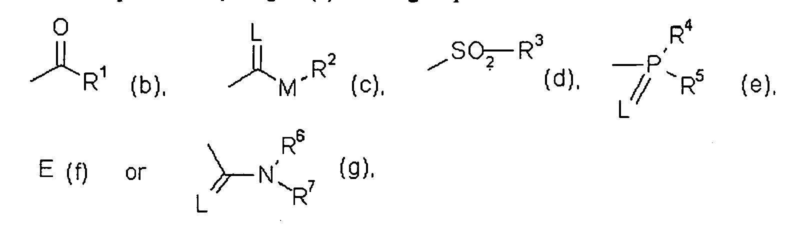 in which
E represents a metal ion equivalent or an ammonium ion and
L and M in each case represent oxygen or sulphur.
R¹ represents C₁-C₁₄-alkyl, C₂-C₁₄-alkenyl, C₁-C₄-alkoxy-C₁-C₆-alkyl, C₁₋C₄-alkylthio-C₁-C₆-alkyl, C₁-C₄-polyalkoxy-C₂-C₄-alkyl or cycloalkyl having 3 to 6 ring atoms which can be interrupted by 1 to 2 oxygen and/or sulphur atoms, in each case optionally substituted by fluorine and/or chlorine,
phenyl which is optionally substituted by fluorine, chlorine, bromine, methyl, ethyl, propyl, i-propyl, methoxy, ethoxy, trifluoromethyl, trifluoromethoxy, methylthio, ethylthio, methylsulphonyl, ethylsulphonyl and/or nitro,
phenyl-C₁-C₃-alkyl which is optionally substituted by fluorine, chlorine, bromine, methyl, ethyl, propyl, i-propyl, methoxy, ethoxy, trifluoromethyl and/or trifluoromethoxy,
thienyl, furanyl, pyridyl, pyrimidyl, thiazolyl or pyrazolyl, in each case optionally substituted by fluorine, chlorine, bromine, methyl and/or ethyl,
phenoxy-C₁-C₄-alkyl which is optionally substituted by fluorine, chlorine, methyl and/or ethyl, or
pyridyloxy-C₁-C₄-alkyl, pyrimidyloxy-C₁-C₄-alkyl or thiazolyloxy-C₁-C₄-alkyl, in each case optionally substituted by fluorine, chlorine, amino, methyl and/or ethyl,
R² represents C₁-C₁₄-alkyl, C₃-C₁₄-alkenyl, C₁-C₄-alkoxy-C₂-C₆-alkyl or C₁-C₄-polyalkoxy-C₂-C₆-alkyl, in each case optionally substituted by fluorine and/or chlorine,
C₃-C₆-cycloalkyl which is optionally substituted by fluorine, chlorine, methyl, ethyl, propyl, iso-propyl and/or methoxy,
or phenyl or benzyl, in each case optionally substituted by fluorine, chlorine, nitro, methyl, ethyl, propyl, i-propyl, methoxy, ethoxy and/or trifluoromethyl.
R³, R⁴ and R⁵ independently of one another represent C₁-C₄-alkyl, C₁-C₄-alkoxy, C₁-C₄-alkylamino, di-(C₁-C₄)-alkylamino or C₁-C₄-alkylthio, in each case optionally substituted by fluorine and/or chlorine, or phenyl, phenoxy or phenylthio, in each case optionally substituted by fluorine, chlorine, bromine, nitro, cyano, C₁-C₂-alkoxy, C₁-C₂-fluoroalkoxy, C₁-C₂-alkylthio, C₁-C₂-fluoroalkylthio and/or C₁-C₃-alkyl, and
R⁶ and R⁷ independently of one another represent hydrogen, or C₁-C₄-alkyl, C₃-C₆-cycloalkyl, C₁-C₄-alkoxy, C₃-C₄-alkenyl or C₁-C₄-alkoxy-C₂-C₄-alkyl, in each case optionally substituted by fluorine, chlorine and/or bromine, or phenyl which is optionally substituted by fluorine, chlorine, bromine, C₁-C₄-halogenoalkyl, C₁-C₄-alkyl and/or C₁-C₄-alkoxy, or benzyl which is optionally substituted by fluorine, chlorine, bromine, C₁-C₄-alkyl, C₁-C₄-halogenoalkyl, and/or C₁-C₄-alkoxy, or together with the N atom to which they are bonded represent a C₃-C₆-alkylene ring which is optionally interrupted by oxygen or sulphur.

2. Process for the preparation of the 1-H-3-aryl-pyrrolidine-2,4-dione derivatives of the formula (I) according to Claim 1, **characterized in that**
(A) to obtain 1-H-3-aryl-pyrrolidine-2,4-diones or their enols of the formula (Ia) 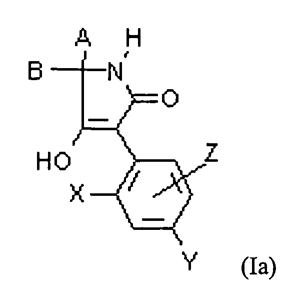 in which
A, B, X, Y and Z have the meaning given in Claim 1,
α) N-acylamino acid esters of the formula (II) 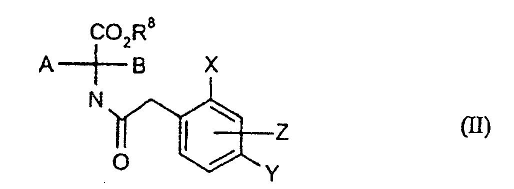 in which
A, B, X, Y and Z have the abovementioned meaning,
and
R⁸ represents alkyl
are subjected to an intramolecular condensation reaction in the presence of a diluent and in the presence of a base;
or
β) to obtain 1-H-3-aryl-pyrrolidine-2,4-diones or their enols of the formula (Ia-a) 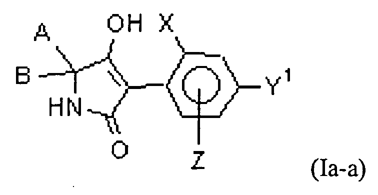 in which
A, B, X and Z have the abovementioned meanings
and
Y¹ represents -OR⁸, where
R⁸ represents alkyl,
N-acylamino acid esters of the formula (IIa) 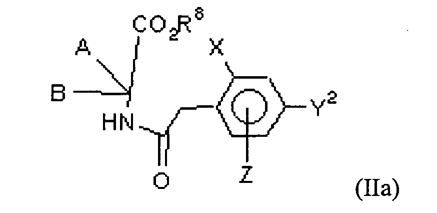 in which
A, B, X and Z have the abovementioned meanings,
Y² represents fluorine and
R⁸ represents alkyl
are subjected to an intramolecular condensation reaction in the presence of a diluent and in the presence of a base;
or
(B) to obtain compounds of the formula (Ib) 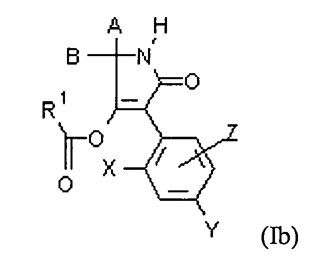 in which
A, B, X, Y, Z and R¹ have the meaning given in Claim 1,
compounds of the formula (Ia), 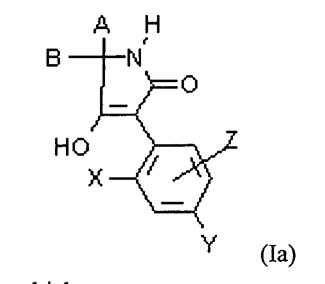 m which
A, B, X, Y and Z have the abovementioned meaning
α) are reacted with acid halides of the general formula (III) 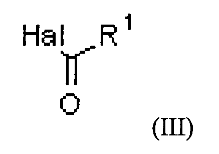 in which
R¹ has the abovementioned meaning and
Hal represents halogen,
if appropriate in the presence of a diluent and if appropriate in the presence of an acid-binding agent,
or
β) are reacted with carboxylic anhydrides of the general formula (IV)
R¹-CO-O-CO-R¹ (IV)
in which
R¹ has the abovementioned meaning,
if appropriate in the presence of a diluent and if appropriate in the presence of an acid-binding agent;
or
(C) to obtain compounds of the formula (Ic-a) 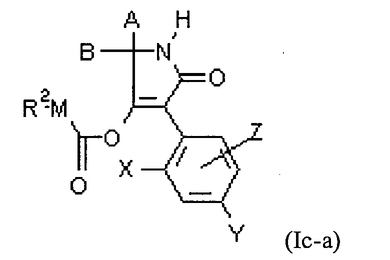 in which
A, B, X, Y, Z and R² have the meaning given in Claim 1
and
M represents oxygen or sulphur,
compounds of the formula (Ia) 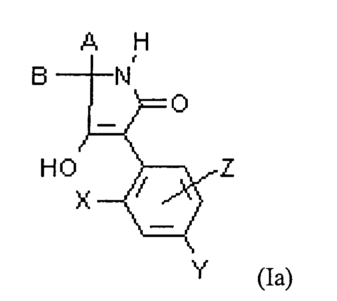 in which
A, B, X, Y and Z have the abovementioned meaning
are reacted with chloroformic esters or chloroformic thioesters of the general formula (V)
R²-M-CO-Cl (V)
in which
R² and M have the abovementioned meaning,
if appropriate in the presence of a diluent and if appropriate in the presence of an acid-binding agent;
or
(D) to obtain compounds of the formula (Ic-b) 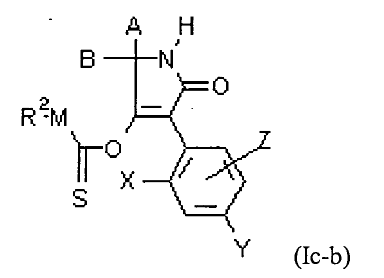 in which
A, B, R², X, Y and Z have the abovementioned meaning
and
M represents oxygen or sulphur,
compounds of the formula (Ia) 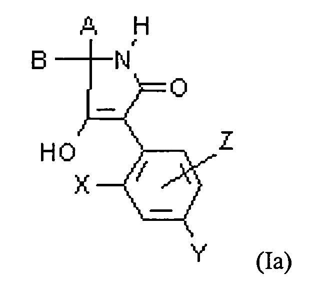 in which
A, B, X, Y and Z have the abovementioned meaning
α) are reacted with chloromonothioformic esters or chlorodithioformic esters of the general formula (VI) 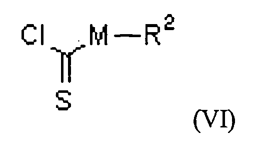 in which
M and R² have the abovementioned meaning,
if appropriate in the presence of a diluent and if appropriate in the presence of an acid-binding agent,
or
β) are reacted with carbon disulphide and subsequently with alkyl halides of the general formula (VII)
R²-Hal (VII)
in which
R² has the abovementioned meaning
and
Hal represents chlorine, bromine or iodine;
or
(E) to obtain compounds of the formula (Id) 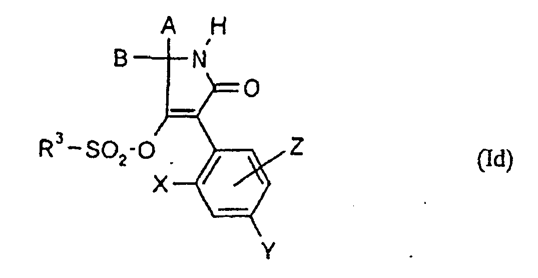 in which
A, B, X, Y, Z and R³ have the meaning given in Claim 1,
compounds of the formula (Ia) 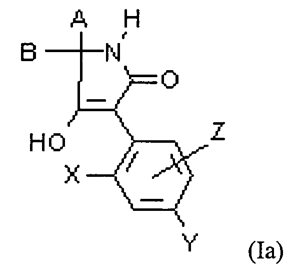 in which
A, B, X, Y and Z have the abovementioned meaning
are reacted with sulphonyl chlorides of the general formula (VIII)
R³-SO₂-Cl (VIII)
in which
R³ has the abovementioned meaning,
if appropriate in the presence of a diluent and if appropriate in the presence of an acid-binding agent;
or
(F) to obtain 3-aryl-pyrrolidine-2,4-diones of the formula (Ie) 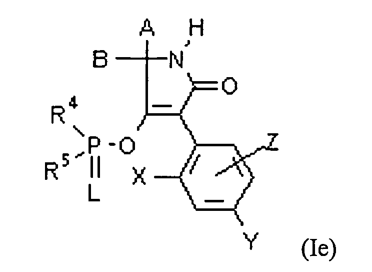 in which
A, B, L, X, Y, Z, R⁴ and R⁵ have the meaning given in Claim 1,
1-H-3-aryl-pyrrolidine-2,4-diones of the formula (Ia) or their enols 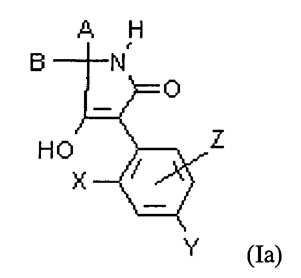 in which
A, B, X, Y and Z have the abovementioned meaning
are reacted with phosphorus compounds of the general formula (IX) 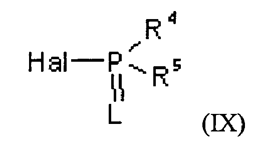 in which
L, R⁴ and R⁵ have the abovementioned meaning
and
Hal represents halogen;
or
(G) to obtain compounds of the formula (If) 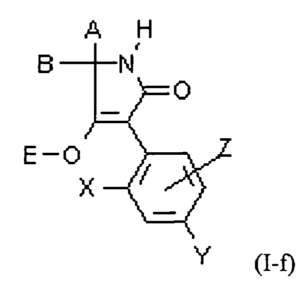 in which
A, B, X, Y and Z have the abovementioned meaning
and
E represents a metal ion equivalent or an ammonium ion,
compounds of the formula (Ia) 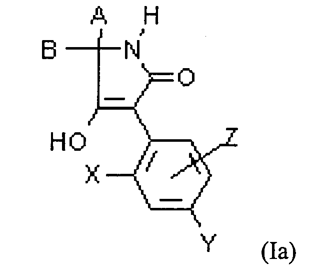 in which
A, B, X, Y and Z have the abovementioned meaning
are reacted with metal compounds or amines of the general formulae (X) and (XI) 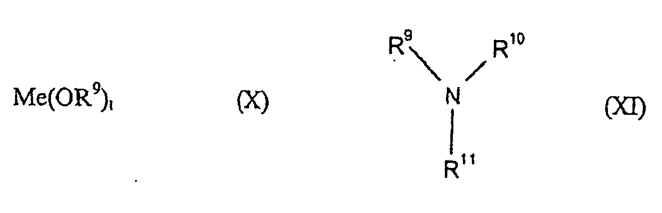 in which
Me represents mono- or divalent metal ions,
t represents the number 1 or 2 and
R⁹, R¹⁰ and R¹¹ independently of one another represent hydrogen and/or alkyl,
if appropriate in the presence of a diluent,
or
(H) to obtain compounds of the formula (Ig) 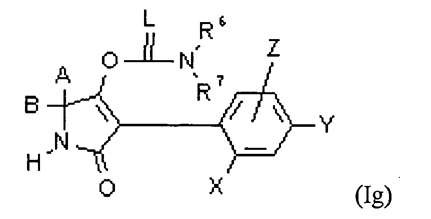 in which
A, B, L, X, Y, Z, R⁶ and R⁷ have the meaning given in Claim 1,
compounds of the formula (Ia) 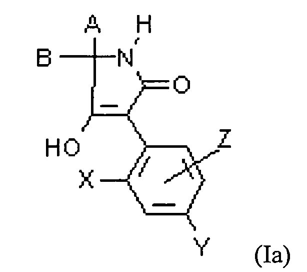 in which
A, B, X, Y and Z have the abovementioned meaning
α) are reacted with isocyanates or isothiocyanates of the general formula (XII)
R⁶-N=C=L (XII)
in which
R⁶ has the abovementioned meaning,
if appropriate in the presence of a diluent and if appropriate in the presence of a catalyst,
or
β) are reacted with carbamoyl chlorides or thiocarbamoyl chlorides of the general formula (XIII) 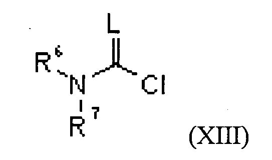 in which
L, R⁶ and R⁷ have the abovementioned meaning,
if appropriate in the presence of a diluent and if appropriate in the presence of an acid-binding agent.

3. Compounds of the formula (II) 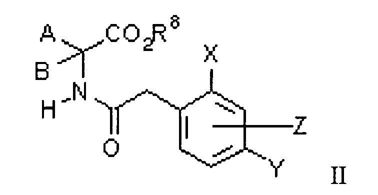 in which
A, B, X, Y and Z have the meaning given in Claim 1 and R⁸ represents alkyl.

4. Process for the preparation of the acyl-amino acid esters of the formula (II) according to Claim 3, **characterized in that** amino acid derivatives of the formula (XIV) 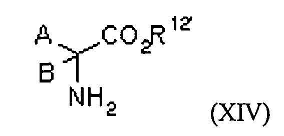 in which
R¹²' represents hydrogen (XIVa) or alkyl (XIVb)
and
A and B have the meaning given in Claim 1,
are acylated with phenylacetyl halides of the formula (XV) 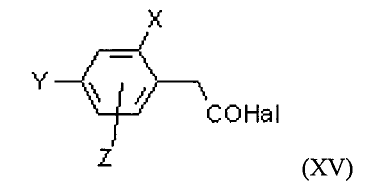 in which
X, Y and Z have the abovementioned meaning and
Hal represents chlorine or bromine,
and, if R¹²' = hydrogen, the acyl amino acids of the formula (IIa) which have formed, 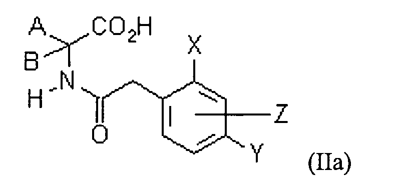 in which
A, B, X, Y and Z have the abovementioned meaning
are esterified or
aminonitriles of the formula (XVI) 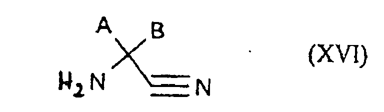 in which
A and B have the abovementioned meaning
are reacted with phenylacetyl halides of the formula (XV) 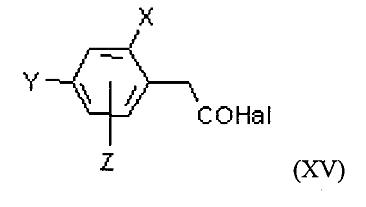 in which
X, Y and Z have the abovementioned meaning and
Hal represents chlorine or bromine,
to give compounds of the formula (XVII) 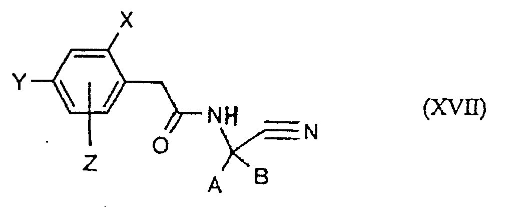 in which
A, B, X, Y and Z have the abovementioned meaning,
and these compounds are subsequently subjected to alcoholysis in the presence of sulphuric acid.

5. Compounds of the formula (XVII) 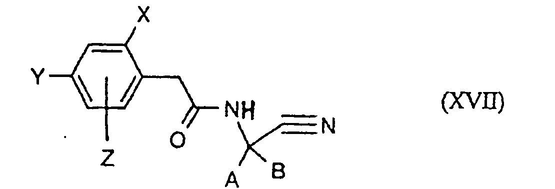 in which
A, B, X, Y and Z have the meaning given in Claim 1.

6. Process for the preparation of compounds of the formula (XVII) according to Claim 5, **characterized in that** aminonitriles of the formula (XVI) 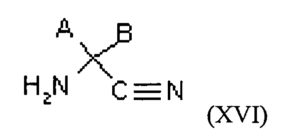 in which
A and B have the meaning given in Claim 1,
are reacted with phenylacetyl halides of the formula (XV) 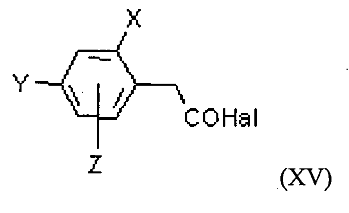 in which
X, Y and Z have the meaning given in Claim 1 and
Hal represents chlorine or bromine.

7. Phenylacetyl halides of the formula (XV) 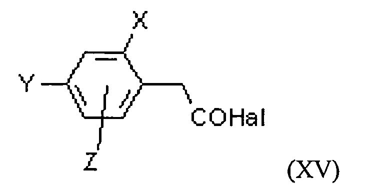 in which
X, Y and Z have the abovementioned meaning and
Hal represents bromine or chlorine,
with the exception of the compound 2-chloro-4-methoxyphenylacetyl chloride.

8. Pesticides **characterized by** a content of at least one 1-H-3-aryl-pyrrolidine-2,4-dione derivative of the formula (I) according to Claim 1.

9. Use of 1-H-3-aryl-pyrrolidine-2,4-dione derivatives of the formula (I) according to Claim 1 for combating pests.

10. Method of combating pests, **characterized in that** 1-H-3-aryl-pyrrolidine-2,4-dione derivatives of the formula (I) according to Claim 1 are allowed to act on pests and/or their environment.

11. Process for the preparation of pesticides, **characterized in that** 1-H-3-arylpyrrolidine-2,4-dione derivatives of the formula (I) according to Claim 1 are mixed with extenders and/or surface-active agents.

12. Compounds of the formula (I) according to Claim 1, in which the substituents have the meanings given in the table:
| **X** | **Y** | **Z** | **A** | **B** | **G** |
|---|---|---|---|---|---|
| Cl | H | 6-OCH₃ | i-C₃H₇ | CH₃ | H |
| Cl | OCH₃ | H | i-C₃H₇ | CH₃ | H |
| OCH₃ | Cl | H | i-C₃H₇ | CH₃ | H |
| Cl | OCH₃ | H | 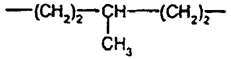 | | H |
| Cl | OCH₃ | H | 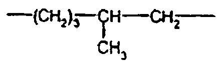 | | H |
| Cl | OCH₃ | H | -(CH₂)₅- | | H |
| Cl | OCH₃ | H | CH₃ | CH₃ | H |
| OCH₃ | Cl | H | 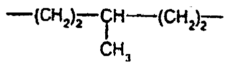 | | H |
| Cl | H | 6-OCH₃ | 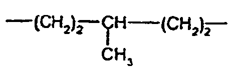 | | H |
| Cl | H | 6-OCH₃ | i-C₃H₇ | CH₃ | 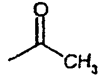 |
| Cl | OCH₃ | H | i-C₃H₇ | CH₃ | 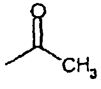 |
| Cl | OCH₃ | H | i-C₃H₇ | CH₃ | 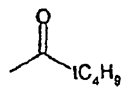 |
| OCH₃ | Cl | H | i-C₃H₇ | CH₃ | 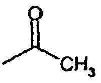 |
| OCH₃ | Cl | H | i-C₃H₇ | CH₃ | 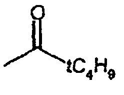 |
| Cl | OCH₃ | H | 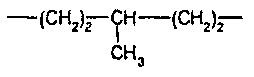 | | 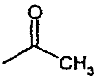 |
| Cl | OCH₃ | H | 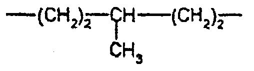 | | 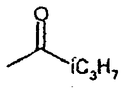 |
| Cl | OCH₃ | H | 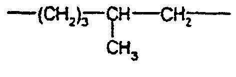 | | 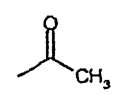 |
| Cl | OCH₃ | H | 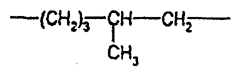 | | 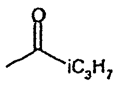 |
| OCH₃ | Cl | H | 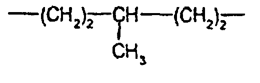 | | 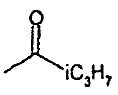 |
| Cl | H | 6-OCH₃ | 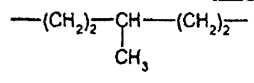 | | 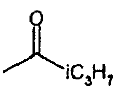 |
| Cl | OCH₃ | H | i-C₃H₇ | CH₃ | 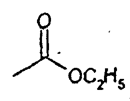 |
| OCH₃ | Cl | H | i-C₃H₇ | CH₃ | 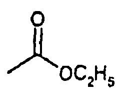 |
| Cl | OCH₃ | H | 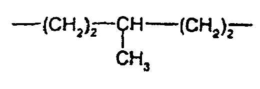 | | 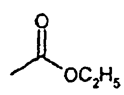 |
| Cl | OCH₃ | H | 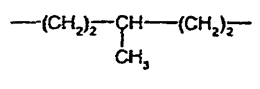 | | 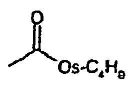 |
| Cl | OCH₃ | H | 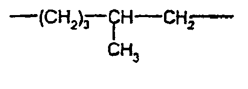 | | 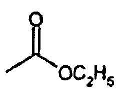 |
| Cl | OCH₃ | H | 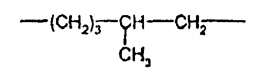 | | 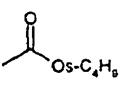 |
| OCH₃ | Cl | H | 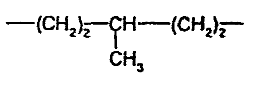 | | 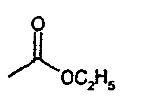 |
| Cl | H | 6-OCH₃ | 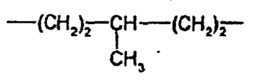 | | 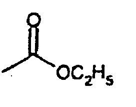 |
| Cl | H | 6-OCH₃ | i-C₃H₇ | CH₃ | 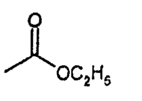 |

## Revendications

1. Dérivés de 1-H-3-aryl-pyrrolidine-2,4-diones de formule (I) : 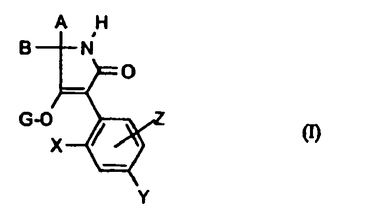 dans laquelle
A représente l'hydrogène, un reste alkyle en C₁ à C₈, alcényle en C₃ ou C₄, (alkoxy en C₁ à C₆) - (alkyle en C₂ à C₄), poly(alkoxy en C₁ à C₄)-(alkyle en C₂ à C₄), (alkylthio en C₁ à C₆) - (alkyle en C₂ à C₄), cycloalkyle à noyau de 3 à 6 atomes pouvant être interrompu par un ou deux atomes d'oxygène et/ou de soufre, chaque reste éventuellement substitué par un halogène, ou un reste phényle, thiényle, pyridyle, imidazolyle, pyrazolyle, triazolyle, indolyle, thiazole ou phényl-(alkyle en C₁ à C₃), chacun éventuellement substitué par un radical fluoro, chloro, bromo, méthyle, éthyle, propyle, isopropyle, méthoxy, éthoxy, trifluorométhyle et/ou nitro,
B représente l'hydrogène, un reste alkyle en C₁ à C₈ ou (alkoxy en C₁ à C₄)alkyle, ou bien
A, B et l'atome de carbone auquel ils sont liés représentent un cycle spiro en C₃ à C₈ saturé ou non saturé, qui est éventuellement substitué une ou plusieurs fois par un radical méthyle, éthyle, propyle, isopropyle, butyle, isobutyle, sec.-butyle, tertiobutyle, cyclohexyle, trifluorométhyle, méthoxy, éthoxy, propoxy, isopropoxy, butoxy, isobutoxy, sec.-butoxy, tertiobutoxy, méthylthio, éthylthio, fluoro, chloro ou phényle et qui est éventuellement interrompu par de l'oxygène ou du soufre, ou bien
A, B et l'atome de carbone auquel ils sont liés représentent un cycle spiro en C₃ à C₆, qui est
substitué par un groupe alkylènediyle éventuellement interrompu par un atome d'oxygène ou de soufre ou par un groupe alkylènedioxyle qui forme un autre cycle spiro pentagonal à heptagonal avec l'atome de carbone auquel il est lié,
ou bien
A, B et l'atome de carbone auquel ils sont liés représentent un cycle spiro en C₃ à C₆ dans lequel deux substituants forment ensemble un cycle pentagonal ou hexagonal saturé ou non saturé, qui peut être interrompu par de l'oxygène ou du soufre,
X représente le chlore,
Y représente un groupe méthoxy,
Z représente l'hydrogène,
ou bien
X représente le chlore,
Y représente l'hydrogène,
Z représente un groupe méthoxy en position 6,
ou bien
X représente un groupe méthoxy,
Y représente le chlore,
Z représente l'hydrogène,
G représente l'hydrogène (a) ou les groupes : 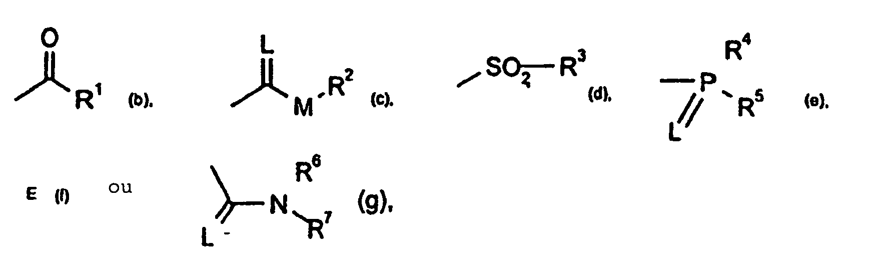 dans lesquels :
E représente un équivalent d'ion métallique ou un ion ammonium, et
L et M représentent chacun l'oxygène ou le soufre,
R¹ représente un reste alkyle en C₁ à C₁₄, alcényle en C₂ à C₁₄, (alkoxy en C₁ à C₄)-(alkyle en C₁ à C₆), (alkylthio en C₁ à C₄)-(alkyle en C₁ à C₆), poly(alkoxy en C₁ à C₄)-(alkyle en C₂ à C₄) ou cycloalkyle à noyau de 3 à 6 atomes pouvant être interrompu par 1 ou 2 atomes d'oxygène et/ou de soufre, chaque reste étant éventuellement substitué par du fluor et/ou du chlore, un reste phényle éventuellement substitué par un radical fluoro, chloro, bromo, méthyle, éthyle, propyle, isopropyle, méthoxy, éthoxy, trifluorométhyle, trifluorométhoxy, méthylthio, éthylthio, méthylsulfonyle, éthylsulfonyle et/ou nitro,
un reste phényl-(alkyle en C₁ à C₃) éventuellement substitué par un radical fluoro, chloro, bromo, méthyle, éthyle, propyle, isopropyle, méthoxy, éthoxy, trifluorométhyle et/ou trifluorométhoxy,
un reste thiényle éventuellement substitué par un radical fluoro, chloro, bromo, méthyle et/ou éthyle, furannyle, pyridyle, pyrimidyle, thiazolyle ou pyrazolyle,
un reste phénoxy-(alkyle en C₁ à C₄) éventuellement substitué par un radical fluoro, chloro, méthyle et/ou éthyle,
un reste pyridyloxy-(alkyle en C₁ à C₄), pyrimidyloxy(alkyle en C₁ à C₄) ou thiazolyloxy-(alkyle en C₁ à C₄), chacun éventuellement substitué par un radical fluoro, chloro, amino, méthyle et/ou éthyle,
R² représente un reste alkyle en C₁ à C₁₄, alcényle en C₃ à C₁₄, (alkoxy en C₁ à C₄) - (alkyle en C₂ à C₆), poly(alkoxy en C₁ à C₄)-(alkyle en C₂ à C₆), chacun éventuellement substitué par du fluor et/ou du chlore, un reste cycloalkyle en C₃ à C₆ éventuellement substitué par un radical fluoro, chloro, méthyle, éthyle, propyle, isopropyle et/ou méthoxy,
ou bien un reste phényle ou benzyle, chacun éventuellement substitué par un radical fluoro, chloro, nitro, méthyle, éthyle, propyle, isopropyle, méthoxy, éthoxy et/ou trifluorométhyle,
R³, R⁴ et R⁵ représentent indépendamment les uns des autres un reste alkyle en C₁ à C₄, alkoxy en C₁ à C₄, alkylamino en C₁ à C₄, di (alkyle en C₁ à C₄)amino, alkylthio en C₁ à C₄, chacun éventuellement substitué par du fluor et/ou du chlore, un reste phényle, phénoxy ou phénylthio, chacun éventuellement substitué par un radical fluoro, chloro, bromo, nitro, cyano, alkoxy en C₁ ou C₂, fluoralkoxy en C₁ ou C₂, alkylthio en C₁ ou C₂, fluoralkylthio en C₁ ou C₂ et/ou alkyle en C₁ à C₃, et
R⁶ et R⁷ représentent indépendamment l'un de l'autre l'hydrogène, un reste alkyle en C₁ à C₄, cycloalkyle en C₃ à C₆, alkoxy en C₁ à C₄, alcényle en C₃ ou C₄, (alkoxy en C₁ à C₄)-(alkyle en C₂ à C₄), chacun éventuellement substitué par du fluor, du chlore et/ou du brome, un reste phényle éventuellement substitué par un radical fluoro, chloro, bromo, halogénalkyle en C₁ à C₄, alkyle en C₁ à C₄ et/ou alkoxy en C₁ à C₄, un reste benzyle éventuellement substitué par un radical fluoro, chloro, bromo, alkyle en C₁ à C₄, halogénalkyle en C₁ à C₄ et/ou alkoxy en C₁ à C₄, forment, conjointement avec l'atome d'azote auquel ils sont liés, un noyau alkylène en C₃ à C₆ éventuellement interrompu par de l'oxygène ou du soufre.

2. Procédé de production des dérivés de 1-H-3-arylpyrrolidine-2,4-diones de formule (I) suivant la revendication 1, **caractérisé en ce que**
(A) pour obtenir des 1-H-3-aryl-pyrrolidine-2,4-diones ou leurs énols de formule (Ia) : 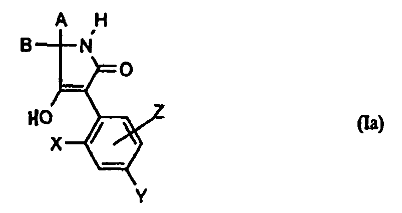 dans laquelle :
A, B, X, Y et Z ont la définition indiquée dans la revendication 1,
α) des esters de N-acylaminoacides de formule (II) : 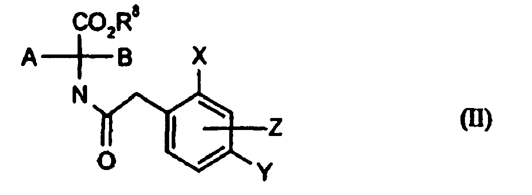 dans laquelle :
A, B, X, Y et Z ont la définition indiquée ci-dessus, et
R⁸ représente un reste alkyle,
sont soumis à une condensation intramoléculaire en présence d'un diluant et en présence d'une base ;
ou bien
β) pour obtenir des 1-H-3-aryl-pyrrolidine-2,4-diones ou leurs énols de formule (Ia-a) : 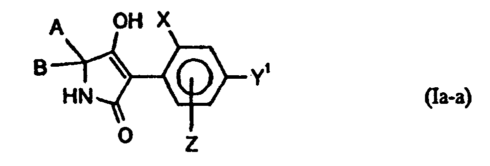 dans laquelle :
A, B, X et Z ont les définitions indiquées ci-dessus, et
Y¹ représente un groupe -OR⁸, dans lequel
R⁸ est un reste alkyle,
des esters de N-acylaminoacides de formule (IIa) : 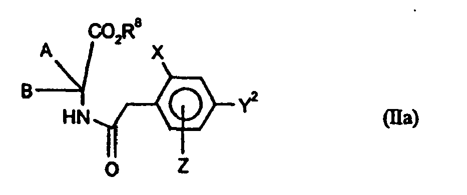 dans laquelle :
A, B, X et Z ont les définitions indiquées ci-dessus,
Y² représente le fluor, et
R⁸ représente un reste alkyle,
sont soumis à une condensation intramoléculaire en présence d'un diluant et en présence d'une base ;
ou bien
(B) pour obtenir des composés de formule (Ib) : 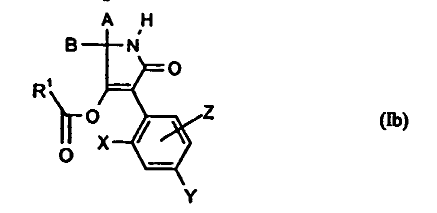 dans laquelle :
A, B, X, Y, Z et R¹ ont la définition indiquée dans la revendication 1,
des composés de formule (Ia) : 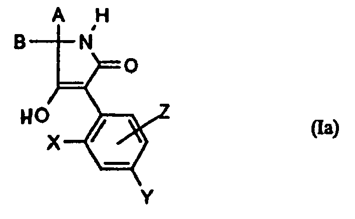 dans laquelle :
A, B, X, Y et Z ont la définition indiquée ci-dessus,
α) sont amenés à réagir avec des halogénures d'acides de formule générale (III) : 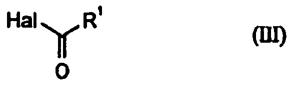 dans laquelle :
R¹ a la définition indiquée ci-dessus et
Hal représente un halogène,
éventuellement en présence d'un diluant et, le cas échéant, en présence d'un accepteur d'acide,
ou bien
β) des anhydrides d'acides carboxyliques de formule générale (IV) :
R¹-CO-O-CO-R¹ (IV)
dans laquelle :
R¹ a la définition indiquée ci-dessus,
sont amenés à réagir éventuellement en présence d'un diluant et, le cas échéant, en présence d'un accepteur d'acide,
ou bien
(C) pour obtenir des composés de formule (Ic-a) : 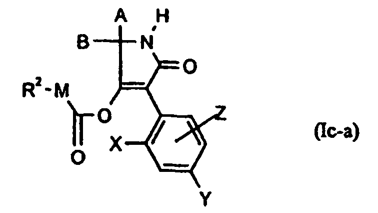 dans laquelle :
A, B, X, Y, Z et R² ont la définition indiquée dans la revendication 1,
et
M représente l'oxygène ou le soufre,
des composés de formule (Ia) : 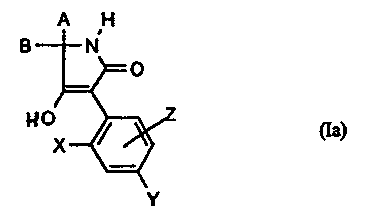 dans laquelle :
A, B, X, Y et Z ont la définition indiquée ci-dessus, sont amenés à réagir avec des esters d'acide chloroformique ou des thioesters d'acide chloroformique de formule générale (V) :
R²-M-CO-Cl (V)
dans laquelle :
R² et M ont la définition indiquée ci-dessus, éventuellement en présence d'un diluant et, le cas échéant, en présence d'un accepteur d'acide ;
ou bien
(D) pour obtenir des composés de formule (Ic-b) : 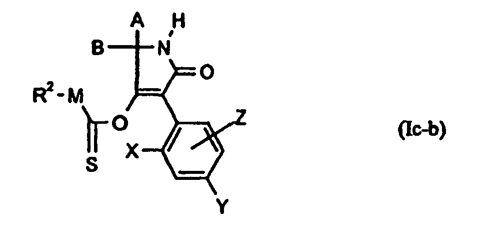 dans laquelle :
A, B, R², X, Y et Z ont la définition indiquée ci-dessus,
et
M représente l'oxygène ou le soufre,
des composés de formule (Ia) : 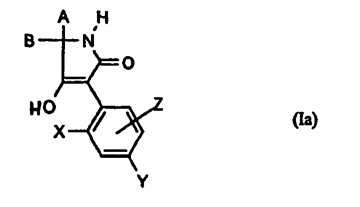 dans laquelle :
A, B, X, Y et Z ont la définition indiquée ci-dessus,
α) sont amenés à réagir avec des esters d'acide chloromonothioformique ou des esters d'acide chlorodithioformiques de formule (VI) : 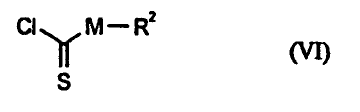 dans laquelle :
M et R² ont la définition indiquée ci-dessus,
éventuellement en présence d'un diluant et, le cas échéant, en présence d'un accepteur d'acide,
ou bien
β) sont amenés à réagir avec le sulfure de carbone puis avec des halogénures d'alkyle de formule générale (VII) :
R²-Hal (VII)
dans laquelle :
R² a la définition indiquée ci-dessus,
et
Hal représente le chlore, le brome ou l'iode ;
ou bien
(E) pour obtenir des composés de formule (Id) : 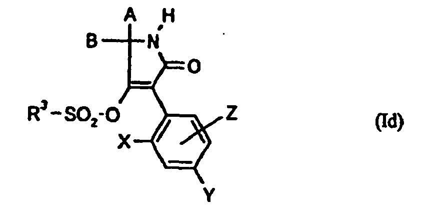 dans laquelle :
A, B, X, Y, Z et R³ ont la définition indiquée dans la revendication 1,
des composés de formule (Ia) : 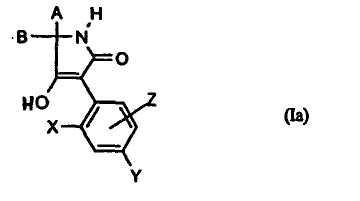 dans laquelle :
A, B, X, Y et Z ont la définition indiquée ci-dessus, sont amenés à réagir avec des chlorures d'acides sulfoniques de formule générale (VIII) :
R³-SO₂-Cl (VIII)
dans laquelle :
R³ a la définition indiquée ci-dessus, éventuellement en présence d'un diluant et, le cas échéant, en présence d'un accepteur d'acide ;
ou bien
(F) pour obtenir des 3-aryl-pyrrolidine-2,4-diones de formule (Ie) : 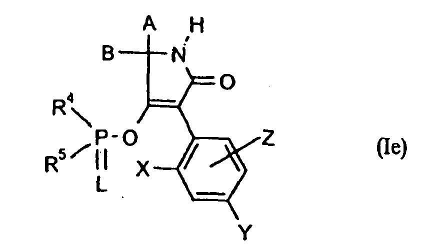 dans laquelle :
A, B, L, X, Y, Z, R⁴ et R⁵ ont la définition indiquée dans la revendication 1,
des 1H-3-aryl-pyrrolidine-2,4-diones de formule (Ia) ou leurs énols : 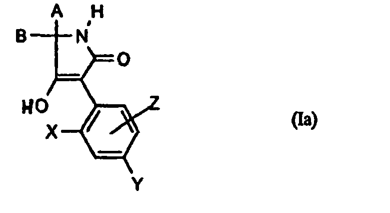 formule dans laquelle :
A, B, X, Y et Z ont la définition indiquée ci-dessus,
sont amenés à réagir avec des composés de phosphore de formule générale (IX) : 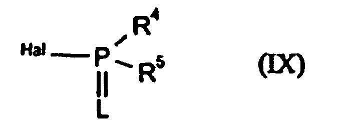 dans laquelle :
L, R⁴ et R⁵ ont la définition indiquée ci-dessus,
et
Hal représente un halogène ;
ou bien
(G) pour obtenir des composés de formule (If) : 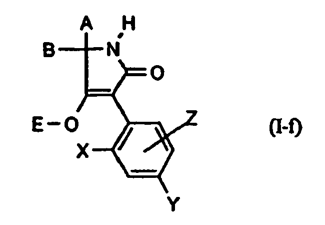 dans laquelle :
A, B, X, Y et Z ont la définition indiquée ci-dessus, et
E représente un équivalent d'ion métallique ou un ion ammonium,
des composés de formule (Ia) : 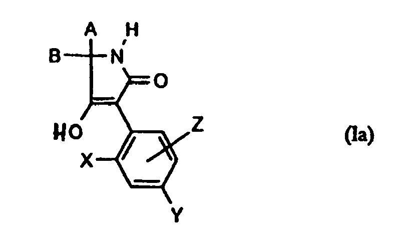 dans laquelle :
A, B, X, Y et Z ont la définition indiquée ci-dessus,
sont amenés à réagir avec des composés métalliques ou des amines de formules générales (X) et (XI) : 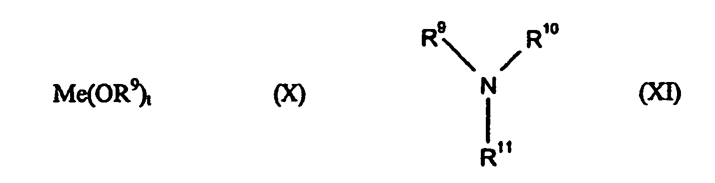 dans lesquelles :
Me représente des ions métalliques monovalents ou divalents,
t représente le nombre 1 ou 2, et
R⁹, R¹⁰ et R¹¹ représentent indépendamment les uns des autres l'hydrogène et/ou un reste alkyle,
éventuellement en présence d'un diluant,
ou bien
(H) pour obtenir des composés de formule (Ig) : 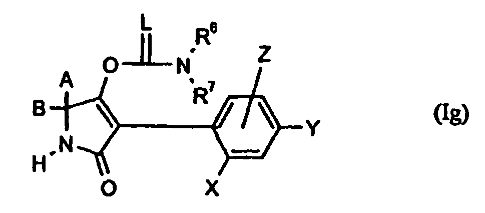 dans laquelle :
A, B, L, X, Y, Z, R⁶ et R⁷ ont la définition indiquée dans la revendication 1,
des composés de formule (Ia) : 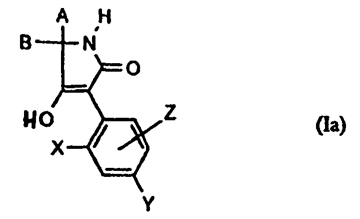 dans laquelle :
A, B, X, Y et Z ont la définition indiquée ci-dessus, sont amenés à réagir
α) avec des isocyanates ou des isothiocyanates de formule générale (XII) :
R⁶-N=C=L (XII)
dans laquelle :
R⁶ a la définition indiquée ci-dessus,
éventuellement en présence d'un diluant et, le cas échéant, en présence d'un catalyseur,
ou bien
b) avec des chlorures d'acides carbamiques ou des chlorures d'acides thiocarbamiques de formule générale (XIII) : 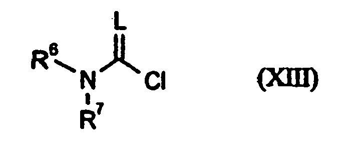 dans laquelle :
L, R⁶ et R⁷ ont la définition indiquée ci-dessus,
éventuellement en présence d'un diluant et, le cas échéant, en présence d'un accepteur d'acide,

3. Composés de formule (II) : 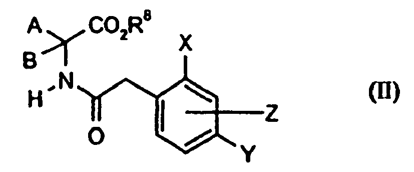 dans laquelle :
A, B, X, Y et Z ont la définition indiquée dans la revendication 1 et R⁸ représente un reste alkyle.

4. Procédé de production des esters d'acylaminoacides de formule (II), suivant la revendication 7, **caractérisé en ce que** des dérivés d'aminoacides de formule (XIV) : 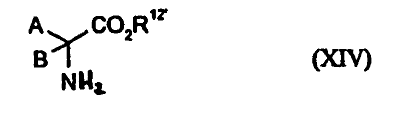 dans laquelle :
R¹²' représente l'hydrogène (XIVa) ou un reste alkyle (XIVb),
et
A et B ont la définition indiquée dans la revendication 1, sont acylés avec des halogénures d'acides phénylacétiques de formules (XV) : 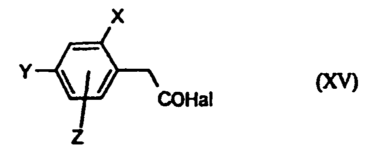 dans laquelle :
X, Y et Z ont la définition indiquée ci-dessus, et
Hal représente le chlore ou le brome,
et les acylaminoacides qui sont alors produits pour R¹²' = hydrogène, de formule (IIa) : 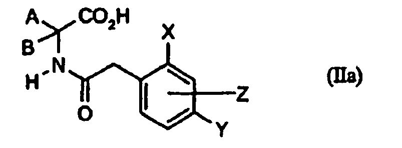 dans laquelle :
A, B, X, Y et Z ont la définition indiquée ci-dessus, sont estérifiés, ou bien
on fait réagir des aminonitriles de formule (XVI) : 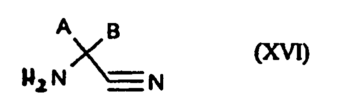 dans laquelle :
A et B ont la définition indiquée ci-dessus,
avec des halogénures d'acide phénylacétique de formule (XV) : 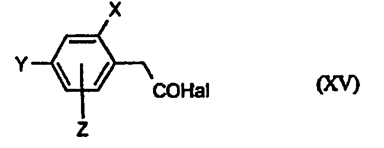 dans laquelle :
X, Y et Z ont la définition indiquée ci-dessus, et
Hal représente le chlore ou le brome,
pour obtenir des composés de formule (XVII) : 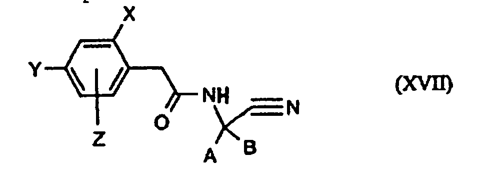 dans laquelle :
A, B, X, Y et Z ont la définition indiquée ci-dessus, puis on soumet ces composés à une alcoolyse sulfurique.

5. Composés de formule (XVII) : 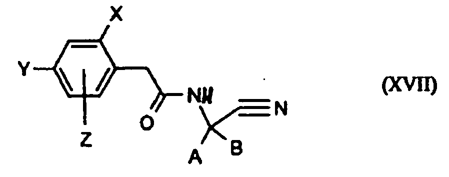 dans laquelle :
dans laquelle:
A, B, X, Y et Z ont la définition indiquée dans la revendication 1.

6. Procédé de production de composés de formule (XVII) suivant la revendication 9, **caractérisé en ce qu'**on fait réagir des aminonitriles de formule (XVI) : 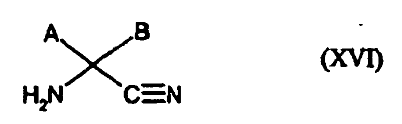 dans laquelle :
A et B ont la définition indiquée dans la revendication 1, avec des halogénures d'acides phénylacétiques de formule (XV) : 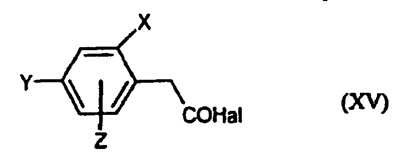 dans laquelle :
X, Y et Z ont la définition indiquée dans la revendication 1, et
Hal représente le chlore ou le brome.

7. Halogénures d'acides phénylacétiques de formule (XV) : 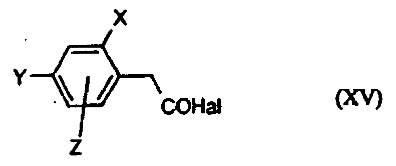 dans laquelle :
X, Y et Z ont la définition indiquée ci-dessus, et
Hal représente le brome ou le chlore,
à l'exception du composé chlorure d'acide 2-chloro-4-méthoxyphénylacétique.

8. Compositions pesticides **caractérisées par** une teneur en au moins un dérivé de 1H-3-aryl-pyrrolidine-2,4-dione de formule (I) suivant la revendication 1.

9. Utilisation de dérivés de 1H-3-aryl-pyrrolidine-2,4-diones de formule (I) suivant la revendication 1 pour combattre des parasites.

10. Procédé de lutte contre des parasites, **caractérisé en ce qu'**on fait agir des dérivés de 1H-3-aryl-pyrrolidine-2,4-diones de formule (I) suivant la revendication 1 sur des parasites et/ou sur leur milieu.

11. Procédé de préparation de compositions pesticides, **caractérisé en ce qu'**on mélange des dérivés de 1H-3-aryl-pyrrolidine-2,4-diones de formule (I) suivant la revendication 1 avec des diluants et/ou des agents tensioactifs.

12. Composés de formule (I) suivant la revendication 1, formule dans laquelle les substituants ont les définitions indiquées dans le tableau :
| **X** | **Y** | **Z** | **A** | **B** | **C** |
|---|---|---|---|---|---|
| Cl | H | 6-OCH₃ | i-C₃H₇ | CH₃ | H |
| Cl | OCH₃ | H | i-C₃H₇ | CH₃ | H |
| OCH₃ | Cl | H | i-C₃H₇ | CH₃ | H |
| Cl | OCH₃ | H | 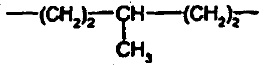 | | H |
| Cl | OCH₃ | H | 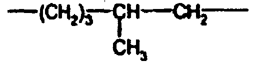 | | H |
| Cl | OCH₃ | H | -(CH₂)₅- | | H |
| Cl | OCH₃ | H | CH₃ | CH₃ | H |
| OCH₃ | Cl | H | 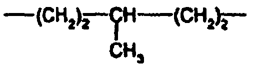 | | H |
| Cl | H | 6-OCH₃ | 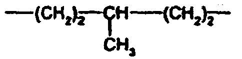 | | H |
| Cl | H | 6-OCH₃ | i-C₃H₇ | CH₃ | 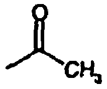 |
| Cl | OCH₃ | H | i-C₃H₇ | CH₃ | 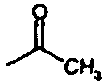 |
| Cl | OCH₃ | H | i-C₃H₇ | CH₃ | 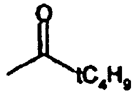 |
| OCH₃ | Cl | H | i-C₃H₇ | CH₃ | 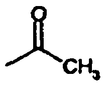 |
| OCH₃ | Cl | H | i-C₃H₇ | CH₃ | 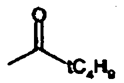 |
| Cl | OCH₃ | H | 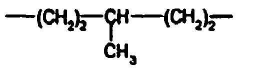 | | 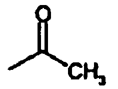 |
| Cl | OCH₃ | H | 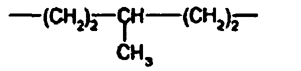 | | 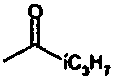 |
| Cl | OCH₃ | H | 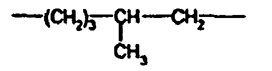 | | 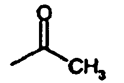 |
| Cl | OCH₃ | H | 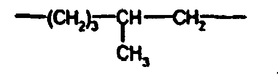 | | 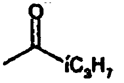 |
| OCH₃ | Cl | H | 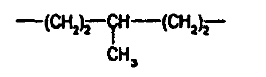 | | 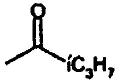 |
| Cl | H | 6-OCH₃ | 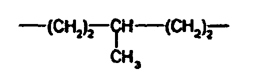 | | 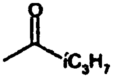 |
| Cl | OCH₃ | H | i-C₃H₇ | CH₃ | 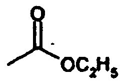 |
| OCH₃ | Cl | H | i-C₃H₇ | CH₃ | 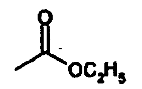 |
| Cl | OCH₃ | H | 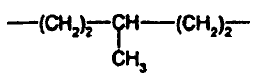 | | 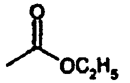 |
| Cl | OCH₃ | H | 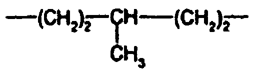 | | 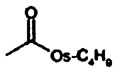 |
| Cl | OCH₃ | H | 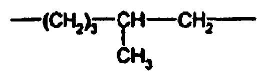 | | 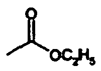 |
| Cl | OCH₃ | H | 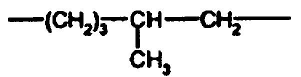 | | 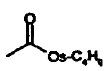 |
| OCH₃ | Cl | H | 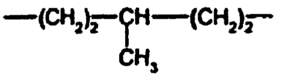 | | 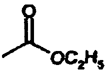 |
| Cl | H | 6-OCH₃ | 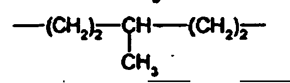 | | 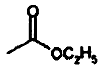 |
| Cl | H | 6-OCH₃ | i-C₃H₇ | CH₃ | 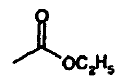 |
